# EUROPEAN PATENT APPLICATION

(11) **EP 3 572 092 A1**
(43) Date of publication of application: **27.11.2019**
(21) Application number: 19167408.4
(22) Date of filing: 29.11.2012
(51) Int. Cl.: A61K 39/12

(54) **RECOMBINANT GALLID HERPESVIRUS 3 (MDV SEROTYPE 2) VECTORS EXPRESSING ANTIGENS OF AVIAN PATHOGENS AND USES THEREOF**

(30) Priority: 30.11.2011 US 201161564877 P; 30.08.2012 US 201261694957 P
(62) Divisional of application: 12801680.5
(71) Applicant: Boehringer Ingelheim Animal Health USA Inc., Duluth, GA 30096 (US)
(72) Inventor: BUBLOT, Michel, 69630 Chaponost (FR); MEBATSION, Teshome, Watkinsville, GA 30677 (US); PRITCHARD, Joyce, Gainesville, GA 30504 (US); LINZ, Perry, Jefferson, GA 30549 (US)
(74) Representative: D Young & Co LLP

(57) **Abstract**

The present invention provides recombinant herpesvirus of turkeys (HVT) vectors that contain and express antigens of avian pathogens, compositions comprising the recombinant HVT vectors, polyvalent vaccines comprising the recombinant HVT vectors and one or more wild type viruses or recombinant vectors. The present invention further provides methods of vaccination against a variety of avian pathogens and method of producing the recombinant HVT vectors.

SEQUENCE LISTING

<110>  Boehringer Ingelheim Animal Health USA Inc.

<120>  Recombinant HVT Vectors Expressing Antigens of Avian Pathogens
       and Uses Thereof

<130>  P105111EPD

<150>  US 61/564,877
<151>  2011-11-30

<150>  US 61/694,957
<151>  2012-08-30

<160>  47

<170>  PatentIn version 3.5

<210>  1
<211>  1665
<212>  DNA
<213>  artificial sequence

<220>
<223>  NDV-F VIId codon-optimized DNA sequence

<400>  1
atgggcagca agcccagcac aagaatccca gcccccctga tgctgatcac ccgcatcatg      60

ctgatcctgg gctgcatcag acccacaagc tccctggatg gacgccccct ggccgctgcc     120

ggcatcgtgg tgaccggcga caaggccgtg aacgtgtaca ccagcagcca gaccggcagc     180

atcatcgtga agctgctgcc caacatgccc agagacaaag aggcctgcgc caaggccccc     240

ctggaagcct acaacagaac cctgaccacc ctgctgaccc ccctgggcga cagcatcaga     300

aagatccagg gctccgtgag cacaagcggc ggaggaaagc agggcagact gatcggcgcc     360

gtgatcggca gcgtggccct gggagtggct acagctgccc agattaccgc tgcagccgcc     420

ctgatccagg ccaaccagaa cgccgccaac atcctgagac tgaaagagag cattgccgcc     480

accaacgagg ccgtgcacga agtgaccgac ggcctgagcc agctgtccgt ggccgtgggc     540

aagatgcagc agttcgtgaa cgaccagttc aacaacaccg ccagagagct ggactgcatc     600

aagatcaccc agcaggtggg cgtggagctg aacctgtacc tgaccgagct gaccacagtg     660

ttcggccccc agatcacaag cccagccctg acacagctga ccatccaggc cctgtacaac     720

ctggctggcg gcaacatgga ctatctgctg acaaagctgg gaatcggcaa caaccagctg     780

tccagcctga tcggaagcgg cctgatcacc ggctacccca tcctgtacga cagccagaca     840

cagctgctgg gcatccaggt gaacctgccc agcgtgggca acctgaacaa catgcgcgcc     900

acctacctgg aaaccctgag cgtgtccacc accaagggct acgccagcgc cctggtgccc     960

aaggtggtga cacaggtggg cagcgtgatc gaggaactgg acaccagcta ctgcatcgag    1020

agcgacctgg acctgtactg caccagaatc gtgaccttcc caatgagccc cggcatctac    1080

agctgcctga gcggcaacac cagcgcctgc atgtacagca agaccgaagg cgcactgaca 1140

acaccctaca tggccctgaa gggaagcgtg atcgccaact gcaagatcac cacctgcaga 1200

tgcaccgacc ccccaggcat catcagccag aactacggcg aggccgtgag cctgatcgat 1260

cgccattcct gtaacgtgct gtccctggac ggcatcacac tgagactgag cggcgagttc 1320

gatgccacct accagaagaa catcagcatc ctggacagcc aggtgatcgt gaccggcaac 1380

ctggacatca gcaccgagct gggcaacgtg aataacagca tcagcaacgc cctggacaga 1440

ctggccgaga gcaacagcaa gctggaaaaa gtgaacgtgc gcctgacatc cacttccgct 1500

ctgatcacct acatcgtgct gaccgtgatc agcctggtgt tcggcgccct gagcctggtg 1560

ctggcctgct acctgatgta caagcagaag gcccagcaga aaaccctgct gtggctgggc 1620

aacaacaccc tggaccagat gagagccacc accagagcct gatga 1665

<210> 2
<211> 553
<212> PRT
<213> artificial sequence

<220>
<223> NDV-F protein sequence from codon-optimized VIId gene

<400> 2
   Met Gly Ser Lys Pro Ser Thr Arg Ile Pro Ala Pro Leu Met Leu Ile
   1 5 10 15
   Thr Arg Ile Met Leu Ile Leu Gly Cys Ile Arg Pro Thr Ser Ser Leu
   20 25 30
   Asp Gly Arg Pro Leu Ala Ala Ala Gly Ile Val Val Thr Gly Asp Lys
   35 40 45
   Ala Val Asn Val Tyr Thr Ser Ser Gln Thr Gly Ser Ile Ile Val Lys
   50 55 60
   Leu Leu Pro Asn Met Pro Arg Asp Lys Glu Ala Cys Ala Lys Ala Pro
   65 70 75 80
   Leu Glu Ala Tyr Asn Arg Thr Leu Thr Thr Leu Leu Thr Pro Leu Gly
   85 90 95
   Asp Ser Ile Arg Lys Ile Gln Gly Ser Val Ser Thr Ser Gly Gly Gly
   100 105 110
   Lys Gln Gly Arg Leu Ile Gly Ala Val Ile Gly Ser Val Ala Leu Gly
   115 120 125
   Val Ala Thr Ala Ala Gln Ile Thr Ala Ala Ala Ala Leu Ile Gln Ala
       130                 135                 140
   Asn Gln Asn Ala Ala Asn Ile Leu Arg Leu Lys Glu Ser Ile Ala Ala
   145                 150                 155                 160
   Thr Asn Glu Ala Val His Glu Val Thr Asp Gly Leu Ser Gln Leu Ser
                   165                 170                 175
   Val Ala Val Gly Lys Met Gln Gln Phe Val Asn Asp Gln Phe Asn Asn
               180                 185                 190
   Thr Ala Arg Glu Leu Asp Cys Ile Lys Ile Thr Gln Gln Val Gly Val
           195                 200                 205
   Glu Leu Asn Leu Tyr Leu Thr Glu Leu Thr Thr Val Phe Gly Pro Gln
       210                 215                 220
   Ile Thr Ser Pro Ala Leu Thr Gln Leu Thr Ile Gln Ala Leu Tyr Asn
   225                 230                 235                 240
   Leu Ala Gly Gly Asn Met Asp Tyr Leu Leu Thr Lys Leu Gly Ile Gly
                   245                 250                 255
   Asn Asn Gln Leu Ser Ser Leu Ile Gly Ser Gly Leu Ile Thr Gly Tyr
               260                 265                 270
   Pro Ile Leu Tyr Asp Ser Gln Thr Gln Leu Leu Gly Ile Gln Val Asn
           275                 280                 285
   Leu Pro Ser Val Gly Asn Leu Asn Asn Met Arg Ala Thr Tyr Leu Glu
       290                 295                 300
   Thr Leu Ser Val Ser Thr Thr Lys Gly Tyr Ala Ser Ala Leu Val Pro
   305                 310                 315                 320
   Lys Val Val Thr Gln Val Gly Ser Val Ile Glu Glu Leu Asp Thr Ser
                   325                 330                 335
   Tyr Cys Ile Glu Ser Asp Leu Asp Leu Tyr Cys Thr Arg Ile Val Thr
               340                 345                 350
   Phe Pro Met Ser Pro Gly Ile Tyr Ser Cys Leu Ser Gly Asn Thr Ser
           355                 360                 365
   Ala Cys Met Tyr Ser Lys Thr Glu Gly Ala Leu Thr Thr Pro Tyr Met
       370                 375                 380
   Ala Leu Lys Gly Ser Val Ile Ala Asn Cys Lys Ile Thr Thr Cys Arg
   385                 390                 395                 400
   Cys Thr Asp Pro Pro Gly Ile Ile Ser Gln Asn Tyr Gly Glu Ala Val
                   405                 410                 415
   Ser Leu Ile Asp Arg His Ser Cys Asn Val Leu Ser Leu Asp Gly Ile
               420                 425                 430
   Thr Leu Arg Leu Ser Gly Glu Phe Asp Ala Thr Tyr Gln Lys Asn Ile
           435                 440                 445
   Ser Ile Leu Asp Ser Gln Val Ile Val Thr Gly Asn Leu Asp Ile Ser
       450                 455                 460
   Thr Glu Leu Gly Asn Val Asn Asn Ser Ile Ser Asn Ala Leu Asp Arg
   465                 470                 475                 480
   Leu Ala Glu Ser Asn Ser Lys Leu Glu Lys Val Asn Val Arg Leu Thr
                   485                 490                 495
   Ser Thr Ser Ala Leu Ile Thr Tyr Ile Val Leu Thr Val Ile Ser Leu
               500                 505                 510
   Val Phe Gly Ala Leu Ser Leu Val Leu Ala Cys Tyr Leu Met Tyr Lys
           515                 520                 525
   Gln Lys Ala Gln Gln Lys Thr Leu Leu Trp Leu Gly Asn Asn Thr Leu
       530                 535                 540
   Asp Gln Met Arg Ala Thr Thr Arg Ala
   545                 550

<210>  3
<211>  1662
<212>  DNA
<213>  artificial sequence

<220>
<223>  NDV-F VIId wildtype DNA sequence

<400>  3
atgggctcca aaccttctac caggatccca gcacctctga tgctgatcac ccggattatg      60

ctgatattgg gctgtatccg tccgacaagc tctcttgacg gcaggcctct tgcagctgca     120

ggaattgtag taacaggaga taaggcagtc aatgtataca cttcgtctca gacagggtca     180

atcatagtca agttgctccc gaatatgccc agggataagg aggcgtgtgc aaaagcccca     240

ttagaggcat ataacagaac actgactact ttgctcactc ctcttggcga ctccatccgc 300

aagatccaag ggtctgtgtc cacatctgga ggaggcaagc aaggccgcct gataggtgct 360

gttattggca gtgtagctct tggggttgca acagcggcac agataacagc agctgcggcc 420

ctaatacaag ccaaccagaa tgccgccaac atcctccggc ttaaggagag cattgctgca 480

accaatgaag ctgtgcatga agtcaccgac ggattatcac aactatcagt ggcagttggg 540

aagatgcagc agtttgtcaa tgaccagttt aataatacgg cgcgagaatt ggactgtata 600

aaaatcacac aacaggttgg tgtagaactc aacctatacc taactgaatt gactacagta 660

ttcgggccac agatcacctc ccctgcatta actcagctga ccatccaggc actttataat 720

ttagctggtg gcaatatgga ttacttatta actaagttag gtatagggaa caatcaactc 780

agctcgttaa ttggtagcgg cctgatcact ggttacccta tactgtatga ctcacagact 840

caactcttgg gcatacaagt gaatttaccc tcagtcggga acttaaataa tatgcgtgcc 900

acctatttgg agaccttatc tgtaagtaca accaaaggat atgcctcagc acttgtcccg 960

aaagtagtga cacaagtcgg ttccgtgata gaagagcttg acacctcata ctgtatagag 1020

tccgatctgg atttatattg tactagaata gtgacattcc ccatgtcccc aggtatttat 1080

tcctgtttga gcggcaacac atcagcttgc atgtattcaa agactgaagg cgcactcact 1140

acgccgtata tggcccttaa aggctcagtt attgccaatt gtaaaataac aacatgtaga 1200

tgtacagacc ctcctggtat catatcgcaa aattatggag aagctgtatc cctgatagat 1260

agacattcgt gcaatgtctt atcattagac gggataactc taaggctcag tggggaattt 1320

gatgcaactt atcaaaagaa catctcaata ctagattctc aagtcatcgt gacaggcaat 1380

cttgatatat caactgaact tggaaacgtc aacaattcaa tcagcaatgc cttggatagg 1440

ttggcagaaa gcaacagcaa gctagaaaaa gtcaatgtca gactaaccag cacatctgct 1500

ctcattacct atattgttct aactgtcatt tctctagttt tcggtgcact tagtctggtg 1560

ttagcgtgtt acctgatgta caaacagaag gcacaacaaa agaccttgct atggcttggg 1620

aataataccc tcgatcagat gagagccact acaagagcat ga 1662

<210> 4
<211> 553
<212> PRT
<213> artificial sequence

<220>
<223> NDV-F protein from wildtype VIId DNA sequence

<400> 4

Met Gly Ser Lys Pro Ser Thr Arg Ile Pro Ala Pro Leu Met Leu Ile
1               5                   10                  15

Thr Arg Ile Met Leu Ile Leu Gly Cys Ile Arg Pro Thr Ser Ser Leu
                20                  25                  30

Asp Gly Arg Pro Leu Ala Ala Ala Gly Ile Val Val Thr Gly Asp Lys
            35                  40                  45

Ala Val Asn Val Tyr Thr Ser Ser Gln Thr Gly Ser Ile Ile Val Lys
    50                  55                  60

Leu Leu Pro Asn Met Pro Arg Asp Lys Glu Ala Cys Ala Lys Ala Pro
65                  70                  75                  80

Leu Glu Ala Tyr Asn Arg Thr Leu Thr Thr Leu Leu Thr Pro Leu Gly
                85                  90                  95

Asp Ser Ile Arg Lys Ile Gln Gly Ser Val Ser Thr Ser Gly Gly Gly
            100                 105                 110

Lys Gln Gly Arg Leu Ile Gly Ala Val Ile Gly Ser Val Ala Leu Gly
        115                 120                 125

Val Ala Thr Ala Ala Gln Ile Thr Ala Ala Ala Ala Leu Ile Gln Ala
    130                 135                 140

Asn Gln Asn Ala Ala Asn Ile Leu Arg Leu Lys Glu Ser Ile Ala Ala
145                 150                 155                 160

Thr Asn Glu Ala Val His Glu Val Thr Asp Gly Leu Ser Gln Leu Ser
                165                 170                 175

Val Ala Val Gly Lys Met Gln Gln Phe Val Asn Asp Gln Phe Asn Asn
            180                 185                 190

Thr Ala Arg Glu Leu Asp Cys Ile Lys Ile Thr Gln Gln Val Gly Val
        195                 200                 205

Glu Leu Asn Leu Tyr Leu Thr Glu Leu Thr Thr Val Phe Gly Pro Gln
    210                 215                 220

Ile Thr Ser Pro Ala Leu Thr Gln Leu Thr Ile Gln Ala Leu Tyr Asn
225                 230                 235                 240

Leu Ala Gly Gly Asn Met Asp Tyr Leu Leu Thr Lys Leu Gly Ile Gly
                245                 250                 255

Asn Asn Gln Leu Ser Ser Leu Ile Gly Ser Gly Leu Ile Thr Gly Tyr
            260                 265                 270

Pro Ile Leu Tyr Asp Ser Gln Thr Gln Leu Leu Gly Ile Gln Val Asn
        275                 280                 285

Leu Pro Ser Val Gly Asn Leu Asn Asn Met Arg Ala Thr Tyr Leu Glu
    290                 295                 300

Thr Leu Ser Val Ser Thr Thr Lys Gly Tyr Ala Ser Ala Leu Val Pro
305                 310                 315                 320

Lys Val Val Thr Gln Val Gly Ser Val Ile Glu Glu Leu Asp Thr Ser
                325                 330                 335

Tyr Cys Ile Glu Ser Asp Leu Asp Leu Tyr Cys Thr Arg Ile Val Thr
            340                 345                 350

Phe Pro Met Ser Pro Gly Ile Tyr Ser Cys Leu Ser Gly Asn Thr Ser
        355                 360                 365

Ala Cys Met Tyr Ser Lys Thr Glu Gly Ala Leu Thr Thr Pro Tyr Met
    370                 375                 380

Ala Leu Lys Gly Ser Val Ile Ala Asn Cys Lys Ile Thr Thr Cys Arg
385                 390                 395                 400

Cys Thr Asp Pro Pro Gly Ile Ile Ser Gln Asn Tyr Gly Glu Ala Val
                405                 410                 415

Ser Leu Ile Asp Arg His Ser Cys Asn Val Leu Ser Leu Asp Gly Ile
            420                 425                 430

Thr Leu Arg Leu Ser Gly Glu Phe Asp Ala Thr Tyr Gln Lys Asn Ile
        435                 440                 445

Ser Ile Leu Asp Ser Gln Val Ile Val Thr Gly Asn Leu Asp Ile Ser
    450                 455                 460

Thr Glu Leu Gly Asn Val Asn Asn Ser Ile Ser Asn Ala Leu Asp Arg
465                 470                 475                 480

Leu Ala Glu Ser Asn Ser Lys Leu Glu Lys Val Asn Val Arg Leu Thr
                485                 490                 495

Ser Thr Ser Ala Leu Ile Thr Tyr Ile Val Leu Thr Val Ile Ser Leu
            500                 505                 510

Val Phe Gly Ala Leu Ser Leu Val Leu Ala Cys Tyr Leu Met Tyr Lys
        515                 520                 525

Gln Lys Ala Gln Gln Lys Thr Leu Leu Trp Leu Gly Asn Asn Thr Leu
    530                 535                 540

Asp Gln Met Arg Ala Thr Thr Arg Ala
545                 550

<210>  5
<211>  1665
<212>  DNA
<213>  artificial sequence

<220>
<223>  NDV-F Ca02 codon-optimized DNA sequence

<400>  5
atgggcagca agcccagcac ctggatcagc gtgaccctga tgctgatcac cagaaccatg      60

ctgatcctga gctgcatctg ccccacaagc agcctggacg gcagacccct ggccgctgcc     120

ggcatcgtgg tgaccggcga caaggccgtg aacatctaca ccagcagcca gaccggcagc     180

atcatcatca agctgctgcc caacatgccc aaggacaaag aggcctgcgc caaggccccc     240

ctggaagcct acaacagaac cctgaccacc ctgctgaccc ccctgggcga cagcatcaga     300

agaatccagg gcagcgccac cacaagcggc ggaggaaagc agggcagact ggtgggcgct     360

atcatcggga gcgtggccct gggcgtggcc acagctgccc agattaccgc tgcagccgcc     420

ctgattcagg ccaatcagaa cgccgccaac atcctgagac tgaaagagag cattgccgcc     480

accaacgacg ccgtgcacga agtgacaaac ggactgtccc agctggctgt cgctgtcggc     540

aagatgcagc agttcgtgaa caaccagttc aacaacaccg ccagagagct ggactgcatc     600

aagatcgccc agcaggtggg cgtggagctg aacctgtacc tgaccgagct gaccacagtg     660

ttcggccccc agatcacaag ccccgctctg acccagctga caatccaggc cctgtacaac     720

ctggctggcg gcaacatgga ctatctgctg actaagctgg gagtgggcaa caaccagctg     780

tccagcctga tcgggtccgg gctgatcaca ggcaacccca tcctgtacga cagccagaca     840

cagctgctgg gcatccagat caacctgcca tccgtgggaa gcctgaacaa catgagagcc     900

acctacctgg aaaccctgag cgtgtccacc accaagggct tcgccagcgc cctggtgccc     960

aaggtggtga cacaggtggg cagcgtgatc gaggaactgg acaccagcta ctgcatcgag    1020

agcgacatcg acctgtactg caccagagtg gtgaccttcc caatgagccc cggcatctac    1080

agctgcctga gcggcaacac cagcgcctgc atgtacagca agaccgaagg agcactgaca    1140

acaccctaca tggccctgaa gggaagcgtg atcgccaact gcaagatgac cacctgcaga    1200

tgcgccgacc ccccaggcat catcagccag aactacggcg aggccgtgag cctgatcgac    1260

aaacattcct gtagcgtgct gtccctggat ggcatcacac tgagactgag cggcgagttc    1320

gacgccacct accagaagaa catcagcatc ctggacagcc aggtgatcgt gaccggcaac     1380

ctggacatca gcaccgagct gggcaacgtg aacaacagca tcagcagcac cctggacaag     1440

ctggccgagt ccaacaacaa gctgaacaaa gtgaacgtga acctgaccag cacaagcgcc     1500

ctgatcacct acatcgtgct ggccatcgtg tccctggcct tcggcgtgat cagcctggtg     1560

ctggcctgct acctgatgta caagcagaga gcccagcaga aaaccctgct gtggctgggc     1620

aataacaccc tggaccagat gagggccacc accagaacct gatga                     1665

<210>  6
<211>  553
<212>  PRT
<213>  artificial sequence

<220>
<223>  NDV-F protein sequence from codon-optimized CA02 gene

<400>  6

Met Gly Ser Lys Pro Ser Thr Trp Ile Ser Val Thr Leu Met Leu Ile
1               5                   10                  15

Thr Arg Thr Met Leu Ile Leu Ser Cys Ile Cys Pro Thr Ser Ser Leu
            20                  25                  30

Asp Gly Arg Pro Leu Ala Ala Ala Gly Ile Val Val Thr Gly Asp Lys
        35                  40                  45

Ala Val Asn Ile Tyr Thr Ser Ser Gln Thr Gly Ser Ile Ile Ile Lys
    50                  55                  60

Leu Leu Pro Asn Met Pro Lys Asp Lys Glu Ala Cys Ala Lys Ala Pro
65                  70                  75                  80

Leu Glu Ala Tyr Asn Arg Thr Leu Thr Thr Leu Leu Thr Pro Leu Gly
                85                  90                  95

Asp Ser Ile Arg Arg Ile Gln Gly Ser Ala Thr Thr Ser Gly Gly Gly
            100                 105                 110

Lys Gln Gly Arg Leu Val Gly Ala Ile Ile Gly Ser Val Ala Leu Gly
        115                 120                 125

Val Ala Thr Ala Ala Gln Ile Thr Ala Ala Ala Ala Leu Ile Gln Ala
    130                 135                 140

Asn Gln Asn Ala Ala Asn Ile Leu Arg Leu Lys Glu Ser Ile Ala Ala
145                 150                 155                 160

Thr Asn Asp Ala Val His Glu Val Thr Asn Gly Leu Ser Gln Leu Ala
                165                 170                 175

Val Ala Val Gly Lys Met Gln Gln Phe Val Asn Asn Gln Phe Asn Asn
            180                 185                 190

Thr Ala Arg Glu Leu Asp Cys Ile Lys Ile Ala Gln Gln Val Gly Val
        195                 200                 205

Glu Leu Asn Leu Tyr Leu Thr Glu Leu Thr Thr Val Phe Gly Pro Gln
    210                 215                 220

Ile Thr Ser Pro Ala Leu Thr Gln Leu Thr Ile Gln Ala Leu Tyr Asn
225                 230                 235                 240

Leu Ala Gly Gly Asn Met Asp Tyr Leu Leu Thr Lys Leu Gly Val Gly
                245                 250                 255

Asn Asn Gln Leu Ser Ser Leu Ile Gly Ser Gly Leu Ile Thr Gly Asn
            260                 265                 270

Pro Ile Leu Tyr Asp Ser Gln Thr Gln Leu Leu Gly Ile Gln Ile Asn
        275                 280                 285

Leu Pro Ser Val Gly Ser Leu Asn Asn Met Arg Ala Thr Tyr Leu Glu
    290                 295                 300

Thr Leu Ser Val Ser Thr Thr Lys Gly Phe Ala Ser Ala Leu Val Pro
305                 310                 315                 320

Lys Val Val Thr Gln Val Gly Ser Val Ile Glu Glu Leu Asp Thr Ser
                325                 330                 335

Tyr Cys Ile Glu Ser Asp Ile Asp Leu Tyr Cys Thr Arg Val Val Thr
            340                 345                 350

Phe Pro Met Ser Pro Gly Ile Tyr Ser Cys Leu Ser Gly Asn Thr Ser
        355                 360                 365

Ala Cys Met Tyr Ser Lys Thr Glu Gly Ala Leu Thr Thr Pro Tyr Met
    370                 375                 380

Ala Leu Lys Gly Ser Val Ile Ala Asn Cys Lys Met Thr Thr Cys Arg
385                 390                 395                 400

Cys Ala Asp Pro Pro Gly Ile Ile Ser Gln Asn Tyr Gly Glu Ala Val
                405                 410                 415

Ser Leu Ile Asp Lys His Ser Cys Ser Val Leu Ser Leu Asp Gly Ile
            420                 425                 430

Thr Leu Arg Leu Ser Gly Glu Phe Asp Ala Thr Tyr Gln Lys Asn Ile
        435                 440                 445

Ser Ile Leu Asp Ser Gln Val Ile Val Thr Gly Asn Leu Asp Ile Ser
    450                 455                 460

Thr Glu Leu Gly Asn Val Asn Asn Ser Ile Ser Ser Thr Leu Asp Lys
465                 470                 475                 480

Leu Ala Glu Ser Asn Asn Lys Leu Asn Lys Val Asn Val Asn Leu Thr
                485                 490                 495

Ser Thr Ser Ala Leu Ile Thr Tyr Ile Val Leu Ala Ile Val Ser Leu
            500                 505                 510

Ala Phe Gly Val Ile Ser Leu Val Leu Ala Cys Tyr Leu Met Tyr Lys
        515                 520                 525

Gln Arg Ala Gln Gln Lys Thr Leu Leu Trp Leu Gly Asn Asn Thr Leu
    530                 535                 540

Asp Gln Met Arg Ala Thr Thr Arg Thr
545                 550

<210>  7
<211>  1362
<212>  DNA
<213>  artificial sequence

<220>
<223>  IBDV DNA sequence encoding VP2

<400>  7
atgacaaacc tgcaagatca aacccaacag attgttccgt tcatacggag ccttctgatg      60

ccaacaaccg gaccggcgtc cattccggac gacaccctgg agaagcacac tctcaggtca     120

gagacctcga cctacaattt gactgtgggg gacacagggt cagggctaat tgtctttttc     180

cctggattcc ctggctcaat tgtgggtgct cactacacac tgcagagcaa tgggaactac     240

aagttcgatc agatgctcct gactgcccag aacctaccgg ccagctacaa ctactgcaga     300

ctagtgagtc ggagtctcac agtgaggtca agcacactcc ctggtggcgt ttatgcacta     360

aacggcacca taaacgccgt gaccttccaa ggaagcctga gtgaactgac agatgttagc     420

tacaatgggt tgatgtctgc aacagccaac atcaacgaca aaattgggaa tgtcctggta     480

ggggaagggg tcactgtcct cagcctaccc acatcatatg atcttgggta tgtgaggctt     540

ggtgacccca ttcccgctat agggcttgac ccaaaaatgg tagctacatg cgacagcagt 600

gacaggccca gagtctacac cataactgca gccgatgatt accaattctc atcacagtac 660

caaccaggtg gggtaacaat cacactgttc tcagccaaca ttgatgctat cacaagcctc 720

agcattgggg gagagctcgt gtttcaaaca agcgtccaag gccttgtact gggcgccacc 780

atctacctta taggctttga tgggactgcg gtaatcacca gagctgtagc cgcagataat 840

gggctgacgg ccggcaccga caatcttatg ccattcaatc ttgtcattcc aaccaatgag 900

ataacccagc caatcacatc catcaaactg gagatagtga cctccaaaag tggtggtcag 960

gcaggggatc agatgtcatg gtcggcaagt gggagcctag cagtgacgat ccatggtggc 1020

aactatccag gggccctccg tcccgtcaca ctagtagcct acgaaagagt ggcaacagga 1080

tccgtcgtta cggtcgctgg ggtgagtaac ttcgagctga ttccaaatcc tgaactagca 1140

aagaacctgg ttacagaata cggccgattt gacccaggag ccatgaacta cacaaaattg 1200

atactgagtg agagggaccg tcttggcatc aagaccgtct ggccaacaag ggagtacact 1260

gattttcgtg agtacttcat ggaggtggcc gacctcaact ctcccctgaa gattgcagga 1320

gcatttggct tcaaagacat aatccgggct ataaggaggt aa 1362

<210> 8
<211> 453
<212> PRT
<213> artificial sequence

<220>
<223> IBDV VP2 protein

<400> 8

Met Thr Asn Leu Gln Asp Gln Thr Gln Gln Ile Val Pro Phe Ile Arg
1               5                   10                  15

Ser Leu Leu Met Pro Thr Thr Gly Pro Ala Ser Ile Pro Asp Asp Thr
            20                  25                  30

Leu Glu Lys His Thr Leu Arg Ser Glu Thr Ser Thr Tyr Asn Leu Thr
        35                  40                  45

Val Gly Asp Thr Gly Ser Gly Leu Ile Val Phe Phe Pro Gly Phe Pro
    50                  55                  60

Gly Ser Ile Val Gly Ala His Tyr Thr Leu Gln Ser Asn Gly Asn Tyr
65                  70                  75                  80

Lys Phe Asp Gln Met Leu Leu Thr Ala Gln Asn Leu Pro Ala Ser Tyr
                85                  90                  95

Asn Tyr Cys Arg Leu Val Ser Arg Ser Leu Thr Val Arg Ser Ser Thr
                100                 105                 110

Leu Pro Gly Gly Val Tyr Ala Leu Asn Gly Thr Ile Asn Ala Val Thr
        115                 120                 125

Phe Gln Gly Ser Leu Ser Glu Leu Thr Asp Val Ser Tyr Asn Gly Leu
    130                 135                 140

Met Ser Ala Thr Ala Asn Ile Asn Asp Lys Ile Gly Asn Val Leu Val
145                 150                 155                 160

Gly Glu Gly Val Thr Val Leu Ser Leu Pro Thr Ser Tyr Asp Leu Gly
                165                 170                 175

Tyr Val Arg Leu Gly Asp Pro Ile Pro Ala Ile Gly Leu Asp Pro Lys
            180                 185                 190

Met Val Ala Thr Cys Asp Ser Ser Asp Arg Pro Arg Val Tyr Thr Ile
        195                 200                 205

Thr Ala Ala Asp Asp Tyr Gln Phe Ser Ser Gln Tyr Gln Pro Gly Gly
    210                 215                 220

Val Thr Ile Thr Leu Phe Ser Ala Asn Ile Asp Ala Ile Thr Ser Leu
225                 230                 235                 240

Ser Ile Gly Gly Glu Leu Val Phe Gln Thr Ser Val Gln Gly Leu Val
                245                 250                 255

Leu Gly Ala Thr Ile Tyr Leu Ile Gly Phe Asp Gly Thr Ala Val Ile
            260                 265                 270

Thr Arg Ala Val Ala Ala Asp Asn Gly Leu Thr Ala Gly Thr Asp Asn
        275                 280                 285

Leu Met Pro Phe Asn Leu Val Ile Pro Thr Asn Glu Ile Thr Gln Pro
    290                 295                 300

Ile Thr Ser Ile Lys Leu Glu Ile Val Thr Ser Lys Ser Gly Gly Gln
305                 310                 315                 320

Ala Gly Asp Gln Met Ser Trp Ser Ala Ser Gly Ser Leu Ala Val Thr
                325                 330                 335

Ile His Gly Gly Asn Tyr Pro Gly Ala Leu Arg Pro Val Thr Leu Val
            340                 345                 350

Ala Tyr Glu Arg Val Ala Thr Gly Ser Val Val Thr Val Ala Gly Val
        355                 360                 365

Ser Asn Phe Glu Leu Ile Pro Asn Pro Glu Leu Ala Lys Asn Leu Val
    370                 375                 380

Thr Glu Tyr Gly Arg Phe Asp Pro Gly Ala Met Asn Tyr Thr Lys Leu
385                 390                 395                 400

Ile Leu Ser Glu Arg Asp Arg Leu Gly Ile Lys Thr Val Trp Pro Thr
                405                 410                 415

Arg Glu Tyr Thr Asp Phe Arg Glu Tyr Phe Met Glu Val Ala Asp Leu
            420                 425                 430

Asn Ser Pro Leu Lys Ile Ala Gly Ala Phe Gly Phe Lys Asp Ile Ile
        435                 440                 445

Arg Ala Ile Arg Arg
    450

<210>  9
<211>  368
<212>  DNA
<213>  artificial sequence

<220>
<223>  SV40 promoter

<400>  9
gaattcgagc tcggtacagc ttggctgtgg aatgtgtgtc agttagggtg tggaaagtcc      60

ccaggctccc cagcaggcag aagtatgcaa agcatgcatc tcaattagtc agcaaccagg     120

tgtggaaagt ccccaggctc cccagcaggc agaagtatgc aaagcatgca tctcaattag     180

tcagcaacca tagtcccgcc cctaactccg cccatcccgc ccctaactcc gcccagttcc     240

gcccattctc cgccccatgg ctgactaatt ttttttattt atgcagaggc cgaggccgcc     300

tcggcctctg agctattcca gaagtagtga ggaggctttt ttggaggcct aggcttttgc     360

aaaaagct                                                              368

<210>  10
<211>  1391
<212>  DNA
<213>  artificial sequence

<220>
<223>  CMV-IE promoter

<400>  10

aactccgccc gttttatgac tagaaccaat agtttttaat gccaaatgca ctgaaatccc 60

ctaatttgca aagccaaacg ccccctatgt gagtaatacg gggacttttt acccaatttc 120

ccaagcggaa agccccctaa tacactcata tggcatatga atcagcacgg tcatgcactc 180

taatggcggc ccatagggac tttccacata gggggcgttc accatttccc agcatagggg 240

tggtgactca atggccttta cccaagtaca ttgggtcaat gggaggtaag ccaatgggtt 300

tttcccatta ctggcaagca cactgagtca aatgggactt tccactgggt tttgcccaag 360

tacattgggt caatgggagg tgagccaatg ggaaaaaccc attgctgcca agtacactga 420

ctcaataggg actttccaat gggtttttcc attgttggca agcatataag gtcaatgtgg 480

gtgagtcaat agggactttc cattgtattc tgcccagtac ataaggtcaa tagggggtga 540

atcaacagga aagtcccatt ggagccaagt acactgcgtc aatagggact ttccattggg 600

ttttgcccag tacataaggt caatagggga tgagtcaatg ggaaaaaccc attggagcca 660

agtacactga ctcaataggg actttccatt gggttttgcc cagtacataa ggtcaatagg 720

gggtgagtca acaggaaagt cccattggag ccaagtacat tgagtcaata gggactttcc 780

aatgggtttt gcccagtaca taaggtcaat gggaggtaag ccaatgggtt tttcccatta 840

ctggcacgta tactgagtca ttagggactt tccaatgggt tttgcccagt acataaggtc 900

aataggggtg aatcaacagg aaagtcccat tggagccaag tacactgagt caatagggac 960

tttccattgg gttttgccca gtacaaaagg tcaatagggg gtgagtcaat gggtttttcc 1020

cattattggc acgtacataa ggtcaatagg ggtgagtcat tgggtttttc cagccaattt 1080

aattaaaacg ccatgtactt tcccaccatt gacgtcaatg ggctattgaa actaatgcaa 1140

cgtgaccttt aaacggtact ttcccatagc tgattaatgg gaaagtaccg ttctcgagcc 1200

aatacacgtc aatgggaagt gaaagggcag ccaaaacgta acaccgcccc ggttttcccc 1260

tggaaattcc atattggcac gcattctatt ggctgagctg cgttctacgt gggtataaga 1320

ggcgcgacca gcgtcggtac cgtcgcagtc ttcggtctga ccaccgtaga acgcagagct 1380

cctcgctgca g 1391

<210> 11
<211> 218
<212> DNA
<213> artificial sequence

<220>
<223> SV40 polyA signal

<400> 11
ggggatccag acatgataag atacattgat gagtttggac aaaccacaac tagaatgcag 60

tgaaaaaaat gctttatttg tgaaatttgt gatgctattg ctttatttgt aaccattata 120

agctgcaata aacaagttaa caacaacaat tgcattgatt ttatgtttca ggttcagggg 180

gaggtgtggg aggttttttc ggatcctcta gagtcgac 218

<210> 12
<211> 155
<212> DNA
<213> artificial sequence

<220>
<223> Synthetic polyA signal

<400> 12
aataaaatat ctttattttc attacatctg tgtgttggtt ttttgtgtga atcgatagta 60

ctaacatacg ctctccatca aaacaaaacg aaacaaaaca aactagcaaa ataggctgtc 120

cccagtgcaa gtgcaggtgc cagaacattt ctctt 155

<210> 13
<211> 22
<212> DNA
<213> artificial sequence

<220>
<223> primer

<400> 13
cgaacaaact tcatcgctat gc 22

<210> 14
<211> 22
<212> DNA
<213> artificial sequence

<220>
<223> primer

<400> 14
taactcaaat gcgaagcgtt gc 22

<210> 15
<211> 21
<212> DNA
<213> artificial sequence

<220>
<223> primer

<400> 15
actgacaaca ccctacatgg c 21

<210> 16
<211> 19
<212> DNA
<213> artificial sequence

<220>
<223> VIioptF RP primer

<400>  16
gccagcacca ggctcaggg                                                    19


<210>  17
<211>  18
<212>  DNA
<213>  artificial sequence

<220>
<223>  primer

<400>  17
agcttggctg tggaatgt                                                     18


<210>  18
<211>  4344
<212>  DNA
<213>  artificial sequence

<220>
<223>  Partial plasmid pHM103+Fopt DNA sequence

<400>  18
gagctcaggg tatgatactc agctgttatt gtggccgacc aggaggactc caatgcttag      60

cattcataag aacgctagag atgctattta acgatgtgct gtcgtctaaa gaatttgtgc     120

atttagcctt taaatgtaaa accaatgacg cattcactac gctcgtgcgt gcaatttctg     180

ggccagggta tgcatattcc ataacagaaa tcgacacttg agaagaggat ctgactgttt     240

gggataaagg tcgtttgggt ctgtcctagc gatataattt atatgacgat atacattaaa     300

catctgtgtg cagtacttag gtatttaatc atgtcgatga aatgttatgt gtaaatatcg     360

gacaatatag ataacgggca cgctgctatt gtaacgtgcg cccgcgcgct agtgctgact     420

aatagtgtgg atgatgtata cagtatatta caaacggaaa tgatacgtaa taaattatgt     480

actcttattg atttataaaa acatacatgc agtgttgcta tgtcacataa ttagcctcgc     540

ccgtctacgc tccactgaag ataatgggct cccgctgttc aaaaaaatca gcgtgcgtcg     600

ataagacttt ggtgcagtct cttcggggtc gcaatttaga tttgccgcat ggagggtatc     660

tggggatttt tgccaatgct ggagcgacga ctgtacgatt cgtcccatcg ggatctagca     720

gaccaatgat gttgacacac atcggccatg catgtacgga cggtctattg cgcgagtttg     780

ttattttcga aggacaagat ggaagtgtat atggaaccga caataatgtt agtttgcatt     840

tcttagggcg gaatctacat gatatcttat ccaagcgggg tatgagccag agagatgtga     900

tggtcataaa gggtaaattt tttagatctg aaataacgca gttgcccaaa caacgatcgc     960

gattaaaaga aaaatcggat ggttcaatta ggacatgcat ggattctgtg cgcataaacc    1020

ataaccgcag cactgttggg cacttcggta actcaaatgc gaagcgttgc acgtctgcga    1080

taactacgcc tactatgcac attgttactc ctgcatctta aaaatatatc ctgtagtaat    1140

tttcacagca atgtcataac atcatctcgc taaagaatga cctgggattg gagaagtaat 1200

gaatatttgc aaccaatgca ttgaataaac taacattaaa cgaattcgag ctcggtacag 1260

cttggctgtg gaatgtgtgt cagttagggt gtggaaagtc cccaggctcc ccagcaggca 1320

gaagtatgca aagcatgcat ctcaattagt cagcaaccag gtgtggaaag tccccaggct 1380

ccccagcagg cagaagtatg caaagcatgc atctcaatta gtcagcaacc atagtcccgc 1440

ccctaactcc gcccatcccg cccctaactc cgcccagttc cgcccattct ccgccccatg 1500

gctgactaat tttttttatt tatgcagagg ccgaggccgc ctcggcctct gagctattcc 1560

agaagtagtg aggaggcttt tttggaggcc taggcttttg caaaaagctg cggccgccac 1620

catgggcagc aagcccagca caagaatccc agcccccctg atgctgatca cccgcatcat 1680

gctgatcctg ggctgcatca gacccacaag ctccctggat ggacgccccc tggccgctgc 1740

cggcatcgtg gtgaccggcg acaaggccgt gaacgtgtac accagcagcc agaccggcag 1800

catcatcgtg aagctgctgc ccaacatgcc cagagacaaa gaggcctgcg ccaaggcccc 1860

cctggaagcc tacaacagaa ccctgaccac cctgctgacc cccctgggcg acagcatcag 1920

aaagatccag ggctccgtga gcacaagcgg cggaggaaag cagggcagac tgatcggcgc 1980

cgtgatcggc agcgtggccc tgggagtggc tacagctgcc cagattaccg ctgcagccgc 2040

cctgatccag gccaaccaga acgccgccaa catcctgaga ctgaaagaga gcattgccgc 2100

caccaacgag gccgtgcacg aagtgaccga cggcctgagc cagctgtccg tggccgtggg 2160

caagatgcag cagttcgtga acgaccagtt caacaacacc gccagagagc tggactgcat 2220

caagatcacc cagcaggtgg gcgtggagct gaacctgtac ctgaccgagc tgaccacagt 2280

gttcggcccc cagatcacaa gcccagccct gacacagctg accatccagg ccctgtacaa 2340

cctggctggc ggcaacatgg actatctgct gacaaagctg ggaatcggca acaaccagct 2400

gtccagcctg atcggaagcg gcctgatcac cggctacccc atcctgtacg acagccagac 2460

acagctgctg ggcatccagg tgaacctgcc cagcgtgggc aacctgaaca acatgcgcgc 2520

cacctacctg gaaaccctga gcgtgtccac caccaagggc tacgccagcg ccctggtgcc 2580

caaggtggtg acacaggtgg gcagcgtgat cgaggaactg gacaccagct actgcatcga 2640

gagcgacctg gacctgtact gcaccagaat cgtgaccttc ccaatgagcc ccggcatcta 2700

cagctgcctg agcggcaaca ccagcgcctg catgtacagc aagaccgaag gcgcactgac 2760

aacaccctac atggccctga agggaagcgt gatcgccaac tgcaagatca ccacctgcag 2820

atgcaccgac cccccaggca tcatcagcca gaactacggc gaggccgtga gcctgatcga 2880

tcgccattcc tgtaacgtgc tgtccctgga cggcatcaca ctgagactga gcggcgagtt 2940

cgatgccacc taccagaaga acatcagcat cctggacagc caggtgatcg tgaccggcaa 3000

cctggacatc agcaccgagc tgggcaacgt gaataacagc atcagcaacg ccctggacag 3060

actggccgag agcaacagca agctggaaaa agtgaacgtg cgcctgacat ccacttccgc 3120

tctgatcacc tacatcgtgc tgaccgtgat cagcctggtg ttcggcgccc tgagcctggt 3180

gctggcctgc tacctgatgt acaagcagaa ggcccagcag aaaaccctgc tgtggctggg 3240

caacaacacc ctggaccaga tgagagccac caccagagcc tgatgagcgg ccgcggggat 3300

ccagacatga taagatacat tgatgagttt ggacaaacca caactagaat gcagtgaaaa 3360

aaatgcttta tttgtgaaat ttgtgatgct attgctttat ttgtaaccat tataagctgc 3420

aataaacaag ttaacaacaa caattgcatt gattttatgt ttcaggttca gggggaggtg 3480

tgggaggttt tttcggatcc tctagagtcg acaattattt tatttaataa catatagccc 3540

aaagacctct atgaacattt agtttcccgt atactcaacg gcgcgtgtac acacgcatct 3600

ctttgcatag cgatgaagtt tgttcggcag cagaaaatgc agatatccaa caatctggag 3660

aaaacttatc atcacagtgg cagtggaaac ataccccctc tatattcatg gtataattat 3720

cgtctacagc gtccaggata gtggcgtgag aaaatggaga tctgcagccc tcctttccat 3780

ggcatgccgc tttattgttc attaaacgca caatggtctc aacgccagat atgggcatag 3840

attctgaaga acccgttgac aatccgaaga agaaggcgtg caggtctttg gaagactcgc 3900

acgttggtct tataatgtat gatcgagatg tcaccctaat gccacatggt acaggcttat 3960

cgcggtcatg gcgatcggac ttgtaatttg caacgatggg caaaggatcg acgacatgcc 4020

aaacattctg aacccgtaga gatgttaacg atgacgagga tgaatatccc atgctcgctg 4080

ccatagtatc aagtacaccg cgaataagga cgcgtccaac atcgttatat gcacacaatg 4140

ggctacacgt gactaacacc cccgaatatt agtcatatgt gagtttcagt ctggctccca 4200

tatagcctgt agactatttg tggtttaagt gtgaacgagg cgctgtgaac gagactcggg 4260

ccgattgtaa gaacaagcaa atgcactttc catttaacaa gaagtgtaga gagaatactc 4320

aacctctttg gatgtatcct cgag 4344

<210> 19
<211> 4085
<212> DNA
<213> artificial sequence

<220>
<223> Partial plasmid pSB1 44cds SV FCAopt sequence for vSB1-009

<400> 19
cttttgtcat gctcggagct ctgatcgcat cttatcatta cgtctgcata gcaacgtctg 60

gagacgtgac gtggaagacc gggtttttag ttgtggcggc agggacgatt gccggcatca 120

cggctccgta tggagacatt tctcctctag ccggctttct ttcggcgtat acggcgttag 180

ctattcacgt ggtcagagac gccagtcggt ctctaatgaa cacgtgctac taccgtgcac 240

gtcgggaaat tactgtgaac ggtgcatatc gcctcggtcg cgcgcgtctc ccgcccagca 300

cggacgccga ggcgacgcgc gaagaagacg tatccagtta cgatacgctg ggggggaata 360

ttcctacgat aattctgagc ctcatagcgg tcatctcgat tccagccata gccagctttc 420

aaaagtacat gtcgaacgca actaagcacc agtcaacatt gactgacacg ttacgcagta 480

tatgcggttt cttggtgggt acaagtgtcg cgatattcct tccgtcgcgc taccacgagg 540

ttctgttccg tccaattctt gtattactgt taatattcgg ggcaatggct actaccttag 600

ccggcttcgg tttacttctc gggccgacat tgttttccgc gacagccgcg gttctgtgct 660

gctacacttg tataaatgta cgcaacgcga atagcggaat aaagcaattg gcggccgccg 720

cagctggtaa atgcatatta ggaactgcca tctcgagcat gttggtttgc gtgttaatac 780

aatattcctg atcgcggagc gattaatttt tatatcatgt gctcatagcg ttctttcgaa 840

ctgcgaataa aactttcgtg gctactaaag gggcctatcg tgggtttatg cgctgtcgaa 900

aacatgaaag ggccgattta aagctaagtt gcgcaggcag aggccactcc atatacgctc 960

tcggagacgc ggctcgcacg ccagctgaaa tattttcccc cctgcaggtc gacccaattc 1020

gagctcggta cagcttggct gtggaatgtg tgtcagttag ggtgtggaaa gtccccaggc 1080

tccccagcag gcagaagtat gcaaagcatg catctcaatt agtcagcaac caggtgtgga 1140

aagtccccag gctccccagc aggcagaagt atgcaaagca tgcatctcaa ttagtcagca 1200

accatagtcc cgcccctaac tccgcccatc ccgcccctaa ctccgcccag ttccgcccat 1260

tctccgcccc atggctgact aatttttttt atttatgcag aggccgaggc cgcctcggcc 1320

tctgagctat tccagaagta gtgaggaggc ttttttggag gcctaggctt ttgcaaaaag 1380

ctcccggggc ggccgccacc atgggcagca agcccagcac ctggatcagc gtgaccctga 1440

tgctgatcac cagaaccatg ctgatcctga gctgcatctg ccccacaagc agcctggacg 1500

gcagacccct ggccgctgcc ggcatcgtgg tgaccggcga caaggccgtg aacatctaca 1560

ccagcagcca gaccggcagc atcatcatca agctgctgcc caacatgccc aaggacaaag 1620

aggcctgcgc caaggccccc ctggaagcct acaacagaac cctgaccacc ctgctgaccc 1680

ccctgggcga cagcatcaga agaatccagg gcagcgccac cacaagcggc ggaggaaagc 1740

agggcagact ggtgggcgct atcatcggga gcgtggccct gggcgtggcc acagctgccc 1800

agattaccgc tgcagccgcc ctgattcagg ccaatcagaa cgccgccaac atcctgagac 1860

tgaaagagag cattgccgcc accaacgacg ccgtgcacga agtgacaaac ggactgtccc 1920

agctggctgt cgctgtcggc aagatgcagc agttcgtgaa caaccagttc aacaacaccg 1980

ccagagagct ggactgcatc aagatcgccc agcaggtggg cgtggagctg aacctgtacc 2040

tgaccgagct gaccacagtg ttcggccccc agatcacaag ccccgctctg acccagctga 2100

caatccaggc cctgtacaac ctggctggcg gcaacatgga ctatctgctg actaagctgg 2160

gagtgggcaa caaccagctg tccagcctga tcgggtccgg gctgatcaca ggcaacccca 2220

tcctgtacga cagccagaca cagctgctgg gcatccagat caacctgcca tccgtgggaa 2280

gcctgaacaa catgagagcc acctacctgg aaaccctgag cgtgtccacc accaagggct 2340

tcgccagcgc cctggtgccc aaggtggtga cacaggtggg cagcgtgatc gaggaactgg 2400

acaccagcta ctgcatcgag agcgacatcg acctgtactg caccagagtg gtgaccttcc 2460

caatgagccc cggcatctac agctgcctga gcggcaacac cagcgcctgc atgtacagca 2520

agaccgaagg agcactgaca acaccctaca tggccctgaa gggaagcgtg atcgccaact 2580

gcaagatgac cacctgcaga tgcgccgacc ccccaggcat catcagccag aactacggcg 2640

aggccgtgag cctgatcgac aaacattcct gtagcgtgct gtccctggat ggcatcacac 2700

tgagactgag cggcgagttc gacgccacct accagaagaa catcagcatc ctggacagcc 2760

aggtgatcgt gaccggcaac ctggacatca gcaccgagct gggcaacgtg aacaacagca 2820

tcagcagcac cctggacaag ctggccgagt ccaacaacaa gctgaacaaa gtgaacgtga 2880

acctgaccag cacaagcgcc ctgatcacct acatcgtgct ggccatcgtg tccctggcct 2940

tcggcgtgat cagcctggtg ctggcctgct acctgatgta caagcagaga gcccagcaga 3000

aaaccctgct gtggctgggc aataacaccc tggaccagat gagggccacc accagaacct 3060

gatgagcggc cgcgatacct gcaggtttgc ggtgacattg atctggctca ttatatgccc 3120

cgagctcttg taacatcgcg gacgcgattt ccgtagtagg cacatctcaa atgcaaaagc 3180

ggcatgtcaa ccgtataggt acatccggcc ctgcttacag tcggtagggc atatatccac 3240

cggaaaactt cagctttaga ctcctcaggt gatgaggaat agtatgtaac cctctagcag 3300

tacggtattt ctaaaaaaag gtagatcctt ttccacacgg cacagactaa ataacgtaca 3360

ctacacaggt tctctcgaac ttcgtttgga ccggaattat tccctcggca gcgcctaaaa 3420

agcaaacctc tagagtagat aagtgtcagt gaacctaggc cttctttgtt ccacggctgg 3480

aaagctaagg gacgaggtac acgcgacccc agccacgcac gaacagagtt taacggaagc 3540

gtcgtttgcg ggataaggtt gtcggacccc gcgggtccgt tgaaaagtgg ctgcgcgcct 3600

accgacgaat acgtcggtaa caattttaga aatcgaatat gactgcgagt accgtacaat 3660

cgcgaaatac ggtctctata tagctactcg gtccttaaat atgtaagtat gatgtcccct 3720

actcccgaag acgaccgcga cttggtcgca gtacgtgggc tgctccggat gatggacgag 3780

accacatctg agcgacacaa acgttcgcgt tcaggatgcc cccggttgtt atgcggttgt 3840

acgatcggga tcgctcttac tgtgttcgtc atcacagcta cggtcgtgct agcttcgctg 3900

tttgcattct cttacatgtc cctggagtcc ggtacatgtc ctcacgaatg gatcggttta 3960

ggctatagtt gtatgcgcgc gatggggagc aacgctaccg agctagaagc cctagatacg 4020

tgctcccgac ataacagcaa gcttgtcgac tttactcatg cgaaaattct aatcgaagct 4080

atcgc 4085

<210> 20
<211> 4335
<212> DNA
<213> artificial sequence

<220>
<223> Partial plasmid pHVT US2 SV-Fopt-SynPA for vHVT306

<400> 20
taaaatggga tctatcatta cattcgttaa gagtctggat aattttactg tttgccagct 60

tcgatcttgg aacgtactgt ggatagtgcc ttacttggaa tcgtgaaaat ttgaaacgtc 120

cattatttgg atatcttccg gttgtcccat atcccgccct ggtaccgctc ggataccttg 180

cccgtatgga ttcgtattga cagtcgcgca atcggggacc aacaacgcgt gggtccacac 240

tcattcggaa attttccgat gattctgaat atttattgcc gctcgttacg agtcgttgga 300

catatctgta atacatttct tcttctgaag gatcgctgca catttgatct atacattggc 360

caggatgttc aagtctcaga tgttgcattc tggcacagca caactttatg gcatttccga 420

tgtaatcgtc cggcagccct gggggagttc tatattcgca tattgggatg gtaaggacaa 480

tagcagatct cgcaacctcc agggaggcta taataacgtt tttaaaggat ggatttctca 540

taaaaatctg tcgcaaatta cactgagaat atcctttact agcgccgatt gagagcatcg 600

tcgtccaatt ttctaaatgg aaagaaaaca aggcgggcaa gagtgttcca aacattttca 660

ttttcggcga atctctcaaa tcccatggcg tgcaattgat tgcaaaattg gcacttccgt 720

tcacgtttgt atctccaaac tctaagacac ttttaattga aaaactacgt tctagtgtgg 780

aaagaaacct ataggcagac catagaacta tttgacacca catatctttt tgtatgtcaa 840

actgaccatg atcgtatgtt gctgaatgca ctagggcaat tcgctcgcgc gactccatac 900

attgaataat tccacacgtc agctcatcgg ttagcaaggt ccagtagttg aagtcattta 960

tttttccccg cggctggcca aatctacctc tgggaatatc caagttgtcg aatatgatcg 1020

caccggctct ggtcatggtg aaggaacttg tagcataaag acgcaggtat cataggggta 1080

atattttttt attcactcac atactaaaag taacgcatat tagcaccatg tatgggctat 1140

caattgacat ttgcgtagca ctacatcacg attatgtaca acataatggg acaacatatg 1200

cctgcaggtc gacccaattc gagctcggta cagcttggct gtggaatgtg tgtcagttag 1260

ggtgtggaaa gtccccaggc tccccagcag gcagaagtat gcaaagcatg catctcaatt 1320

agtcagcaac caggtgtgga aagtccccag gctccccagc aggcagaagt atgcaaagca 1380

tgcatctcaa ttagtcagca accatagtcc cgcccctaac tccgcccatc ccgcccctaa 1440

ctccgcccag ttccgcccat tctccgcccc atggctgact aatttttttt atttatgcag 1500

aggccgaggc cgcctcggcc tctgagctat tccagaagta gtgaggaggc ttttttggag 1560

gcctaggctt ttgcaaaaag ctcccggggc ggccgccacc atgggcagca agcccagcac 1620

aagaatccca gcccccctga tgctgatcac ccgcatcatg ctgatcctgg gctgcatcag 1680

acccacaagc tccctggatg gacgccccct ggccgctgcc ggcatcgtgg tgaccggcga 1740

caaggccgtg aacgtgtaca ccagcagcca gaccggcagc atcatcgtga agctgctgcc 1800

caacatgccc agagacaaag aggcctgcgc caaggccccc ctggaagcct acaacagaac 1860

cctgaccacc ctgctgaccc ccctgggcga cagcatcaga aagatccagg gctccgtgag 1920

cacaagcggc ggaggaaagc agggcagact gatcggcgcc gtgatcggca gcgtggccct 1980

gggagtggct acagctgccc agattaccgc tgcagccgcc ctgatccagg ccaaccagaa 2040

cgccgccaac atcctgagac tgaaagagag cattgccgcc accaacgagg ccgtgcacga 2100

agtgaccgac ggcctgagcc agctgtccgt ggccgtgggc aagatgcagc agttcgtgaa 2160

cgaccagttc aacaacaccg ccagagagct ggactgcatc aagatcaccc agcaggtggg 2220

cgtggagctg aacctgtacc tgaccgagct gaccacagtg ttcggccccc agatcacaag 2280

cccagccctg acacagctga ccatccaggc cctgtacaac ctggctggcg gcaacatgga 2340

ctatctgctg acaaagctgg gaatcggcaa caaccagctg tccagcctga tcggaagcgg 2400

cctgatcacc ggctacccca tcctgtacga cagccagaca cagctgctgg gcatccaggt 2460

gaacctgccc agcgtgggca acctgaacaa catgcgcgcc acctacctgg aaaccctgag 2520

cgtgtccacc accaagggct acgccagcgc cctggtgccc aaggtggtga cacaggtggg 2580

cagcgtgatc gaggaactgg acaccagcta ctgcatcgag agcgacctgg acctgtactg 2640

caccagaatc gtgaccttcc caatgagccc cggcatctac agctgcctga gcggcaacac 2700

cagcgcctgc atgtacagca agaccgaagg cgcactgaca acaccctaca tggccctgaa 2760

gggaagcgtg atcgccaact gcaagatcac cacctgcaga tgcaccgacc ccccaggcat 2820

catcagccag aactacggcg aggccgtgag cctgatcgat cgccattcct gtaacgtgct 2880

gtccctggac ggcatcacac tgagactgag cggcgagttc gatgccacct accagaagaa 2940

catcagcatc ctggacagcc aggtgatcgt gaccggcaac ctggacatca gcaccgagct 3000

gggcaacgtg aataacagca tcagcaacgc cctggacaga ctggccgaga gcaacagcaa 3060

gctggaaaaa gtgaacgtgc gcctgacatc cacttccgct ctgatcacct acatcgtgct 3120

gaccgtgatc agcctggtgt tcggcgccct gagcctggtg ctggcctgct acctgatgta 3180

caagcagaag gcccagcaga aaaccctgct gtggctgggc aacaacaccc tggaccagat 3240

gagagccacc accagagcct gatgagcggc cgcgatatca ataaaatatc tttattttca 3300

ttacatctgt gtgttggttt tttgtgtgaa tcgatagtac taacatacgc tctccatcaa 3360

aacaaaacga aacaaaacaa actagcaaaa taggctgtcc ccagtgcaag tgcaggtgcc 3420

agaacatttc tcttctagac ctgcaggccc gggcaagtag atgcaatttc ctcacactag 3480

ttgggtttat ctactattga attttcccct atctgtgata cacttgggag cctctacaag 3540

catattgcca tcatgtacgt ttttatctac tgtcttaacg cccatgggaa cggaggcgtc 3600

gtcgtcatgt attggacggc aacataggca gcaacacaaa ttgcgtttag gtggggtgca 3660

tgtggactcg ataccaagcc cctgcagctg gggaacgtct ggtggagagc cgataatttg 3720

atatacgcac gccatattac tgtcgttgaa gtacgcctta tcttctatgt tttcaaattt 3780

aggttcccaa gtggacgtga gaagtgtttg tatctcacat ggaatggccc aaggcattcc 3840

agcccaggtg cctggtactt taatggcaaa caaacgtttt ggtagaggta ttgattctat 3900

tgcagttctg cagatatctg cagccccgag tatccacagg ctatacgata cgttatcgga 3960

ggcctccgat tctagcatta catagccggt cagtagatcc tgccattcgg tagcgcaacc 4020

ggctacatct tcaaacagtc tcacaataaa tgcatctctc gttcctgcca atccggaacc 4080

gggcatacca ctcccgcctg ccgatttaat tctcacaatt gggcgatgcc ggcggggcaa 4140

aacgaatgtg gatttggcaa accgacacag gtctgctgta cggactaata tgggcacacc 4200

cacatcattc ttcagatgct ccatgcattg ttctatgaga aagatccata gggtggaggc 4260

agcgtcacga gatcgcccag gcaatcgatc gcattcgtct agtaaagtga cgagagttat 4320

catgcacaca cccat 4335

<210> 21
<211> 5381
<212> DNA
<213> artificial sequence

<220>
<223> plasmid pCD046+NDV-F wt for vHVT110

<400> 21
gagctcaggg tatgatactc agctgttatt gtggccgacc aggaggactc caatgcttag 60

cattcataag aacgctagag atgctattta acgatgtgct gtcgtctaaa gaatttgtgc 120

atttagcctt taaatgtaaa accaatgacg cattcactac gctcgtgcgt gcaatttctg 180

ggccagggta tgcatattcc ataacagaaa tcgacacttg agaagaggat ctgactgttt 240

gggataaagg tcgtttgggt ctgtcctagc gatataattt atatgacgat atacattaaa 300

catctgtgtg cagtacttag gtatttaatc atgtcgatga aatgttatgt gtaaatatcg 360

gacaatatag ataacgggca cgctgctatt gtaacgtgcg cccgcgcgct agtgctgact 420

aatagtgtgg atgatgtata cagtatatta caaacggaaa tgatacgtaa taaattatgt 480

actcttattg atttataaaa acatacatgc agtgttgcta tgtcacataa ttagcctcgc 540

ccgtctacgc tccactgaag ataatgggct cccgctgttc aaaaaaatca gcgtgcgtcg 600
ataagacttt ggtgcagtct cttcggggtc gcaatttaga tttgccgcat ggagggtatc 660
tggggatttt tgccaatgct ggagcgacga ctgtacgatt cgtcccatcg ggatctagca 720
gaccaatgat gttgacacac atcggccatg catgtacgga cggtctattg cgcgagtttg 780
ttattttcga aggacaagat ggaagtgtat atggaaccga caataatgtt agtttgcatt 840
tcttagggcg gaatctacat gatatcttat ccaagcgggg tatgagccag agagatgtga 900
tggtcataaa gggtaaattt tttagatctg aaataacgca gttgcccaaa caacgatcgc 960
gattaaaaga aaaatcggat ggttcaatta ggacatgcat ggattctgtg cgcataaacc 1020
ataaccgcag cactgttggg cacttcggta actcaaatgc gaagcgttgc acgtctgcga 1080
taactacgcc tactatgcac attgttactc ctgcatctta aaatatatcc ctgtagtaat 1140
tttcacagca atgtcataac atcatctcgc taaagaatga cctgggattg gagaagtaat 1200
gaatatttgc aaccaatgca ttgaataaac taacattaaa cgaattcaat agtggatccc 1260
ccaactccgc ccgttttatg actagaacca atagttttta atgccaaatg cactgaaatc 1320
ccctaatttg caaagccaaa cgccccctat gtgagtaata cggggacttt ttacccaatt 1380
tcccacgcgg aaagccccct aatacactca tatggcatat gaatcagcac ggtcatgcac 1440
tctaatggcg gcccataggg actttccaca tagggggcgt tcaccatttc ccagcatagg 1500
ggtggtgact caatggcctt tacccaagta cattgggtca atgggaggta agccaatggg 1560
tttttcccat tactggcaag cacactgagt caaatgggac tttccactgg gttttgccca 1620
agtacattgg gtcaatggga ggtgagccaa tgggaaaaac ccattgctgc caagtacact 1680
gactcaatag ggactttcca atgggttttt ccattgttgg caagcatata aggtcaatgt 1740
gggtgagtca atagggactt tccattgtat tctgcccagt acataaggtc aatagggggt 1800
gaatcaacag gaaagtccca ttggagccaa gtacactgcg tcaataggga ctttccattg 1860
ggttttgccc agtacataag gtcaataggg gatgagtcaa tgggaaaaac ccattggagc 1920
caagtacact gactcaatag ggactttcca ttgggttttg cccagtacat aaggtcaata 1980
gggggtgagt caacaggaaa gttccattgg agccaagtac attgagtcaa tagggacttt 2040
ccaatgggtt ttgcccagta cataaggtca atgggaggta agccaatggg tttttcccat 2100
tactggcacg tatactgagt cattagggac tttccaatgg gttttgccca gtacataagg 2160
tcaatagggg tgaatcaaca ggaaagtccc attggagcca agtacactga gtcaataggg 2220
actttccatt gggttttgcc cagtacaaaa ggtcaatagg gggtgagtca atgggttttt 2280
cccattattg gcacgtacat aaggtcaata ggggtgagtc attgggtttt tccagccaat 2340
ttaattaaaa cgccatgtac tttcccacca ttgacgtcaa tgggctattg aaactaatgc 2400
aacgtgacct ttaaacggta ctttcccata gctgattaat gggaaagtac cgttctcgag 2460

ccaatacacg tcaatgggaa gtgaaagggc agccaaaacg taacaccgcc ccggttttcc 2520

cctggaaatt ccatattggc acgcattcta ttggctgagc tgcgttctac gtgggtataa 2580

gaggcgcgac cagcgtcggt accgtcgcag tcttcggtct gaccaccgta gaacgcagag 2640

ctcctcgctg caggcggccg catgggctcc aaaccttcta ccaggatccc agcacctctg 2700

atgctgatca cccggattat gctgatattg ggctgtatcc gtccgacaag ctctcttgac 2760

ggcaggcctc ttgcagctgc aggaattgta gtaacaggag ataaggcagt caatgtatac 2820

acttcgtctc agacagggtc aatcatagtc aagttgctcc cgaatatgcc cagggataag 2880

gaggcgtgtg caaaagcccc attagaggca tataacagaa cactgactac tttgctcact 2940

cctcttggcg actccatccg caagatccaa gggtctgtgt ccacatctgg aggaggcaag 3000

caaggccgcc tgataggtgc tgttattggc agtgtagctc ttggggttgc aacagcggca 3060

cagataacag cagctgcggc cctaatacaa gccaaccaga atgccgccaa catcctccgg 3120

cttaaggaga gcattgctgc aaccaatgaa gctgtgcatg aagtcaccga cggattatca 3180

caactatcag tggcagttgg gaagatgcag cagtttgtca atgaccagtt taataatacg 3240

gcgcgagaat tggactgtat aaaaatcaca caacaggttg gtgtagaact caacctatac 3300

ctaactgaat tgactacagt attcgggcca cagatcacct cccctgcatt aactcagctg 3360

accatccagg cactttataa tttagctggt ggcaatatgg attacttatt aactaagtta 3420

ggtataggga acaatcaact cagctcgtta attggtagcg gcctgatcac tggttaccct 3480

atactgtatg actcacagac tcaactcttg ggcatacaag tgaatttacc ctcagtcggg 3540

aacttaaata atatgcgtgc cacctatttg gagaccttat ctgtaagtac aaccaaagga 3600

tatgcctcag cacttgtccc gaaagtagtg acacaagtcg gttccgtgat agaagagctt 3660

gacacctcat actgtataga gtccgatctg gatttatatt gtactagaat agtgacattc 3720

cccatgtccc caggtattta ttcctgtttg agcggcaaca catcagcttg catgtattca 3780

aagactgaag gcgcactcac tacgccgtat atggccctta aaggctcagt tattgccaat 3840

tgtaaaataa caacatgtag atgtacagac cctcctggta tcatatcgca aaattatgga 3900

gaagctgtat ccctgataga tagacattcg tgcaatgtct tatcattaga cgggataact 3960

ctaaggctca gtggggaatt tgatgcaact tatcaaaaga acatctcaat actagattct 4020

caagtcatcg tgacaggcaa tcttgatata tcaactgaac ttggaaacgt caacaattca 4080

atcagcaatg ccttggatag gttggcagaa agcaacagca agctagaaaa agtcaatgtc 4140

agactaacca gcacatctgc tctcattacc tatattgttc taactgtcat ttctctagtt 4200

ttcggtgcac ttagtctggt gttagcgtgt tacctgatgt acaaacagaa ggcacaacaa 4260

aagaccttgc tatggcttgg gaataatacc ctcgatcaga tgagagccac tacaagagca 4320

tgagcggccg cggggatcca gacatgataa gatacattga tgagtttgga caaaccacaa 4380

ctagaatgca gtgaaaaaaa tgctttattt gtgaaatttg tgatgctatt gctttatttg 4440

taaccattat aagctgcaat aaacaagtta acaacaacaa ttgcattgat tttatgtttc 4500

aggttcaggg ggaggtgtgg gaggtttttt cggatcctct agagtcgaca attattttat 4560

ttaataacat atagcccaaa gacctctatg aacatttagt ttcccgtata ctcaacggcg 4620

cgtgtacaca cgcatctctt tgcatagcga tgaagtttgt tcggcagcag aaaatgcaga 4680

tatccaacaa tctggagaaa acttatcatc acagtggcag tggaaacata ccccctctat 4740

attcatggta taattatcgt ctacagcgtc caggatagtg gcgtgagaaa atggagatct 4800

gcagccctcc tttccatggc atgccgcttt attgttcatt aaacgcacaa tggtctcaac 4860

gccagatatg ggcatagatt ctgaagaacc cgttgacaat ccgaagaaga aggcgtgcag 4920

gtctttggaa gactcgcacg ttggtcttat aatgtatgat cgagatgtca ccctaatgcc 4980

acatggtaca ggcttatcgc ggtcatggcg atcggacttg taatttgcaa cgatgggcaa 5040

aggatcgacg acatgccaaa cattctgaac ccgtagagat gttaacgatg acgaggatga 5100

atatcccatg ctcgctgcca tagtatcaag tacaccgcga ataaggacgc gtccaacatc 5160

gttatatgca cacaatgggc tacacgtgac taacaccccc gaatattagt catatgtgag 5220

tttcagtctg gctcccatat agcctgtaga ctatttgtgg tttaagtgtg aacgaggcgc 5280

tgtgaacgag actcgggccg attgtaagaa caagcaaatg cactttccat ttaacaagaa 5340

gtgtagagag aatactcaac ctctttggat gtatcctcga g 5381

<210> 22
<211> 4337
<212> DNA
<213> artificial sequence

<220>
<223> Partial plasmid pHM103+NDV-F wt sequence for vHVT111

<400> 22
gagctcaggg tatgatactc agctgttatt gtggccgacc aggaggactc caatgcttag 60

cattcataag aacgctagag atgctattta acgatgtgct gtcgtctaaa gaatttgtgc 120

atttagcctt taaatgtaaa accaatgacg cattcactac gctcgtgcgt gcaatttctg 180

ggccagggta tgcatattcc ataacagaaa tcgacacttg agaagaggat ctgactgttt 240

gggataaagg tcgtttgggt ctgtcctagc gatataattt atatgacgat atacattaaa 300

catctgtgtg cagtacttag gtatttaatc atgtcgatga aatgttatgt gtaaatatcg 360

gacaatatag ataacgggca cgctgctatt gtaacgtgcg cccgcgcgct agtgctgact 420

aatagtgtgg atgatgtata cagtatatta caaacggaaa tgatacgtaa taaattatgt 480

actcttattg atttataaaa acatacatgc agtgttgcta tgtcacataa ttagcctcgc 540

ccgtctacgc tccactgaag ataatgggct cccgctgttc aaaaaaatca gcgtgcgtcg 600

ataagacttt ggtgcagtct cttcggggtc gcaatttaga tttgccgcat ggagggtatc 660

tggggatttt tgccaatgct ggagcgacga ctgtacgatt cgtcccatcg ggatctagca 720

gaccaatgat gttgacacac atcggccatg catgtacgga cggtctattg cgcgagtttg 780

ttattttcga aggacaagat ggaagtgtat atggaaccga caataatgtt agtttgcatt 840

tcttagggcg gaatctacat gatatcttat ccaagcgggg tatgagccag agagatgtga 900

tggtcataaa gggtaaattt tttagatctg aaataacgca gttgcccaaa caacgatcgc 960

gattaaaaga aaaatcggat ggttcaatta ggacatgcat ggattctgtg cgcataaacc 1020

ataaccgcag cactgttggg cacttcggta actcaaatgc gaagcgttgc acgtctgcga 1080

taactacgcc tactatgcac attgttactc ctgcatctta aaaatatatc ctgtagtaat 1140

tttcacagca atgtcataac atcatctcgc taaagaatga cctgggattg gagaagtaat 1200

gaatatttgc aaccaatgca ttgaataaac taacattaaa cgaattcgag ctcggtacag 1260

cttggctgtg gaatgtgtgt cagttagggt gtggaaagtc cccaggctcc ccagcaggca 1320

gaagtatgca aagcatgcat ctcaattagt cagcaaccag gtgtggaaag tccccaggct 1380

ccccagcagg cagaagtatg caaagcatgc atctcaatta gtcagcaacc atagtcccgc 1440

ccctaactcc gcccatcccg cccctaactc cgcccagttc cgcccattct ccgccccatg 1500

gctgactaat tttttttatt tatgcagagg ccgaggccgc ctcggcctct gagctattcc 1560

agaagtagtg aggaggcttt tttggaggcc taggcttttg caaaaagctg cggccgcatg 1620

ggctccaaac cttctaccag gatcccagca cctctgatgc tgatcacccg gattatgctg 1680

atattgggct gtatccgtcc gacaagctct cttgacggca ggcctcttgc agctgcagga 1740

attgtagtaa caggagataa ggcagtcaat gtatacactt cgtctcagac agggtcaatc 1800

atagtcaagt tgctcccgaa tatgcccagg gataaggagg cgtgtgcaaa agccccatta 1860

gaggcatata acagaacact gactactttg ctcactcctc ttggcgactc catccgcaag 1920

atccaagggt ctgtgtccac atctggagga ggcaagcaag gccgcctgat aggtgctgtt 1980

attggcagtg tagctcttgg ggttgcaaca gcggcacaga taacagcagc tgcggcccta 2040

atacaagcca accagaatgc cgccaacatc ctccggctta aggagagcat tgctgcaacc 2100

aatgaagctg tgcatgaagt caccgacgga ttatcacaac tatcagtggc agttgggaag 2160

atgcagcagt ttgtcaatga ccagtttaat aatacggcgc gagaattgga ctgtataaaa 2220

atcacacaac aggttggtgt agaactcaac ctatacctaa ctgaattgac tacagtattc 2280

gggccacaga tcacctcccc tgcattaact cagctgacca tccaggcact ttataattta 2340

gctggtggca atatggatta cttattaact aagttaggta tagggaacaa tcaactcagc 2400

tcgttaattg gtagcggcct gatcactggt taccctatac tgtatgactc acagactcaa 2460
ctcttgggca tacaagtgaa tttaccctca gtcgggaact taaataatat gcgtgccacc 2520
tatttggaga ccttatctgt aagtacaacc aaaggatatg cctcagcact tgtcccgaaa 2580
gtagtgacac aagtcggttc cgtgatagaa gagcttgaca cctcatactg tatagagtcc 2640
gatctggatt tatattgtac tagaatagtg acattcccca tgtccccagg tatttattcc 2700
tgtttgagcg gcaacacatc agcttgcatg tattcaaaga ctgaaggcgc actcactacg 2760
ccgtatatgg cccttaaagg ctcagttatt gccaattgta aaataacaac atgtagatgt 2820
acagaccctc ctggtatcat atcgcaaaat tatggagaag ctgtatccct gatagataga 2880
cattcgtgca atgtcttatc attagacggg ataactctaa ggctcagtgg ggaatttgat 2940
gcaacttatc aaaagaacat ctcaatacta gattctcaag tcatcgtgac aggcaatctt 3000
gatatatcaa ctgaacttgg aaacgtcaac aattcaatca gcaatgcctt ggataggttg 3060
gcagaaagca acagcaagct agaaaaagtc aatgtcagac taaccagcac atctgctctc 3120
attacctata ttgttctaac tgtcatttct ctagttttcg gtgcacttag tctggtgtta 3180
gcgtgttacc tgatgtacaa acagaaggca caacaaaaga ccttgctatg gcttgggaat 3240
aataccctcg atcagatgag agccactaca agagcatgag cggccgcggg gatccagaca 3300
tgataagata cattgatgag tttggacaaa ccacaactag aatgcagtga aaaaaatgct 3360
ttatttgtga aatttgtgat gctattgctt tatttgtaac cattataagc tgcaataaac 3420
aagttaacaa caacaattgc attgatttta tgtttcaggt tcagggggag gtgtgggagg 3480
ttttttcgga tcctctagag tcgacaatta ttttatttaa taacatatag cccaaagacc 3540
tctatgaaca tttagtttcc cgtatactca acggcgcgtg tacacacgca tctctttgca 3600
tagcgatgaa gtttgttcgg cagcagaaaa tgcagatatc caacaatctg gagaaaactt 3660
atcatcacag tggcagtgga aacatacccc ctctatattc atggtataat tatcgtctac 3720
agcgtccagg atagtggcgt gagaaaatgg agatctgcag ccctcctttc catggcatgc 3780
cgctttattg ttcattaaac gcacaatggt ctcaacgcca gatatgggca tagattctga 3840
agaacccgtt gacaatccga agaagaaggc gtgcaggtct ttggaagact cgcacgttgg 3900
tcttataatg tatgatcgag atgtcaccct aatgccacat ggtacaggct tatcgcggtc 3960
atggcgatcg gacttgtaat ttgcaacgat gggcaaagga tcgacgacat gccaaacatt 4020
ctgaacccgt agagatgtta acgatgacga ggatgaatat cccatgctcg ctgccatagt 4080
atcaagtaca ccgcgaataa ggacgcgtcc aacatcgtta tatgcacaca atgggctaca 4140
cgtgactaac acccccgaat attagtcata tgtgagtttc agtctggctc ccatatagcc 4200
tgtagactat ttgtggttta agtgtgaacg aggcgctgtg aacgagactc gggccgattg 4260
taagaacaag caaatgcact ttccatttaa caagaagtgt agagagaata ctcaacctct 4320

ttggatgtat cctcgag 4337

<210> 23
<211> 4344
<212> DNA
<213> artificial sequence

<220>
<223> Partial plasmid pHM103+NDV-F CA02 for vHVT116

<400> 23
gagctcaggg tatgatactc agctgttatt gtggccgacc aggaggactc caatgcttag 60

cattcataag aacgctagag atgctattta acgatgtgct gtcgtctaaa gaatttgtgc 120

atttagcctt taaatgtaaa accaatgacg cattcactac gctcgtgcgt gcaatttctg 180

ggccagggta tgcatattcc ataacagaaa tcgacacttg agaagaggat ctgactgttt 240

gggataaagg tcgtttgggt ctgtcctagc gatataattt atatgacgat atacattaaa 300

catctgtgtg cagtacttag gtatttaatc atgtcgatga aatgttatgt gtaaatatcg 360

gacaatatag ataacgggca cgctgctatt gtaacgtgcg cccgcgcgct agtgctgact 420

aatagtgtgg atgatgtata cagtatatta caaacggaaa tgatacgtaa taaattatgt 480

actcttattg atttataaaa acatacatgc agtgttgcta tgtcacataa ttagcctcgc 540

ccgtctacgc tccactgaag ataatgggct cccgctgttc aaaaaaatca gcgtgcgtcg 600

ataagacttt ggtgcagtct cttcggggtc gcaatttaga tttgccgcat ggagggtatc 660

tggggatttt tgccaatgct ggagcgacga ctgtacgatt cgtcccatcg ggatctagca 720

gaccaatgat gttgacacac atcggccatg catgtacgga cggtctattg cgcgagtttg 780

ttattttcga aggacaagat ggaagtgtat atggaaccga caataatgtt agtttgcatt 840

tcttagggcg gaatctacat gatatcttat ccaagcgggg tatgagccag agagatgtga 900

tggtcataaa gggtaaattt tttagatctg aaataacgca gttgcccaaa caacgatcgc 960

gattaaaaga aaaatcggat ggttcaatta ggacatgcat ggattctgtg cgcataaacc 1020

ataaccgcag cactgttggg cacttcggta actcaaatgc gaagcgttgc acgtctgcga 1080

taactacgcc tactatgcac attgttactc ctgcatctta aaaatatatc ctgtagtaat 1140

tttcacagca atgtcataac atcatctcgc taaagaatga cctgggattg gagaagtaat 1200

gaatatttgc aaccaatgca ttgaataaac taacattaaa cgaattcgag ctcggtacag 1260

cttggctgtg gaatgtgtgt cagttagggt gtggaaagtc cccaggctcc ccagcaggca 1320

gaagtatgca aagcatgcat ctcaattagt cagcaaccag gtgtggaaag tccccaggct 1380

ccccagcagg cagaagtatg caaagcatgc atctcaatta gtcagcaacc atagtcccgc 1440

ccctaactcc gcccatcccg cccctaactc cgcccagttc cgcccattct ccgccccatg 1500

gctgactaat tttttttatt tatgcagagg ccgaggccgc ctcggcctct gagctattcc 1560
agaagtagtg aggaggcttt tttggaggcc taggcttttg caaaaagctg cggccgccac 1620
catgggcagc aagcccagca cctggatcag cgtgaccctg atgctgatca ccagaaccat 1680
gctgatcctg agctgcatct gccccacaag cagcctggac ggcagacccc tggccgctgc 1740
cggcatcgtg gtgaccggcg acaaggccgt gaacatctac accagcagcc agaccggcag 1800
catcatcatc aagctgctgc ccaacatgcc caaggacaaa gaggcctgcg ccaaggcccc 1860
cctggaagcc tacaacagaa ccctgaccac cctgctgacc cccctgggcg acagcatcag 1920
aagaatccag ggcagcgcca ccacaagcgg cggaggaaag cagggcagac tggtgggcgc 1980
tatcatcggg agcgtggccc tgggcgtggc cacagctgcc cagattaccg ctgcagccgc 2040
cctgattcag gccaatcaga acgccgccaa catcctgaga ctgaaagaga gcattgccgc 2100
caccaacgac gccgtgcacg aagtgacaaa cggactgtcc cagctggctg tcgctgtcgg 2160
caagatgcag cagttcgtga acaaccagtt caacaacacc gccagagagc tggactgcat 2220
caagatcgcc cagcaggtgg gcgtggagct gaacctgtac ctgaccgagc tgaccacagt 2280
gttcggcccc cagatcacaa gccccgctct gacccagctg acaatccagg ccctgtacaa 2340
cctggctggc ggcaacatgg actatctgct gactaagctg ggagtgggca acaaccagct 2400
gtccagcctg atcgggtccg ggctgatcac aggcaacccc atcctgtacg acagccagac 2460
acagctgctg ggcatccaga tcaacctgcc atccgtggga agcctgaaca acatgagagc 2520
cacctacctg gaaaccctga gcgtgtccac caccaagggc ttcgccagcg ccctggtgcc 2580
caaggtggtg acacaggtgg gcagcgtgat cgaggaactg gacaccagct actgcatcga 2640
gagcgacatc gacctgtact gcaccagagt ggtgaccttc ccaatgagcc ccggcatcta 2700
cagctgcctg agcggcaaca ccagcgcctg catgtacagc aagaccgaag gagcactgac 2760
aacaccctac atggccctga agggaagcgt gatcgccaac tgcaagatga ccacctgcag 2820
atgcgccgac cccccaggca tcatcagcca gaactacggc gaggccgtga gcctgatcga 2880
caaacattcc tgtagcgtgc tgtccctgga tggcatcaca ctgagactga gcggcgagtt 2940
cgacgccacc taccagaaga acatcagcat cctggacagc caggtgatcg tgaccggcaa 3000
cctggacatc agcaccgagc tgggcaacgt gaacaacagc atcagcagca ccctggacaa 3060
gctggccgag tccaacaaca agctgaacaa agtgaacgtg aacctgacca gcacaagcgc 3120
cctgatcacc tacatcgtgc tggccatcgt gtccctggcc ttcggcgtga tcagcctggt 3180
gctggcctgc tacctgatgt acaagcagag agcccagcag aaaaccctgc tgtggctggg 3240
caataacacc ctggaccaga tgagggccac caccagaacc tgatgagcgg ccgcggggat 3300
ccagacatga taagatacat tgatgagttt ggacaaacca caactagaat gcagtgaaaa 3360
aaatgcttta tttgtgaaat ttgtgatgct attgctttat ttgtaaccat tataagctgc 3420

aataaacaag ttaacaacaa caattgcatt gattttatgt ttcaggttca gggggaggtg 3480

tgggaggttt tttcggatcc tctagagtcg acaattattt tatttaataa catatagccc 3540

aaagacctct atgaacattt agtttcccgt atactcaacg gcgcgtgtac acacgcatct 3600

ctttgcatag cgatgaagtt tgttcggcag cagaaaatgc agatatccaa caatctggag 3660

aaaacttatc atcacagtgg cagtggaaac ataccccctc tatattcatg gtataattat 3720

cgtctacagc gtccaggata gtggcgtgag aaaatggaga tctgcagccc tcctttccat 3780

ggcatgccgc tttattgttc attaaacgca caatggtctc aacgccagat atgggcatag 3840

attctgaaga acccgttgac aatccgaaga agaaggcgtg caggtctttg gaagactcgc 3900

acgttggtct tataatgtat gatcgagatg tcaccctaat gccacatggt acaggcttat 3960

cgcggtcatg gcgatcggac ttgtaatttg caacgatggg caaaggatcg acgacatgcc 4020

aaacattctg aacccgtaga gatgttaacg atgacgagga tgaatatccc atgctcgctg 4080

ccatagtatc aagtacaccg cgaataagga cgcgtccaac atcgttatat gcacacaatg 4140

ggctacacgt gactaacacc cccgaatatt agtcatatgt gagtttcagt ctggctccca 4200

tatagcctgt agactatttg tggtttaagt gtgaacgagg cgctgtgaac gagactcggg 4260

ccgattgtaa gaacaagcaa atgcactttc catttaacaa gaagtgtaga gagaatactc 4320

aacctctttg gatgtatcct cgag 4344

<210> 24
<211> 3988
<212> DNA
<213> artificial sequence

<220>
<223> Partial plasmid HVTIG2 SV Fwt SbfI sequence for vHVT301

<400> 24
tgtttcgcac catatccaag ctggctgtcc ctaagagctt attcctgcaa gacctcatac 60

ggaataattg cccgaccaat acttattacg gacataggta ggccgataaa tattatgttg 120

actggaggat ggaaaggagg ttttgtaaca gctacatcgc tcgttcatca gcaagcgata 180

ctttggatat ccgagcttca aaagccgcat aaaccccgct ttatttctga atacgcccca 240

acagtaacac atgcgtggtt cctggcactt ggaacgccgt gttttatagg caagaacata 300

ctacccaaag aggtcttggg atttctggcg cgtcgttgca atgaagaaat gaattctttg 360

ttccttgaaa tgccgacaac tctaaaaacg gtattcgagc accattactt tacgcgtgga 420

tctgaagtaa atccagcgtt gttgatggag cctaacagat ttttgcaact gatggattcg 480

cggaaaatcc tatgtttata cgaatccgct atgtgcgaca accccggagc tcagggtatg 540

atactcagct gttattgtgg ccgaccagga ggactccaat gcttagcatt cataagaacg 600

ctagagatgc tatttaacga tgtgctgtcg tctaaagaat ttgtgcattt agcctttaaa 660
tgtaaaacca atgacgcatt cactacgctc gtgcgtgcaa tttctgggcc agggtatgca 720
tattccataa cagaaatcga cacttgagaa gaggatctga ctgtttggga taaaggtcgt 780
ttgggtctgt cctagcgata taatttatat gacgatatac attaaacatc tgtgtgcagt 840
acttaggtat ttaatcatgt cgatgaaatg ttatgtgtaa atatcggaca atatagataa 900
cgggcacgct gctattgtaa cgtgcgcccg cgcgctagtg ctgactaata gtgtggatga 960
tgtatacagt atattacaaa cggaaatgat acgtaataaa cctgcaggtc gacccaattc 1020
gagctcggta cagcttggct gtggaatgtg tgtcagttag ggtgtggaaa gtccccaggc 1080
tccccagcag gcagaagtat gcaaagcatg catctcaatt agtcagcaac caggtgtgga 1140
aagtccccag gctccccagc aggcagaagt atgcaaagca tgcatctcaa ttagtcagca 1200
accatagtcc cgcccctaac tccgcccatc ccgcccctaa ctccgcccag ttccgcccat 1260
tctccgcccc atggctgact aatttttttt atttatgcag aggccgaggc cgcctcggcc 1320
tctgagctat tccagaagta gtgaggaggc ttttttggag gcctaggctt ttgcaaaaag 1380
ctgcggccgc atgggctcca aaccttctac caggatccca gcacctctga tgctgatcac 1440
ccggattatg ctgatattgg gctgtatccg tccgacaagc tctcttgacg gcaggcctct 1500
tgcagctgca ggaattgtag taacaggaga taaggcagtc aatgtataca cttcgtctca 1560
gacagggtca atcatagtca agttgctccc gaatatgccc agggataagg aggcgtgtgc 1620
aaaagcccca ttagaggcat ataacagaac actgactact ttgctcactc ctcttggcga 1680
ctccatccgc aagatccaag ggtctgtgtc cacatctgga ggaggcaagc aaggccgcct 1740
gataggtgct gttattggca gtgtagctct tggggttgca acagcggcac agataacagc 1800
agctgcggcc ctaatacaag ccaaccagaa tgccgccaac atcctccggc ttaaggagag 1860
cattgctgca accaatgaag ctgtgcatga agtcaccgac ggattatcac aactatcagt 1920
ggcagttggg aagatgcagc agtttgtcaa tgaccagttt aataatacgg cgcgagaatt 1980
ggactgtata aaaatcacac aacaggttgg tgtagaactc aacctatacc taactgaatt 2040
gactacagta ttcgggccac agatcacctc ccctgcatta actcagctga ccatccaggc 2100
actttataat ttagctggtg gcaatatgga ttacttatta actaagttag gtatagggaa 2160
caatcaactc agctcgttaa ttggtagcgg cctgatcact ggttacccta tactgtatga 2220
ctcacagact caactcttgg gcatacaagt gaatttaccc tcagtcggga acttaaataa 2280
tatgcgtgcc acctatttgg agaccttatc tgtaagtaca accaaaggat atgcctcagc 2340
acttgtcccg aaagtagtga cacaagtcgg ttccgtgata gaagagcttg acacctcata 2400
ctgtatagag tccgatctgg atttatattg tactagaata gtgacattcc ccatgtcccc 2460
aggtatttat tcctgtttga gcggcaacac atcagcttgc atgtattcaa agactgaagg 2520

cgcactcact acgccgtata tggcccttaa aggctcagtt attgccaatt gtaaaataac 2580

aacatgtaga tgtacagacc ctcctggtat catatcgcaa aattatggag aagctgtatc 2640

cctgatagat agacattcgt gcaatgtctt atcattagac gggataactc taaggctcag 2700

tggggaattt gatgcaactt atcaaaagaa catctcaata ctagattctc aagtcatcgt 2760

gacaggcaat cttgatatat caactgaact tggaaacgtc aacaattcaa tcagcaatgc 2820

cttggatagg ttggcagaaa gcaacagcaa gctagaaaaa gtcaatgtca gactaaccag 2880

cacatctgct ctcattacct atattgttct aactgtcatt tctctagttt tcggtgcact 2940

tagtctggtg ttagcgtgtt acctgatgta caaacagaag gcacaacaaa agaccttgct 3000

atggcttggg aataataccc tcgatcagat gagagccact acaagagcat gagcggccgc 3060

ggggatccag acatgataag atacattgat gagtttggac aaaccacaac tagaatgcag 3120

tgaaaaaaat gctttatttg tgaaatttgt gatgctattg ctttatttgt aaccattata 3180

agctgcaata aacaagttaa caacaacaat tgcattgatt ttatgtttca ggttcagggg 3240

gaggtgtggg aggttttttc ggatcctcta gaggggatta atcctgcagg ttatgtactc 3300

ttattgattt ataaaaacat acatgcagtg ttgctatgtc acataattag cctcgcccgt 3360

ctacgctcca ctgaagataa tgggctcccg ctgttcaaaa aaatcagcgt gcgtcgataa 3420

gactttggtg cagtctcttc ggggtcgcaa tttagatttg ccgcatggag ggtatctggg 3480

gatttttgcc aatgctggag cgacgactgt acgattcgtc ccatcgggat ctagcagacc 3540

aatgatgttg acacacatcg gccatgcatg tacggacggt ctattgcgcg agtttgttat 3600

tttcgaagga caagatggaa gtgtatatgg aaccgacaat aatgttagtt tgcatttctt 3660

agggcggaat ctacatgata tcttatccaa gcggggtatg agccagagag atgtgatggt 3720

cataaagggt aaatttttta gatctgaaat aacgcagttg cccaaacaac gatcgcgatt 3780

aaaagaaaaa tcggatggtt caattaggac atgcatggat tctgtgcgca taaaccataa 3840

ccgcagcact gttgggcact tcggtaactc aaatgcgaag cgttgcacgt ctgcgataac 3900

tacgcctact atgcacattg ttactcctgc atcttaaaaa tatatcctgt agtaattttc 3960

acagcaatgt cataacatca tctcgcta 3988

<210> 25
<211> 3707
<212> DNA
<213> artificial sequence

<220>
<223> Partial plasmid pHVTUS10 cds F opt plasmid for vHVT302

<400> 25
tcccttacgg cggatcgaaa cgacattagg catactcggg taccattttg cattccgatc 60

agcacggatg aaattaggca ggaatgcggt ttatattatg cggcattgga caaacgatat 120

ggcattgatt ggcagtttat gaatgtcttc atgttgggcg taaacggatt cctattggtt 180

cagaagacaa cgacgatata tttagagaga aaaagctacc cagcatagga taaacacaca 240

ttgagcattg agagacatag gtatcggtat ggatgggaaa actacacacg tgaacaccaa 300

acgacttata tactcgagcg gtgatactac tgagcaagaa tgcactgcat ctgagccact 360

gaatgaagac tgtgatgaaa atgtgaccat cgatggaatt ggagaagaat atgcgcagtt 420

cttcatgtcc ccgcaatggg tcccaaatct acatcgcttg agcgaggata ccaaaaaggt 480

ataccgatgt atggtttcca acagactcaa ttattttccc tattatgagg cgttcaggcg 540

gtctttgttt gatatgtata tgctaggtcg gttggggcgt cgacttaagc gatctgactg 600

ggagactatt atgcatctgt caccaacgca aagtcggcgt ctacatagaa ctttaagatt 660

tgtggagcgt agaattatcc catctaacag ttatatacgc acatcgggcc acgttccgcc 720

ttcgagggca cttccgacag atacgaattt aaagatggat gaataattaa attggaaaga 780

gtaactacat taatcgagcg tcatgacggc gtcccgtgaa aatgggaatt ttctactcga 840

aacaccgtga catttgacag acctggaatt gttattctga tatatagtgg gtgtgtctgg 900

ccggcaacat acataatgtg catgcgaaac cactttttca gtgtacgctg acattgtgca 960

acacggaggg gtagcatcta catacaatat atgttgatta cctgcagggc ggccgccacc 1020

atgggcagca agcccagcac aagaatccca gcccccctga tgctgatcac ccgcatcatg 1080

ctgatcctgg gctgcatcag acccacaagc tccctggatg gacgccccct ggccgctgcc 1140

ggcatcgtgg tgaccggcga caaggccgtg aacgtgtaca ccagcagcca gaccggcagc 1200

atcatcgtga agctgctgcc caacatgccc agagacaaag aggcctgcgc caaggccccc 1260

ctggaagcct acaacagaac cctgaccacc ctgctgaccc ccctgggcga cagcatcaga 1320

aagatccagg gctccgtgag cacaagcggc ggaggaaagc agggcagact gatcggcgcc 1380

gtgatcggca gcgtggccct gggagtggct acagctgccc agattaccgc tgcagccgcc 1440

ctgatccagg ccaaccagaa cgccgccaac atcctgagac tgaaagagag cattgccgcc 1500

accaacgagg ccgtgcacga agtgaccgac ggcctgagcc agctgtccgt ggccgtgggc 1560

aagatgcagc agttcgtgaa cgaccagttc aacaacaccg ccagagagct ggactgcatc 1620

aagatcaccc agcaggtggg cgtggagctg aacctgtacc tgaccgagct gaccacagtg 1680

ttcggccccc agatcacaag cccagccctg acacagctga ccatccaggc cctgtacaac 1740

ctggctggcg gcaacatgga ctatctgctg acaaagctgg gaatcggcaa caaccagctg 1800

tccagcctga tcggaagcgg cctgatcacc ggctacccca tcctgtacga cagccagaca 1860

cagctgctgg gcatccaggt gaacctgccc agcgtgggca acctgaacaa catgcgcgcc 1920

acctacctgg aaaccctgag cgtgtccacc accaagggct acgccagcgc cctggtgccc 1980

aaggtggtga cacaggtggg cagcgtgatc gaggaactgg acaccagcta ctgcatcgag 2040

agcgacctgg acctgtactg caccagaatc gtgaccttcc caatgagccc cggcatctac 2100

agctgcctga gcggcaacac cagcgcctgc atgtacagca agaccgaagg cgcactgaca 2160

acaccctaca tggccctgaa gggaagcgtg atcgccaact gcaagatcac cacctgcaga 2220

tgcaccgacc ccccaggcat catcagccag aactacggcg aggccgtgag cctgatcgat 2280

cgccattcct gtaacgtgct gtccctggac ggcatcacac tgagactgag cggcgagttc 2340

gatgccacct accagaagaa catcagcatc ctggacagcc aggtgatcgt gaccggcaac 2400

ctggacatca gcaccgagct gggcaacgtg aataacagca tcagcaacgc cctggacaga 2460

ctggccgaga gcaacagcaa gctggaaaaa gtgaacgtgc gcctgacatc cacttccgct 2520

ctgatcacct acatcgtgct gaccgtgatc agcctggtgt tcggcgccct gagcctggtg 2580

ctggcctgct acctgatgta caagcagaag gcccagcaga aaaccctgct gtggctgggc 2640

aacaacaccc tggaccagat gagagccacc accagagcct gatgagcggc cgccccgggc 2700

ctgcaggcat aggcacgctc tgatgttaca gaccacaata ccgcatacat ttattgtaag 2760

gttgttaata aaggtttatt ctatgtaaga ctacaatact ttcgacattg cttgtataca 2820

tattaaatac tttctcaagt tcctattaca taaaatggga tctatcatta cattcgttaa 2880

gagtctggat aattttactg tttgccagct tcgatcttgg aacgtactgt ggatagtgcc 2940

ttacttggaa tcgtgaaaat ttgaaacgtc cattatttgg atatcttccg gttgtcccat 3000

atcccgccct ggtaccgctc ggataccttg cccgtatgga ttcgtattga cagtcgcgca 3060

atcggggacc aacaacgcgt gggtccacac tcattcggaa attttccgat gattctgaat 3120

atttattgcc gctcgttacg agtcgttgga catatctgta atacatttct tcttctgaag 3180

gatcgctgca catttgatct atacattggc caggatgttc aagtctcaga tgttgcattc 3240

tggcacagca caactttatg gcatttccga tgtaatcgtc cggcagccct gggggagttc 3300

tatattcgca tattgggatg gtaaggacaa tagcagatct cgcaacctcc agggaggcta 3360

taataacgtt tttaaaggat ggatttctca taaaaatctg tcgcaaatta cactgagaat 3420

atcctttact agcgccgatt gagagcatcg tcgtccaatt ttctaaatgg aaagaaaaca 3480

aggcgggcaa gagtgttcca aacattttca ttttcggcga atctctcaaa tcccatggcg 3540

tgcaattgat tgcaaaattg gcacttccgt tcacgtttgt atctccaaac tctaagacac 3600

ttttaattga aaaactacgt tctagtgtgg aaagaaacct ataggcagac catagaacta 3660

tttgacacca catatctttt tgtatgtcaa actgaccatg atcgtat 3707

<210> 26
<211> 3707
<212> DNA

<213>  artificial sequence

<220>
<223>  Partial plasmid pHVT US20 cds F CA02 opt sequence for vHVT303

<400>  26
tcccttacgg cggatcgaaa cgacattagg catactcggg taccattttg cattccgatc      60

agcacggatg aaattaggca ggaatgcggt ttatattatg cggcattgga caaacgatat     120

ggcattgatt ggcagtttat gaatgtcttc atgttgggcg taaacggatt cctattggtt     180

cagaagacaa cgacgatata tttagagaga aaaagctacc cagcatagga taaacacaca     240

ttgagcattg agagacatag gtatcggtat ggatgggaaa actacacacg tgaacaccaa     300

acgacttata tactcgagcg gtgatactac tgagcaagaa tgcactgcat ctgagccact     360

gaatgaagac tgtgatgaaa atgtgaccat cgatggaatt ggagaagaat atgcgcagtt     420

cttcatgtcc ccgcaatggg tcccaaatct acatcgcttg agcgaggata ccaaaaaggt     480

ataccgatgt atggtttcca acagactcaa ttattttccc tattatgagg cgttcaggcg     540

gtctttgttt gatatgtata tgctaggtcg gttggggcgt cgacttaagc gatctgactg     600

ggagactatt atgcatctgt caccaacgca aagtcggcgt ctacatagaa ctttaagatt     660

tgtggagcgt agaattatcc catctaacag ttatatacgc acatcgggcc acgttccgcc     720

ttcgagggca cttccgacag atacgaattt aaagatggat gaataattaa attggaaaga     780

gtaactacat taatcgagcg tcatgacggc gtcccgtgaa aatgggaatt ttctactcga     840

aacaccgtga catttgacag acctggaatt gttattctga tatatagtgg gtgtgtctgg     900

ccggcaacat acataatgtg catgcgaaac cactttttca gtgtacgctg acattgtgca     960

acacggaggg gtagcatcta catacaatat atgttgatta cctgcagggc ggccgccacc    1020

atgggcagca agcccagcac ctggatcagc gtgaccctga tgctgatcac cagaaccatg    1080

ctgatcctga gctgcatctg ccccacaagc agcctggacg gcagacccct ggccgctgcc    1140

ggcatcgtgg tgaccggcga caaggccgtg aacatctaca ccagcagcca gaccggcagc    1200

atcatcatca agctgctgcc caacatgccc aaggacaaag aggcctgcgc caaggccccc    1260

ctggaagcct acaacagaac cctgaccacc ctgctgaccc ccctgggcga cagcatcaga    1320

agaatccagg gcagcgccac cacaagcggc ggaggaaagc agggcagact ggtgggcgct    1380

atcatcggga gcgtggccct gggcgtggcc acagctgccc agattaccgc tgcagccgcc    1440

ctgattcagg ccaatcagaa cgccgccaac atcctgagac tgaaagagag cattgccgcc    1500

accaacgacg ccgtgcacga agtgacaaac ggactgtccc agctggctgt cgctgtcggc    1560

aagatgcagc agttcgtgaa caaccagttc aacaacaccg ccagagagct ggactgcatc    1620

aagatcgccc agcaggtggg cgtggagctg aacctgtacc tgaccgagct gaccacagtg    1680

ttcggccccc agatcacaag ccccgctctg acccagctga caatccaggc cctgtacaac    1740

ctggctggcg gcaacatgga ctatctgctg actaagctgg gagtgggcaa caaccagctg 1800

tccagcctga tcgggtccgg gctgatcaca ggcaacccca tcctgtacga cagccagaca 1860

cagctgctgg gcatccagat caacctgcca tccgtgggaa gcctgaacaa catgagagcc 1920

acctacctgg aaaccctgag cgtgtccacc accaagggct tcgccagcgc cctggtgccc 1980

aaggtggtga cacaggtggg cagcgtgatc gaggaactgg acaccagcta ctgcatcgag 2040

agcgacatcg acctgtactg caccagagtg gtgaccttcc caatgagccc cggcatctac 2100

agctgcctga gcggcaacac cagcgcctgc atgtacagca agaccgaagg agcactgaca 2160

acaccctaca tggccctgaa gggaagcgtg atcgccaact gcaagatgac cacctgcaga 2220

tgcgccgacc ccccaggcat catcagccag aactacggcg aggccgtgag cctgatcgac 2280

aaacattcct gtagcgtgct gtccctggat ggcatcacac tgagactgag cggcgagttc 2340

gacgccacct accagaagaa catcagcatc ctggacagcc aggtgatcgt gaccggcaac 2400

ctggacatca gcaccgagct gggcaacgtg aacaacagca tcagcagcac cctggacaag 2460

ctggccgagt ccaacaacaa gctgaacaaa gtgaacgtga acctgaccag cacaagcgcc 2520

ctgatcacct acatcgtgct ggccatcgtg tccctggcct tcggcgtgat cagcctggtg 2580

ctggcctgct acctgatgta caagcagaga gcccagcaga aaaccctgct gtggctgggc 2640

aataacaccc tggaccagat gagggccacc accagaacct gatgagcggc cgccccgggc 2700

ctgcaggcat aggcacgctc tgatgttaca gaccacaata ccgcatacat ttattgtaag 2760

gttgttaata aaggtttatt ctatgtaaga ctacaatact ttcgacattg cttgtataca 2820

tattaaatac tttctcaagt tcctattaca taaaatggga tctatcatta cattcgttaa 2880

gagtctggat aattttactg tttgccagct tcgatcttgg aacgtactgt ggatagtgcc 2940

ttacttggaa tcgtgaaaat ttgaaacgtc cattatttgg atatcttccg gttgtcccat 3000

atcccgccct ggtaccgctc ggataccttg cccgtatgga ttcgtattga cagtcgcgca 3060

atcggggacc aacaacgcgt gggtccacac tcattcggaa attttccgat gattctgaat 3120

atttattgcc gctcgttacg agtcgttgga catatctgta atacatttct tcttctgaag 3180

gatcgctgca catttgatct atacattggc caggatgttc aagtctcaga tgttgcattc 3240

tggcacagca caactttatg gcatttccga tgtaatcgtc cggcagccct gggggagttc 3300

tatattcgca tattgggatg gtaaggacaa tagcagatct cgcaacctcc agggaggcta 3360

taataacgtt tttaaaggat ggatttctca taaaaatctg tcgcaattta cactgagaat 3420

atcctttact agcgccgatt gagagcatcg tcgtccaatt ttctaaatgg aaagaaaaca 3480

aggcgggcaa gagtgttcca aacattttca ttttcggcga atctctcaaa tcccatggcg 3540

tgcaattgat tgcaaaattg gcacttccgt tcacgtttgt atctccaaac tctaagacac 3600

ttttaattga aaaactacgt tctagtgtgg aaagaaacct ataggcagac catagaacta 3660

tttgacacca catatctttt tgtatgtcaa actgaccatg atcgtat 3707

<210> 27
<211> 3946
<212> DNA
<213> artificial sequence

<220>
<223> Partial plasmid HVT IG2 SVFopt syn tail sequence for vHVT304

<400> 27
tgtttcgcac catatccaag ctggctgtcc ctaagagctt attcctgcaa gacctcatac 60

ggaataattg cccgaccaat acttattacg gacataggta ggccgataaa tattatgttg 120

actggaggat ggaaaggagg ttttgtaaca gctacatcgc tcgttcatca gcaagcgata 180

ctttggatat ccgagcttca aaagccgcat aaaccccgct ttatttctga atacgcccca 240

acagtaacac atgcgtggtt cctggcactt ggaacgccgt gttttatagg caagaacata 300

ctacccaaag aggtcttggg atttctggcg cgtcgttgca atgaagaaat gaattctttg 360

ttccttgaaa tgccgacaac tctaaaaacg gtattcgagc accattactt tacgcgtgga 420

tctgaagtaa atccagcgtt gttgatggag cctaacagat ttttgcaact gatggattcg 480

cggaaaatcc tatgtttata cgaatccgct atgtgcgaca accccggagc tcagggtatg 540

atactcagct gttattgtgg ccgaccagga ggactccaat gcttagcatt cataagaacg 600

ctagagatgc tatttaacga tgtgctgtcg tctaaagaat ttgtgcattt agcctttaaa 660

tgtaaaacca atgacgcatt cactacgctc gtgcgtgcaa tttctgggcc agggtatgca 720

tattccataa cagaaatcga cacttgagaa gaggatctga ctgtttggga taaaggtcgt 780

ttgggtctgt cctagcgata taatttatat gacgatatac attaaacatc tgtgtgcagt 840

acttaggtat ttaatcatgt cgatgaaatg ttatgtgtaa atatcggaca atatagataa 900

cgggcacgct gctattgtaa cgtgcgcccg cgcgctagtg ctgactaata gtgtggatga 960

tgtatacagt atattacaaa cggaaatgat acgtaataaa cctgcaggtc gacccaattc 1020

gagctcggta cagcttggct gtggaatgtg tgtcagttag ggtgtggaaa gtccccaggc 1080

tccccagcag gcagaagtat gcaaagcatg catctcaatt agtcagcaac caggtgtgga 1140

aagtccccag gctccccagc aggcagaagt atgcaaagca tgcatctcaa ttagtcagca 1200

accatagtcc cgcccctaac tccgcccatc ccgcccctaa ctccgcccag ttccgcccat 1260

tctccgcccc atggctgact aatttttttt atttatgcag aggccgaggc cgcctcggcc 1320

tctgagctat tccagaagta gtgaggaggc ttttttggag gcctaggctt ttgcaaaaag 1380

ctcccggggc ggccgccacc atgggcagca agcccagcac aagaatccca gcccccctga 1440

tgctgatcac ccgcatcatg ctgatcctgg gctgcatcag acccacaagc tccctggatg 1500

gacgccccct ggccgctgcc ggcatcgtgg tgaccggcga caaggccgtg aacgtgtaca 1560

ccagcagcca gaccggcagc atcatcgtga agctgctgcc caacatgccc agagacaaag 1620

aggcctgcgc caaggccccc ctggaagcct acaacagaac cctgaccacc ctgctgaccc 1680

ccctgggcga cagcatcaga aagatccagg gctccgtgag cacaagcggc ggaggaaagc 1740

agggcagact gatcggcgcc gtgatcggca gcgtggccct gggagtggct acagctgccc 1800

agattaccgc tgcagccgcc ctgatccagg ccaaccagaa cgccgccaac atcctgagac 1860

tgaaagagag cattgccgcc accaacgagg ccgtgcacga agtgaccgac ggcctgagcc 1920

agctgtccgt ggccgtgggc aagatgcagc agttcgtgaa cgaccagttc aacaacaccg 1980

ccagagagct ggactgcatc aagatcaccc agcaggtggg cgtggagctg aacctgtacc 2040

tgaccgagct gaccacagtg ttcggccccc agatcacaag cccagccctg acacagctga 2100

ccatccaggc cctgtacaac ctggctggcg gcaacatgga ctatctgctg acaaagctgg 2160

gaatcggcaa caaccagctg tccagcctga tcggaagcgg cctgatcacc ggctacccca 2220

tcctgtacga cagccagaca cagctgctgg gcatccaggt gaacctgccc agcgtgggca 2280

acctgaacaa catgcgcgcc acctacctgg aaaccctgag cgtgtccacc accaagggct 2340

acgccagcgc cctggtgccc aaggtggtga cacaggtggg cagcgtgatc gaggaactgg 2400

acaccagcta ctgcatcgag agcgacctgg acctgtactg caccagaatc gtgaccttcc 2460

caatgagccc cggcatctac agctgcctga gcggcaacac cagcgcctgc atgtacagca 2520

agaccgaagg cgcactgaca acaccctaca tggccctgaa gggaagcgtg atcgccaact 2580

gcaagatcac cacctgcaga tgcaccgacc ccccaggcat catcagccag aactacggcg 2640

aggccgtgag cctgatcgat cgccattcct gtaacgtgct gtccctggac ggcatcacac 2700

tgagactgag cggcgagttc gatgccacct accagaagaa catcagcatc ctggacagcc 2760

aggtgatcgt gaccggcaac ctggacatca gcaccgagct gggcaacgtg aataacagca 2820

tcagcaacgc cctggacaga ctggccgaga gcaacagcaa gctggaaaaa gtgaacgtgc 2880

gcctgacatc cacttccgct ctgatcacct acatcgtgct gaccgtgatc agcctggtgt 2940

tcggcgccct gagcctggtg ctggcctgct acctgatgta caagcagaag gcccagcaga 3000

aaaccctgct gtggctgggc aacaacaccc tggaccagat gagagccacc accagagcct 3060

gatgagcggc cgcgatatca ataaaatatc tttattttca ttacatctgt gtgttggttt 3120

tttgtgtgaa tcgatagtac taacatacgc tctccatcaa aacaaaacga aacaaaacaa 3180

actagcaaaa taggctgtcc ccagtgcaag tgcaggtgcc agaacatttc tcttctagac 3240

ctgcaggtta tgtactctta ttgatttata aaaacataca tgcagtgttg ctatgtcaca 3300

taattagcct cgcccgtcta cgctccactg aagataatgg gctcccgctg ttcaaaaaaa 3360

tcagcgtgcg tcgataagac tttggtgcag tctcttcggg gtcgcaattt agatttgccg 3420

catggagggt atctggggat ttttgccaat gctggagcga cgactgtacg attcgtccca 3480

tcgggatcta gcagaccaat gatgttgaca cacatcggcc atgcatgtac ggacggtcta 3540

ttgcgcgagt ttgttatttt cgaaggacaa gatggaagtg tatatggaac cgacaataat 3600

gttagtttgc atttcttagg gcggaatcta catgatatct tatccaagcg gggtatgagc 3660

cagagagatg tgatggtcat aaagggtaaa ttttttagat ctgaaataac gcagttgccc 3720

aaacaacgat cgcgattaaa agaaaaatcg gatggttcaa ttaggacatg catggattct 3780

gtgcgcataa accataaccg cagcactgtt gggcacttcg gtaactcaaa tgcgaagcgt 3840

tgcacgtctg cgataactac gcctactatg cacattgtta ctcctgcatc ttaaaaatat 3900

atcctgtagt aattttcaca gcaatgtcat aacatcatct cgctaa 3946

<210> 28
<211> 4654
<212> DNA
<213> artificial sequence

<220>
<223> Partial plasmid pHVT US2 SV-FCA02 opt-synPA for vHVT307

<400> 28
tatctccaca tcgtattcag gcccacggaa gtcttcgtta tcgaagctat tgttactagt 60

atctggcgac atcgacggtt ctgcaaccgt cgtaccgctt tcgatatttt cacagacaat 120

acccatattc gaggcactta ctttcgaaga ctcaacatct acttccatcg ccgccacgta 180

tgtaatttcg ggacgttgga tgatataaaa tatatagtac gcgtccgggt atacacctgt 240

gcgaaagtag tacgagaccg gcagtcaaaa agacgtttcc gatcttccac agctccagtt 300

attcggaagg cgtgggcatg ggtgtgtgca tgataactct cgtcacttta ctagacgaat 360

gcgatcgatt gcctgggcga tctcgtgacg ctgcctccac cctatggatc tttctcatag 420

aacaatgcat ggagcatctg aagaatgatg tgggtgtgcc catattagtc cgtacagcag 480

acctgtgtcg gtttgccaaa tccacattcg ttttgccccg ccggcatcgc ccaattgtga 540

gaattaaatc ggcaggcggg agtggtatgc ccggttccgg attggcagga acgagagatg 600

catttattgt gagactgttt gaagatgtag ccggttgcgc taccgaatgg caggatctac 660

tgaccggcta tgtaatgcta gaatcggagg cctccgataa cgtatcgtat agcctgtgga 720

tactcggggc tgcagatatc tgcagaactg caatagaatc aatacctcta ccaaaacgtt 780

tgtttgccat taaagtacca ggcacctggg ctggaatgcc ttgggccatt ccatgtgaga 840

tacaaacact tctcacgtcc acttgggaac ctaaatttga aaacatagaa gataaggcgt 900

acttcaacga cagtaatatg gcgtgcgtat atcaaattat cggctctcca ccagacgttc 960

cccagctgca ggggcttggt atcgagtcca catgcacccc acctaaacgc aatttgtgtt 1020

gctgcctatg ttgccgtcca atacatgacg acgacgcctc cgttcccatg ggcgttaaga 1080

cagtagataa aaacgtacat gatggcaata tgcttgtaga ggctcccaag tgtatcacag 1140

ataggggaaa attcaatagt agataaaccc aactagtgtg aggaaattgc atctacttgc 1200

ccccgggcct gcaggtcgac ccaattcgag ctcggtacag cttggctgtg gaatgtgtgt 1260

cagttagggt gtggaaagtc cccaggctcc ccagcaggca gaagtatgca aagcatgcat 1320

ctcaattagt cagcaaccag gtgtggaaag tccccaggct ccccagcagg cagaagtatg 1380

caaagcatgc atctcaatta gtcagcaacc atagtcccgc ccctaactcc gcccatcccg 1440

cccctaactc cgcccagttc cgcccattct ccgccccatg gctgactaat tttttttatt 1500

tatgcagagg ccgaggccgc ctcggcctct gagctattcc agaagtagtg aggaggcttt 1560

tttggaggcc taggcttttg caaaaagctc ccggggcggc cgccaccatg ggcagcaagc 1620

ccagcacctg gatcagcgtg accctgatgc tgatcaccag aaccatgctg atcctgagct 1680

gcatctgccc cacaagcagc ctggacggca gacccctggc cgctgccggc atcgtggtga 1740

ccggcgacaa ggccgtgaac atctacacca gcagccagac cggcagcatc atcatcaagc 1800

tgctgcccaa catgcccaag gacaaagagg cctgcgccaa ggcccccctg gaagcctaca 1860

acagaaccct gaccaccctg ctgacccccc tgggcgacag catcagaaga atccagggca 1920

gcgccaccac aagcggcgga ggaaagcagg gcagactggt gggcgctatc atcgggagcg 1980

tggccctggg cgtggccaca gctgcccaga ttaccgctgc agccgccctg attcaggcca 2040

atcagaacgc cgccaacatc ctgagactga aagagagcat tgccgccacc aacgacgccg 2100

tgcacgaagt gacaaacgga ctgtcccagc tggctgtcgc tgtcggcaag atgcagcagt 2160

tcgtgaacaa ccagttcaac aacaccgcca gagagctgga ctgcatcaag atcgcccagc 2220

aggtgggcgt ggagctgaac ctgtacctga ccgagctgac cacagtgttc ggcccccaga 2280

tcacaagccc cgctctgacc cagctgacaa tccaggccct gtacaacctg gctggcggca 2340

acatggacta tctgctgact aagctgggag tgggcaacaa ccagctgtcc agcctgatcg 2400

ggtccgggct gatcacaggc aaccccatcc tgtacgacag ccagacacag ctgctgggca 2460

tccagatcaa cctgccatcc gtgggaagcc tgaacaacat gagagccacc tacctggaaa 2520

ccctgagcgt gtccaccacc aagggcttcg ccagcgccct ggtgcccaag gtggtgacac 2580

aggtgggcag cgtgatcgag gaactggaca ccagctactg catcgagagc gacatcgacc 2640

tgtactgcac cagagtggtg accttcccaa tgagccccgg catctacagc tgcctgagcg 2700

gcaacaccag cgcctgcatg tacagcaaga ccgaaggagc actgacaaca ccctacatgg 2760

ccctgaaggg aagcgtgatc gccaactgca agatgaccac ctgcagatgc gccgaccccc 2820

caggcatcat cagccagaac tacggcgagg ccgtgagcct gatcgacaaa cattcctgta 2880

gcgtgctgtc cctggatggc atcacactga gactgagcgg cgagttcgac gccacctacc 2940

agaagaacat cagcatcctg gacagccagg tgatcgtgac cggcaacctg gacatcagca 3000

ccgagctggg caacgtgaac aacagcatca gcagcaccct ggacaagctg gccgagtcca 3060

acaacaagct gaacaaagtg aacgtgaacc tgaccagcac aagcgccctg atcacctaca 3120

tcgtgctggc catcgtgtcc ctggccttcg gcgtgatcag cctggtgctg gcctgctacc 3180

tgatgtacaa gcagagagcc cagcagaaaa ccctgctgtg gctgggcaat aacaccctgg 3240

accagatgag ggccaccacc agaacctgat gagcggccgc gatatcaata aaatatcttt 3300

attttcatta catctgtgtg ttggtttttt gtgtgaatcg atagtactaa catacgctct 3360

ccatcaaaac aaaacgaaac aaaacaaact agcaaaatag gctgtcccca gtgcaagtgc 3420

aggtgccaga acatttctct tctagacctg caggcatatg ttgtcccatt atgttgtaca 3480

taatcgtgat gtagtgctac gcaaatgtca attgatagcc catacatggt gctaatatgc 3540

gttactttta gtatgtgagt gaataaaaaa atattacccc tatgatacct gcgtctttat 3600

gctacaagtt ccttcaccat gaccagagcc ggtgcgatca tattcgacaa cttggatatt 3660

cccagaggta gatttggcca gccgcgggga aaaataaatg acttcaacta ctggaccttg 3720

ctaaccgatg agctgacgtg tggaattatt caatgtatgg agtcgcgcga gcgaattgcc 3780

ctagtgcatt cagcaacata cgatcatggt cagtttgaca tacaaaaaga tatgtggtgt 3840

caaatagttc tatggtctgc ctataggttt ctttccacac tagaacgtag tttttcaatt 3900

aaaagtgtct tagagtttgg agatacaaac gtgaacggaa gtgccaattt tgcaatcaat 3960

tgcacgccat gggatttgag agattcgccg aaaatgaaaa tgtttggaac actcttgccc 4020

gccttgtttt ctttccattt agaaaattgg acgacgatgc tctcaatcgg cgctagtaaa 4080

ggatattctc agtgtaattt gcgacagatt tttatgagaa atccatcctt taaaaacgtt 4140

attatagcct ccctggaggt tgcgagatct gctattgtcc ttaccatccc aatatgcgaa 4200

tatagaactc ccccagggct gccggacgat tacatcggaa atgccataaa gttgtgctgt 4260

gccagaatgc aacatctgag acttgaacat cctggccaat gtatagatca aatgtgcagc 4320

gatccttcag aagaagaaat gtattacaga tatgtccaac gactcgtaac gagcggcaat 4380

aaatattcag aatcatcgga aaatttccga atgagtgtgg acccacgcgt tgttggtccc 4440

cgattgcgcg actgtcaata cgaatccata cgggcaaggt atccgagcgg taccagggcg 4500

ggatatggga caaccggaag atatccaaat aatggacgtt tcaaattttc acgattccaa 4560

gtaaggcact atccacagta cgttccaaga tcgaagctgg caaacagtaa aattatccag 4620

actcttaacg aatgtaatga tagatcccat ttta 4654

<210> 29
<211> 5381

<212>  DNA
<213>  artificial sequence

<220>
<223>  partial plasmid pCD046+NDV-F VII YZCQ sequence for HVT112

<400>  29
gagctcaggg tatgatactc agctgttatt gtggccgacc aggaggactc caatgcttag      60

cattcataag aacgctagag atgctattta acgatgtgct gtcgtctaaa gaatttgtgc     120

atttagcctt taaatgtaaa accaatgacg cattcactac gctcgtgcgt gcaatttctg     180

ggccagggta tgcatattcc ataacagaaa tcgacacttg agaagaggat ctgactgttt     240

gggataaagg tcgtttgggt ctgtcctagc gatataattt atatgacgat atacattaaa     300

catctgtgtg cagtacttag gtatttaatc atgtcgatga aatgttatgt gtaaatatcg     360

gacaatatag ataacgggca cgctgctatt gtaacgtgcg cccgcgcgct agtgctgact     420

aatagtgtgg atgatgtata cagtatatta caaacggaaa tgatacgtaa taaattatgt     480

actcttattg atttataaaa acatacatgc agtgttgcta tgtcacataa ttagcctcgc     540

ccgtctacgc tccactgaag ataatgggct cccgctgttc aaaaaaatca gcgtgcgtcg     600

ataagacttt ggtgcagtct cttcggggtc gcaatttaga tttgccgcat ggagggtatc     660

tggggatttt tgccaatgct ggagcgacga ctgtacgatt cgtcccatcg ggatctagca     720

gaccaatgat gttgacacac atcggccatg catgtacgga cggtctattg cgcgagtttg     780

ttattttcga aggacaagat ggaagtgtat atggaaccga caataatgtt agtttgcatt     840

tcttagggcg gaatctacat gatatcttat ccaagcgggg tatgagccag agagatgtga     900

tggtcataaa gggtaaattt tttagatctg aaataacgca gttgcccaaa caacgatcgc     960

gattaaaaga aaaatcggat ggttcaatta ggacatgcat ggattctgtg cgcataaacc    1020

ataaccgcag cactgttggg cacttcggta actcaaatgc gaagcgttgc acgtctgcga    1080

taactacgcc tactatgcac attgttactc ctgcatctta aaaatatatc ctgtagtaat    1140

tttcacagca atgtcataac atcatctcgc taaagaatga cctgggattg gagaagtaat    1200

gaatatttgc aaccaatgca ttgaataaac taacattaaa cgaattcaat agtggatccc    1260

ccaactccgc ccgttttatg actagaacca atagttttta atgccaaatg cactgaaatc    1320

ccctaatttg caaagccaaa cgccccctat gtgagtaata cggggacttt ttacccaatt    1380

tcccacgcgg aaagccccct aatacactca tatggcatat gaatcagcac ggtcatgcac    1440

tctaatggcg gcccataggg actttccaca tagggggcgt tcaccatttc ccagcatagg    1500

ggtggtgact caatggcctt tacccaagta cattgggtca atgggaggta agccaatggg    1560

tttttcccat tactggcaag cacactgagt caaatgggac tttccactgg gttttgccca    1620

agtacattgg gtcaatggga ggtgagccaa tgggaaaaac ccattgctgc caagtacact    1680

gactcaatag ggactttcca atgggttttt ccattgttgg caagcatata aggtcaatgt 1740

gggtgagtca atagggactt tccattgtat tctgcccagt acataaggtc aatagggggt 1800

gaatcaacag gaaagtccca ttggagccaa gtacactgcg tcaataggga ctttccattg 1860

ggttttgccc agtacataag gtcaataggg gatgagtcaa tgggaaaaac ccattggagc 1920

caagtacact gactcaatag ggactttcca ttgggttttg cccagtacat aaggtcaata 1980

gggggtgagt caacaggaaa gttccattgg agccaagtac attgagtcaa tagggacttt 2040

ccaatgggtt ttgcccagta cataaggtca atgggaggta agccaatggg tttttcccat 2100

tactggcacg tatactgagt cattagggac tttccaatgg gttttgccca gtacataagg 2160

tcaatagggg tgaatcaaca ggaaagtccc attggagcca agtacactga gtcaataggg 2220

actttccatt gggttttgcc cagtacaaaa ggtcaatagg gggtgagtca atgggttttt 2280

cccattattg gcacgtacat aaggtcaata ggggtgagtc attgggtttt tccagccaat 2340

ttaattaaaa cgccatgtac tttcccacca ttgacgtcaa tgggctattg aaactaatgc 2400

aacgtgacct ttaaacggta ctttcccata gctgattaat gggaaagtac cgttctcgag 2460

ccaatacacg tcaatgggaa gtgaaagggc agccaaaacg taacaccgcc ccggttttcc 2520

cctggaaatt ccatattggc acgcattcta ttggctgagc tgcgttctac gtgggtataa 2580

gaggcgcgac cagcgtcggt accgtcgcag tcttcggtct gaccaccgta gaacgcagag 2640

ctcctcgctg caggcggccg catgggctct aaaccttcta ccaggatccc agcacctctg 2700

atgctgatca cccggattat gctgatattg gactgtatcc gtccgacaag ctctcttgac 2760

ggcaggcctc ttgcagctgc aggaattgta gtaacaggag ataaggcagt caatgtatat 2820

acctcgtctc agacagggtc aatcatagtc aagttgctcc cgaatatgcc caaggataag 2880

gaggcgtgtg cgaaagaccc attagaggca tataacagaa cactgactac tttgctcact 2940

cctcttggcg aatccatccg caagatccaa gggtctgtgt ccacgtctgg aggaggcaag 3000

caaggccgcc tgataggtgc tgttattggt agtgtagctc ttggggttgc aacagcggca 3060

caaataacag cagctgcggc cctaatacaa gccaaccaga atgctgccaa catccttcgg 3120

cttaaggaga gcattgctgc aaccaatgaa gctgtgcatg aagtcaccga cggattatca 3180

caactatcag tggcagttgg gaagatgcag cagtttgtca atgaccagtt taataataca 3240

gcgcgagaat tggactgtat aaaaatcaca caacaggttg gtgtagaact caacctatac 3300

ctaactgaat tgactacagt attcgggcca cagatcacct cccctgcatt aactcagctg 3360

accatccagg cactttataa tttagctggt ggcaatatgg attacttatt aactaagtta 3420

ggtataggga acaatcaact cagctcatta attggcagcg gcctgatcac tggttaccct 3480

atattgtatg actcacagac tcaactcttg ggcatacaag tgaatttgcc ctcagtcggg 3540

aacttaaata atatgcgtgc cacctattta gagaccttat ctgtaagtac agccaaagga 3600

tatgcctcag cacttgttcc aaaagtagtg acacaagtcg gttctgtgat agaagagctt 3660

gacacctcat actgtataga gtccgatctg gatttatatt gtactagaat agtgacattc 3720

cccatgtccc caggtattta ttcctgttta agcggcaaca catcagcttg catgtattca 3780

aagactgaag gcgcactcac tacgccgtat atggccctta aaggctcagt tattgccaat 3840

tgtaagataa caacatgtag atgtacagac cctcctggta tcatatcgca aaattatgga 3900

gaagctgtat ccctgataga tagacattcg tgcaatgtct tatcattaga cgggataact 3960

ctgaggctca gtggagaatt tgatgcaact tatcaaaaga acatctcaat actagattct 4020

caagtcatcg tgacaggcaa tcttgatata tcaactgaac ttggaaacgt caacaattca 4080

atcagcaatg ccttggataa gttggcaaaa agcaacagca agctagaaaa agtcaatgtc 4140

agactaacca gcacatccgc tctcattacc tatattgttc tgactgtcat ttctctagtt 4200

ttcggtgcac taagtctggg tttaacatgt tacctgatgt acaaacaaaa ggcacaacaa 4260

aagaccttgc tatggcttgg gaataatacc ctcgatcaga tgagagccac tacaagagca 4320

tgagcggccg cggggatcca gacatgataa gatacattga tgagtttgga caaaccacaa 4380

ctagaatgca gtgaaaaaaa tgctttattt gtgaaatttg tgatgctatt gctttatttg 4440

taaccattat aagctgcaat aaacaagtta acaacaacaa ttgcattgat tttatgtttc 4500

aggttcaggg ggaggtgtgg gaggtttttt cggatcctct agagtcgaca attattttat 4560

ttaataacat atagcccaaa gacctctatg aacatttagt ttcccgtata ctcaacggcg 4620

cgtgtacaca cgcatctctt tgcatagcga tgaagtttgt tcggcagcag aaaatgcaga 4680

tatccaacaa tctggagaaa acttatcatc acagtggcag tggaaacata ccccctctat 4740

attcatggta taattatcgt ctacagcgtc caggatagtg gcgtgagaaa atggagatct 4800

gcagccctcc tttccatggc atgccgcttt attgttcatt aaacgcacaa tggtctcaac 4860

gccagatatg ggcatagatt ctgaagaacc cgttgacaat ccgaagaaga aggcgtgcag 4920

gtctttggaa gactcgcacg ttggtcttat aatgtatgat cgagatgtca ccctaatgcc 4980

acatggtaca ggcttatcgc ggtcatggcg atcggacttg taatttgcaa cgatgggcaa 5040

aggatcgacg acatgccaaa cattctgaac ccgtagagat gttaacgatg acgaggatga 5100

atatcccatg ctcgctgcca tagtatcaag tacaccgcga ataaggacgc gtccaacatc 5160

gttatatgca cacaatgggc tacacgtgac taacaccccc gaatattagt catatgtgag 5220

tttcagtctg gctcccatat agcctgtaga ctatttgtgg tttaagtgtg aacgaggcgc 5280

tgtgaacgag actcgggccg attgtaagaa caagcaaatg cactttccat ttaacaagaa 5340

gtgtagagag aatactcaac ctctttggat gtatcctcga g 5381

<210> 30

<211> 5381
<212> DNA
<213> artificial sequence

<220>
<223> partial plasmid pCD046+Texas NDV-F sequence for HVT113

<400> 30
gagctcaggg tatgatactc agctgttatt gtggccgacc aggaggactc caatgcttag 60

cattcataag aacgctagag atgctattta acgatgtgct gtcgtctaaa gaatttgtgc 120

atttagcctt taaatgtaaa accaatgacg cattcactac gctcgtgcgt gcaatttctg 180

ggccagggta tgcatattcc ataacagaaa tcgacacttg agaagaggat ctgactgttt 240

gggataaagg tcgtttgggt ctgtcctagc gatataattt atatgacgat atacattaaa 300

catctgtgtg cagtacttag gtatttaatc atgtcgatga aatgttatgt gtaaatatcg 360

gacaatatag ataacgggca cgctgctatt gtaacgtgcg cccgcgcgct agtgctgact 420

aatagtgtgg atgatgtata cagtatatta caaacggaaa tgatacgtaa taaattatgt 480

actcttattg atttataaaa acatacatgc agtgttgcta tgtcacataa ttagcctcgc 540

ccgtctacgc tccactgaag ataatgggct cccgctgttc aaaaaaatca gcgtgcgtcg 600

ataagacttt ggtgcagtct cttcggggtc gcaatttaga tttgccgcat ggagggtatc 660

tggggatttt tgccaatgct ggagcgacga ctgtacgatt cgtcccatcg ggatctagca 720

gaccaatgat gttgacacac atcggccatg catgtacgga cggtctattg cgcgagtttg 780

ttattttcga aggacaagat ggaagtgtat atggaaccga caataatgtt agtttgcatt 840

tcttagggcg gaatctacat gatatcttat ccaagcgggg tatgagccag agagatgtga 900

tggtcataaa gggtaaattt tttagatctg aaataacgca gttgcccaaa caacgatcgc 960

gattaaaaga aaaatcggat ggttcaatta ggacatgcat ggattctgtg cgcataaacc 1020

ataaccgcag cactgttggg cacttcggta actcaaatgc gaagcgttgc acgtctgcga 1080

taactacgcc tactatgcac attgttactc ctgcatctta aaaatatatc ctgtagtaat 1140

tttcacagca atgtcataac atcatctcgc taaagaatga cctgggattg gagaagtaat 1200

gaatatttgc aaccaatgca ttgaataaac taacattaaa cgaattcaat agtggatccc 1260

ccaactccgc ccgttttatg actagaacca atagttttta atgccaaatg cactgaaatc 1320

ccctaatttg caaagccaaa cgccccctat gtgagtaata cggggacttt ttacccaatt 1380

tcccacgcgg aaagccccct aatacactca tatggcatat gaatcagcac ggtcatgcac 1440

tctaatggcg gcccataggg actttccaca tagggggcgt tcaccatttc ccagcatagg 1500

ggtggtgact caatggcctt tacccaagta cattgggtca atgggaggta agccaatggg 1560

tttttcccat tactggcaag cacactgagt caaatgggac tttccactgg gttttgccca 1620

agtacattgg gtcaatggga ggtgagccaa tgggaaaaac ccattgctgc caagtacact 1680

gactcaatag ggactttcca atgggttttt ccattgttgg caagcatata aggtcaatgt 1740

gggtgagtca atagggactt tccattgtat tctgcccagt acataaggtc aatagggggt 1800

gaatcaacag gaaagtccca ttggagccaa gtacactgcg tcaataggga ctttccattg 1860

ggttttgccc agtacataag gtcaataggg gatgagtcaa tgggaaaaac ccattggagc 1920

caagtacact gactcaatag ggactttcca ttgggttttg cccagtacat aaggtcaata 1980

gggggtgagt caacaggaaa gttccattgg agccaagtac attgagtcaa tagggacttt 2040

ccaatgggtt ttgcccagta cataaggtca atgggaggta agccaatggg tttttcccat 2100

tactggcacg tatactgagt cattagggac tttccaatgg gttttgccca gtacataagg 2160

tcaatagggg tgaatcaaca ggaaagtccc attggagcca agtacactga gtcaataggg 2220

actttccatt gggttttgcc cagtacaaaa ggtcaatagg gggtgagtca atgggttttt 2280

cccattattg gcacgtacat aaggtcaata ggggtgagtc attgggtttt tccagccaat 2340

ttaattaaaa cgccatgtac tttcccacca ttgacgtcaa tgggctattg aaactaatgc 2400

aacgtgacct ttaaacggta ctttcccata gctgattaat gggaaagtac cgttctcgag 2460

ccaatacacg tcaatgggaa gtgaaagggc agccaaaacg taacaccgcc ccggttttcc 2520

cctggaaatt ccatattggc acgcattcta ttggctgagc tgcgttctac gtgggtataa 2580

gaggcgcgac cagcgtcggt accgtcgcag tcttcggtct gaccaccgta gaacgcagag 2640

ctcctcgctg caggcggccg catgggctcc agatcttcta ccaggatccc ggtacctcta 2700

atgctgatca tccgaaccgc gctgacactg agctgtatcc gtctgacaag ctctcttgat 2760

ggcaggcctc ttgcggctgc agggatcgtg gtaacaggag ataaagcagt caacatatac 2820

acctcatccc agacagggtc aatcatagtt aagttactcc cgaatatgcc caaggacaaa 2880

gaggtgtgtg caaaagcccc attggaggca tacaacagga cactgactac tttactcacc 2940

ccccttggtg attctatccg caggatacaa gagtctgtga ctacttccgg aggaggcaag 3000

caaggccgcc tgataggtgc cattatcggc agtgtagctc ttggggttgc gacagctgca 3060

cagataacag cagcttcggc cctgatacaa gccaaccaga atgctgccaa catcctccgg 3120

cttaaagaga gcattgctgc aaccaatgaa gctgtgcacg aggtcactga cggattatca 3180

caactagcag tggcagtagg gaagatgcaa cagtttgtca atgaccagtt caataataca 3240

gcgcaagaat tggactgtat aaaaattgca cagcaggtcg gtgtagaact caacttgtac 3300

ctaactgaat tgactacagt atttgggcca caaatcactt cccctgcctt aactcagctg 3360

actatccaag cgctttacaa tctagctggt ggtaatatgg attacttgct gactaagtta 3420

ggtgtaggga acaaccaact cagctcatta attggtagcg gcttgatcac cggcaaccct 3480

attctgtacg actcacagac tcagatcttg ggtatacagg taactttgcc ttcagttggg 3540

aacctgaata atatgcgtgc cacctacctg gagaccttat ctgtaagcac aaccaaggga 3600

tttgcctcag cacttgtccc aaaagtggtg acacaggtcg gttccgtgat agaagaactt 3660

gacacctcat actgtatagg gaccgacttg gatttatact gtacaagaat agtgacattc 3720

cctatgtctc ctggtattta ttcttgtctg agcggtaata catcggcttg catgtattca 3780

aagactgaag gcgcacttac tacgccatat atggctctca aaggctcagt tattgccaat 3840

tgcaagctga caacatgtag atgtgcagat cccccaggta tcatatcgca aaattatgga 3900

gaagctgtgt ccttaataga taggcactca tgcaacgtct tatccttaga cgggataact 3960

ctgaggctca gtggggaatt tgatgcaacc tatcaaaaga atatctctat actagattct 4020

caagttatag tgacaggcaa tcttgatata tcaactgagc ttgggaatgt caacaactca 4080

ataagtaatg ccctgaataa gttagaggaa agcaacagca aactagacaa agtcaatgtc 4140

aaactgacca gcacatctgc tctcattacc tacatcgttt taactgtcat atctcttgtt 4200

tttggtgtac ttagcctggt tctagcatgc tacctgatgt acaagcaaaa ggcacaacaa 4260

aagaccttgt tatggcttgg gaataatacc cttgatcaga tgagagccac tacaaaaata 4320

tgagcggccg cggggatcca gacatgataa gatacattga tgagtttgga caaaccacaa 4380

ctagaatgca gtgaaaaaaa tgctttattt gtgaaatttg tgatgctatt gctttatttg 4440

taaccattat aagctgcaat aaacaagtta acaacaacaa ttgcattgat tttatgtttc 4500

aggttcaggg ggaggtgtgg gaggtttttt cggatcctct agagtcgaca attattttat 4560

ttaataacat atagcccaaa gacctctatg aacatttagt ttcccgtata ctcaacggcg 4620

cgtgtacaca cgcatctctt tgcatagcga tgaagtttgt tcggcagcag aaaatgcaga 4680

tatccaacaa tctggagaaa acttatcatc acagtggcag tggaaacata ccccctctat 4740

attcatggta taattatcgt ctacagcgtc caggatagtg gcgtgagaaa atggagatct 4800

gcagccctcc tttccatggc atgccgcttt attgttcatt aaacgcacaa tggtctcaac 4860

gccagatatg ggcatagatt ctgaagaacc cgttgacaat ccgaagaaga aggcgtgcag 4920

gtctttggaa gactcgcacg ttggtcttat aatgtatgat cgagatgtca ccctaatgcc 4980

acatggtaca ggcttatcgc ggtcatggcg atcggacttg taatttgcaa cgatgggcaa 5040

aggatcgacg acatgccaaa cattctgaac ccgtagagat gttaacgatg acgaggatga 5100

atatcccatg ctcgctgcca tagtatcaag tacaccgcga ataaggacgc gtccaacatc 5160

gttatatgca cacaatgggc tacacgtgac taacaccccc gaatattagt catatgtgag 5220

tttcagtctg gctcccatat agcctgtaga ctatttgtgg tttaagtgtg aacgaggcgc 5280

tgtgaacgag actcgggccg attgtaagaa caagcaaatg cactttccat ttaacaagaa 5340

gtgtagagag aatactcaac ctctttggat gtatcctcga g 5381

<210> 31
<211> 4600
<212> DNA
<213> artificial sequence

<220>
<223> partial plasmid pHM119 sequence for vHVT039

<400> 31
gagctcaggg tatgatactc agctgttatt gtggccgacc aggaggactc caatgcttag 60

cattcataag aacgctagag atgctattta acgatgtgct gtcgtctaaa gaatttgtgc 120

atttagcctt taaatgtaaa accaatgacg cattcactac gctcgtgcgt gcaatttctg 180

ggccagggta tgcatattcc ataacagaaa tcgacacttg agaagaggat ctgactgttt 240

gggataaagg tcgtttgggt ctgtcctagc gatataattt atatgacgat atacattaaa 300

catctgtgtg cagtacttag gtatttaatc atgtcgatga aatgttatgt gtaaatatcg 360

gacaatatag ataacgggca cgctgctatt gtaacgtgcg cccgcgcgct agtgctgact 420

aatagtgtgg atgatgtata cagtatatta caaacggaaa tgatacgtaa taaattatgt 480

actcttattg atttataaaa acatacatgc agtgttgcta tgtcacataa ttagcctcgc 540

ccgtctacgc tccactgaag ataatgggct cccgctgttc aaaaaaatca gcgtgcgtcg 600

ataagacttt ggtgcagtct cttcggggtc gcaatttaga tttgccgcat ggagggtatc 660

tggggatttt tgccaatgct ggagcgacga ctgtacgatt cgtcccatcg ggatctagca 720

gaccaatgat gttgacacac atcggccatg catgtacgga cggtctattg cgcgagtttg 780

ttattttcga aggacaagat ggaagtgtat atggaaccga caataatgtt agtttgcatt 840

tcttagggcg gaatctacat gatatcttat ccaagcgggg tatgagccag agagatgtga 900

tggtcataaa gggtaaattt tttagatctg aaataacgca gttgcccaaa caacgatcgc 960

gattaaaaga aaaatcggat ggttcaatta ggacatgcat ggattctgtg cgcataaacc 1020

ataaccgcag cactgttggg cacttcggta actcaaatgc gaagcgttgc acgtctgcga 1080

taactacgcc tactatgcac attgttactc ctgcatctta aaaatatatc ctgtagtaat 1140

tttcacagca atgtcataac atcatctcgc taaagaatga cctgggattg gagaagtaat 1200

gaatatttgc aaccaatgca ttgaataaac taacattaaa cgaattccga tgtttagtca 1260

cgatagacat cggttcgccc agccgtcgaa tacagcatta tattttagtg ttgaaaatgt 1320

agggctgctt cctcacttaa aggaggaaat ggctcgattc atgtttcata gcagtagaaa 1380

aacagattgg accgtcagta agtttagagg gttttatgac tttagcacta tagataatgt 1440

aactgcggcc catcgcatgg cttggaaata tatcaaagaa ctgatttttg caacagcttt 1500

attttcttct gtatttaaat gtggcgaatt gcacatctgt cgtgccgaca gtttgcagat 1560

caacagcaat ggagactatg tatggaaaaa tggaatatat ataacatatg aaaccgaata 1620

tccacttata atgattctgg ggtcagaatc aagcacttca gaaacgcaaa atatgactgc 1680

aattattgat acagatgttt tttcgttgct ttattctatt ttgcagtata tggcccccgt 1740

tacggcagat caggtgcgag tagaacagat taccaacagc cacgccccca tctgacccgt 1800

ccaatattct tgtgtccctg cattttatct cacacaattt atgaacagca tcattaagat 1860

catctcactg cggccgcaag atgggctcca gatcttctac caggatcccg gtacctctaa 1920

tgctgatcat ccgaaccgcg ctgacactga gctgtatccg tctgacaagc tctcttgatg 1980

gcaggcctct tgcggctgca gggatcgtgg taacaggaga taaagcagtc aacatataca 2040

cctcatccca gacagggtca atcatagtta agttactccc gaatatgccc aaggacaaag 2100

aggtgtgtgc aaaagcccca ttggaggcat acaacaggac actgactact ttactcaccc 2160

cccttggtga ttctatccgc aggatacaag agtctgtgac tacttccgga ggaaggagac 2220

agagacgctt tataggtgcc attatcggca gtgtagctct tggggttgcg acagctgcac 2280

agataacagc agcttcggcc ctgatacaag ccaaccagaa tgctgccaac atcctccggc 2340

ttaaagagag cattgctgca accaatgaag ctgtgcacga ggtcactgac ggattatcac 2400

aactagcagt ggcagtaggg aagatgcaac agtttgtcaa tgaccagttc aataatacag 2460

cgcaagaatt ggactgtata aaaattgcac agcaggtcgg tgtagaactc aacttgtacc 2520

taactgaatt gactacagta tttgggccac aaatcacttc ccctgcctta actcagctga 2580

ctatccaagc gctttacaat ctagctggtg gtaatatgga ttacttgctg actaagttag 2640

gtgtagggaa caaccaactc agctcattaa ttggtagcgg cttgatcacc ggcaacccta 2700

ttctgtacga ctcacagact cagatcttgg gtatacaggt aactttgcct tcagttggga 2760

acctgaataa tatgcgtgcc acctacctgg agaccttatc tgtaagcaca accaagggat 2820

ttgcctcagc acttgtccca aaagtggtga cacaggtcgg ttccgtgata gaagaacttg 2880

acacctcata ctgtataggg accgacttgg atttatactg tacaagaata gtgacattcc 2940

ctatgtctcc tggtatttat tcttgtctga gcggtaatac atcggcttgc atgtattcaa 3000

agactgaagg cgcacttact acgccatata tggctctcaa aggctcagtt attgccaatt 3060

gcaagctgac aacatgtaga tgtgcagatc ccccaggtat catatcgcaa aattatggag 3120

aagctgtgtc cttaatagat aggcactcat gcaacgtctt atccttagac gggataactc 3180

tgaggctcag tggggaattt gatgcaacct atcaaaagaa tatctctata ctagattctc 3240

aagttatagt gacaggcaat cttgatatat caactgagct tgggaatgtc aacaactcaa 3300

taagtaatgc cctgaataag ttagaggaaa gcaacagcaa actagacaaa gtcaatgtca 3360

aactgaccag cacatctgct ctcattacct acatcgtttt aactgtcata tctcttgttt 3420

ttggtgtact tagcctggtt ctagcatgct acctgatgta caagcaaaag gcacaacaaa 3480

agaccttgtt atggcttggg aataataccc ttgatcagat gagagccact acaaaaatat 3540

gagcggccgc ggggatccag acatgataag atacattgat gagtttggac aaaccacaac 3600

tagaatgcag tgaaaaaaat gctttatttg tgaaatttgt gatgctattg ctttatttgt 3660

aaccattata agctgcaata aacaagttaa caacaacaat tgcattcatt ttatgtttca 3720

ggttcagggg gaggtgtggg aggttttttc ggatcctcta gagtcgacaa ttattttatt 3780

taataacata tagcccaaag acctctatga acatttagtt tcccgtatac tcaacggcgc 3840

gtgtacacac gcatctcttt gcatagcgat gaagtttgtt cggcagcaga aaatgcagat 3900

atccaacaat ctggagaaaa cttatcatca cagtggcagt ggaaacatac cccctctata 3960

ttcatggtat aattatcgtc tacagcgtcc aggatagtgg cgtgagaaaa tggagatctg 4020

cagccctcct ttccatggca tgccgcttta ttgttcatta aacgcacaat ggtctcaacg 4080

ccagatatgg gcatagattc tgaagaaccc gttgacaatc cgaagaagaa ggcgtgcagg 4140

tctttggaag actcgcacgt tggtcttata atgtatgatc gagatgtcac cctaatgcca 4200

catggtacag gcttatcgcg gtcatggcga tcggacttgt aatttgcaac gatgggcaaa 4260

ggatcgacga catgccaaac attctgaacc cgtagagatg ttaacgatga cgaggatgaa 4320

tatcccatgc tcgctgccat agtatcaagt acaccgcgaa taaggacgcg tccaacatcg 4380

ttatatgcac acaatgggct acacgtgact aacacccccg aatattagtc atatgtgagt 4440

ttcagtctgg ctcccatata gcctgtagac tatttgtggt ttaagtgtga acgaggcgct 4500

gtgaacgaga ctcgggccga ttgtaagaac aagcaaatgc actttccatt taacaagaag 4560

tgtagagaga atactcaacc tctttggatg tatcctcgag 4600

<210> 32
<211> 1662
<212> DNA
<213> artificial sequence

<220>
<223> NDV Texas F gene (wild type non-modified)

<400> 32
atgggctcca gatcttctac caggatcccg gtacctctaa tgctgatcat ccgaaccgcg 60

ctgacactga gctgtatccg tctgacaagc tctcttgatg gcaggcctct tgcggctgca 120

gggatcgtgg taacaggaga taaagcagtc aacatataca cctcatccca gacagggtca 180

atcatagtta agttactccc gaatatgccc aaggacaaag aggtgtgtgc aaaagcccca 240

ttggaggcat acaacaggac actgactact ttactcaccc cccttggtga ttctatccgc 300

aggatacaag agtctgtgac tacttccgga ggaaggagac agagacgctt tataggtgcc 360

attatcggca gtgtagctct tggggttgcg acagctgcac agataacagc agcttcggcc 420

ctgatacaag ccaaccagaa tgctgccaac atcctccggc ttaaagagag cattgctgca 480

accaatgaag ctgtgcacga ggtcactgac ggattatcac aactagcagt ggcagtaggg 540

aagatgcaac agtttgtcaa tgaccagttc aataatacag cgcaagaatt ggactgtata 600

aaaattgcac agcaggtcgg tgtagaactc aacttgtacc taactgaatt gactacagta 660

tttgggccac aaatcacttc ccctgcctta actcagctga ctatccaagc gctttacaat 720

ctagctggtg gtaatatgga ttacttgctg actaagttag gtgtagggaa caaccaactc 780

agctcattaa ttggtagcgg cttgatcacc ggcaacccta ttctgtacga ctcacagact 840

cagatcttgg gtatacaggt aactttgcct tcagttggga acctgaataa tatgcgtgcc 900

acctacctgg agaccttatc tgtaagcaca accaagggat ttgcctcagc acttgtccca 960

aaagtggtga cacaggtcgg ttccgtgata gaagaacttg acacctcata ctgtataggg 1020

accgacttgg atttatactg tacaagaata gtgacattcc ctatgtctcc tggtatttat 1080

tcttgtctga gcggtaatac atcggcttgc atgtattcaa agactgaagg cgcacttact 1140

acgccatata tggctctcaa aggctcagtt attgccaatt gcaagctgac aacatgtaga 1200

tgtgcagatc ccccaggtat catatcgcaa aattatggag aagctgtgtc cttaatagat 1260

aggcactcat gcaacgtctt atccttagac gggataactc tgaggctcag tggggaattt 1320

gatgcaacct atcaaaagaa tatctctata ctagattctc aagttatagt gacaggcaat 1380

cttgatatat caactgagct tgggaatgtc aacaactcaa taagtaatgc cctgaataag 1440

ttagaggaaa gcaacagcaa actagacaaa gtcaatgtca aactgaccag cacatctgct 1500

ctcattacct acatcgtttt aactgtcata tctcttgttt ttggtgtact tagcctggtt 1560

ctagcatgct acctgatgta caagcaaaag gcacaacaaa agaccttgtt atggcttggg 1620

aataataccc ttgatcagat gagagccact acaaaaatat ga 1662

<210> 33
<211> 553
<212> PRT
<213> artificial sequence

<220>
<223> NDV Texas F protein (wild type non-modified)

<400> 33

Met Gly Ser Arg Ser Ser Thr Arg Ile Pro Val Pro Leu Met Leu Ile
1               5                   10                  15

Ile Arg Thr Ala Leu Thr Leu Ser Cys Ile Arg Leu Thr Ser Ser Leu
            20                  25                  30

Asp Gly Arg Pro Leu Ala Ala Ala Gly Ile Val Val Thr Gly Asp Lys
        35                  40                  45

Ala Val Asn Ile Tyr Thr Ser Ser Gln Thr Gly Ser Ile Ile Val Lys
    50                  55                  60

Leu Leu Pro Asn Met Pro Lys Asp Lys Glu Val Cys Ala Lys Ala Pro
65                  70                  75                  80

Leu Glu Ala Tyr Asn Arg Thr Leu Thr Thr Leu Leu Thr Pro Leu Gly
                85                  90                  95

Asp Ser Ile Arg Arg Ile Gln Glu Ser Val Thr Thr Ser Gly Gly Arg
            100                 105                 110

Arg Gln Arg Arg Phe Ile Gly Ala Ile Ile Gly Ser Val Ala Leu Gly
        115                 120                 125

Val Ala Thr Ala Ala Gln Ile Thr Ala Ala Ser Ala Leu Ile Gln Ala
    130                 135                 140

Asn Gln Asn Ala Ala Asn Ile Leu Arg Leu Lys Glu Ser Ile Ala Ala
145                 150                 155                 160

Thr Asn Glu Ala Val His Glu Val Thr Asp Gly Leu Ser Gln Leu Ala
                165                 170                 175

Val Ala Val Gly Lys Met Gln Gln Phe Val Asn Asp Gln Phe Asn Asn
            180                 185                 190

Thr Ala Gln Glu Leu Asp Cys Ile Lys Ile Ala Gln Gln Val Gly Val
        195                 200                 205

Glu Leu Asn Leu Tyr Leu Thr Glu Leu Thr Thr Val Phe Gly Pro Gln
    210                 215                 220

Ile Thr Ser Pro Ala Leu Thr Gln Leu Thr Ile Gln Ala Leu Tyr Asn
225                 230                 235                 240

Leu Ala Gly Gly Asn Met Asp Tyr Leu Leu Thr Lys Leu Gly Val Gly
                245                 250                 255

Asn Asn Gln Leu Ser Ser Leu Ile Gly Ser Gly Leu Ile Thr Gly Asn
            260                 265                 270

Pro Ile Leu Tyr Asp Ser Gln Thr Gln Ile Leu Gly Ile Gln Val Thr
        275                 280                 285

Leu Pro Ser Val Gly Asn Leu Asn Asn Met Arg Ala Thr Tyr Leu Glu
    290                 295                 300

Thr Leu Ser Val Ser Thr Thr Lys Gly Phe Ala Ser Ala Leu Val Pro
305                 310                 315                 320

Lys Val Val Thr Gln Val Gly Ser Val Ile Glu Glu Leu Asp Thr Ser
                325                 330                 335

Tyr Cys Ile Gly Thr Asp Leu Asp Leu Tyr Cys Thr Arg Ile Val Thr
            340                 345                 350

Phe Pro Met Ser Pro Gly Ile Tyr Ser Cys Leu Ser Gly Asn Thr Ser
        355                 360                 365

Ala Cys Met Tyr Ser Lys Thr Glu Gly Ala Leu Thr Thr Pro Tyr Met
    370                 375                 380

Ala Leu Lys Gly Ser Val Ile Ala Asn Cys Lys Leu Thr Thr Cys Arg
385                 390                 395                 400

Cys Ala Asp Pro Pro Gly Ile Ile Ser Gln Asn Tyr Gly Glu Ala Val
                405                 410                 415

Ser Leu Ile Asp Arg His Ser Cys Asn Val Leu Ser Leu Asp Gly Ile
            420                 425                 430

Thr Leu Arg Leu Ser Gly Glu Phe Asp Ala Thr Tyr Gln Lys Asn Ile
        435                 440                 445

Ser Ile Leu Asp Ser Gln Val Ile Val Thr Gly Asn Leu Asp Ile Ser
    450                 455                 460

Thr Glu Leu Gly Asn Val Asn Asn Ser Ile Ser Asn Ala Leu Asn Lys
465                 470                 475                 480

Leu Glu Glu Ser Asn Ser Lys Leu Asp Lys Val Asn Val Lys Leu Thr
                485                 490                 495

Ser Thr Ser Ala Leu Ile Thr Tyr Ile Val Leu Thr Val Ile Ser Leu
            500                 505                 510

Val Phe Gly Val Leu Ser Leu Val Leu Ala Cys Tyr Leu Met Tyr Lys
        515                 520                 525

Gln Lys Ala Gln Gln Lys Thr Leu Leu Trp Leu Gly Asn Asn Thr Leu
    530                 535                 540

Asp Gln Met Arg Ala Thr Thr Lys Ile
545                 550

<210> 34
<211> 1662
<212> DNA
<213> artificial sequence

<220>
<223> NDV-F YZCQ wildtype DNA sequence

<400> 34
atgggctcca gatcttctac caggatcccg gtacctctaa tgctgatcat ccgaaccgcg 60

ctgacactga gctgtatccg tctgacaagc tctcttgatg gcaggcctct tgcggctgca 120

gggatcgtgg taacaggaga taaagcagtc aacatataca cctcatccca gacagggtca 180

atcatagtta agttactccc gaatatgccc aaggacaaag aggtgtgtgc aaaagcccca 240

ttggaggcat acaacaggac actgactact ttactcaccc cccttggtga ttctatccgc 300

aggatacaag agtctgtgac tacttccgga ggaggcaagc aaggccgcct gataggtgcc 360

attatcggca gtgtagctct tggggttgcg acagctgcac agataacagc agcttcggcc 420

ctgatacaag ccaaccagaa tgctgccaac atcctccggc ttaaagagag cattgctgca 480

accaatgaag ctgtgcacga ggtcactgac ggattatcac aactagcagt ggcagtaggg 540

aagatgcaac agtttgtcaa tgaccagttc aataatacag cgcaagaatt ggactgtata 600

aaaattgcac agcaggtcgg tgtagaactc aacttgtacc taactgaatt gactacagta 660

tttgggccac aaatcacttc ccctgcctta actcagctga ctatccaagc gctttacaat 720

ctagctggtg gtaatatgga ttacttgctg actaagttag gtgtagggaa caaccaactc 780

agctcattaa ttggtagcgg cttgatcacc ggcaacccta ttctgtacga ctcacagact 840

cagatcttgg gtatacaggt aactttgcct tcagttggga acctgaataa tatgcgtgcc 900

acctacctgg agaccttatc tgtaagcaca accaagggat ttgcctcagc acttgtccca 960

aaagtggtga cacaggtcgg ttccgtgata gaagaacttg acacctcata ctgtataggg 1020

accgacttgg atttatactg tacaagaata gtgacattcc ctatgtctcc tggtatttat 1080

tcttgtctga gcggtaatac atcggcttgc atgtattcaa agactgaagg cgcacttact 1140

acgccatata tggctctcaa aggctcagtt attgccaatt gcaagctgac aacatgtaga 1200

tgtgcagatc ccccaggtat catatcgcaa aattatggag aagctgtgtc cttaatagat 1260

aggcactcat gcaacgtctt atccttagac gggataactc tgaggctcag tggggaattt 1320

gatgcaacct atcaaaagaa tatctctata ctagattctc aagttatagt gacaggcaat 1380

cttgatatat caactgagct tgggaatgtc aacaactcaa taagtaatgc cctgaataag 1440

ttagaggaaa gcaacagcaa actagacaaa gtcaatgtca aactgaccag cacatctgct 1500

ctcattacct acatcgtttt aactgtcata tctcttgttt ttggtgtact tagcctggtt 1560

ctagcatgct acctgatgta caagcaaaag gcacaacaaa agaccttgtt atggcttggg 1620

aataataccc ttgatcagat gagagccact acaaaaatat ga 1662

<210> 35
<211> 553
<212> PRT
<213> artificial sequence

<220>
<223> NDV-F protein from wildtype YZCQ strain (Amino Acid Sequence of
      NDV-F of Texas strain with lentogenic cleavage site sequence)

<400> 35

Met Gly Ser Arg Ser Ser Thr Arg Ile Pro Val Pro Leu Met Leu Ile
1               5                   10                  15

Ile Arg Thr Ala Leu Thr Leu Ser Cys Ile Arg Leu Thr Ser Ser Leu
            20                  25                  30

Asp Gly Arg Pro Leu Ala Ala Ala Gly Ile Val Val Thr Gly Asp Lys
        35                  40                  45

Ala Val Asn Ile Tyr Thr Ser Ser Gln Thr Gly Ser Ile Ile Val Lys
    50                  55                  60

Leu Leu Pro Asn Met Pro Lys Asp Lys Glu Val Cys Ala Lys Ala Pro
65                  70                  75                  80

Leu Glu Ala Tyr Asn Arg Thr Leu Thr Thr Leu Leu Thr Pro Leu Gly
                85                  90                  95

Asp Ser Ile Arg Arg Ile Gln Glu Ser Val Thr Thr Ser Gly Gly Gly
            100                 105                 110

Lys Gln Gly Arg Leu Ile Gly Ala Ile Ile Gly Ser Val Ala Leu Gly
        115                 120                 125

Val Ala Thr Ala Ala Gln Ile Thr Ala Ala Ser Ala Leu Ile Gln Ala
    130                 135                 140

Asn Gln Asn Ala Ala Asn Ile Leu Arg Leu Lys Glu Ser Ile Ala Ala
145                 150                 155                 160

Thr Asn Glu Ala Val His Glu Val Thr Asp Gly Leu Ser Gln Leu Ala
                165                 170                 175

Val Ala Val Gly Lys Met Gln Gln Phe Val Asn Asp Gln Phe Asn Asn
            180                 185                 190

Thr Ala Gln Glu Leu Asp Cys Ile Lys Ile Ala Gln Gln Val Gly Val
        195                 200                 205

Glu Leu Asn Leu Tyr Leu Thr Glu Leu Thr Thr Val Phe Gly Pro Gln
    210                 215                 220

Ile Thr Ser Pro Ala Leu Thr Gln Leu Thr Ile Gln Ala Leu Tyr Asn
225                 230                 235                 240

Leu Ala Gly Gly Asn Met Asp Tyr Leu Leu Thr Lys Leu Gly Val Gly
                245                 250                 255

Asn Asn Gln Leu Ser Ser Leu Ile Gly Ser Gly Leu Ile Thr Gly Asn
            260                 265                 270

Pro Ile Leu Tyr Asp Ser Gln Thr Gln Ile Leu Gly Ile Gln Val Thr
        275                 280                 285

Leu Pro Ser Val Gly Asn Leu Asn Asn Met Arg Ala Thr Tyr Leu Glu
    290                 295                 300

Thr Leu Ser Val Ser Thr Thr Lys Gly Phe Ala Ser Ala Leu Val Pro
305                 310                 315                 320

Lys Val Val Thr Gln Val Gly Ser Val Ile Glu Glu Leu Asp Thr Ser
                325                 330                 335

Tyr Cys Ile Gly Thr Asp Leu Asp Leu Tyr Cys Thr Arg Ile Val Thr
            340                 345                 350

Phe Pro Met Ser Pro Gly Ile Tyr Ser Cys Leu Ser Gly Asn Thr Ser
        355                 360                 365

Ala Cys Met Tyr Ser Lys Thr Glu Gly Ala Leu Thr Thr Pro Tyr Met
    370                 375                 380

Ala Leu Lys Gly Ser Val Ile Ala Asn Cys Lys Leu Thr Thr Cys Arg
385                 390                 395                 400

Cys Ala Asp Pro Pro Gly Ile Ile Ser Gln Asn Tyr Gly Glu Ala Val
                405                 410                 415

Ser Leu Ile Asp Arg His Ser Cys Asn Val Leu Ser Leu Asp Gly Ile
            420                 425                 430

Thr Leu Arg Leu Ser Gly Glu Phe Asp Ala Thr Tyr Gln Lys Asn Ile
        435                 440                 445

Ser Ile Leu Asp Ser Gln Val Ile Val Thr Gly Asn Leu Asp Ile Ser
    450                 455                 460

Thr Glu Leu Gly Asn Val Asn Asn Ser Ile Ser Asn Ala Leu Asn Lys
465                 470                 475                 480

Leu Glu Glu Ser Asn Ser Lys Leu Asp Lys Val Asn Val Lys Leu Thr
                485                 490                 495

Ser Thr Ser Ala Leu Ile Thr Tyr Ile Val Leu Thr Val Ile Ser Leu
            500                 505                 510

Val Phe Gly Val Leu Ser Leu Val Leu Ala Cys Tyr Leu Met Tyr Lys
        515                 520                 525

Gln Lys Ala Gln Gln Lys Thr Leu Leu Trp Leu Gly Asn Asn Thr Leu
    530                 535                 540

Asp Gln Met Arg Ala Thr Thr Lys Ile
545                 550

<210>  36
<211>  1662
<212>  DNA
<213>  artificial sequence

<220>
<223>  NDV-F Texas wildtype DNA sequence

<400>  36
atgggctcta aaccttctac caggatccca gcacctctga tgctgatcac ccggattatg      60

ctgatattgg actgtatccg tccgacaagc tctcttgacg gcaggcctct tgcagctgca     120

ggaattgtag taacaggaga taaggcagtc aatgtatata cctcgtctca gacagggtca     180

atcatagtca agttgctccc gaatatgccc aaggataagg aggcgtgtgc gaaagaccca     240

ttagaggcat ataacagaac actgactact ttgctcactc ctcttggcga atccatccgc     300

aagatccaag ggtctgtgtc cacgtctgga ggaggcaagc aaggccgcct gataggtgct     360

gttattggta gtgtagctct tggggttgca acagcggcac aaataacagc agctgcggcc     420

ctaatacaag ccaaccagaa tgctgccaac atccttcggc ttaaggagag cattgctgca     480

accaatgaag ctgtgcatga agtcaccgac ggattatcac aactatcagt ggcagttggg     540

aagatgcagc agtttgtcaa tgaccagttt aataatacag cgcgagaatt ggactgtata     600

aaaatcacac aacaggttgg tgtagaactc aacctatacc taactgaatt gactacagta     660

ttcgggccac agatcacctc ccctgcatta actcagctga ccatccaggc actttataat     720

ttagctggtg gcaatatgga ttacttatta actaagttag gtatagggaa caatcaactc 780

agctcattaa ttggcagcgg cctgatcact ggttacccta tattgtatga ctcacagact 840

caactcttgg gcatacaagt gaatttgccc tcagtcggga acttaaataa tatgcgtgcc 900

acctatttag agaccttatc tgtaagtaca gccaaaggat atgcctcagc acttgttcca 960

aaagtagtga cacaagtcgg ttctgtgata gaagagcttg acacctcata ctgtatagag 1020

tccgatctgg atttatattg tactagaata gtgacattcc ccatgtcccc aggtatttat 1080

tcctgtttaa gcggcaacac atcagcttgc atgtattcaa agactgaagg cgcactcact 1140

acgccgtata tggcccttaa aggctcagtt attgccaatt gtaagataac aacatgtaga 1200

tgtacagacc ctcctggtat catatcgcaa aattatggag aagctgtatc cctgatagat 1260

agacattcgt gcaatgtctt atcattagac gggataactc tgaggctcag tggagaattt 1320

gatgcaactt atcaaaagaa catctcaata ctagattctc aagtcatcgt gacaggcaat 1380

cttgatatat caactgaact tggaaacgtc aacaattcaa tcagcaatgc cttggataag 1440

ttggcaaaaa gcaacagcaa gctagaaaaa gtcaatgtca gactaaccag cacatccgct 1500

ctcattacct atattgttct gactgtcatt tctctagttt tcggtgcact aagtctgggt 1560

ttaacatgtt acctgatgta caaacaaaag gcacaacaaa agaccttgct atggcttggg 1620

aataataccc tcgatcagat gagagccact acaagagcat ga 1662

<210> 37
<211> 553
<212> PRT
<213> artificial sequence

<220>
<223> NDV-F protein from wildtype Texas strain (Amino Acid Sequence of
NDV-F VIId wt YZCQ with lentogenic cleavage site sequence)

<400> 37

Met Gly Ser Lys Pro Ser Thr Arg Ile Pro Ala Pro Leu Met Leu Ile
1               5                   10                  15

Thr Arg Ile Met Leu Ile Leu Asp Cys Ile Arg Pro Thr Ser Ser Leu
            20                  25                  30

Asp Gly Arg Pro Leu Ala Ala Ala Gly Ile Val Val Thr Gly Asp Lys
        35                  40                  45

Ala Val Asn Val Tyr Thr Ser Ser Gln Thr Gly Ser Ile Ile Val Lys
    50                  55                  60

Leu Leu Pro Asn Met Pro Lys Asp Lys Glu Ala Cys Ala Lys Asp Pro
65                  70                  75                  80

Leu Glu Ala Tyr Asn Arg Thr Leu Thr Thr Leu Leu Thr Pro Leu Gly
                85                  90                  95

Glu Ser Ile Arg Lys Ile Gln Gly Ser Val Ser Thr Ser Gly Gly Gly
            100                 105                 110

Lys Gln Gly Arg Leu Ile Gly Ala Val Ile Gly Ser Val Ala Leu Gly
        115                 120                 125

Val Ala Thr Ala Ala Gln Ile Thr Ala Ala Ala Ala Leu Ile Gln Ala
    130                 135                 140

Asn Gln Asn Ala Ala Asn Ile Leu Arg Leu Lys Glu Ser Ile Ala Ala
145                 150                 155                 160

Thr Asn Glu Ala Val His Glu Val Thr Asp Gly Leu Ser Gln Leu Ser
                165                 170                 175

Val Ala Val Gly Lys Met Gln Gln Phe Val Asn Asp Gln Phe Asn Asn
            180                 185                 190

Thr Ala Arg Glu Leu Asp Cys Ile Lys Ile Thr Gln Gln Val Gly Val
        195                 200                 205

Glu Leu Asn Leu Tyr Leu Thr Glu Leu Thr Thr Val Phe Gly Pro Gln
    210                 215                 220

Ile Thr Ser Pro Ala Leu Thr Gln Leu Thr Ile Gln Ala Leu Tyr Asn
225                 230                 235                 240

Leu Ala Gly Gly Asn Met Asp Tyr Leu Leu Thr Lys Leu Gly Ile Gly
                245                 250                 255

Asn Asn Gln Leu Ser Ser Leu Ile Gly Ser Gly Leu Ile Thr Gly Tyr
            260                 265                 270

Pro Ile Leu Tyr Asp Ser Gln Thr Gln Leu Leu Gly Ile Gln Val Asn
        275                 280                 285

Leu Pro Ser Val Gly Asn Leu Asn Asn Met Arg Ala Thr Tyr Leu Glu
    290                 295                 300

Thr Leu Ser Val Ser Thr Ala Lys Gly Tyr Ala Ser Ala Leu Val Pro
305                 310                 315                 320

Lys Val Val Thr Gln Val Gly Ser Val Ile Glu Glu Leu Asp Thr Ser
                325                 330                 335

Tyr Cys Ile Glu Ser Asp Leu Asp Leu Tyr Cys Thr Arg Ile Val Thr
            340                 345                 350

Phe Pro Met Ser Pro Gly Ile Tyr Ser Cys Leu Ser Gly Asn Thr Ser
        355                 360                 365

Ala Cys Met Tyr Ser Lys Thr Glu Gly Ala Leu Thr Thr Pro Tyr Met
    370                 375                 380

Ala Leu Lys Gly Ser Val Ile Ala Asn Cys Lys Ile Thr Thr Cys Arg
385                 390                 395                 400

Cys Thr Asp Pro Pro Gly Ile Ile Ser Gln Asn Tyr Gly Glu Ala Val
                405                 410                 415

Ser Leu Ile Asp Arg His Ser Cys Asn Val Leu Ser Leu Asp Gly Ile
            420                 425                 430

Thr Leu Arg Leu Ser Gly Glu Phe Asp Ala Thr Tyr Gln Lys Asn Ile
        435                 440                 445

Ser Ile Leu Asp Ser Gln Val Ile Val Thr Gly Asn Leu Asp Ile Ser
    450                 455                 460

Thr Glu Leu Gly Asn Val Asn Asn Ser Ile Ser Asn Ala Leu Asp Lys
465                 470                 475                 480

Leu Ala Lys Ser Asn Ser Lys Leu Glu Lys Val Asn Val Arg Leu Thr
                485                 490                 495

Ser Thr Ser Ala Leu Ile Thr Tyr Ile Val Leu Thr Val Ile Ser Leu
            500                 505                 510

Val Phe Gly Ala Leu Ser Leu Gly Leu Thr Cys Tyr Leu Met Tyr Lys
        515                 520                 525

Gln Lys Ala Gln Gln Lys Thr Leu Leu Trp Leu Gly Asn Asn Thr Leu
    530                 535                 540

Asp Gln Met Arg Ala Thr Thr Arg Ala
545                 550

<210>  38
<211>  622
<212>  DNA
<213>  artificial sequence

<220>
<223>  MDV gB promoter

<400>  38
cgatgtttag tcacgataga catcggttcg cccagccgtc gaatacagca ttatatttta      60

gtgttgaaaa tgtagggctg cttcctcact taaaggagga aatggctcga ttcatgtttc     120

atagcagtag aaaaacagat tggaccgtca gtaagtttag agggttttat gactttagca     180

ctatagataa tgtaactgcg gcccatcgca tggcttggaa atatatcaaa gaactgattt     240

ttgcaacagc tttattttct tctgtattta aatgtggcga attgcacatc tgtcgtgccg     300

acagtttgca gatcaacagc aatggagact atgtatggaa aaatggaata tatataacat     360

atgaaaccga atatccactt ataatgattc tggggtcaga atcaagcact tcagaaacgc     420

aaaatatgac tgcaattatt gatacagatg ttttttcgtt gctttattct attttgcagt     480

atatggcccc cgttacggca gatcaggtgc gagtagaaca gattaccaac agccacgccc     540

ccatctgacc cgtccaatat tcttgtgtcc ctgcatttta tctcacacaa tttatgaaca     600

gcatcattaa gatcatctca ct                                              622


<210>  39
<211>  4850
<212>  DNA
<213>  artificial sequence

<220>
<223>  Partial plasmid SORF3-US2 gpVar-Ewtsyn sequence for vHVT202

<400>  39
taaaatggga tctatcatta cattcgttaa gagtctggat aattttactg tttgccagct      60

tcgatcttgg aacgtactgt ggatagtgcc ttacttggaa tcgtgaaaat ttgaaacgtc     120

cattatttgg atatcttccg gttgtcccat atcccgccct ggtaccgctc ggataccttg     180

cccgtatgga ttcgtattga cagtcgcgca atcggggacc aacaacgcgt gggtccacac     240

tcattcggaa attttccgat gattctgaat atttattgcc gctcgttacg agtcgttgga     300

catatctgta atacatttct tcttctgaag gatcgctgca catttgatct atacattggc     360

caggatgttc aagtctcaga tgttgcattc tggcacagca caactttatg gcatttccga     420

tgtaatcgtc cggcagccct gggggagttc tatattcgca tattgggatg gtaaggacaa     480

tagcagatct cgcaacctcc agggaggcta taataacgtt tttaaaggat ggatttctca     540

taaaaatctg tcgcaaatta cactgagaat atcctttact agcgccgatt gagagcatcg     600

tcgtccaatt ttctaaatgg aaagaaaaca aggcgggcaa gagtgttcca aacattttca     660

ttttcggcga atctctcaaa tcccatggcg tgcaattgat tgcaaaattg gcacttccgt     720

tcacgtttgt atctccaaac tctaagacac ttttaattga aaaactacgt tctagtgtgg     780

aaagaaacct ataggcagac catagaacta tttgacacca catatctttt tgtatgtcaa     840

actgaccatg atcgtatgtt gctgaatgca ctagggcaat tcgctcgcgc gactccatac 900

attgaataat tccacacgtc agctcatcgg ttagcaaggt ccagtagttg aagtcattta 960

tttttccccg cggctggcca aatctacctc tgggaatatc caagttgtcg aatatgatcg 1020

caccggctct ggtcatggtg aaggaacttg tagcataaag acgcaggtat cataggggta 1080

atattttttt attcactcac atactaaaag taacgcatat tagcaccatg tatgggctat 1140

caattgacat ttgcgtagca ctacatcacg attatgtaca acataatggg acaacatatg 1200

cctgcaggtt agtcatatgt tacttggcag aggccgcatg gaaagtccct ggacgtggga 1260

catctgatta atacgtgagg aggtcagcca tgttcttttt ggcaaaggac tacggtcatt 1320

ggacgtttga ttggcatggg atagggtcag ccagagttaa cagtgttctt ttggcaaagg 1380

gatacgtgga aagtcccggg ccatttacag taaactgata cggggacaaa gcacagccat 1440

atttagtcat gtattgcttg gcagagggtc tatggaaagt ccctggacgt gggacgtctg 1500

attaatatga aagaaggtca gccagaggta gctgtgtcct ttttggcaaa gggatacggt 1560

tatgggacgt ttgattggac tgggataggg tcagccagag ttaacagtgt tcttttggca 1620

aaggaaacgt ggaaagtccc gggccattta cagtaaactg atactgggac aaagtacacc 1680

catatttagt catgttcttt ttggcaaaga gcatctggaa agtcccgggc agcattatag 1740

tcacttggca gagggaaagg gtcactcaga gttaagtaca tctttccagg gccaatattc 1800

cagtaaatta cacttagttt tatgcaaatc agccacaaag gggattttcc cggtcaatta 1860

tgactttttc cttagtcatg cggtatccaa ttactgccaa attggcagta catactaggt 1920

gattcactga catttggccg tcctctggaa agtccctgga aaccgctcaa gtactgtatc 1980

atggtgactt tgcatttttg gagagcacgc cccactccac cattggtcca cgtaccctat 2040

gggggagtgg tttatgagta tataaggggc tccggtttag aagccgggca gagcggccgc 2100

atgacaaacc tgcaagatca aacccaacag attgttccgt tcatacggag ccttctgatg 2160

ccaacaaccg gaccggcgtc cattccggac gacaccctgg agaagcacac tctcaggtca 2220

gagacctcga cctacaattt gactgtgggg gacacagggt cagggctaat tgtctttttc 2280

cctggattcc ctggctcaat tgtgggtgct cactacacac tgcagagcaa tgggaactac 2340

aagttcgatc agatgctcct gactgcccag aacctaccgg ccagctacaa ctactgcagg 2400

ctagtgagtc ggagtctcac agtaaggtca agcacactcc ctggtggcgt ttatgcacta 2460

aacggcacca taaacgccgt gaccttccaa ggaagcctga gtgaactgac agatgttagc 2520

tacaacgggt tgatgtctgc aacagccaac atcaacgaca aaattgggaa cgtcctagta 2580

ggggaagggg taaccgtcct cagcttaccc acatcatatg atcttgggta tgtgaggctt 2640

ggtgacccca tacccgctat agggcttgac ccaaaaatgg tagcaacatg tgacagcagt 2700

gacaggccca gagtctacac cataactgca gccgataatt accaattctc atcacagtac 2760

caaacaggtg gggtaacaat cacactgttc tcagccaaca ttgatgccat cacaagtctc 2820

agcgttgggg gagagctcgt gttcaaaaca agcgtccaaa gccttgtact gggcgccacc 2880

atctacctta taggctttga tgggactgcg gtaatcacca gagctgtggc cgcaaacaat 2940

gggctgacgg ccggcatcga caatcttatg ccattcaatc ttgtgattcc aaccaatgag 3000

ataacccagc caatcacatc catcaaactg gagatagtga cctccaaaag tgatggtcag 3060

gcaggggaac agatgtcatg gtcggcaagt gggagcctag cagtgacgat ccatggtggc 3120

aactatccag gagccctccg tcccgtcaca ctagtggcct acgaaagagt ggcaacagga 3180

tctgtcgtta cggtcgctgg ggtgagcaac ttcgagctga tcccaaatcc tgaactagca 3240

aagaacctgg ttacagaata tggccgattt gacccaggag ccatgaacta cacgaaattg 3300

atactgagtg agagggaccg ccttggcatc aagaccgtct ggccaacaag ggagtacact 3360

gactttcgtg agtacttcat ggaggtggcc gacctcaact ctcccctgaa gattgcagga 3420

gcatttggct tcaaagacat aatccgggcc ataaggaggt gagcggccgc gatatcaata 3480

aaatatcttt attttcatta catctgtgtg ttggtttttt gtgtgaatcg atagtactaa 3540

catacgctct ccatcaaaac aaaacgaaac aaaacaaact agcaaaatag gctgtcccca 3600

gtgcaagtgc aggtgccaga acatttctct tctagacctg caggcccggg gcaagtagat 3660

gcaatttcct cacactagtt gggtttatct actattgaat tttcccctat ctgtgataca 3720

cttgggagcc tctacaagca tattgccatc atgtacgttt ttatctactg tcttaacgcc 3780

catgggaacg gaggcgtcgt cgtcatgtat tggacggcaa cataggcagc aacacaaatt 3840

gcgtttaggt ggggtgcatg tggactcgat accaagcccc tgcagctggg gaacgtctgg 3900

tggagagccg ataatttgat atacgcacgc catattactg tcgttgaagt acgccttatc 3960

ttctatgttt tcaaatttag gttcccaagt ggacgtgaga agtgtttgta tctcacatgg 4020

aatggcccaa ggcattccag cccaggtgcc tggtacttta atggcaaaca aacgttttgg 4080

tagaggtatt gattctattg cagttctgca gatatctgca gccccgagta tccacaggct 4140

atacgatacg ttatcggagg cctccgattc tagcattaca tagccggtca gtagatcctg 4200

ccattcggta gcgcaaccgg ctacatcttc aaacagtctc acaataaatg catctctcgt 4260

tcctgccaat ccggaaccgg gcataccact cccgcctgcc gatttaattc tcacaattgg 4320

gcgatgccgg cggggcaaaa cgaatgtgga tttggcaaac cgacacaggt ctgctgtacg 4380

gactaatatg ggcacaccca catcattctt cagatgctcc atgcattgtt ctatgagaaa 4440

gatccatagg gtggaggcag cgtcacgaga tcgcccaggc aatcgatcgc attcgtctag 4500

taaagtgacg agagttatca tgcacacacc catgcccacg ccttccgaat aactggagct 4560

gtggaagatc ggaaacgtct ttttgactgc cggtctcgta ctactttcgc acaggtgtat 4620

acccggacgc gtactatata ttttatatca tccaacgtcc cgaaattaca tacgtggcgg 4680

cgatggaagt agatgttgag tcttcgaaag taagtgcctc gaatatgggt attgtctgtg 4740

aaaatatcga aagcggtacg acggttgcag aaccgtcgat gtcgccagat actagtaaca 4800

atagcttcga taacgaagac ttccgtgggc ctgaatacga tgtggagata 4850

<210> 40
<211> 4943
<212> DNA
<213> artificial sequence

<220>
<223> Partial plasmid SB1US2 gpVIIdwtsyn sequence for vSB1-010

<400> 40
tctcgtctaa aacgctccag tgctttacag ttcgataatc tggacctggg gacgcgtata 60

ggatcgttcc tccacatgcg ctgctgtcgg tatctcgaat ccccggtatt cagttgaatc 120

gttggcggag tgtcctcctg gactctgcaa tgttccctag ccgtcttcac tatctcgtgc 180

aaggctctat aatacagttc ctctgcagac ccgtcgttgc tcttcccttc tgcgtcgtta 240

gttatttctg taggctccag acgatttgcc tgcatttgtg cgcaacataa tctgattgca 300

ttccctatct cgtcttccgg taatcccata ggtgttcggt attcgcagat aggtagagaa 360

agcaccactg caaatcgtgc aatttccatt gccccaacca atattttttt taagaacggc 420

atcgccgtta atgtacctcg ggcattgtga cgatcgaaac ccttatggat gcctaaagag 480

agcattgcgg tccagttctc caggtgaaaa gagaatagcg cgggtagaaa cgggccgatt 540

agttttatct tcgccgcgtc cctaatatcc caagttctgc agtataactt ccatcgtccg 600

ttttcgacaa ggtccggcgc gacatagttt gaaatgtcat ctatcagaaa catctcgccc 660

atcgtagaaa aaaacctgta cgcagaccat aaaaccattc ggtaccacat atccttgtgt 720

atatcaaacg atatgttggt tatgtcgttg gcggatgttg tatgaaatag agctaagcgt 780

tctctggatt ccacgcactg aacgattccg ttagtcaatt catctgctaa cataggccaa 840

aagtttattc gtgttacttt tctcggcggt ttggcaaaac gcccccttgg cacatccatg 900

tcattaaata cagcggcata actcctactc atgtgttcca tagcccaggt ttctgttcgg 960

tctgctacta cgatcagatc agtggcgcga tcagatgcgt gggatgaatg aagtgtatcc 1020

gaaagcagtt ttgagatata cgctaaactg tacgacgatt gtggcactaa acgaagcttt 1080

gcgcgacccc catcccacgc cctgcaggtt agtcatatgt tacttggcag aggccgcatg 1140

gaaagtccct ggacgtggga catctgatta atacgtgagg aggtcagcca tgttcttttt 1200

ggcaaaggac tacggtcatt ggacgtttga ttggcatggg atagggtcag ccagagttaa 1260

cagtgttctt ttggcaaagg gatacgtgga aagtcccggg ccatttacag taaactgata 1320

cggggacaaa gcacagccat atttagtcat gtattgcttg gcagagggtc tatggaaagt 1380
ccctggacgt gggacgtctg attaatatga aagaaggtca gccagaggta gctgtgtcct 1440
ttttggcaaa gggatacggt tatgggacgt ttgattggac tgggataggg tcagccagag 1500
ttaacagtgt tcttttggca aaggaaacgt ggaaagtccc gggccattta cagtaaactg 1560
atactgggac aaagtacacc catatttagt catgttcttt ttggcaaaga gcatctggaa 1620
agtcccgggc agcattatag tcacttggca gagggaaagg gtcactcaga gttaagtaca 1680
tctttccagg gccaatattc cagtaaatta cacttagttt tatgcaaatc agccacaaag 1740
gggattttcc cggtcaatta tgactttttc cttagtcatg cggtatccaa ttactgccaa 1800
attggcagta catactaggt gattcactga catttggccg tcctctggaa agtccctgga 1860
aaccgctcaa gtactgtatc atggtgactt tgcatttttg gagagcacgc cccactccac 1920
cattggtcca cgtaccctat gggggagtgg tttatgagta tataaggggc tccggtttag 1980
aagccgggca gagcggccgc atgggctcca aaccttctac caggatccca gcacctctga 2040
tgctgatcac ccggattatg ctgatattgg gctgtatccg tccgacaagc tctcttgacg 2100
gcaggcctct tgcagctgca ggaattgtag taacaggaga taaggcagtc aatgtataca 2160
cttcgtctca gacagggtca atcatagtca agttgctccc gaatatgccc agggataagg 2220
aggcgtgtgc aaaagcccca ttagaggcat ataacagaac actgactact ttgctcactc 2280
ctcttggcga ctccatccgc aagatccaag ggtctgtgtc cacatctgga ggaggcaagc 2340
aaggccgcct gataggtgct gttattggca gtgtagctct tggggttgca acagcggcac 2400
agataacagc agctgcggcc ctaatacaag ccaaccagaa tgccgccaac atcctccggc 2460
ttaaggagag cattgctgca accaatgaag ctgtgcatga agtcaccgac ggattatcac 2520
aactatcagt ggcagttggg aagatgcagc agtttgtcaa tgaccagttt aataatacgg 2580
cgcgagaatt ggactgtata aaaatcacac aacaggttgg tgtagaactc aacctatacc 2640
taactgaatt gactacagta ttcgggccac agatcacctc ccctgcatta actcagctga 2700
ccatccaggc actttataat ttagctggtg gcaatatgga ttacttatta actaagttag 2760
gtatagggaa caatcaactc agctcgttaa ttggtagcgg cctgatcact ggttacccta 2820
tactgtatga ctcacagact caactcttgg gcatacaagt gaatttaccc tcagtcggga 2880
acttaaataa tatgcgtgcc acctatttgg agaccttatc tgtaagtaca accaaaggat 2940
atgcctcagc acttgtcccg aaagtagtga cacaagtcgg ttccgtgata gaagagcttg 3000
acacctcata ctgtatagag tccgatctgg atttatattg tactagaata gtgacattcc 3060
ccatgtcccc aggtatttat tcctgtttga gcggcaacac atcagcttgc atgtattcaa 3120
agactgaagg cgcactcact acgccgtata tggcccttaa aggctcagtt attgccaatt 3180
gtaaaataac aacatgtaga tgtacagacc ctcctggtat catatcgcaa aattatggag 3240

aagctgtatc cctgatagat agacattcgt gcaatgtctt atcattagac gggataactc 3300

taaggctcag tggggaattt gatgcaactt atcaaaagaa catctcaata ctagattctc 3360

aagtcatcgt gacaggcaat cttgatatat caactgaact tggaaacgtc aacaattcaa 3420

tcagcaatgc cttggatagg ttggcagaaa gcaacagcaa gctagaaaaa gtcaatgtca 3480

gactaaccag cacatctgct ctcattacct atattgttct aactgtcatt tctctagttt 3540

tcggtgcact tagtctggtg ttagcgtgtt acctgatgta caaacagaag gcacaacaaa 3600

agaccttgct atggcttggg aataataccc tcgatcagat gagagccact acaagagcat 3660

gagcggccgc gatatcaata aaatatcttt attttcatta catctgtgtg ttggtttttt 3720

gtgtgaatcg atagtactaa catacgctct ccatcaaaac aaaacgaaac aaaacaaact 3780

agcaaaatag gctgtcccca gtgcaagtgc aggtgccaga acatttctct tctagacctg 3840

caggggagtc tgtgcaaggt taatgaccct cgcagttcat tcggaagtta taactgccgc 3900

cttcgcacat ttctttttgt cctgttttgt attgccataa cagataggaa ttgaaacctg 3960

atcctcctgt tttttgcagc atggccagca acagaatact ttgtcggatc gactacttgc 4020

gcgagatggt tccgttcttg gaggtttcgg cgggtcgggt ggagaaccta ttattttata 4080

cacacacgtc ataccgttgt cgcgaaaatg ttctttgtct tctgccgtct cgaacgtcgg 4140

ttcccacgta gacgttagga gcgttggaat ggtatcagga agagcccacg gcatgccgga 4200

ccaagtaccc gctactttga ccgcgagcag tctcttcggt aatgggatgt attccagagc 4260

agcgcggcag agatcagcgg cccccactat ccacagactg tatgaagtgt tttctgaaac 4320

atcggactcc aacatcaaat atccagacat aacatcttgc cattcggaag cacatccgcc 4380

gacatcttca aatagcctaa ctataaacga gtctctagtt cctgctaacc cagtacctcg 4440

aatgccagtc ccatccggtg ggttcgtcct gataatcggt ctctgacgcc gaggaagaac 4500

taaaaggggt ctggaaaagc ggaacagatc tgcagaccga acgactacag acacgcccac 4560

atcatcatgt atctgttcca tgcattgctt tatgagaaaa atccataagg ccgaggcggc 4620

atctctagat ctcccgggga gtctctcgca ctcatctagg agagtgacga cagttatcat 4680

agacacgccc atttgtgcac caaacgaaaa gttcctgtac tggtggagcg tcggcgcggg 4740

aatcggtccg tgctctgaaa ccagtgtcta gacagaagac catccggtaa attctggtgt 4800

atgaactgac ggtctccaga cgaacgtcga agacattaac gatggaaact aacgagcttt 4860

cttcaaaagt gtctgattac aacgctaata gaccttacga aactatacgc agcgatacca 4920

gtgacacaga tccgtcggtg tcg 4943

<210> 41
<211> 1362
<212> DNA

<213>  artificial sequence

<220>
<223>  IBDV DNA encoding VP2 protein of IBDV E strain

<400>  41
atgacaaacc tgcaagatca aacccaacag attgttccgt tcatacggag ccttctgatg      60

ccaacaaccg gaccggcgtc cattccggac gacaccctgg agaagcacac tctcaggtca     120

gagacctcga cctacaattt gactgtgggg gacacagggt cagggctaat tgtctttttc     180

cctggattcc ctggctcaat tgtgggtgct cactacacac tgcagagcaa tgggaactac     240

aagttcgatc agatgctcct gactgcccag aacctaccgg ccagctacaa ctactgcagg     300

ctagtgagtc ggagtctcac agtaaggtca agcacactcc ctggtggcgt ttatgcacta     360

aacggcacca taaacgccgt gaccttccaa ggaagcctga gtgaactgac agatgttagc     420

tacaacgggt tgatgtctgc aacagccaac atcaacgaca aaattgggaa cgtcctagta     480

ggggaagggg taaccgtcct cagcttaccc acatcatatg atcttgggta tgtgaggctt     540

ggtgacccca tacccgctat agggcttgac ccaaaaatgg tagcaacatg tgacagcagt     600

gacaggccca gagtctacac cataactgca gccgataatt accaattctc atcacagtac     660

caaacaggtg gggtaacaat cacactgttc tcagccaaca ttgatgccat cacaagtctc     720

agcgttgggg gagagctcgt gttcaaaaca agcgtccaaa gccttgtact gggcgccacc     780

atctacctta taggctttga tgggactgcg gtaatcacca gagctgtggc cgcaaacaat     840

gggctgacgg ccggcatcga caatcttatg ccattcaatc ttgtgattcc aaccaatgag     900

ataacccagc caatcacatc catcaaactg gagatagtga cctccaaaag tgatggtcag     960

gcaggggaac agatgtcatg gtcggcaagt gggagcctag cagtgacgat ccatggtggc    1020

aactatccag gagccctccg tcccgtcaca ctagtggcct acgaaagagt ggcaacagga    1080

tctgtcgtta cggtcgctgg ggtgagcaac ttcgagctga tcccaaatcc tgaactagca    1140

aagaacctgg ttacagaata tggccgattt gacccaggag ccatgaacta cacgaaattg    1200

atactgagtg agagggaccg ccttggcatc aagaccgtct ggccaacaag ggagtacact    1260

gactttcgtg agtacttcat ggaggtggcc gacctcaact ctcccctgaa gattgcagga    1320

gcatttggct tcaaagacat aatccgggcc ataaggaggt ga                       1362


<210>  42
<211>  453
<212>  PRT
<213>  artificial sequence

<220>
<223>  IBDV VP2 protein of IBDV E strain

<400>  42

Met Thr Asn Leu Gln Asp Gln Thr Gln Gln Ile Val Pro Phe Ile Arg
1               5                   10                  15

Ser Leu Leu Met Pro Thr Thr Gly Pro Ala Ser Ile Pro Asp Asp Thr
            20                  25                  30

Leu Glu Lys His Thr Leu Arg Ser Glu Thr Ser Thr Tyr Asn Leu Thr
        35                  40                  45

Val Gly Asp Thr Gly Ser Gly Leu Ile Val Phe Phe Pro Gly Phe Pro
    50                  55                  60

Gly Ser Ile Val Gly Ala His Tyr Thr Leu Gln Ser Asn Gly Asn Tyr
65                  70                  75                  80

Lys Phe Asp Gln Met Leu Leu Thr Ala Gln Asn Leu Pro Ala Ser Tyr
                85                  90                  95

Asn Tyr Cys Arg Leu Val Ser Arg Ser Leu Thr Val Arg Ser Ser Thr
            100                 105                 110

Leu Pro Gly Gly Val Tyr Ala Leu Asn Gly Thr Ile Asn Ala Val Thr
        115                 120                 125

Phe Gln Gly Ser Leu Ser Glu Leu Thr Asp Val Ser Tyr Asn Gly Leu
    130                 135                 140

Met Ser Ala Thr Ala Asn Ile Asn Asp Lys Ile Gly Asn Val Leu Val
145                 150                 155                 160

Gly Glu Gly Val Thr Val Leu Ser Leu Pro Thr Ser Tyr Asp Leu Gly
                165                 170                 175

Tyr Val Arg Leu Gly Asp Pro Ile Pro Ala Ile Gly Leu Asp Pro Lys
            180                 185                 190

Met Val Ala Thr Cys Asp Ser Ser Asp Arg Pro Arg Val Tyr Thr Ile
        195                 200                 205

Thr Ala Ala Asp Asn Tyr Gln Phe Ser Ser Gln Tyr Gln Thr Gly Gly
    210                 215                 220

Val Thr Ile Thr Leu Phe Ser Ala Asn Ile Asp Ala Ile Thr Ser Leu
225                 230                 235                 240

Ser Val Gly Gly Glu Leu Val Phe Lys Thr Ser Val Gln Ser Leu Val
                245                 250                 255

Leu Gly Ala Thr Ile Tyr Leu Ile Gly Phe Asp Gly Thr Ala Val Ile
            260                 265                 270

Thr Arg Ala Val Ala Ala Asn Asn Gly Leu Thr Ala Gly Ile Asp Asn
        275                 280                 285

Leu Met Pro Phe Asn Leu Val Ile Pro Thr Asn Glu Ile Thr Gln Pro
    290                 295                 300

Ile Thr Ser Ile Lys Leu Glu Ile Val Thr Ser Lys Ser Asp Gly Gln
305                 310                 315                 320

Ala Gly Glu Gln Met Ser Trp Ser Ala Ser Gly Ser Leu Ala Val Thr
                325                 330                 335

Ile His Gly Gly Asn Tyr Pro Gly Ala Leu Arg Pro Val Thr Leu Val
            340                 345                 350

Ala Tyr Glu Arg Val Ala Thr Gly Ser Val Val Thr Val Ala Gly Val
        355                 360                 365

Ser Asn Phe Glu Leu Ile Pro Asn Pro Glu Leu Ala Lys Asn Leu Val
    370                 375                 380

Thr Glu Tyr Gly Arg Phe Asp Pro Gly Ala Met Asn Tyr Thr Lys Leu
385                 390                 395                 400

Ile Leu Ser Glu Arg Asp Arg Leu Gly Ile Lys Thr Val Trp Pro Thr
                405                 410                 415

Arg Glu Tyr Thr Asp Phe Arg Glu Tyr Phe Met Glu Val Ala Asp Leu
            420                 425                 430

Asn Ser Pro Leu Lys Ile Ala Gly Ala Phe Gly Phe Lys Asp Ile Ile
        435                 440                 445

Arg Ala Ile Arg Arg
    450

<210>  43
<211>  884
<212>  DNA
<213>  artificial sequence

<220>
<223>  Guinea pig CMV promoter

<400>  43
ttagtcatat gttacttggc agaggccgca tggaaagtcc ctggacgtgg gacatctgat        60

taatacgtga ggaggtcagc catgttcttt ttggcaaagg actacggtca ttggacgttt 120

gattggcatg ggatagggtc agccagagtt aacagtgttc ttttggcaaa gggatacgtg 180

gaaagtcccg ggccatttac agtaaactga tacggggaca aagcacagcc atatttagtc 240

atgtattgct tggcagaggg tctatggaaa gtccctggac gtgggacgtc tgattaatat 300

gaaagaaggt cagccagagg tagctgtgtc ctttttggca aagggatacg gttatgggac 360

gtttgattgg actgggatag ggtcagccag agttaacagt gttcttttgg caaaggaaac 420

gtggaaagtc ccgggccatt tacagtaaac tgatactggg acaaagtaca cccatattta 480

gtcatgttct ttttggcaaa gagcatctgg aaagtcccgg gcagcattat agtcacttgg 540

cagagggaaa gggtcactca gagttaagta catctttcca gggccaatat tccagtaaat 600

tacacttagt tttatgcaaa tcagccacaa aggggatttt cccggtcaat tatgactttt 660

tccttagtca tgcggtatcc aattactgcc aaattggcag tacatactag gtgattcact 720

gacatttggc cgtcctctgg aaagtccctg gaaaccgctc aagtactgta tcatggtgac 780

tttgcatttt tggagagcac gccccactcc accattggtc cacgtaccct atgggggagt 840

ggtttatgag tatataaggg gctccggttt agaagccggg caga 884

<210> 44
<211> 30
<212> DNA
<213> artificial sequence

<220>
<223> primer HM101

<400> 44
ccggaattcc gatgtttagt cacgatagac 30

<210> 45
<211> 35
<212> DNA
<213> artificial sequence

<220>
<223> Primer HM102

<400> 45
ataagagcgg ccgcagtgag atgatcttaa tgatg 35

<210> 46
<211> 38
<212> DNA
<213> artificial sequence

<220>
<223> primer F-ATG

<400> 46

tatagcggcc gcaagatggg ctccagatct tctaccag 38

<210> 47
<211> 34
<212> DNA
<213> artificial sequence

<220>
<223> Primer F-STOP

<400> 47
cgaggcggcc gctcatattt ttgtagtggc tctc 34

## Description

### CROSS-REFERENCE TO RELATED APPLICATIONS

This application claims priority to U.S. provisional application 61/564,877 filed on November 30, 2011 and U.S. provisional application 61/694,957 filed on August 30, 2012.

### FIELD OF THE INVENTION

The invention relates to recombinant viral vectors for the insertion and expression of foreign genes for use as safe immunization vehicles to protect against a variety of pathogens. It also relates to multivalent composition or vaccine comprising one or more recombinant viral vectors for protection against a variety of pathogens. The present invention relates to methods of making and using the recombinant viral vectors.

### BACKGROUND OF THE INVENTION

Poultry vaccination is widely used to protect poultry flocks against devastating diseases including Newcastle disease (ND), infectious bursal disease (IBD), Marek's disease (MD), infectious bronchitis (IB), infectious laryngotracheitis (ILT) and avian influenza (AI). ND is caused by the avian paramyxovirus 1 (APMV-1) also designated ND virus (NDV) belonging to the *Paramyxoviridae* family. MD is caused by Gallid herpesvirus 2 (*Herpesviridae* family) also designated as MD virus serotype 1 (MDV1). IB is caused by IB virus (IBV) belonging to the *Coronaviridae* family, ILT is caused by Gallid herpesvirus 1 (*Herpesviridae* family) also designated ILT virus (ILTV) and AI is caused by AI virus (AIV) belonging to the *Orthomyxoviridae* family.

A number of recombinant avian viral vectors have been proposed with a view to vaccinating birds against these avian pathogens. The viral vectors used comprise avipox viruses, especially fowlpox (EP-A-0,517,292), Marek's virus, such as serotypes 2 and 3 (HVT) (WO-A-87/04463), or alternatively the ITLV, NDV and avian adenovirus. When some of these recombinant avian viral vectors were used for vaccination, they display variable levels of protection.

Several recombinant herpesvirus of turkeys (HVT, also designated Meleagrid herpesvirus 1 or MDV serotype 3) vectors expressing antigens from various pathogens (US patent Nos. 5,980,906, 5,853,733, 6,183,753, 5,187,087) including IBDV, NDV, ILTV and AIV have been developed and licensed. Of particular interest is a HVT vector-expressing IBDV VP2 protective gene that has shown clear advantages over classical IBD vaccines (Bublot et al J.Comp. Path.2007, Vol.137, S81-S84; US 5,980,906). Other HVT vectors of interest are those expressing either NDV (Morgan et al 1992, Avian dis. 36, 858-70; US 6,866,852; US5,650,153) or ILTV (Johnson et al, 2010 Avian Dis 54, 1251-1259; US6,299,882; US5,853,733) protective gene(s). One of the practical problems of using several HVT-based recombinant vaccines together is their interference. Lower protection is induced at least against one of the disease when two HVT recombinants expressing different antigens are mixed (Rudolf Heine 2011; Issues of the Poultry Recombinant Viral Vector Vaccines which May Cause an Effect on the Economic Benefits of those Vaccines; paper presented at the XVII World Veterinary Poultry Association (WVPA) Congress in Cancún, Mexico, August 14-18, 2011; Slacum G, Hein R. and Lynch P., 2009, The compatibility of HVT recombinants with other Marek's disease vaccines, 58th Western Poultry Disease Conference, Sacramento, CA, USA, March 23rd-25th, p 84).

The combination of HVT and SB-1, a Gallid herpesvirus 3 (MDV serotype 2 or MDV-2) vaccine strain, has shown a synergistic effect on MD protection (Witter and Lee, 1984, Avian Pathology 13, 75-92). To address the interference problem, it is of interest to evaluate the HVT virus as a vaccine vector to express one or more protective antigen(s) against a variety of avian pathogens.

The SB-1 genome was cloned and characterized in bacterial artificial chromosome (BAC) (Petherbridge, et al., J. Virol. Methods 158, 11-17, 2009; Singh et al., Research in Veterinary Science 89, 140-145, 2010). The MDV2 SB-1 sequence was recently obtained and analyzed (Spatz and Schat, Virus Gene 42, 331-338, 2011). A glycoprotein E deletion of SB-1 virus was described by Petherbridge, et al. (J. Virol. Methods 158, 11-17, 2009). However, no research has been reported using SB-1 as a viral vector expressing foreign protective genes.

Considering the potential effect of animal pathogens, such as NDV and IBDV on veterinary public health and the economy, efficient methods of preventing infection and protecting animals are needed. There is a need for a solution of combined effective vector vaccines and a suitable method for making the vaccine that could alleviate the problem of interference observed between two HVT-based vector vaccines.

### SUMMARY OF THE INVENTION

The present invention showed surprising results when polyvalent compositions or vaccines comprising single or double HVT vector were effective to protect animals against a variety of avian pathogens without interference. Surprising results were also observed when various combinations of promoters, codon-optimized gene, polyA tails and insertion sites conferred different levels of efficacy and stability to the expression of one or more heterologous genes *in vivo.*

The present invention relates to a recombinant HVT vector comprising one or more heterologous polynucleotides coding for and expressing at least one antigen of an avian pathogen.

The present invention provides a composition or vaccine comprising one or more recombinant HVT vectors comprising one or more heterologous polynucleotides coding for and expressing at least one antigen of an avian pathogen.

The present invention provides a polyvalent composition or vaccine comprising one or more recombinant HVT vectors comprising heterologous polynucleotides coding for and expressing at least one antigen of an avian pathogen and one or more recombinant SB1 vectors comprising heterologous polynucleotides coding for and expressing at least one antigen of an avian pathogen.

The present invention relates to a method of vaccinating an animal, or inducing an immunogenic or protective response in an animal, comprising at least one administration of the composition or vector of the present invention.

### BRIEF DESCRIPTION OF THE DRAWINGS

The following detailed description, given by way of example, and which is not intended to limit the invention to specific embodiments described, may be understood in conjunction with the accompanying figures, incorporated herein by reference, in which:

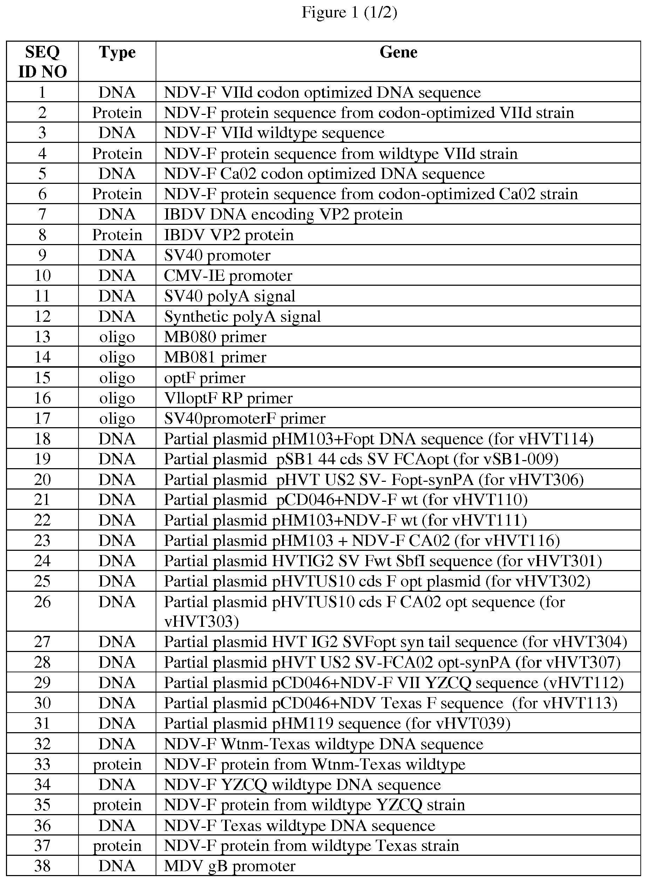

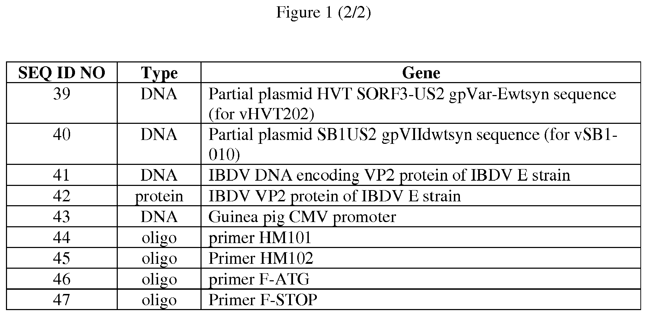

Figure 1 is a table showing the SEQ ID NO assigned to each DNA and protein sequence.
Figure 2 depicts the genome structure of HVT and its insertion sites.
Figure 3 depicts the plasmid map of pHM103.
Figure 4 depicts the PCR analysis results of vHVT114.
Figure 5 shows the dual immunofluorescent assay results.
Figure 6 depicts the Southern blot results of vHVT114.
Figure 7 depicts the immunoprecipitation and Western blot analysis results of vHVT114.
Figure 8 depicts the Western blot analysis of immunoprecipitated sample from vHVT306 infected cells.
Figure 9 depicts the Western blot analysis of immunoprecipitated sample from vSB 1-009 infected cells.
Figure 10 depicts the result of challenge study of vHVT304 and vHVT114 against NDV ZJ1 and CA02.
Figure 11 depicts the viral shedding result after NDV CA02 and ZJ1 challenge.
Figure 12 depicts the viral shedding result after NDV Chimalhuacan challenge.

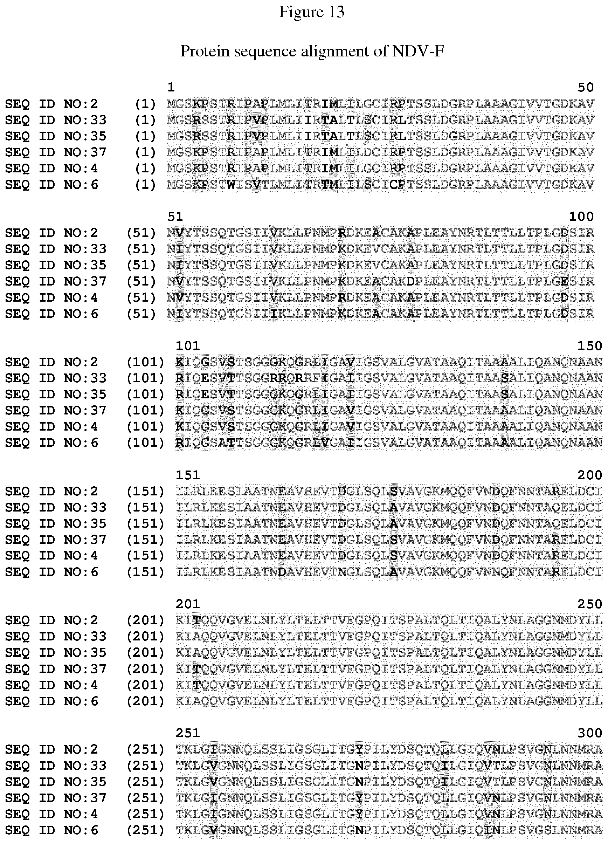

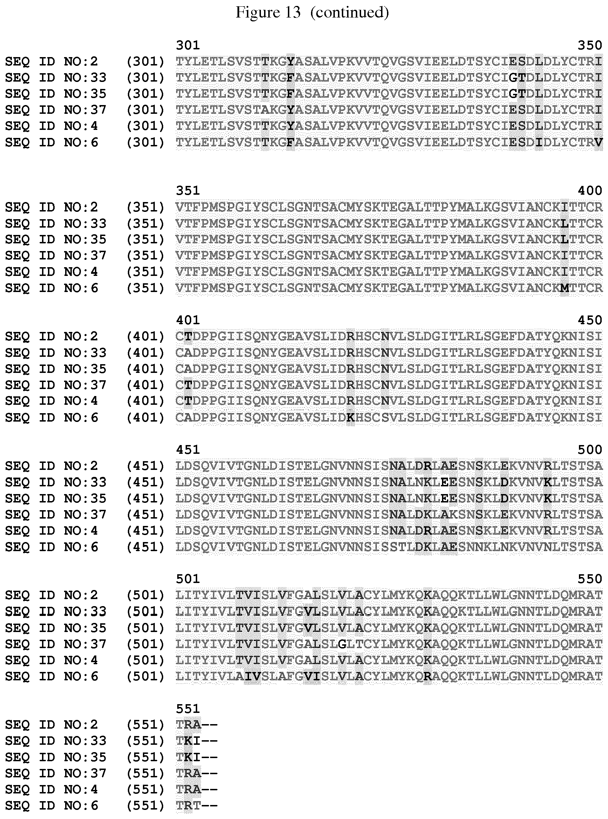

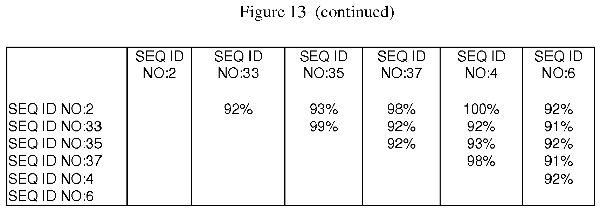

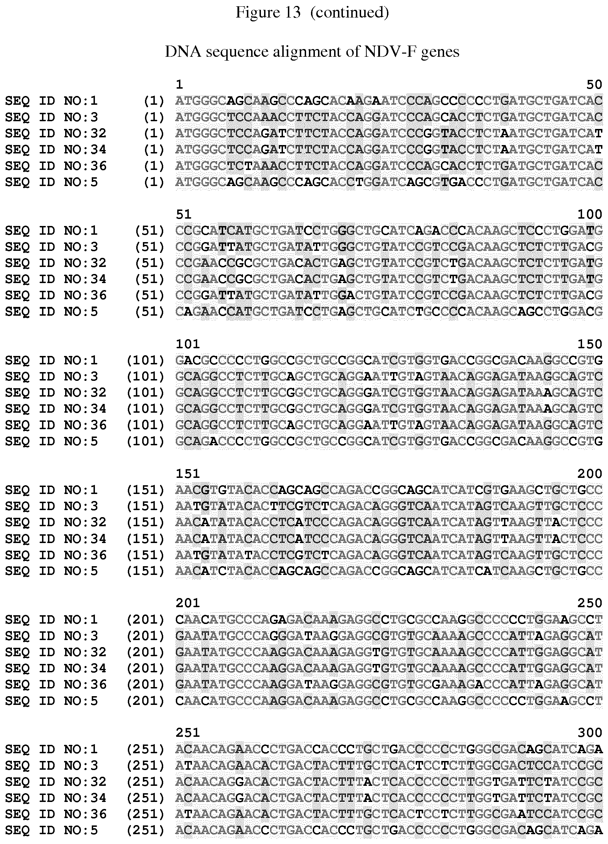

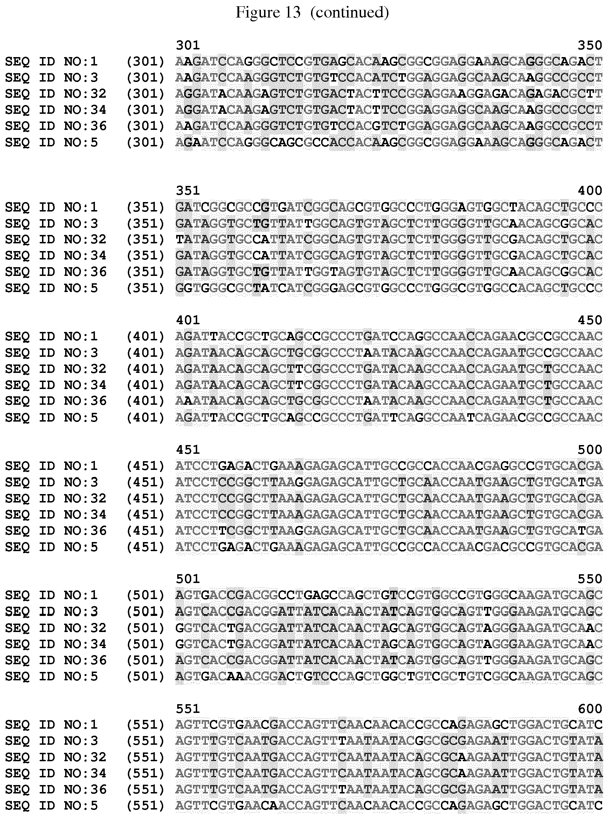

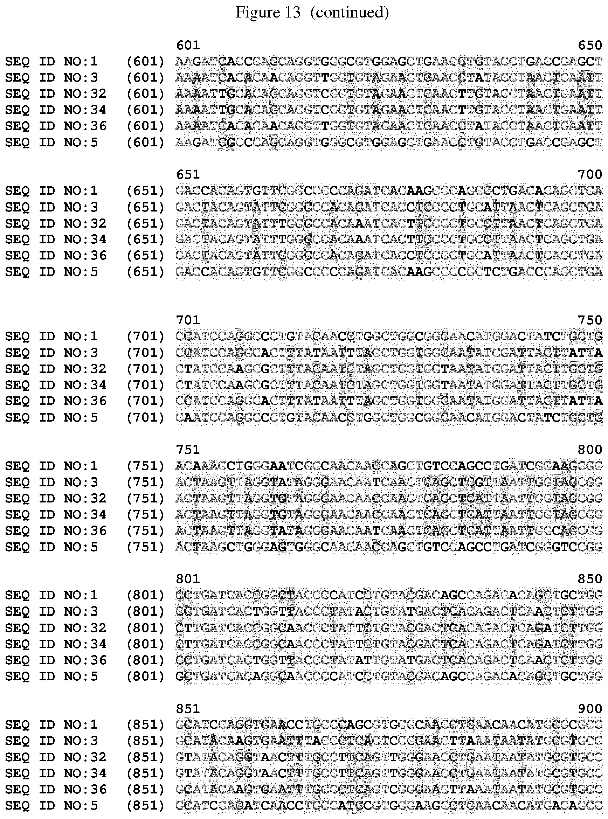

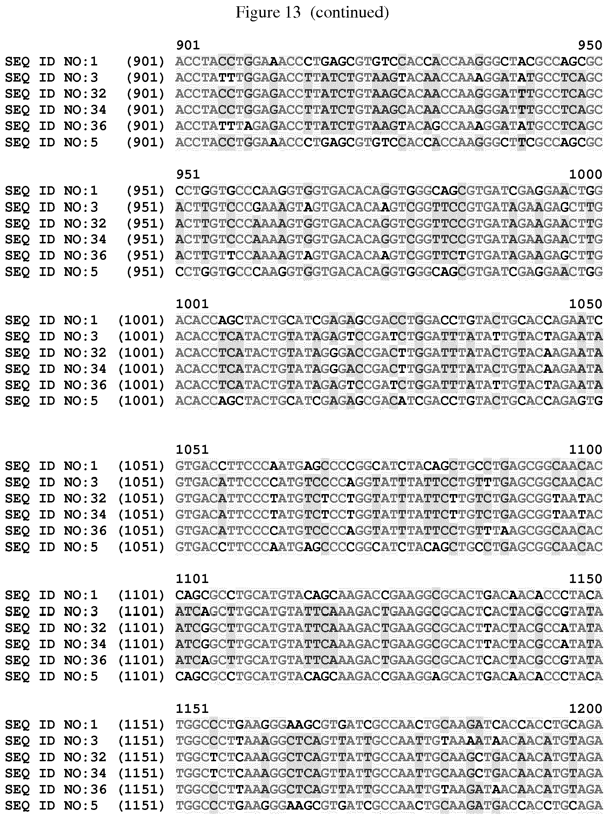

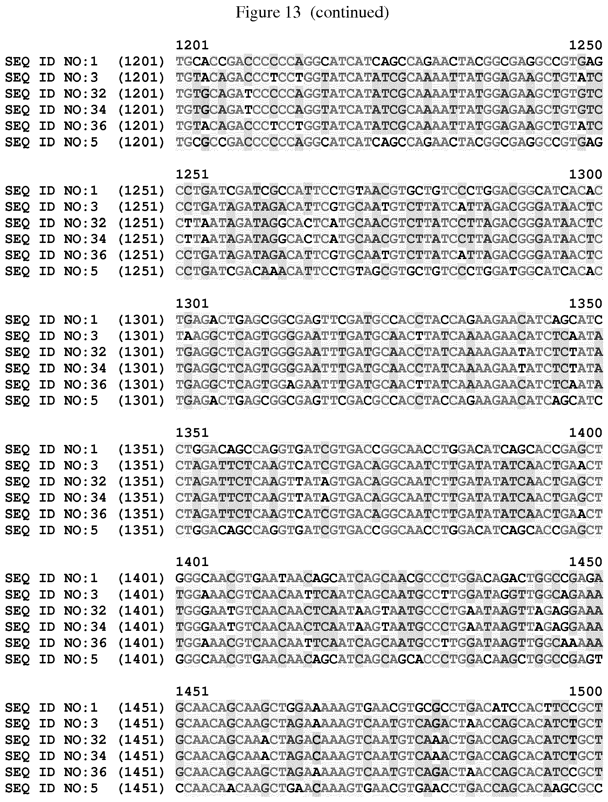

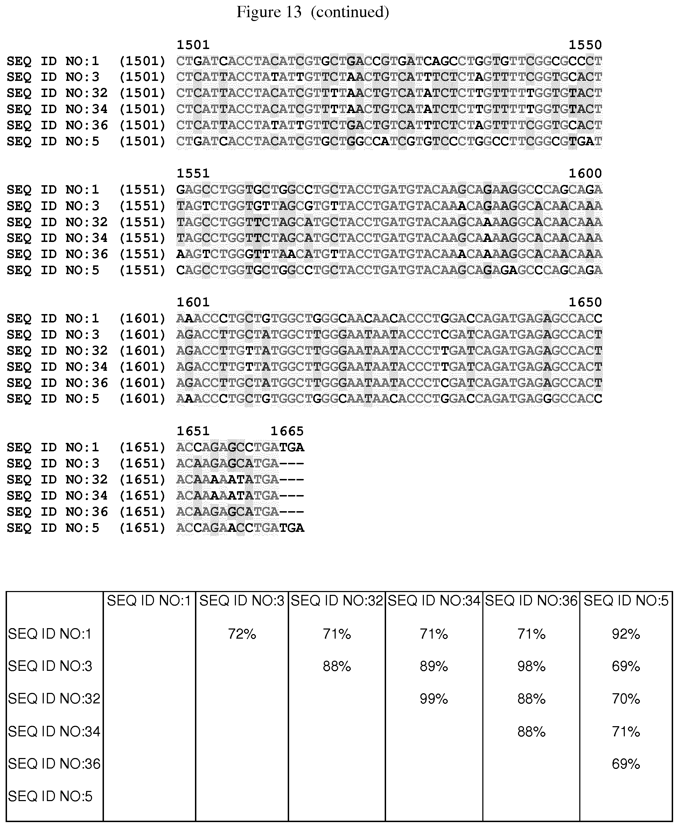

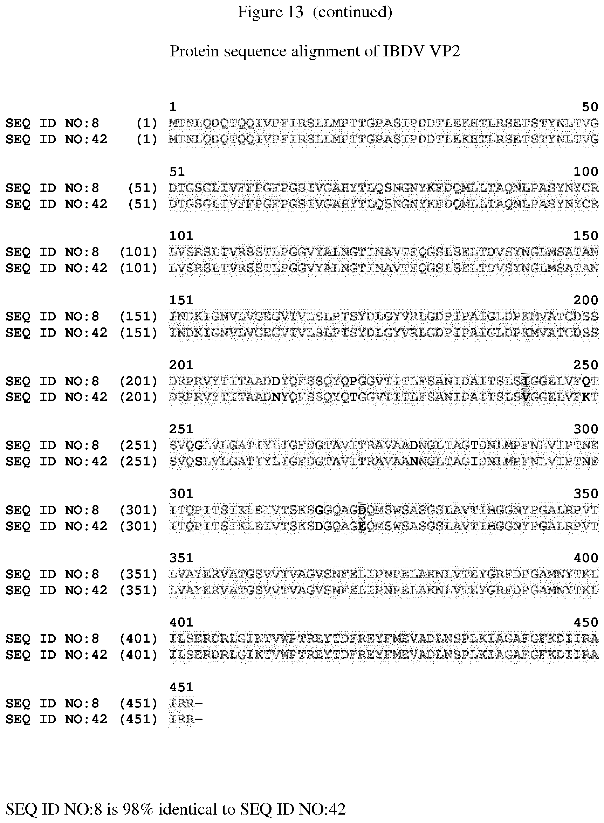

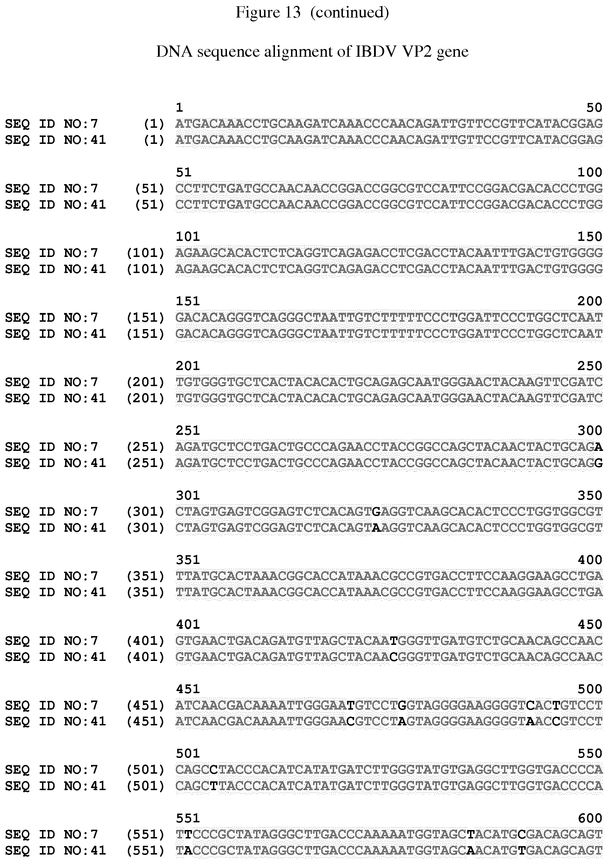

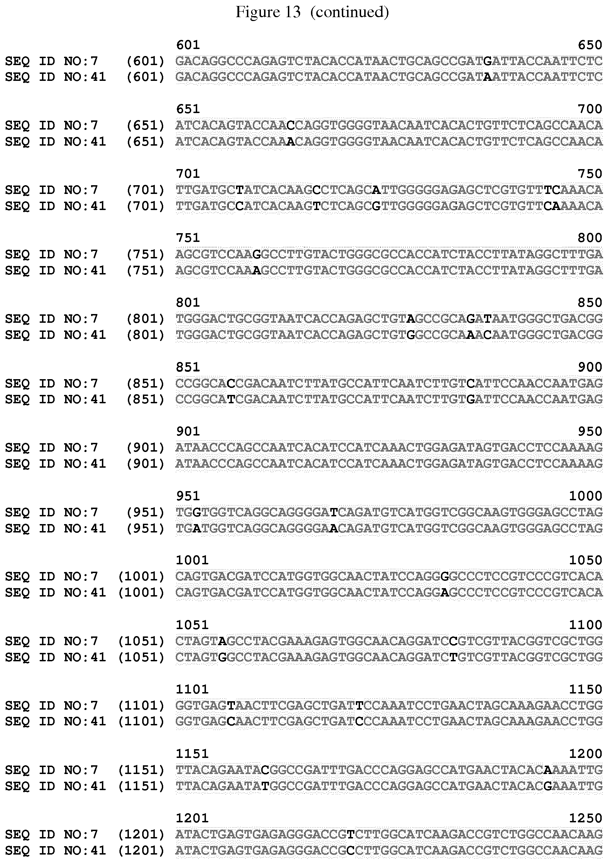

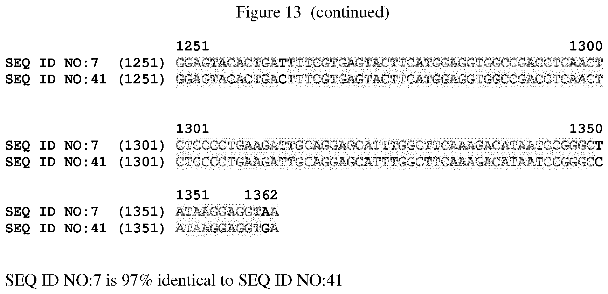

Figure 13 shows the sequence alignment and percentage identity.

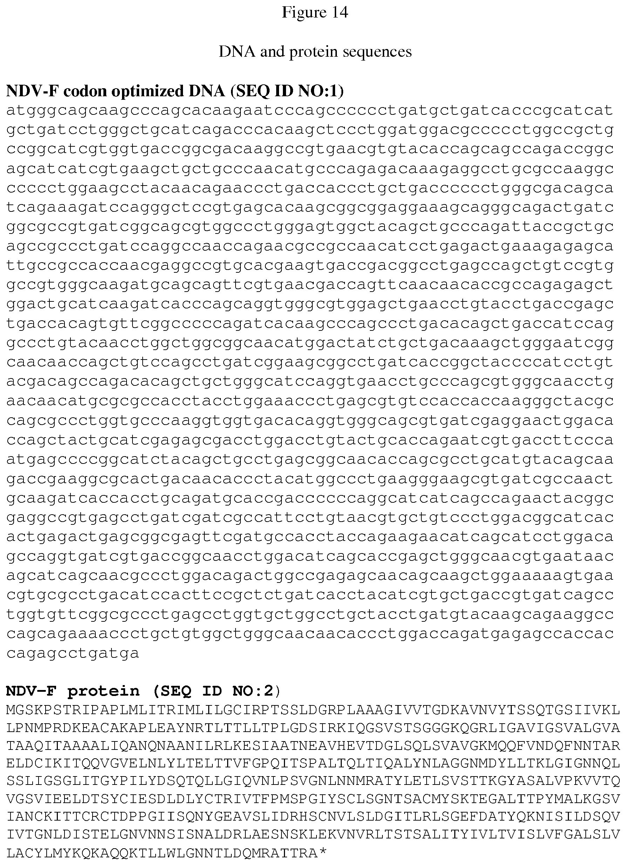

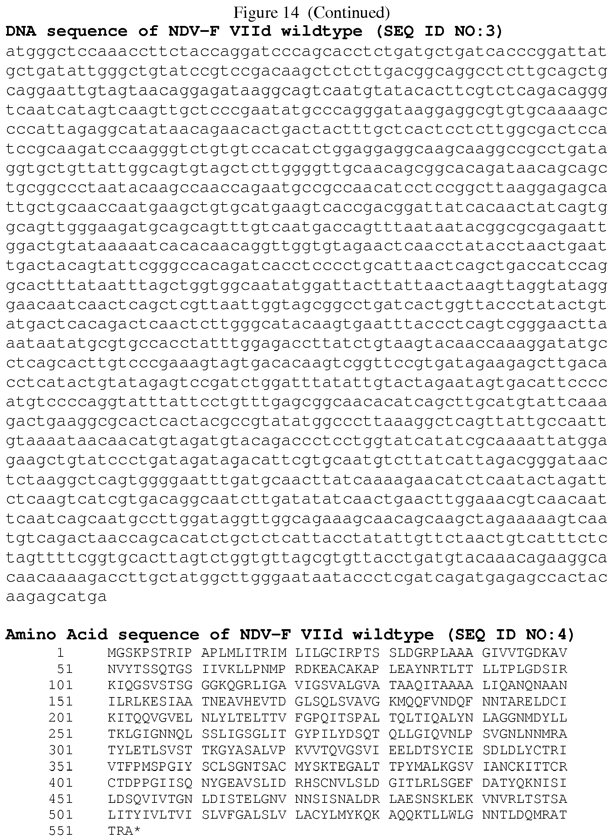

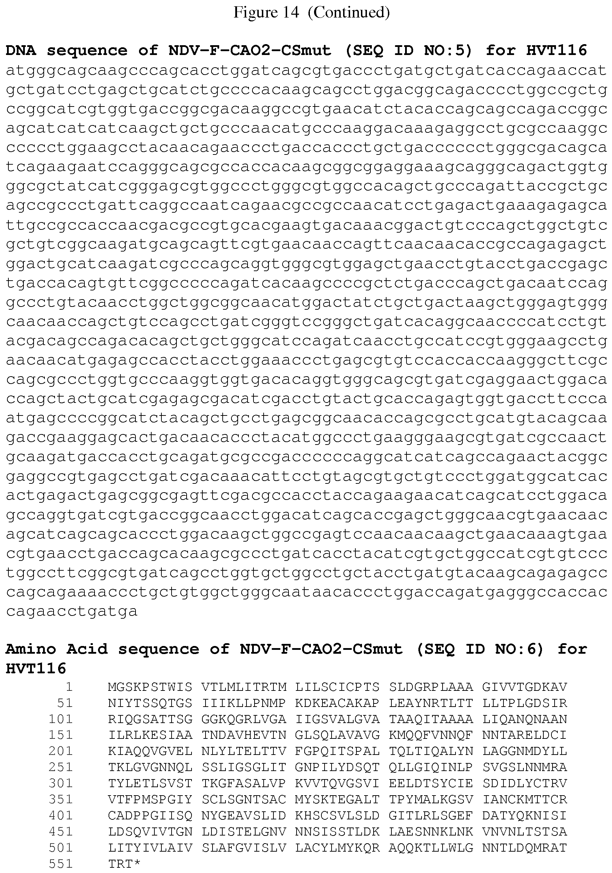

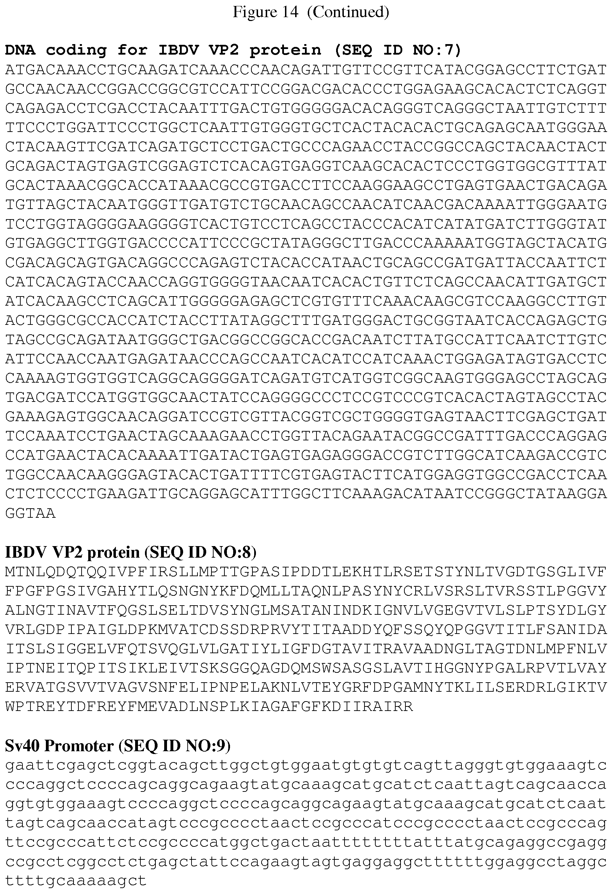

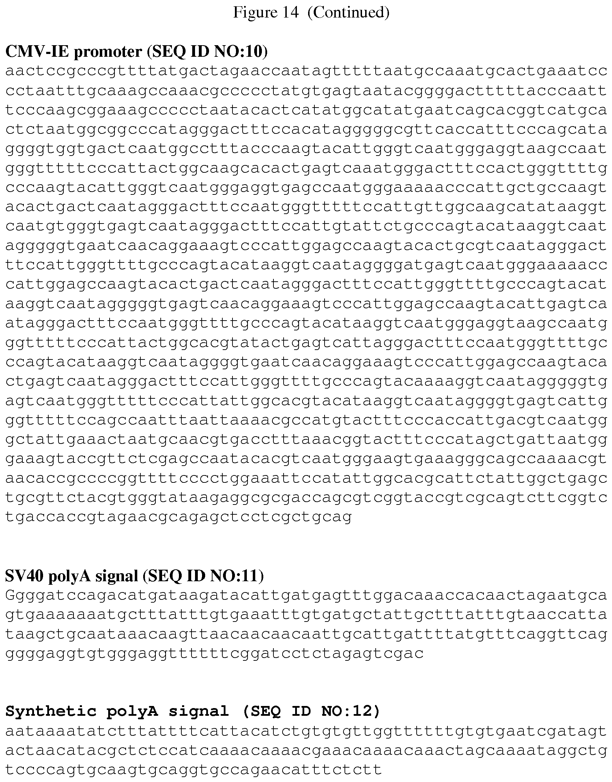

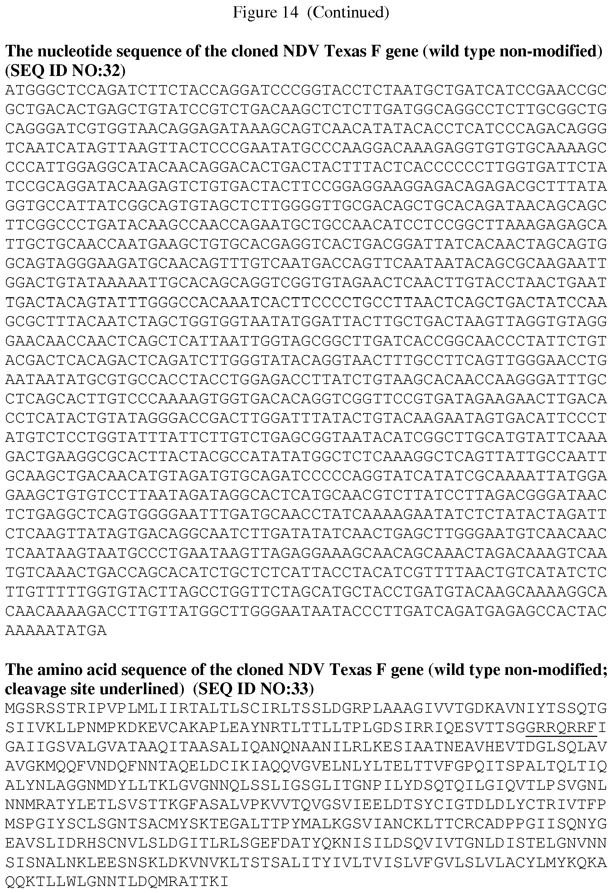

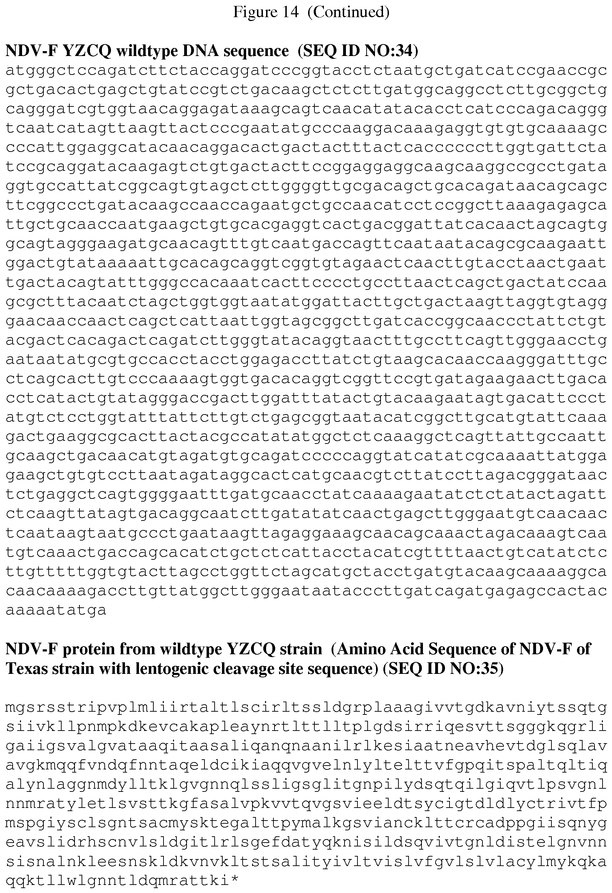

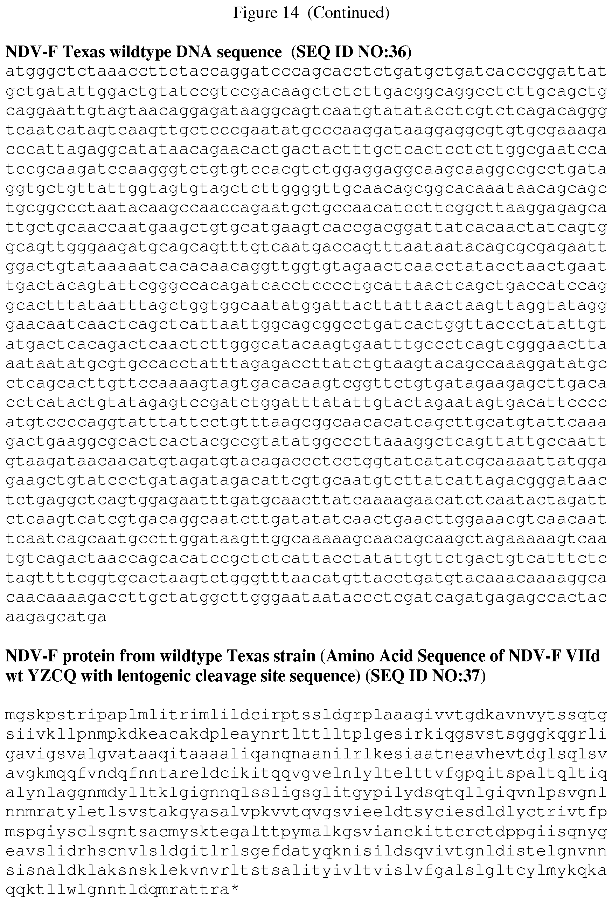

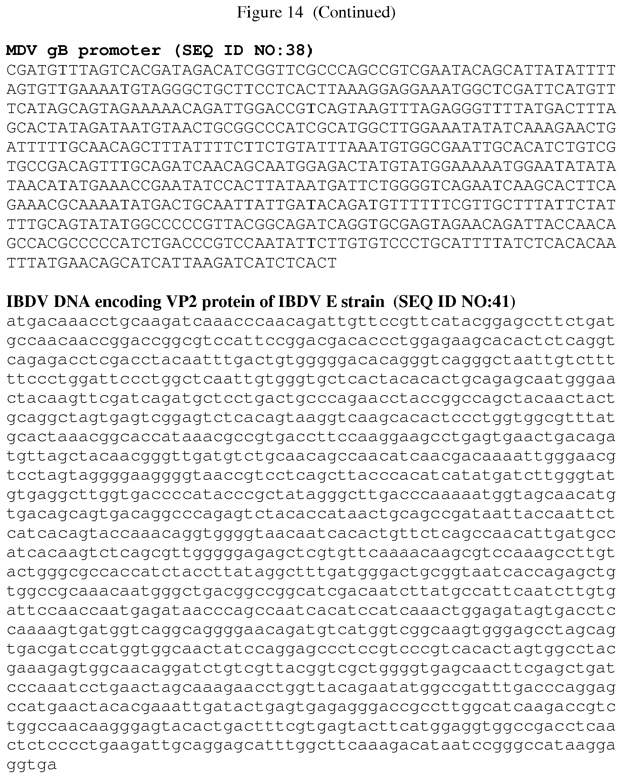

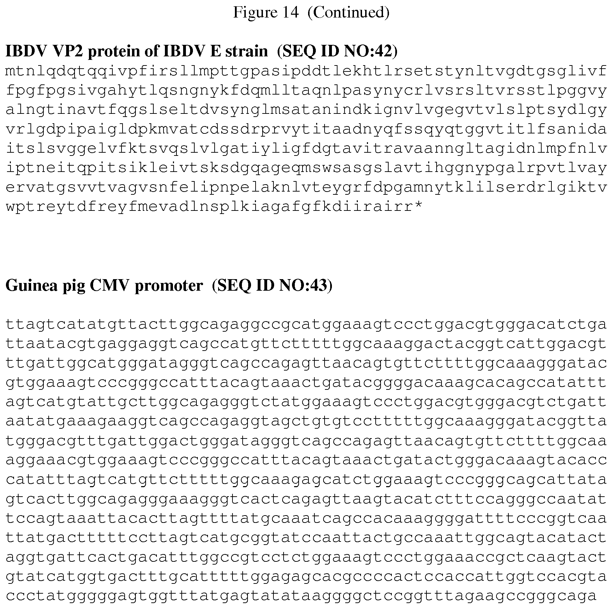

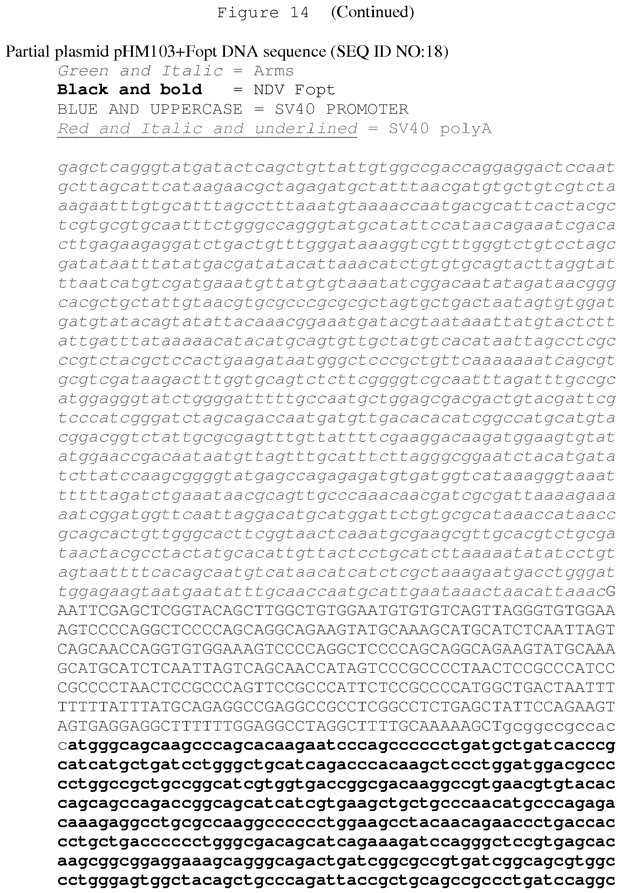

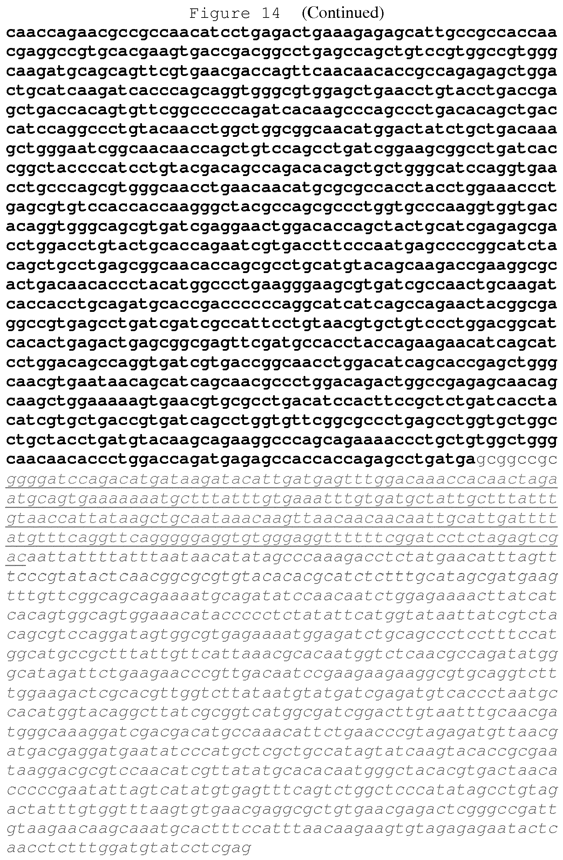

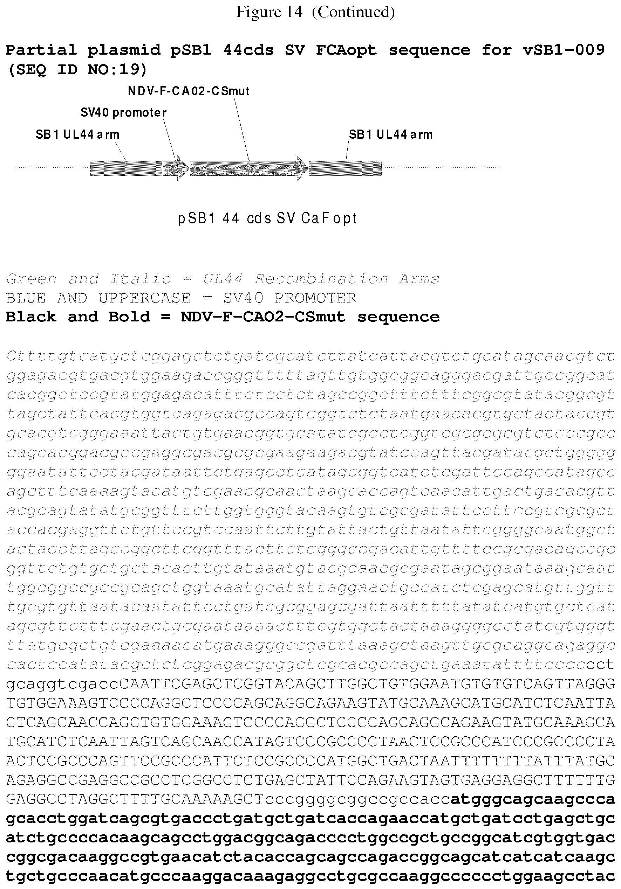

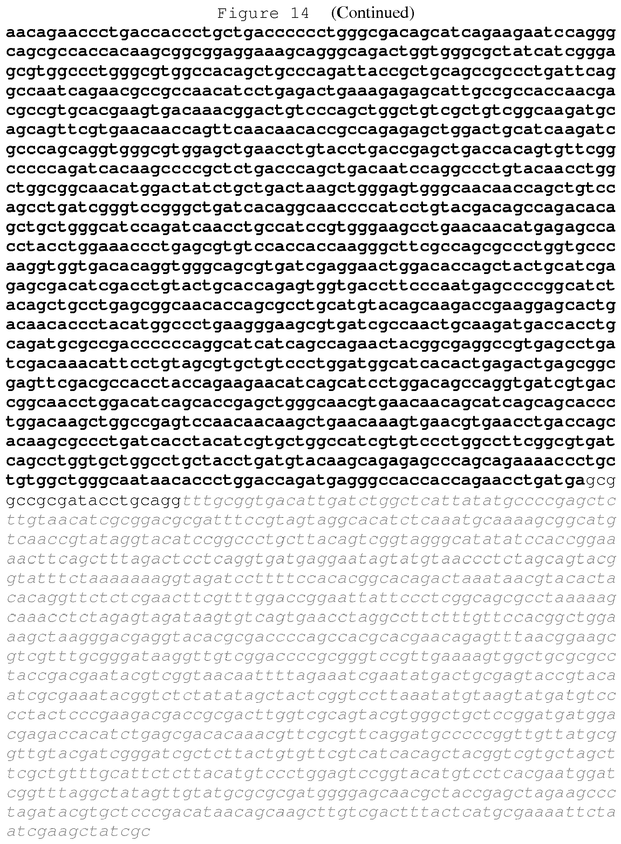

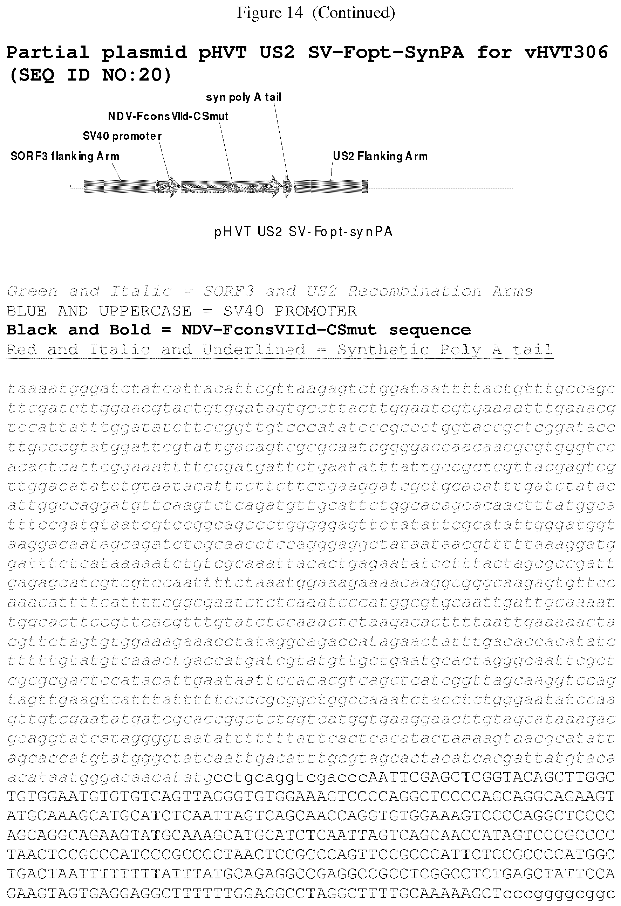

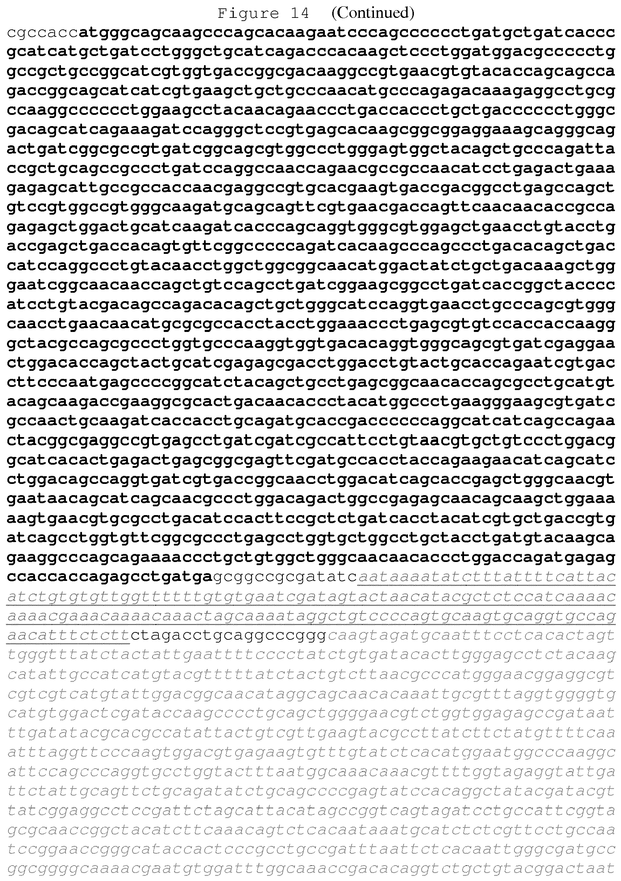

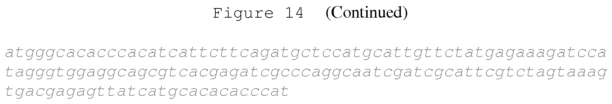

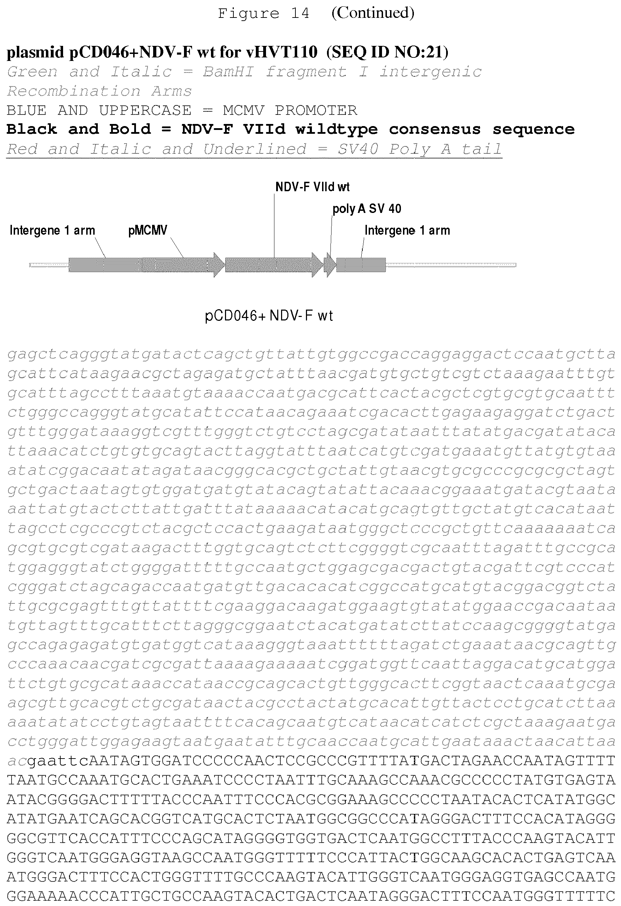

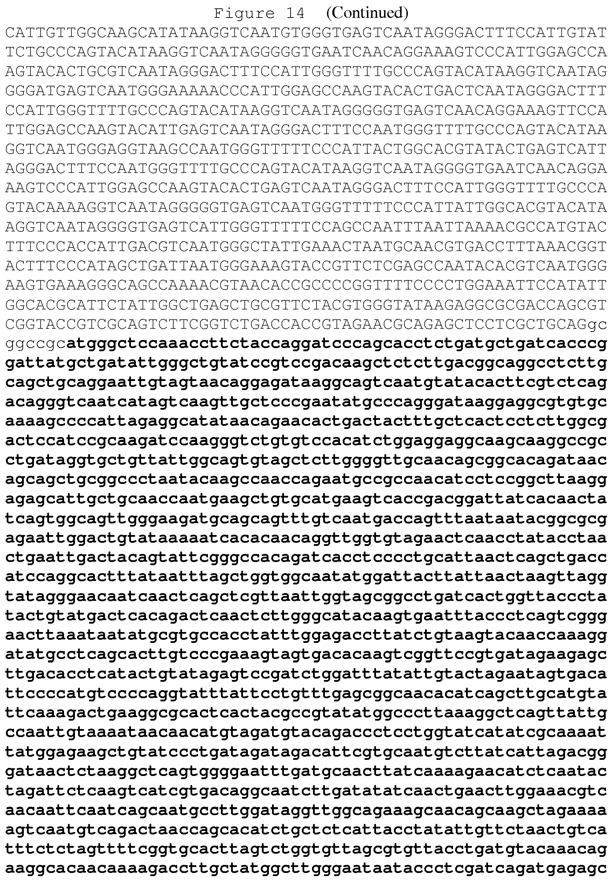

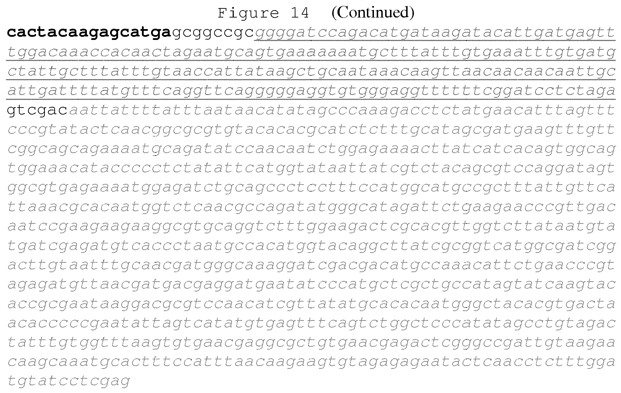

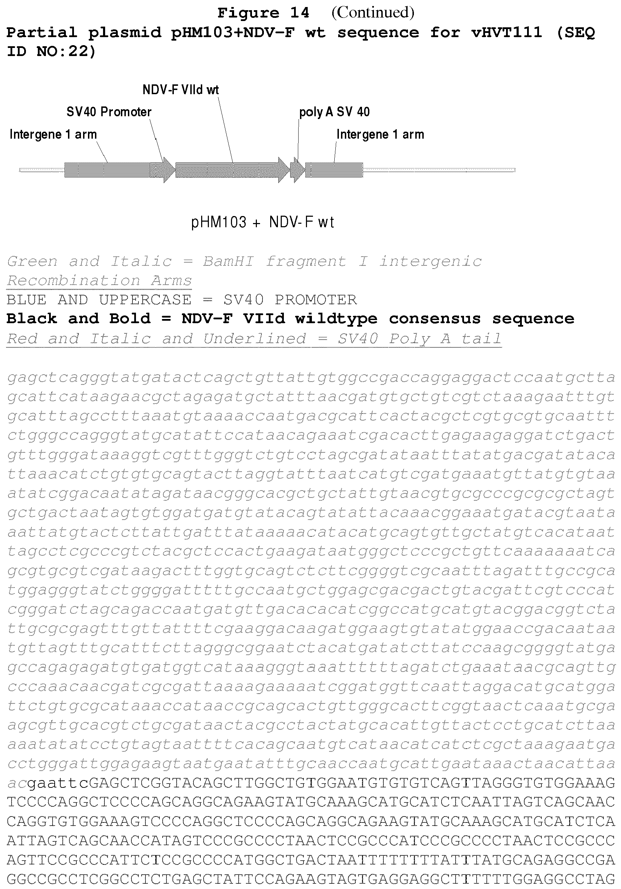

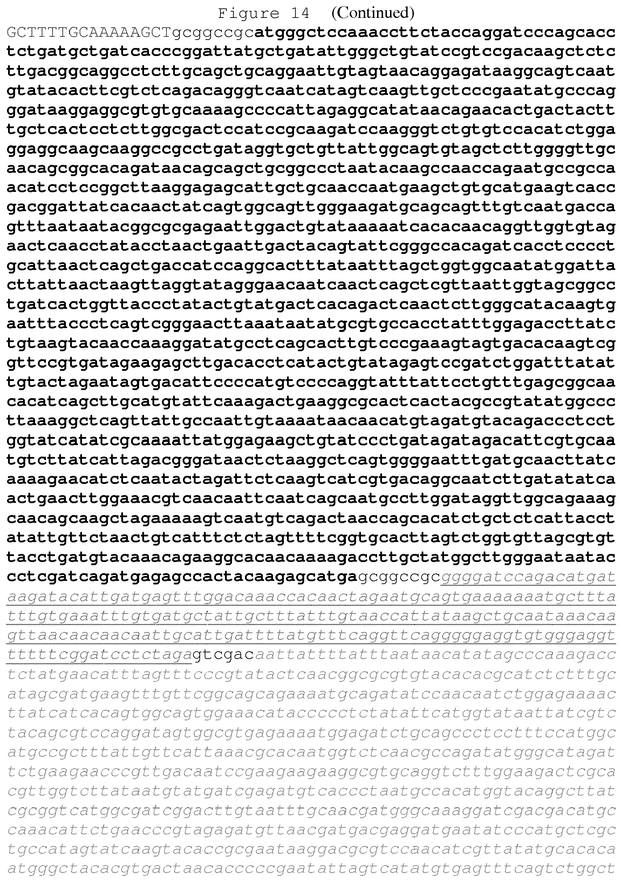

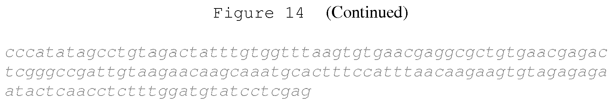

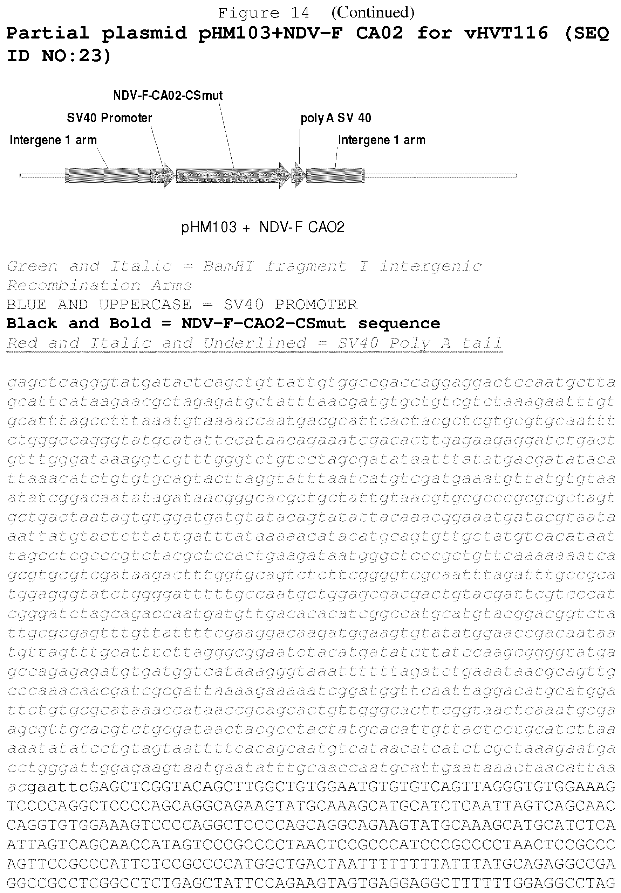

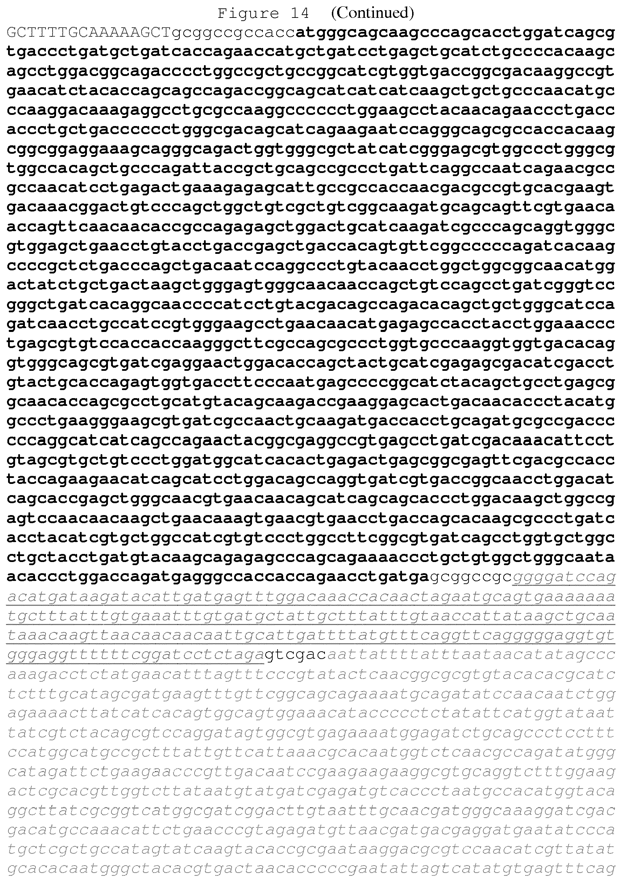

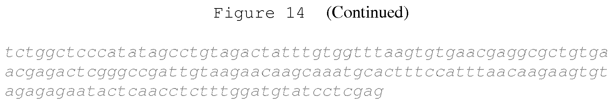

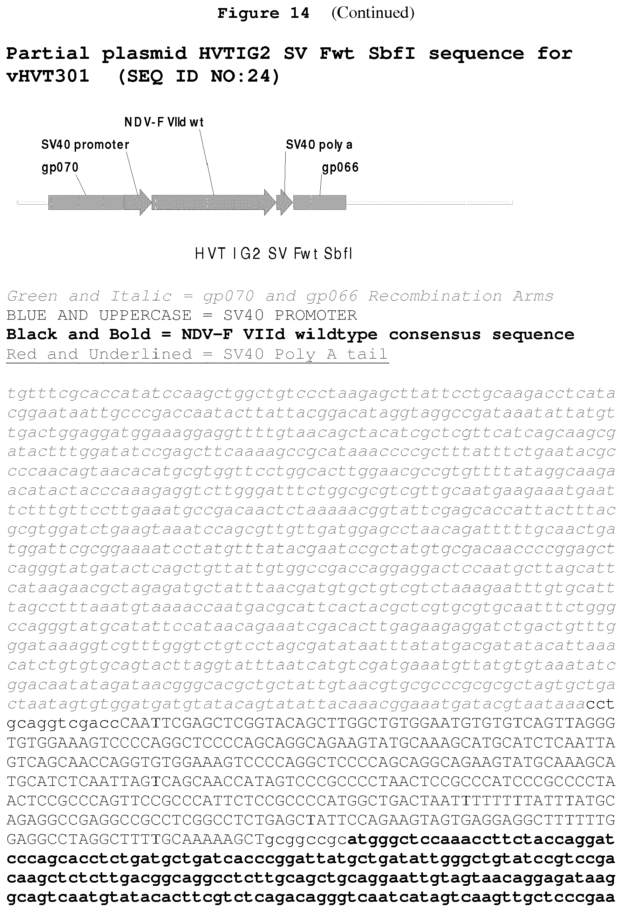

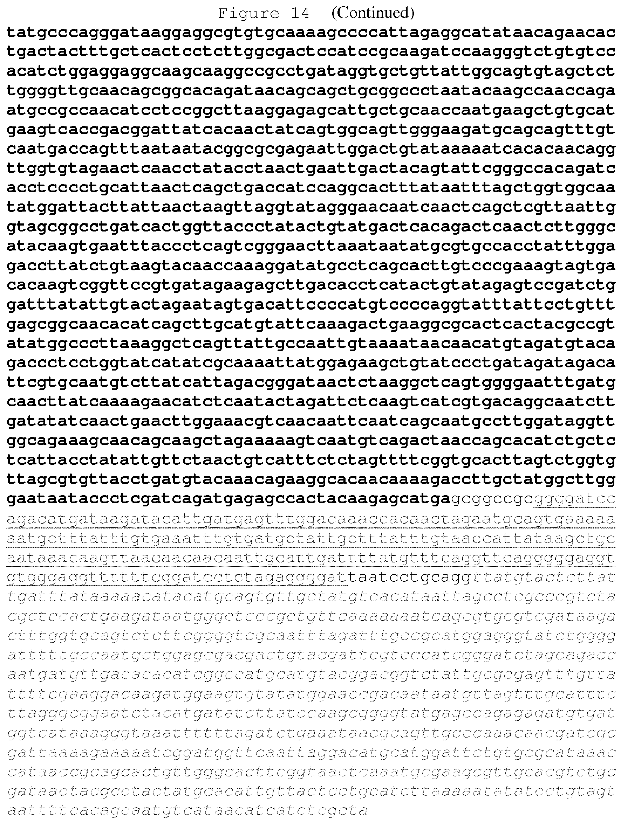

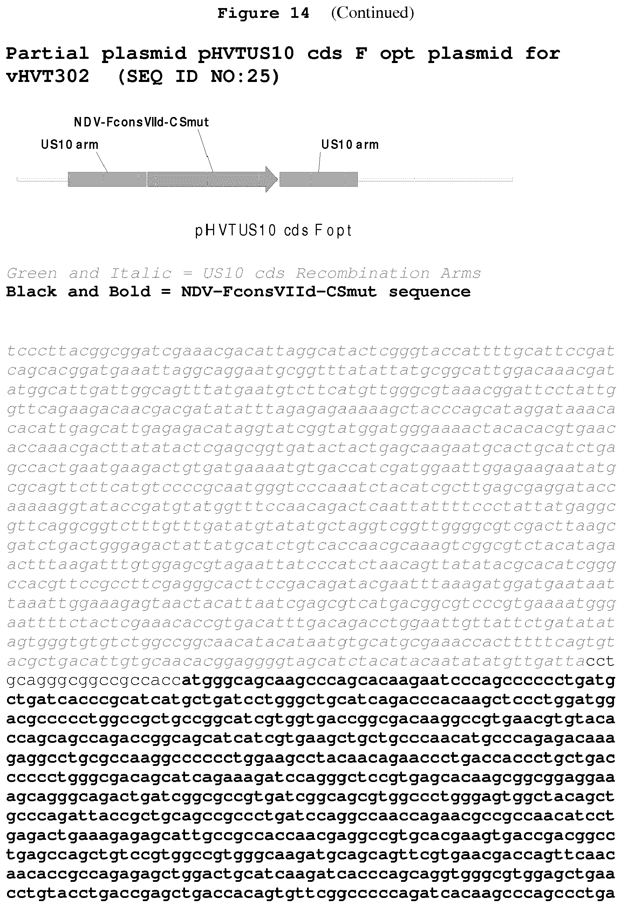

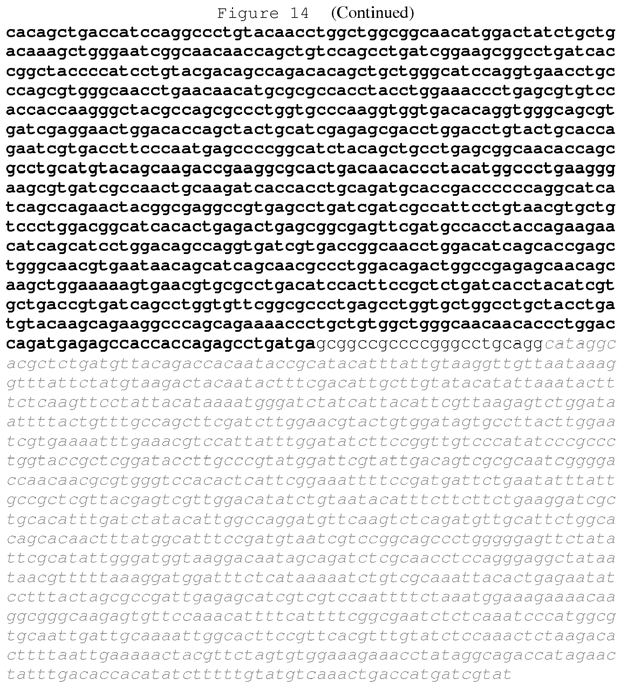

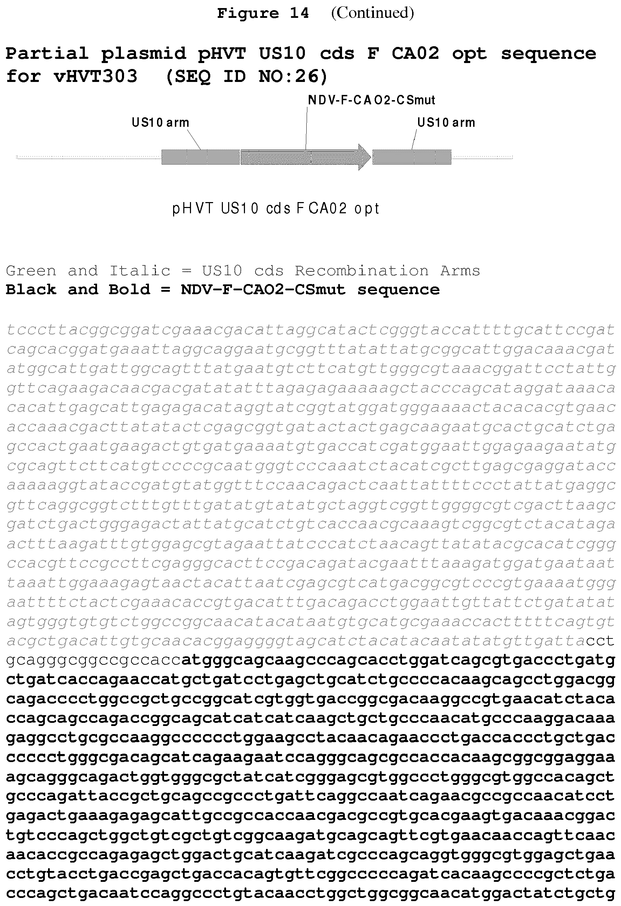

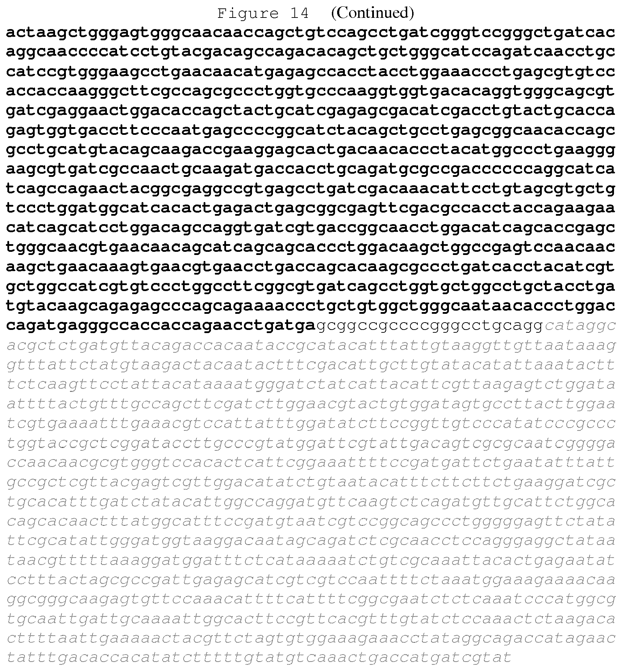

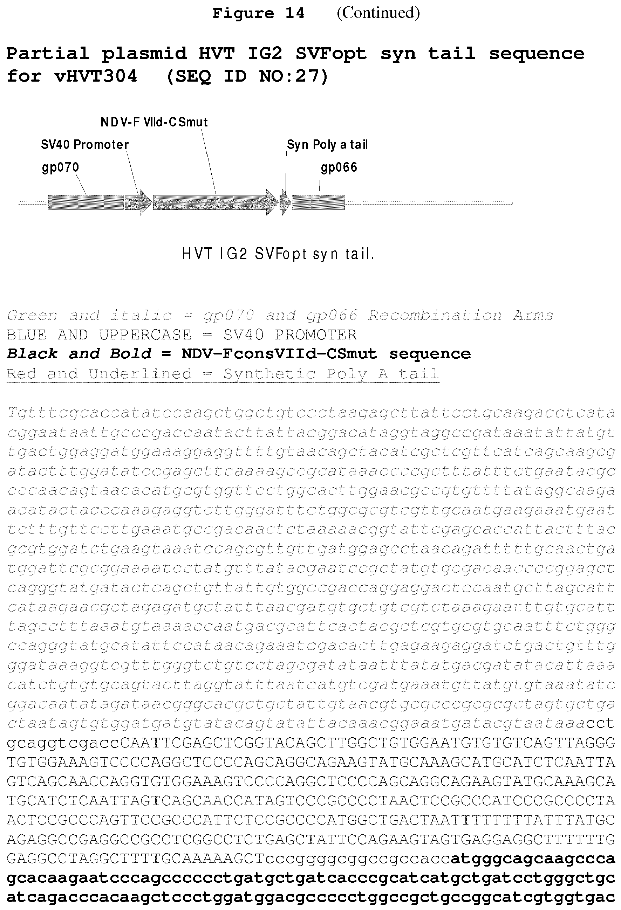

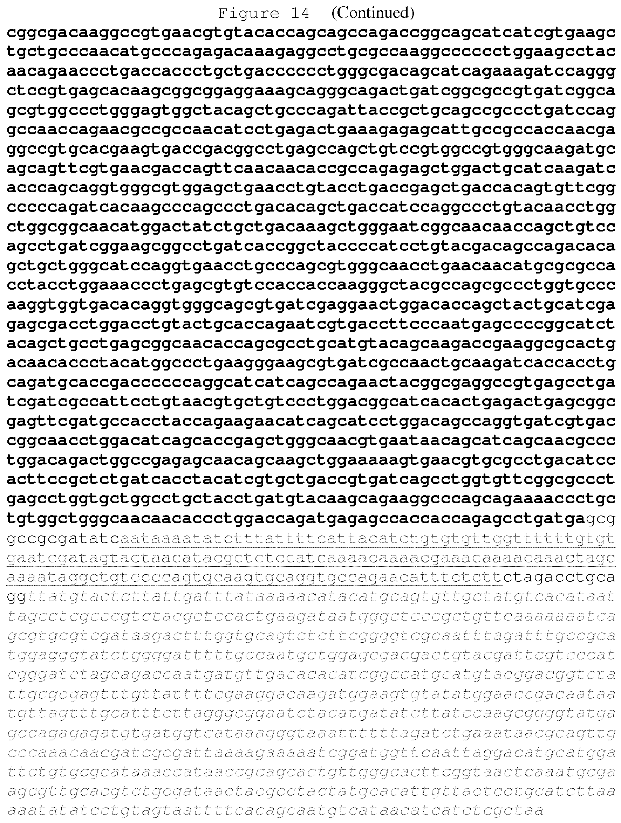

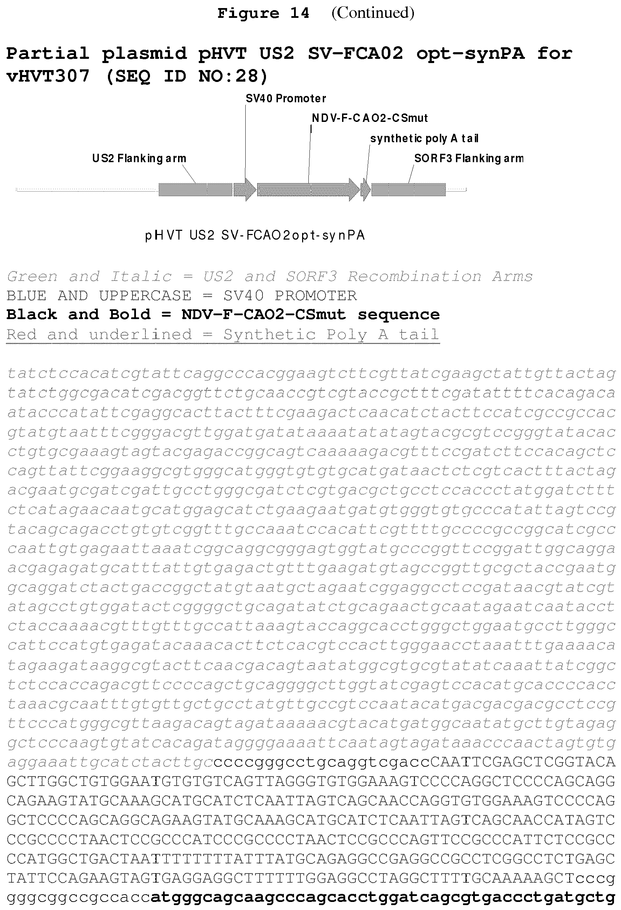

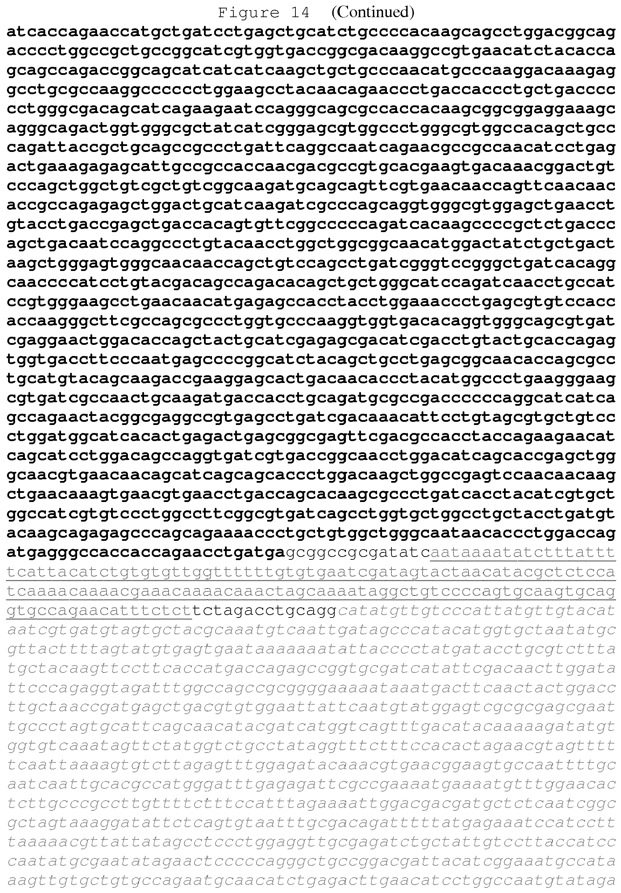

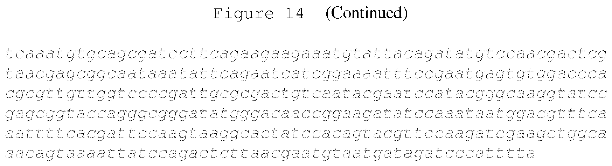

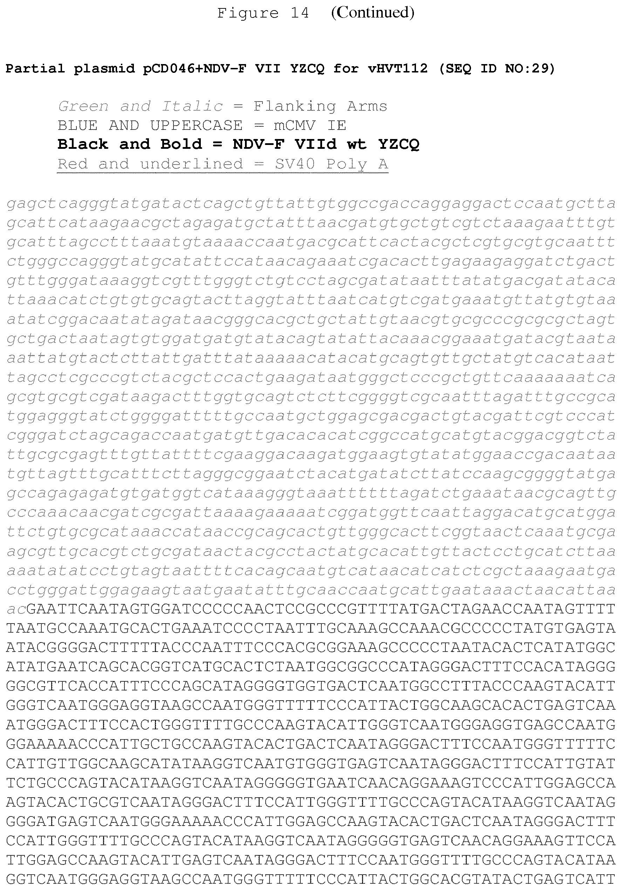

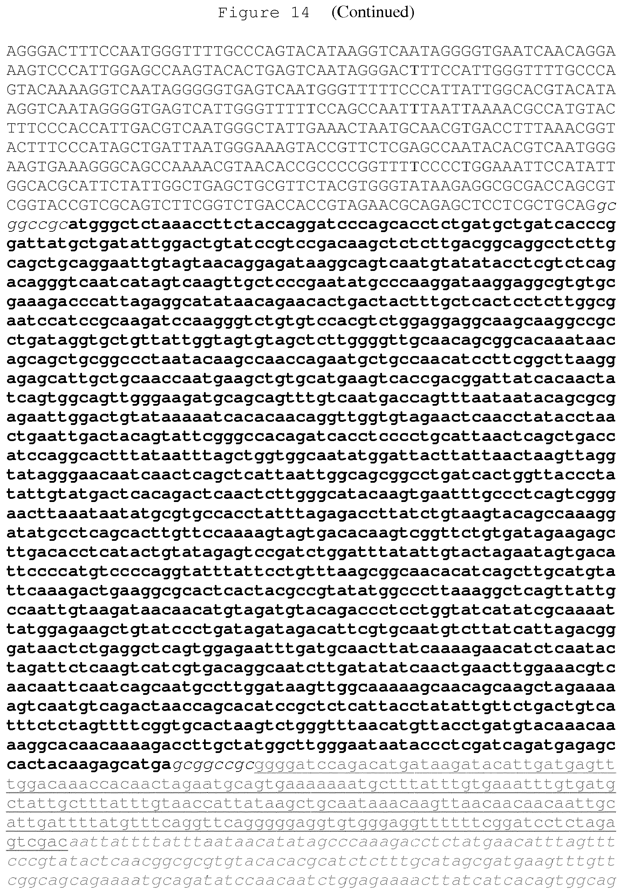

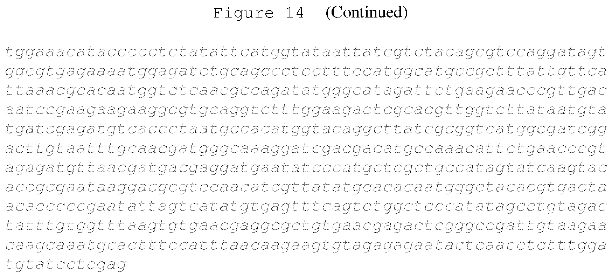

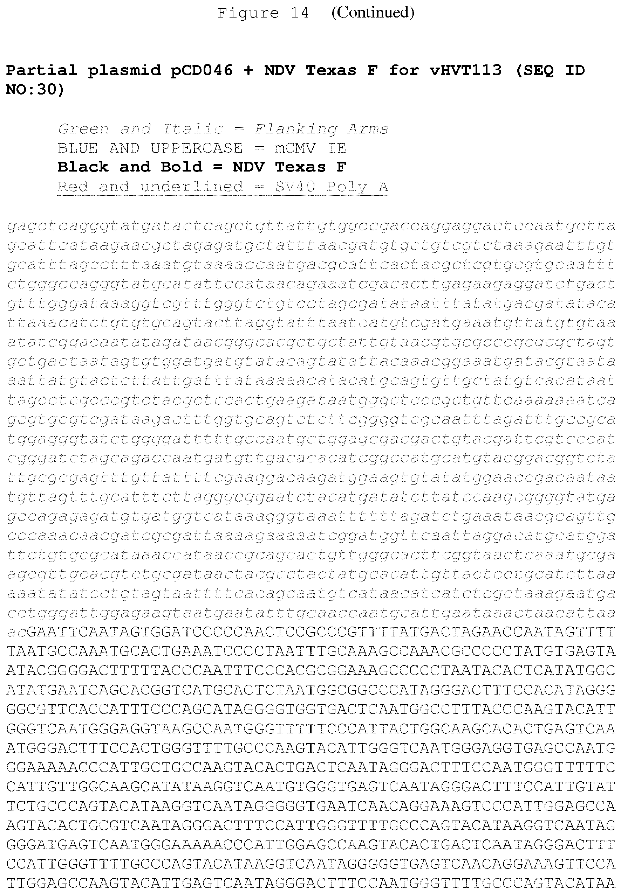

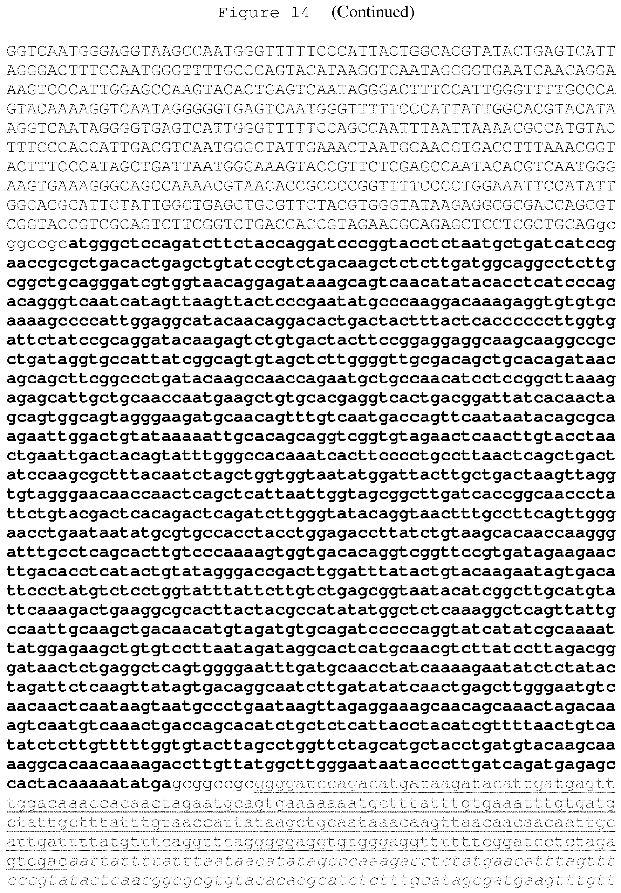

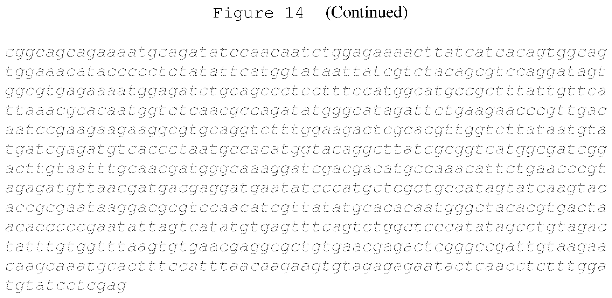

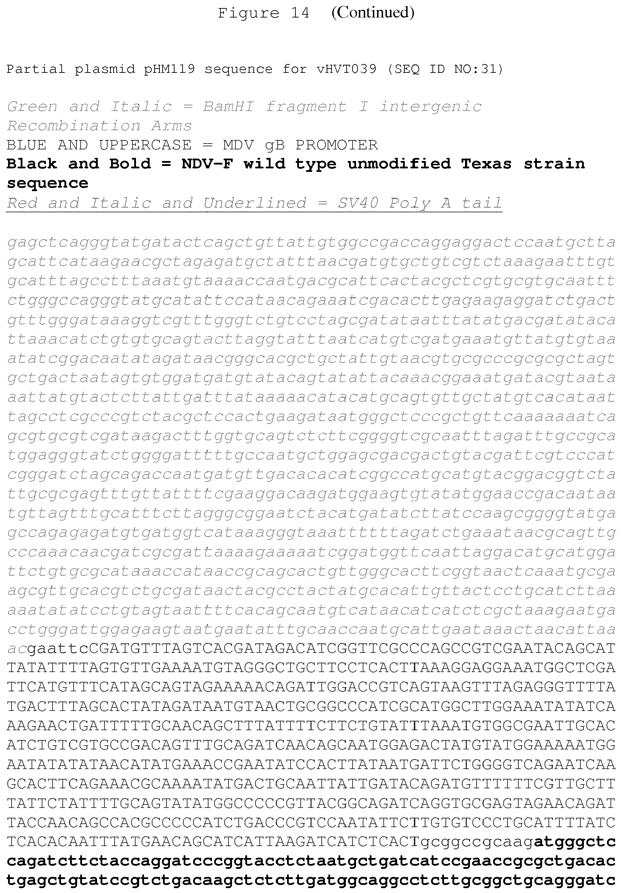

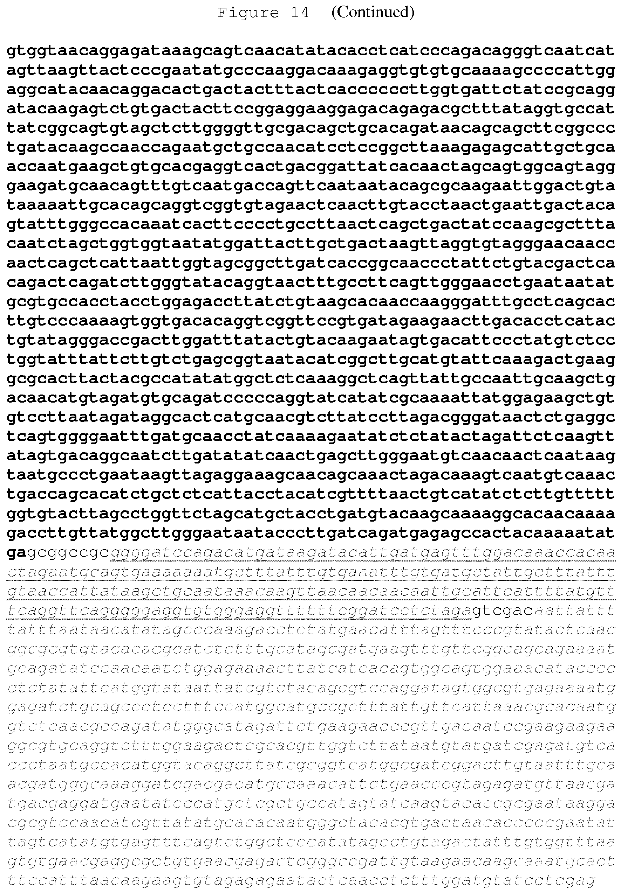

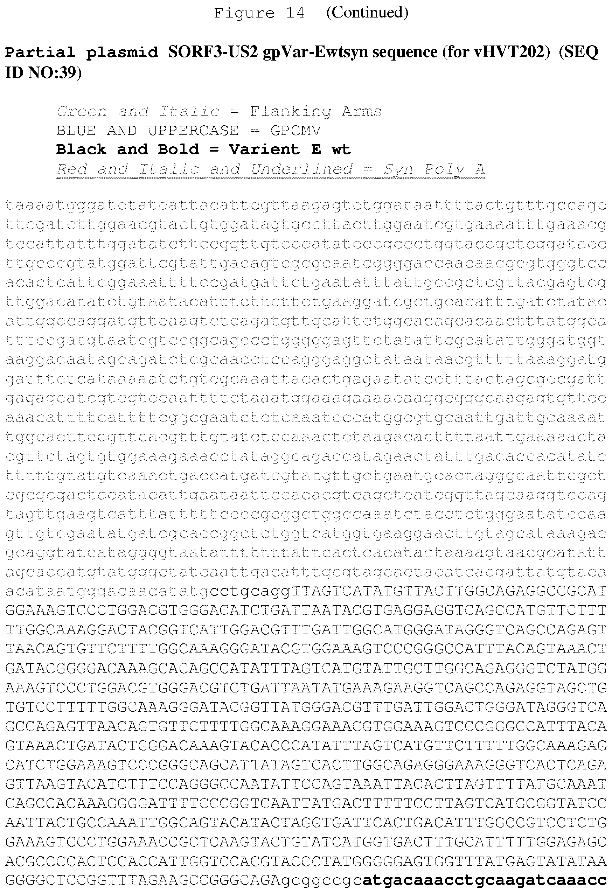

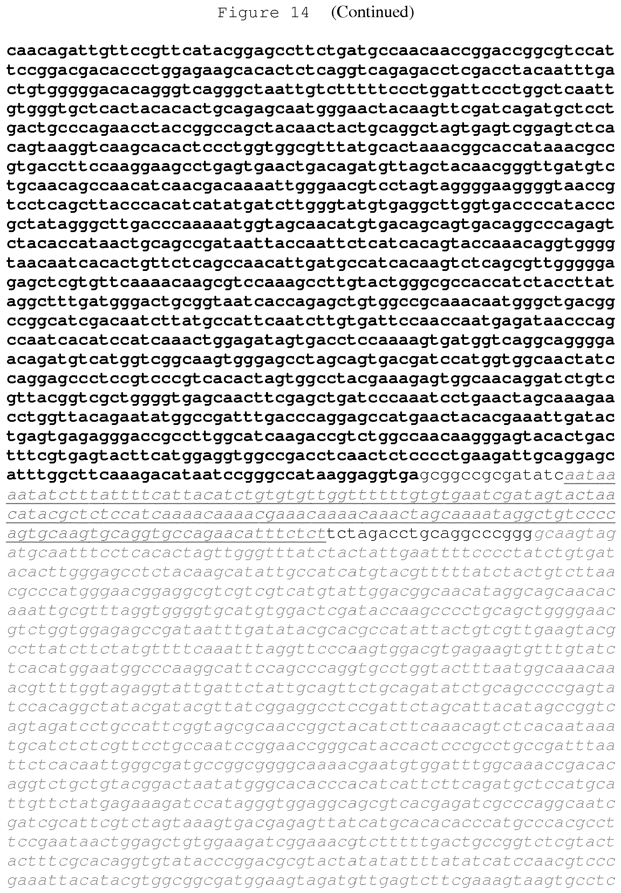

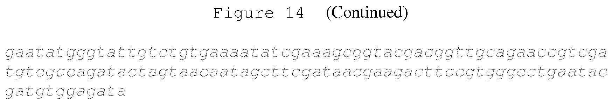

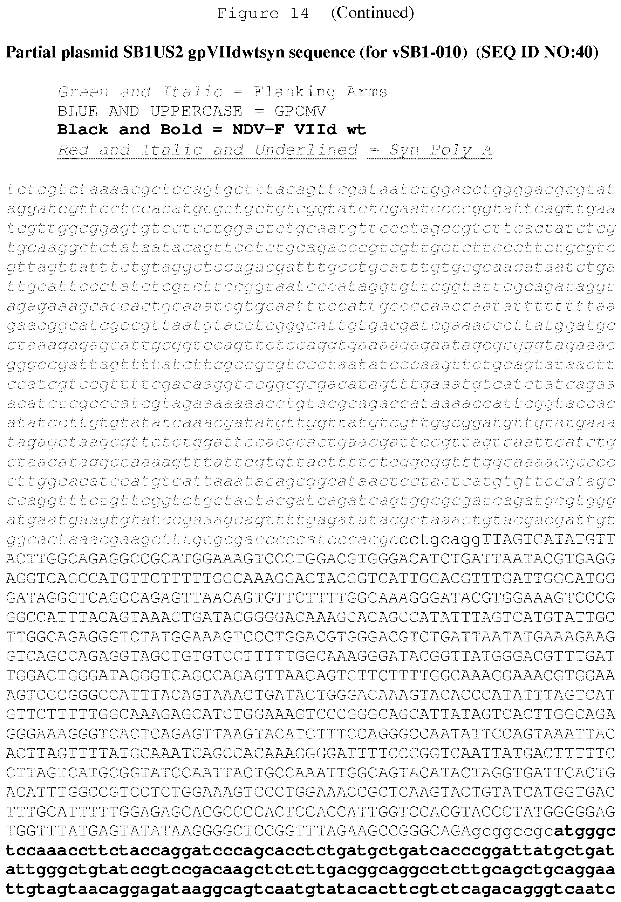

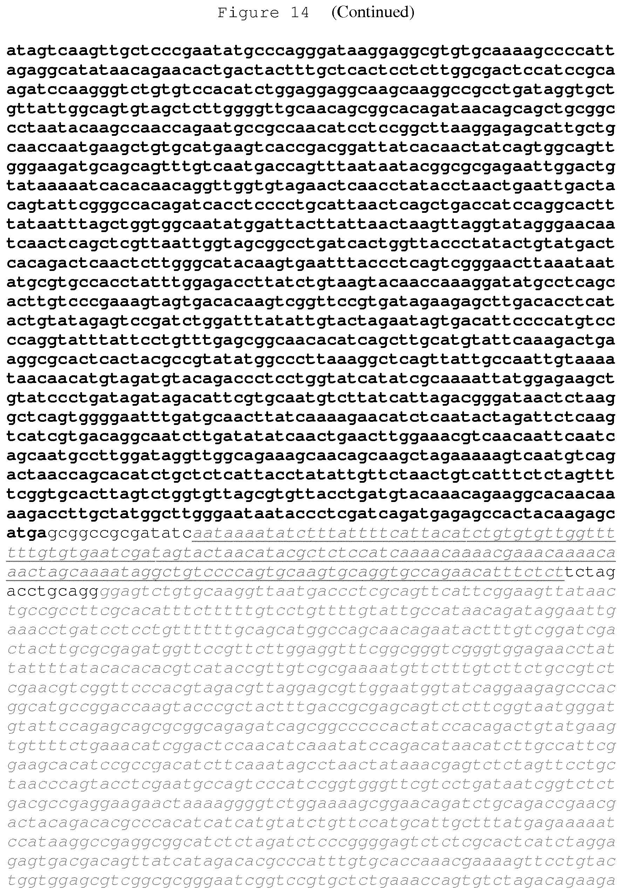

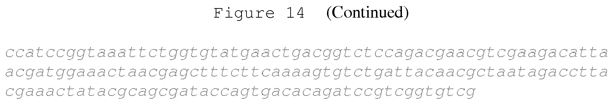

Figure 14 shows the DNA and protein sequences.

### DETAILED DESCRIPTION OF THE INVENTION

It is noted that in this disclosure and particularly in the claims, terms such as "comprises", "comprised", "comprising" and the like can have the meaning attributed to it in U.S. Patent law; e.g., they can mean "includes", "included", "including", and the like; and that terms such as "consisting essentially of" and "consists essentially of' have the meaning ascribed to them in U.S. Patent law, e.g., they allow for elements not explicitly recited, but exclude elements that are found in the prior art or that affect a basic or novel characteristic of the invention.

Unless otherwise noted, technical terms are used according to conventional usage. Definitions of common terms in molecular biology may be found in Benjamin Lewin, Genes V. published by Oxford University Press, 1994 (ISBN 0-19-854287-9); Kendrew et al. (eds.), The Encyclopedia of Molecular Biology, published by Blackwell Science Ltd., 1994 (ISBN 0-632-02182-9); and Robert A. Meyers (ed.), Molecular Biology and Biotechnology: a Comprehensive Desk Reference, published by VCH Publishers, Inc., 1995 (ISBN 1-56081-569-8).

The singular terms "a," "an," and "the" include plural referents unless context clearly indicates otherwise. Similarly, the word "or" is intended to include "and" unless the context clearly indicate otherwise. The word "or" means any one member of a particular list and also includes any combination of members of that list.

The term "animal" is used herein to include all mammals, birds and fish. The animal as used herein may be selected from the group consisting of equine (e.g., horse), canine (e.g., dogs, wolves, foxes, coyotes, jackals), feline (e.g., lions, tigers, domestic cats, wild cats, other big cats, and other felines including cheetahs and lynx), bovine (e.g., cattle), swine (e.g., pig), ovine (e.g., sheep, goats, lamas, bisons), avian (e.g., chicken, duck, goose, turkey, quail, pheasant, parrot, finches, hawk, crow, ostrich, emu and cassowary), primate (e.g., prosimian, tarsier, monkey, gibbon, ape), humans, and fish. The term "animal" also includes an individual animal in all stages of development, including embryonic and fetal stages.

The terms "polypeptide" and "protein" are used interchangeably herein to refer to a polymer of consecutive amino acid residues.

The term "nucleic acid", "nucleotide", and "polynucleotide" are used interchangeably and refer to RNA, DNA, cDNA, or cRNA and derivatives thereof, such as those containing modified backbones. It should be appreciated that the invention provides polynucleotides comprising sequences complementary to those described herein. The "polynucleotide" contemplated in the present invention includes both the forward strand (5' to 3') and reverse complementary strand (3' to 5'). Polynucleotides according to the invention can be prepared in different ways (e.g. by chemical synthesis, by gene cloning etc.) and can take various forms (e.g. linear or branched, single or double stranded, or a hybrid thereof, primers, probes etc.).

The term "genomic DNA" or "genome" is used interchangeably and refers to the heritable genetic information of a host organism. The genomic DNA comprises the DNA of the nucleus (also referred to as chromosomal DNA) but also the DNA of the plastids (e.g., chloroplasts) and other cellular organelles (e.g., mitochondria). The genomic DNA or genome contemplated in the present invention also refers to the RNA of a virus. The RNA may be a positive strand or a negative strand RNA. The term "genomic DNA" contemplated in the present invention includes the genomic DNA containing sequences complementary to those described herein. The term "genomic DNA" also refers to messenger RNA (mRNA), complementary DNA (cDNA), and complementary RNA (cRNA).

The term "gene" is used broadly to refer to any segment of polynucleotide associated with a biological function. Thus, genes or polynucleotides include introns and exons as in genomic sequence, or just the coding sequences as in cDNAs , such as an open reading frame (ORF), starting from the start codon (methionine codon) and ending with a termination signal (stop codon). Genes and polynucleotides can also include regions that regulate their expression, such as transcription initiation, translation and transcription termination. Thus, also included are promoters and ribosome binding regions (in general these regulatory elements lie approximately between 60 and 250 nucleotides upstream of the start codon of the coding sequence or gene; Doree S M *et al.;* Pandher K *et al.;* Chung J Y *et al.*)*,* transcription terminators (in general the terminator is located within approximately 50 nucleotides downstream of the stop codon of the coding sequence or gene; Ward C K *et al.*)*.* Gene or polynucleotide also refers to a nucleic acid fragment that expresses mRNA or functional RNA, or encodes a specific protein, and which includes regulatory sequences.

The term "heterologous DNA" as used herein refers to the DNA derived from a different organism, such as a different cell type or a different species from the recipient. The term also refers a DNA or fragment thereof on the same genome of the host DNA wherein the heterologous DNA is inserted into a region of the genome which is different from its original location.

As used herein, the term "antigen" or "immunogen" means a substance that induces a specific immune response in a host animal. The antigen may comprise a whole organism, killed, attenuated or live; a subunit or portion of an organism; a recombinant vector containing an insert with immunogenic properties; a piece or fragment of DNA capable of inducing an immune response upon presentation to a host animal; a polypeptide, an epitope, a hapten, or any combination thereof. Alternately, the immunogen or antigen may comprise a toxin or antitoxin.

The term "immunogenic protein or peptide" as used herein includes polypeptides that are immunologically active in the sense that once administered to the host, it is able to evoke an immune response of the humoral and/or cellular type directed against the protein. Preferably the protein fragment is such that it has substantially the same immunological activity as the total protein. Thus, a protein fragment according to the invention comprises or consists essentially of or consists of at least one epitope or antigenic determinant. An "immunogenic" protein or polypeptide, as used herein, includes the full-length sequence of the protein, analogs thereof, or immunogenic fragments thereof. By "immunogenic fragment" is meant a fragment of a protein which includes one or more epitopes and thus elicits the immunological response described above. Such fragments can be identified using any number of epitope mapping techniques, well known in the art. For example, linear epitopes may be determined by e.g., concurrently synthesizing large numbers of peptides on solid supports, the peptides corresponding to portions of the protein molecule, and reacting the peptides with antibodies while the peptides are still attached to the supports. Similarly, conformational epitopes are readily identified by determining spatial conformation of amino acids such as by, e.g., x-ray crystallography and 2-dimensional nuclear magnetic resonance.

The term "immunogenic protein or peptide" further contemplates deletions, additions and substitutions to the sequence, so long as the polypeptide functions to produce an immunological response as defined herein. The term "conservative variation" denotes the replacement of an amino acid residue by another biologically similar residue, or the replacement of a nucleotide in a nucleic acid sequence such that the encoded amino acid residue does not change or is another biologically similar residue. In this regard, particularly preferred substitutions will generally be conservative in nature, i.e., those substitutions that take place within a family of amino acids. For example, amino acids are generally divided into four families: (1) acidic--aspartate and glutamate; (2) basic-lysine, arginine, histidine; (3) non-polar--alanine, valine, leucine, isoleucine, proline, phenylalanine, methionine, tryptophan; and (4) uncharged polar--glycine, asparagine, glutamine, cysteine, serine, threonine, tyrosine. Phenylalanine, tryptophan, and tyrosine are sometimes classified as aromatic amino acids. Examples of conservative variations include the substitution of one hydrophobic residue such as isoleucine, valine, leucine or methionine for another hydrophobic residue, or the substitution of one polar residue for another polar residue, such as the substitution of arginine for lysine, glutamic acid for aspartic acid, or glutamine for asparagine, and the like; or a similar conservative replacement of an amino acid with a structurally related amino acid that will not have a major effect on the biological activity. Proteins having substantially the same amino acid sequence as the reference molecule but possessing minor amino acid substitutions that do not substantially affect the immunogenicity of the protein are, therefore, within the definition of the reference polypeptide. All of the polypeptides produced by these modifications are included herein. The term "conservative variation" also includes the use of a substituted amino acid in place of an unsubstituted parent amino acid provided that antibodies raised to the substituted polypeptide also immunoreact with the unsubstituted polypeptide.

The term "epitope" refers to the site on an antigen or hapten to which specific B cells and/or T cells respond. The term is also used interchangeably with "antigenic determinant" or "antigenic determinant site". Antibodies that recognize the same epitope can be identified in a simple immunoassay showing the ability of one antibody to block the binding of another antibody to a target antigen.

An "immunological response" to a composition or vaccine is the development in the host of a cellular and/or antibody-mediated immune response to a composition or vaccine of interest. Usually, an "immunological response" includes but is not limited to one or more of the following effects: the production of antibodies, B cells, helper T cells, and/or cytotoxic T cells, directed specifically to an antigen or antigens included in the composition or vaccine of interest. Preferably, the host will display either a therapeutic or protective immunological response such that resistance to new infection will be enhanced and/or the clinical severity of the disease reduced. Such protection will be demonstrated by either a reduction or lack of symptoms normally displayed by an infected host, a quicker recovery time and/or a lowered viral titer in the infected host.

The terms "recombinant" and "genetically modified" are used interchangeably and refer to any modification, alteration or engineering of a polynucleotide or protein in its native form or structure, or any modification, alteration or engineering of a polynucleotide or protein in its native environment or surrounding. The modification, alteration or engineering of a polynucleotide or protein may include, but is not limited to, deletion of one ore more nucleotides or amino acids, deletion of an entire gene, codon-optimization of a gene, conservative substitution of amino acids, insertion of one or more heterologous polynucleotides.

The term "double HVT construct" or "double HVT vector" refers to an HVT viral vector comprising two heterologous polynucleotides.

The terms "polyvalent vaccine or composition", "combination or combo vaccine or composition" and "multivalent vaccine or composition" are used interchangeably to refer to a composition or vaccine containing more than one composition or vaccines. The polyvalent vaccine or composition may contain two, three, four or more compositions or vaccines. The polyvalent vaccine or composition may comprise recombinant viral vectors, active or attenuated or killed wild-type viruses, or a mixture of recombinant viral vectors and wild-type viruses in active or attenuated or killed forms.

One embodiment of the invention provides a recombinant HVT viral vector comprising one or more heterologous polynucleotides coding for and expressing at least one antigen or polypeptide of an avian pathogen. The HVT strains used for the recombinant viral vector may be any HVT strains, including, but not limited to, the HVT strain FC126 (Igarashi T. et al., J. Gen. Virol. 70, 1789-1804, 1989).

Another embodiment of the invention provides a recombinant SB-1 viral vector comprising one or more heterologous polynucleotides coding for and expressing at least one antigen or polypeptide of an avian pathogen. The SB-1 strains may be any SB-1 strains, including, but not limited to, the commercial Marek's Disease Vaccine (SB-1 vaccine) (Merial Select Inc., Gainesville, GA 30503, USA), the SB-1 strain having the genome sequence as defined by GenBank Accession Number HQ840738.1.

The genes coding for an antigen or polypeptide may be those coding for Newcastle Disease Virus fusion protein (NDV-F), Newcastle Disease Virus hemagglutinin neuraminidase (NDV-HN), Marek's Disease Virus glycoprotein C (gC), Marek's Disease Virus glycoprotein B (gB), Marek's Disease Virus glycoprotein E (gE), Marek's Disease Virus glycoprotein I (gI), Marek's Disease Virus glycoprotein H (gH) or Marek's Disease Virus glycoprotein L (gL), Infectious Bursal Disease Virus (IBDV) VP2, IBDV VPX, IBDV VP3, IBDV VP4, ILTV glycoprotein B, ILTV glycoprotein I, ILTV UL32, ILTV glycoprotein D, ILTV glycoprotein E, ILTV glycoprotein C, influenza hemaglutinin (HA), influenza neuraminidase (NA), protective genes derived from Mycoplasma gallisepticum (MG), or Mycoplasma synoviae (MS), or combinations thereof. The antigen or polypeptide may be any antigen from the poultry pathogen selected form the group consisting of avian encephalomyelitis virus, avian reovirus, avian paramyxovirus, avian metapneumovirus, avian influenza virus, avian adenovirus, fowl pox virus, avian coronavirus, avian rotavirus, chick anemia virus, avian astrovirus, avian parvovirus, coccidiosis (*Eimeria sp.*)*, Campylobacter sp., Salmonella sp., Pasteurella sp., Avibacterium sp., Mycoplasma gallisepticum, Mycoplasma synoviae, Clostridium sp.,* and *E. coli.*

Moreover, homologs of aforementioned antigen or polynucleotides are intended to be within the scope of the present invention. As used herein, the term "homologs" includes orthologs, analogs and paralogs. The term "analogs" refers to two polynucleotides or polypeptides that have the same or similar function, but that have evolved separately in unrelated organisms. The term "orthologs" refers to two polynucleotides or polypeptides from different species, but that have evolved from a common ancestral gene by speciation. Normally, orthologs encode polypeptides having the same or similar functions. The term "paralogs" refers to two polynucleotides or polypeptides that are related by duplication within a genome. Paralogs usually have different functions, but these functions may be related. Analogs, orthologs, and paralogs of a wild-type polypeptide can differ from the wild-type polypeptide by post-translational modifications, by amino acid sequence differences, or by both. In particular, homologs of the invention will generally exhibit at least 80-85%, 85-90%, 90-95%, or 95%, 96%, 97%, 98%, 99% sequence identity, with all or part of the polynucleotide or polypeptide sequences of antigens described above, and will exhibit a similar function.

In one embodiment, the present invention provides a recombinant HVT or SB-1 viral vector comprising one or more heterologous polynucleotides coding for and expressing the NDV-F antigen or polypeptide. In one aspect of the embodiment, the NDV-F antigen or polypeptide has at least 70%, 75%, 80%, 85%, 90%, 95%, 96%, 97%, 98% or 99% sequence identity to a polypeptide having the sequence as set forth in SEQ ID NO:2, 4, 6, 33, 35, or 37, or a conservative variant, an allelic variant, a homolog or an immunogenic fragment comprising at least eight or at least ten consecutive amino acids of one of these polypeptides, or a combination of these polypeptides. In another aspect of the embodiment, the heterologous polynucleotide encoding an NDV-F antigen or polypeptide having at least 70%, 75%, 80%, 85%, 90%, 95%, 96%, 97%, 98% or 99% sequence identity to a polypeptide having the sequence as set forth in SEQ ID NO:2, 4, 6, 33, 35, or 37. In yet another aspect of the embodiment, the heterologous polynucleotide has at least 70%, 75%, 80%, 85%, 90%, 95%, 96%, 97%, 98% or 99% sequence identity to a polynucleotide having the sequence as set forth in SEQ ID NO:1, 3, 5, 32, 34, or 36.

Variants include allelic variants. The term "allelic variant" refers to a polynucleotide or a polypeptide containing polymorphisms that lead to changes in the amino acid sequences of a protein and that exist within a natural population (e.g., a virus species or variety). Such natural allelic variations can typically result in 1- 5% variance in a polynucleotide or a polypeptide. Allelic variants can be identified by sequencing the nucleic acid sequence of interest in a number of different species, which can be readily carried out by using hybridization probes to identify the same gene genetic locus in those species. Any and all such nucleic acid variations and resulting amino acid polymorphisms or variations that are the result of natural allelic variation and that do not alter the functional activity of gene of interest, are intended to be within the scope of the invention.

The term "identity" with respect to sequences can refer to, for example, the number of positions with identical nucleotides or amino acids divided by the number of nucleotides or amino acids in the shorter of the two sequences wherein alignment of the two sequences can be determined in accordance with the Wilbur and Lipman algorithm (Wilbur and Lipman). The sequence identity or sequence similarity of two amino acid sequences, or the sequence identity between two nucleotide sequences can be determined using Vector NTI software package (Invitrogen, 1600 Faraday Ave., Carlsbad, CA). When RNA sequences are said to be similar, or have a degree of sequence identity or homology with DNA sequences, thymidine (T) in the DNA sequence is considered equal to uracil (U) in the RNA sequence. Thus, RNA sequences are within the scope of the invention and can be derived from DNA sequences, by thymidine (T) in the DNA sequence being considered equal to uracil (U) in RNA sequences.

The polynucleotides of the disclosure include sequences that are degenerate as a result of the genetic code, e.g., optimized codon usage for a specific host. As used herein, "optimized" refers to a polynucleotide that is genetically engineered to increase its expression in a given species. To provide optimized polynucleotides coding for NDV-F polypeptides, the DNA sequence of the NDV-F protein gene can be modified to 1) comprise codons preferred by highly expressed genes in a particular species; 2) comprise an A+T or G+C content in nucleotide base composition to that substantially found in said species; 3) form an initiation sequence of said species; or 4) eliminate sequences that cause destabilization, inappropriate polyadenylation, degradation and termination of RNA, or that form secondary structure hairpins or RNA splice sites. Increased expression of NDV F protein in said species can be achieved by utilizing the distribution frequency of codon usage in eukaryotes and prokaryotes, or in a particular species. The term "frequency of preferred codon usage" refers to the preference exhibited by a specific host cell in usage of nucleotide codons to specify a given amino acid. There are 20 natural amino acids, most of which are specified by more than one codon. Therefore, all degenerate nucleotide sequences are included in the disclosure as long as the amino acid sequence of the NDV-F polypeptide encoded by the nucleotide sequence is functionally unchanged.

Successful expression of the heterologous polynucleotides by the recombinant/modified infectious virus requires two conditions. First, the heterologous polynucleotides must be inserted or introduced into a region of the genome of the virus in order that the modified virus remains viable. The second condition for expression of inserted heterologous polynucleotides is the presence of a regulatory sequences allowing expression of the gene in the viral background (for instance: promoter, enhancer, donor and acceptor splicing sites and intron, Kozak translation initiation consensus sequence, polyadenylation signals, untranslated sequence elements).

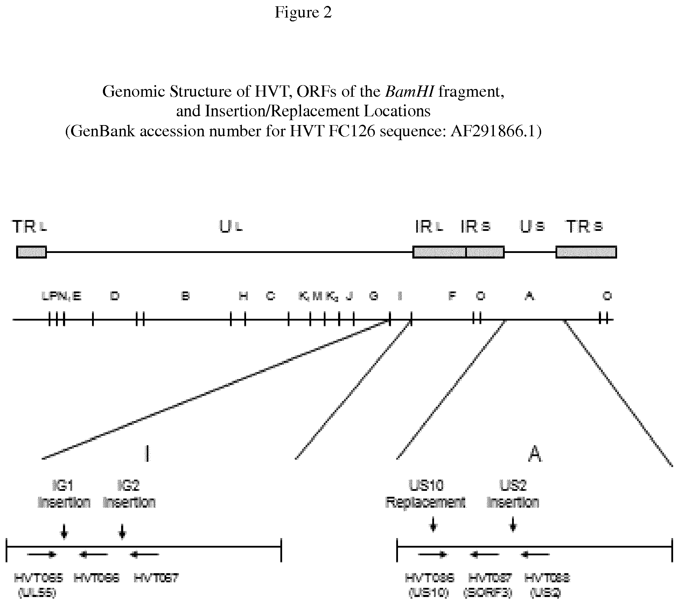

The insertion site may be any non-essential region of the HVT genome, including, but not limited to, the region between the ATG of ORF UL55 and the junction of UL with the adjacent repeat region (US5,980,906), the IG1 locus, the IG2 locus, the IG3 locus, the UL43 locus, the US10 locus, the SORF3/US2 locus (see FIG. 2).

In general, it is advantageous to employ a strong promoter functional in eukaryotic cells. The promoters include, but are not limited to, an immediate early cytomegalovirus (CMV) promoter, guinea pig CMV promoter, an SV40 promoter, Pseudorabies Virus promoters such as that of glycoprotein X promoter, Herpes Simplex Virus-1 such as the alpha 4 promoter, Marek's Disease Viruses (including MDV-1, MDV-2 and HVT) promoters such as those driving glycoproteins gC, gB, gE, or gI expression, Infectious Laryngotracheitis Virus promoters such as those of glycoprotein gB, gE, gI, gD genes, or other herpesvirus promoters.

One embodiment of the invention provides a recombinant HVT vector comprising a heterologous polynucleotide coding for and expressing the NDV-F antigen or polypeptide. In one aspect of the embodiment, the polynucleotide encoding the NDV-F polypeptide is operably linked to the SV40 promoter having the sequence as set forth in SEQ ID NO:9 and therefore the expression of the NDV-F antigen or polypeptide is regulated by the SV40 promoter. In another aspect of the embodiment, the expression of NDV-F antigen or polypeptide is regulated by the SV40 polyA signal having the sequence as set forth in SEQ ID NO:11. In yet another aspect of the embodiment, the polynucleotide encoding the NDV-F polypeptide is operably linked to the MDV gB promoter having the sequence as set forth in SEQ ID NO:38 and therefore the expression of the NDV-F antigen or polypeptide is regulated by the MDV gB promoter.

Another embodiment of the invention provides a recombinant double HVT vector comprising a first heterologous polynucleotide coding for and expressing the NDV-F antigen or polypeptide and a second polynucleotide coding for and expressing the IBDV VP2 antigen or polypeptide. In one aspect of the embodiment, the NDV-F antigen or polypeptide has at least 70%, 75%, 80%, 85%, 90%, 95%, 96%, 97%, 98% or 99% sequence identity to a polypeptide having the sequence as set forth in SEQ ID NO:2, 4, 6, 33, 35, or 37. In another aspect of the embodiment, the IBDV VP2 antigen or polypeptide has at least 70%, 75%, 80%, 85%, 90%, 95%, 96%, 97%, 98% or 99% sequence identity to a polypeptide having the sequence as set forth in SEQ ID NO:8 or 42. In another aspect, the polynucleotide encoding the NDV-F polypeptide is operably linked to the SV40 promoter having the sequence as set forth in SEQ ID NO:9 and the expression of NDV-F antigen or polypeptide is regulated by the SV40 promoter. In yet another aspect, the expression of NDV-F antigen or polypeptide is regulated by the SV40 polyA signal having the sequence as set forth in SEQ ID NO:11, or the synthetic polyA signal having the sequence as set forth in SEQ ID NO:12. In another aspect, the expression of IBDV VP2 antigen or polypeptide is regulated by the CMV-IE promoter having the sequence as set forth in SEQ ID NO:10 and the SV40 polyA signal having the sequence as set forth in SEQ ID NO:11.

Yet another embodiment of the invention provides a recombinant double HVT vector comprising two polynucleotides coding for and expressing the IBDV VP2 antigens or polypeptides. In one aspect of the embodiment, the IBDV VP2 antigen or polypeptide has at least 70%, 75%, 80%, 85%, 90%, 95%, 96%, 97%, 98% or 99% sequence identity to a polypeptide having the sequence as set forth in SEQ ID NO:8 or 42. In one aspect, the polynucleotide encoding a first IBDV VP2 antigen or polypeptide is operably linked to the CMV-IE promoter having the sequence as set forth in SEQ ID NO:10, and the polynucleotide encoding a second IBDV VP2 antigen or polypeptide is operably linked to the guinea pig CMV promoter having the sequence as set forth in SEQ ID NO:43. In another aspect, the expression of a first IBDV VP2 antigen or polypeptide is regulated by the CMV-IE promoter having the sequence as set forth in SEQ ID NO:10 and the SV40 polyA signal having the sequence as set forth in SEQ ID NO:11, and the expression of a second IBDV VP2 antigen or polypeptide is regulated by the guinea pig CMV promoter having the sequence as set forth in SEQ ID NO:43 and the synthetic polyA signal having the sequence as set forth in SEQ ID NO:12. In yet another aspect of the embodiment, the polynucleotides encoding the IBDV VP2 antigen or polypeptide may be inserted in one or more locus regions selected from the group consisting of IG1, IG2, US10, SORF3-US2 and gD of HVT genome. In one embodiment, the present invention relates to a pharmaceutical composition or vaccine comprising one or more recombinant HVT or SB-1 rival vectors of the present invention and a pharmaceutically or veterinarily acceptable carrier, excipient, vehicle or adjuvant.

In another embodiment, the present invention provides a composition or vaccine comprising an HVT viral vector comprising a polynucleotide encoding an NDV-F antigen, an SV40 promoter, and optionally a pharmaceutically or veterinarily acceptable carrier, excipient, vehicle or adjuvant. In another embodiment, the present invention provides a pharmaceutical composition or vaccine comprising a first HVT vector comprising a polynucleotide encoding an NDV-F antigen, a second HVT vector comprising a polynucleotide encoding an IBDV VP2 antigen, and optionally a pharmaceutically or veterinarily acceptable carrier, excipient, vehicle or adjuvant. In another embodiment, the present invention provides a pharmaceutical composition or vaccine comprising an HVT vector comprising a polynucleotide encoding an NDV-F antigen, an SB-1 vector comprising a polynucleotide encoding an NDV-F antigen, optionally a pharmaceutically or veterinarily acceptable carrier, excipient, vehicle or adjuvant. The pharmaceutical composition or vaccine of the present invention may comprise a first HVT vector comprising a polynucleotide encoding an NDV-F antigen, a second HVT vector comprising a polynucleotide encoding an IBDV VP2 antigen, an SB-1 vector comprising a polynucleotide encoding an NDV-F antigen, optionally a pharmaceutically or veterinarily acceptable carrier, excipient, vehicle or adjuvant.

In yet another embodiment, the present invention provides a composition or vaccine comprising a double HVT viral vector comprising: i) a first heterologous polynucleotide coding for and expressing an NDV-F antigen or polypeptide; ii) a second polynucleotide coding for and expressing an IBDV VP2 antigen or polypeptide; and iii) optionally a pharmaceutically or veterinarily acceptable carrier, excipient, vehicle or adjuvant. In another embodiment, the present invention provides a composition or vaccine comprising a double HVT viral vector comprising two polynucleotides coding for and expressing the IBDV VP2 antigens or polypeptides, and optionally a pharmaceutically or veterinarily acceptable carrier, excipient, vehicle or adjuvant. In yet another embodiment, the composition comprising the double HVT viral vector further comprises an HVT vector comprising a polynucleotide encoding an IBDV VP2 antigen, or an SB-1 vector comprising a polynucleotide encoding an NDV-F antigen, or a combination thereof. The pharmaceutically or veterinarily acceptable carriers or adjuvant or vehicles or excipients are well known to the one skilled in the art. For example, a pharmaceutically or veterinarily acceptable carrier or adjuvant or vehicle or excipient can be Marek's disease vaccine diluent used for MD vaccines. Other pharmaceutically or veterinarily acceptable carrier or adjuvant or vehicle or excipients that can be used for methods of this invention include, but are not limited to, 0.9% NaCl (e.g., saline) solution or a phosphate buffer, poly-(L-glutamate) or polyvinylpyrrolidone. The pharmaceutically or veterinarily acceptable carrier or vehicle or excipients may be any compound or combination of compounds facilitating the administration of the vector (or protein expressed from an inventive vector *in vitro*), or facilitating transfection or infection and/or improve preservation of the vector (or protein). Doses and dose volumes are herein discussed in the general description and can also be determined by the skilled artisan from this disclosure read in conjunction with the knowledge in the art, without any undue experimentation.

Optionally other compounds may be added as pharmaceutically or veterinarily acceptable carriers or adjuvant or vehicles or excipients, including, but not limited to, alum; CpG oligonucleotides (ODN), in particular ODN 2006, 2007, 2059, or 2135 (Pontarollo R.A. et al., Vet. Immunol. Immunopath, 2002, 84: 43-59; Wernette C.M. et al., Vet. Immunol. Immunopath, 2002, 84: 223-236; Mutwiri G. et al., Vet. Immunol. Immunopath, 2003, 91: 89-103); polyA-polyU, dimethyldioctadecylammonium bromide (DDA) ("Vaccine Design The Subunit and Adjuvant Approach", edited by Michael F. Powell and Mark J. Newman, *Pharmaceutical Biotechnology,* 6: p.03, p.157); N,N-dioctadecyl-N',N'-bis(2-hydroxyethyl) propanediamine (such as AVRIDINE®) (*Ibid,* p. 148); carbomer, chitosan (*see* US Patent Serial No. 5,980.912 for example).

The pharmaceutical compositions and vaccines according to the invention may comprise or consist essentially of one or more adjuvants. Suitable adjuvants for use in the practice of the present invention are (1) polymers of acrylic or methacrylic acid, maleic anhydride and alkenyl derivative polymers, (2) immunostimulating sequences (ISS), such as oligodeoxyribonucleotide sequences having one or more non-methylated CpG units (Klinman et al., 1996; WO98/16247), (3) an oil in water emulsion, such as the SPT emulsion described on p 147 of "Vaccine Design, The Subunit and Adjuvant Approach" published by M. Powell, M. Newman, Plenum Press 1995, and the emulsion MF59 described on p 183 of the same work, (4) cation lipids containing a quaternary ammonium salt, e.g., DDA (5) cytokines, (6) aluminum hydroxide or aluminum phosphate, (7) saponin or (8) other adjuvants discussed in any document cited and incorporated by reference into the instant application, or (9) any combinations or mixtures thereof.

Another aspect of the invention relates to a method for inducing an immunological response in an animal against one or more antigens or a protective response in an animal against one or more avian pathogens, which method comprises inoculating the animal at least once with the vaccine or pharmaceutical composition of the present invention. Yet another aspect of the invention relates to a method for inducing an immunological response in an animal to one or more antigens or a protective response in an animal against one or more avian pathogens in a prime-boost administration regimen, which is comprised of at least one primary administration and at least one booster administration using at least one common polypeptide, antigen, epitope or immunogen. The immunological composition or vaccine used in primary administration may be same, may be different in nature from those used as a booster.

The avian pathogens may be Newcastle Disease Virus (NDV), Infectious Bursal Disease Virus (*i.e.,* IBDV or Gumboro Disease virus), Marek's Disease Virus (MDV), Infectious Laryngotracheitis Virus (ILTV), avian encephalomyelitis virus, avian reovirus, avian paramyxovirus, avian metapneumovirus, avian influenza virus, avian adenovirus, fowl pox virus, avian coronavirus, avian rotavirus, avian parvovirus, avian astrovirus and chick anemia virus coccidiosis (*Eimeria sp.*)*, Campylobacter sp., Salmonella sp., Mycoplasma gallisepticum, Mycoplasma synoviae, Pasteurella sp., Avibacterium sp., E. coli* or *Clostridium sp.*

Usually, one administration of the vaccine is performed either at one day-of-age by the subcutaneous or intramuscular route or *in ovo* in 17-19 day-old embryo. A second administration can be done within the first 10 days of age. The animals are preferably at least 17-day-embryo or one day old at the time of the first administration.

A variety of administration routes in day-old chicks may be used such as subcutaneously or intramuscularly, intradermally, transdermally. The *in ovo* vaccination can be performed in the amniotic sac and/or the embryo. Commercially available *in ovo* and SC administration devices can be used for vaccination.

The invention will now be further described by way of the following non-limiting examples.

### EXAMPLES

Construction of DNA inserts, plasmids and recombinant viral vectors was carried out using the standard molecular biology techniques described by J. Sambrook et al. (Molecular Cloning: A Laboratory Manual, 2nd Edition, Cold Spring Harbor Laboratory, Cold Spring Harbor, New York, 1989).

### Example 1 Construction of recombinant vHVT114 expressing NDV-F

### Preparation of donor plasmid pHM103+Fopt

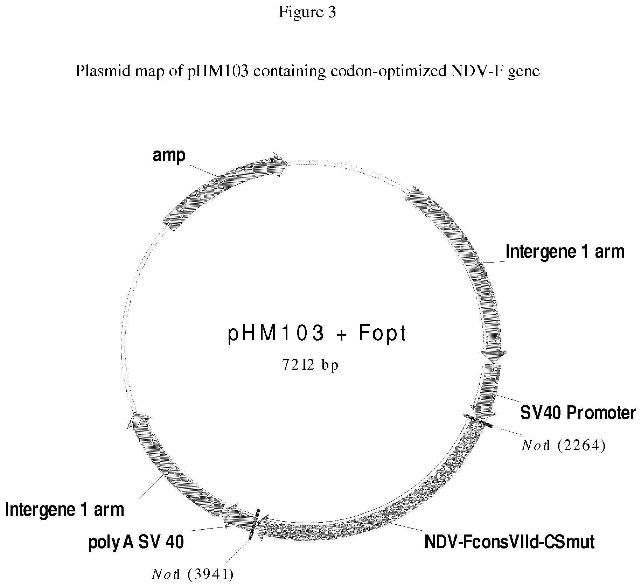

The plasmid pHM103 (Merial Limited) containing the Intergenic I arms of HVT FC126 (see FIG.2), SV40 promoter and SV40 poly A was digested with NotI, dephosphorylated, and the 5.6kb fragment was gel extracted. A NotI flanked 1.7 kb fragment of a chemically synthesized codon-optimized genotype VIId NDV-F gene (SEQ ID NO:1, coding for SEQ ID NO:2) was also NotI digested and the 1.7kb fragment was gel extracted. The 5.6 and 1.7kb fragments were ligated to create pHM103+Fopt (FIG. 3).

### Generation of recombinant HVT viral vector

An in vitro recombination (IVR) was performed by co-electroporation of secondary chicken embryo fibroblast cells (2° CEF cells) using pHM103+Fopt as the donor plasmid and viral DNA isolated from the HVT strain FC126. Co-electroporation was performed using 1x10⁷ 2° CEF in 300ul Opti-MEM and shocked at 150 volts with 950 capacitance in a 2mm electroporation cuvette. The transfected cells were seeded into 96-well plate and incubated for 5 days. The cells grown in the 96-well plate were then duplicated into two 96-well plates. One set of 96-well plates was used for IFA using chicken polyclonal sera against NDV-F to identify positive wells containing recombinants and another set of 96-well plates was used for recovering the infected cells from the positive wells.

The recombinant viral purification was performed first by 96-well plate duplication and IFA selection for the wells containing the most IFA positive plaques with the least amount of IFA negative plaques. Wells matching those criteria were then harvested and adjusted to 1ml in DMEM+2% FBS. From the 1ml stock, 5-20ul were removed and mixed with 1x10⁷ CEFs in 10ml DMEM+2% FBS and aliquoted onto a new 96-well plate to have single HVT plaques per well. The supernatant of the wells that contained single plaques were tested for the absence of parental virus by PCR. After five rounds of plaque purification, a recombinant virus designated as vHVT114 was isolated and the purity was tested by IFA and PCR to confirm NDV-F expression and the absence of parental virus.

### PCR analysis of recombinant vHVT114

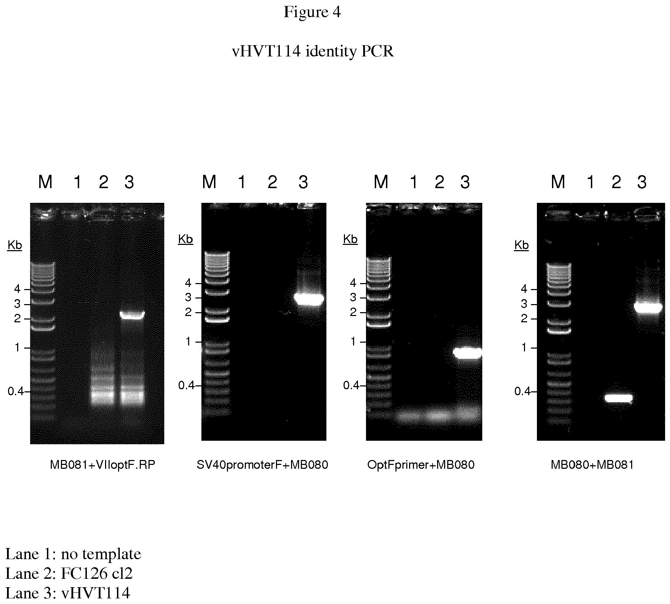

DNA was extracted from vHVT114 by phenol/chloroform extraction, ethanol precipitated, and was resuspended in 20mM HEPES. PCR primers (shown in Table 1) were designed to specifically identify the presence of the codon optimized NDV-F, the SV40 promoter, as well as, the purity of the recombinant virus from FC126 CL2 parental virus. PCR was performed using 200ng of DNA template along with the specified primers pairs indicted in Table 1. PCR cycling conditions are as follows: 94°C for 2 mins; 30 cycles of 94°C for 30 secs, 55°C for 30 secs, 68°C for 3 mins; 68°C for 5 mins. The expected PCR products are shown in Table 2. The PCR results are shown in Figure 4. As shown in Figure 4, the sizes of PCR products after gel electrophoresis correspond well with the expected sizes and the banding patterns.

**Table 1**

| primer | SEQ ID NO | Sequence 5'-3' |
|---|---|---|
| MB080 | 13 | CGA ACA AAC TTC ATC GCT ATG C |
| MB081 | 14 | TAA CTC AAA TGC GAA GCG TTG C |
| optF | 15 | ACT GAC AAC ACC CTA CAT GGC |
| V11optF RP | 16 | GCC AGC ACC AGG CTC AGG G |
| SV40promoterF | 17 | AGC TTG GCT GTG GAA TGT |

**Table 2**

| Primer pairs | Expected size (bp) | |
|---|---|---|
| | FC126 CL21 | vHVT114 |
| MB081 + VIIoptF.RP | -- | 2138 |
| SV40promoterF + MB080 | -- | 2368 |
| OptFprimer + MB080 | -- | 872 |
| MB080 + MB081 | 323 | 2578 |

### Expression analysis of recombinant vHVT114

Immunofluorescence testing was performed using the vHVT114 which was passaged over ten times beyond an experimental pre-master seed (pre-MSV). The pre-MSV and pre-MSV+12 materials were diluted 1:100 in media. Fifty microliters of the diluted virus was added to 10 ml of DMEM+2% FBS with 1x10⁷ CEFs and then aliquoted onto a 96 well plate (100ul/well). The plates were incubated for 3 days at 37°C+5%CO₂ until viral plaques were visible. The plates were fixed with 95% ice-cold acetone for three minutes and washed three times with PBS. Chicken anti-sera against Newcastle Disease Virus (lot#C0139, Charles Rivers Laboratory) at 1:1000 were added along with monoclonal antibody L-78 (Merial Limited) at 1:3000 and the plates were incubated at 37°C for 1 hour. After the 1 hour incubation the plates were washed three times with PBS and FITC anti-chicken (cat# F8888, Sigma) was added along with Alexz Fluor 568 donkey anti-mouse (IgG) (cat# A 10037, Molecular Probe) at 1:500. Again the plates were incubated at 37°C for 1 hour. After the 1 hour incubation the cells were rinsed three times with PBS. A small amount of PBS was added to prevent the monolayer from drying and causing auto fluorescence. The cells were then visualized with a fluorescent microscope using both the tetramethylrhodamine isothiocyanate (TRITC) and fluorescein isothiocyanate (FITC) filters in combination.

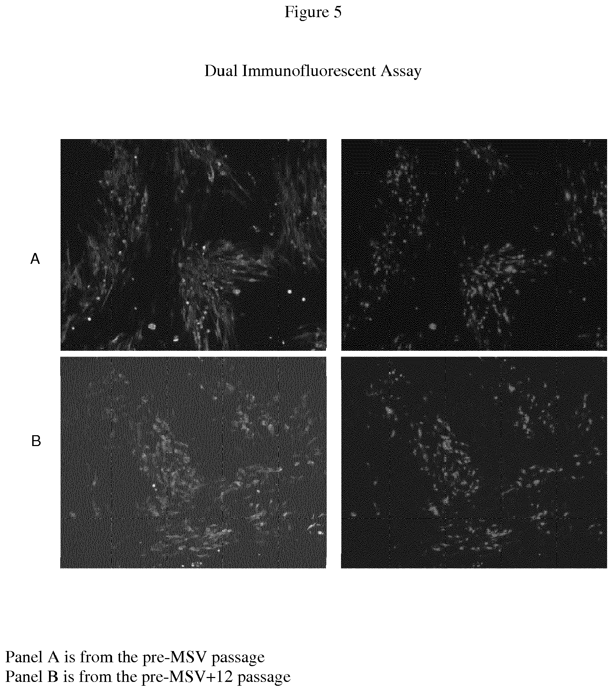

The vHVT114 viral plaques were visualized using both the TRITC and FITC filters for the dual staining. The FITC test showed the NDV-F expression and the TRITC test showed the HVT expression. Because of the small wells of the 96 well plates, each well was recorded with the plaques first counted with the TRITC filter and then recounted with the FITC filter. Over 500 plaques were counted for the pre-MSV and pre-MSV+12 passage. All the plaques were positive for both the FITC and TRITC on both plates. (FIG. 5)

### Southern blot analysis of recombinant vHVT114

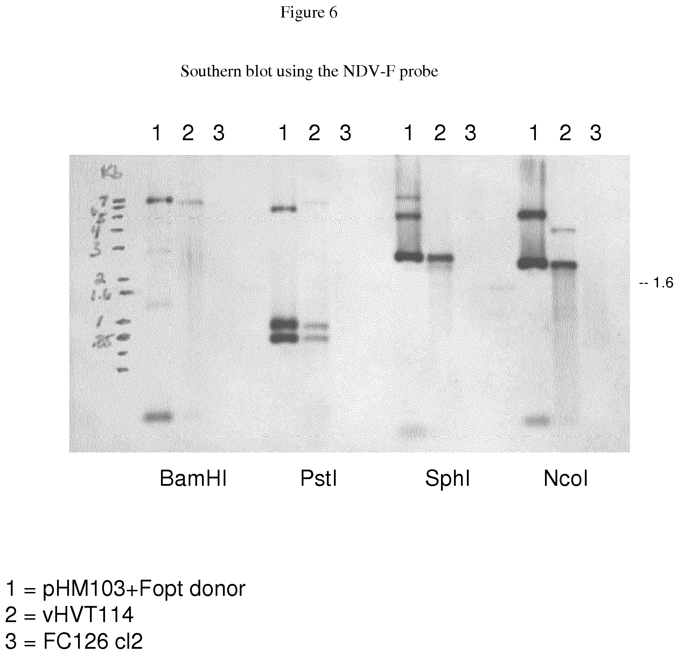

Total genomic DNA was extracted from HVT FC126 and vHVT114 according to the standard genomic DNA extraction protocol. For each restriction digest, 3 µg of genomic DNA (1 ng for the donor plasmid) was used with a total digestion volume of 20 µl for each sample. The genomic DNA of HVT FC126 (negative control), pHM103+Fopt donor plasmid, and vHVT114 were each digested overnight at 37°C with *BamH*I*, Pst*I*, Sph*I*, and Nco*I restriction endonucleases. The restriction fragments of HVT FC126 (negative control), pHM103+Fopt donor plasmid, and vHVT114 genomic DNA were separated by a 1% agarose gel and transferred to a positively charged Nylon membrane. Following the North2South Chemiluminescent Hybridization and Detection Kit (Thermo Scientific) manufacturers' instructions, the membrane was pre-hybridized for 1 hr and then hybridized with a biotinylated NDV-F probe overnight at 55°C. Following the overnight hybridization, several stringency washes were performed until the membrane was placed in blocking buffer with the addition of Streptavidin-HRP. After rinsing the membrane of any unbound Streptavidin-HRP the substrate solution of Luminal and peroxide were added. The membrane was then exposed to X-ray film and developed. Areas where the biotinylated probe bound to the DNA were chemiluminescent and were captured by the X-ray film. Table 3 shows the expected Southern blot bands using the NDV-F probe. The Southern blot results showed the digestion patterns as expected (Fig. 6).

**Table 3**

| NDV-F Probe | | | |
|---|---|---|---|
| **Restriction Endonuclease** | **Donor plasmid pHM103+Fopt** | **vHVT114** | **FC126 CL2** |
| BamHI | 7.014 | 6.630 | --- |
| | 0.198 | 1.259 | |
| | | 0.198 | |
| PstI | 5.481 | 6.359 | --- |
| | 0.947 | 0.947 | |
| | 0.784 | 0.784 | |
| SphI | 4.763 | 2.377 | --- |
| | 2.377 | 2.119 | |
| | 0.072 | 0.072 | |
| NcoI | 4.931 | 3.753 | --- |
| | 2.157 | 2.157 | |
| | 0.124 | 0.124 | |

### Sequence analysis of the inserted region in recombinant vHVT114

Analysis of vHVT114 genomic DNA region was performed by PCR amplification. Total of 10 primers were used to amplify the entire cassette, as well as, beyond the flanking BamHI-I arms used in the donor plasmid. The 4.727 kb PCR product was gel purified and the entire fragment was sequenced using the sequencing primers. The sequence result confirmed that the vHVT114 contains the correct SV40 promoter, the codon-optimized NDV-F and the SV40 polyA sequences that match exactly the sequence described for the donor plasmid pHM103+Fopt in SEQ ID NO:18.

### Western blot analysis of recombinant vHVT114

Approximately 2x10⁶ chicken fibroblast cells were infected at ∼0.1 MOI with vHVT114 Pre-MSV. After two days of incubation at 37°C, infected as well as uninfected cells were harvested using a cell scraper after removing the media and rinsing with PBS. The cells were harvested with 1ml of PBS and centrifuged. The cell pellets were lysed by following the Pierce Classic IP Kit (cat#26146, Thermo Scientific). 100 µl of the anti-NDV-F monoclonal antibody 001C3 (Merial Limited) was used to form the immune complex. The antibody/lysate sample was added to Protein A/G Plus Agarose to capture the immune complex. The immune complex was washed three times to remove non-bound material and then eluted in 50ul volume using sample buffer elution under non-reducing condition. After boiling for 5 minutes, 10 µl of the samples were loaded into a 10% Acrylamide gel (Invitrogen). The PAGE gel was run in MOPS buffer (Invitrogen) at 200volts for 1 hour. Then the gel was transferred onto a PVDF membrane.

The Protein Detector Western Blot Kit TMB System (KPL, cat# 54-11-50) was used for blotting the PVDF membrane by using the reagents and following manufacturer's directions. After blocking the membrane for 1 hour at room temperature, the membrane was then rinsed three times in IX Wash Buffer, five minutes each and then soaked in blocking buffer containing 1:1000 dilution of chicken serum raised against NDV virus (Lot # C0139, Charles River Laboratories). After washing three times in a washing buffer, the membrane was incubated with a peroxidase labeled goat anti-chicken IgG (KPL, cat# 14-24-06) at a dilution of 1:2000 for 1 hour at room temperature. The membrane was then rinsed three times in IX Wash Buffer, five minutes each. 5ml of TMB membrane peroxidase substrate was added to the membrane and gently rocked for about 1 minute. The developing reaction was stopped by placing the membrane into water.

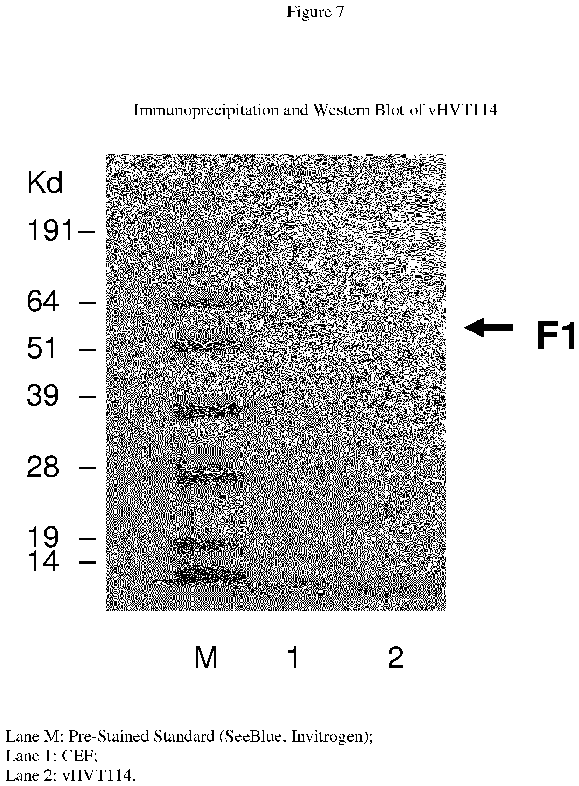

The immunoprecipitation and Western blot technique detected an approximately 55 kD protein in vHVT114 sample that corresponds to the expected size of F1 component of the NDV-F protein (FIG. 7).

### Example 2 Construction of recombinant vHVT110, vHVT111, vHVT112, vHVT113 and vHVT116 expressing NDV-F

Generation and characterization of HVT recombinants vHVT110, vHVT111, vHVT112, vHVT113, and vHVT116 was essentially done in the same way as for vHVT114 described in example 1. Table 4 shows the features unique to each construct around the expression cassettes, including the respective sequences.

**Table 4 Characteristics of the expression cassettes of single HVT recombinants**

| Name | Parental virus | Promoter | F gene | Poly-A | Locus |
|---|---|---|---|---|---|
| vHVT039 | HVT | MDV gB | Wtnm-Texas | SV40 | IG1 |
| vHVT110 | HVT | mCMV IE | Wt-VIId | SV40 | IG1 |
| vHVT111 | HVT | SV40 | Wt-VIId | SV40 | IG1 |
| vHVT112 | HVT | MCMV IE | Wt-YZCQ | SV40 | IG1 |
| vHVT113 | HVT | MCMV IE | Wt-Texas | SV40 | IG1 |
| vHVT114 | HVT | SV40 | Opt-VIId | SV40 | IG1 |
| vHVT116 | HVT | SV40 | Opt-Ca02 | SV40 | IG1 |

### vHVT110

The plasmid pCD046 (Merial proprietary material) containing the Intergenic I arms of HVT FC126, mouse CMV promoter and SV40 poly A was digested with NotI, dephosphorylated, and a 6.6kb fragment was gel extracted. A NotI flanked 1.7 kb fragment of a chemically synthesized NDV-F gene containing wild-type F sequence (SEQ ID NO:3, coding for SEQ ID NO:4) was also NotI digested and the 1.7 kb fragment was gel extracted. The 6.6 and 1.7 kb fragments were ligated to create a donor plasmid pCD046+NDV-F wt (SEQ ID NO:21 for vHVT110) used in transfection to generate recombinant vHVT110. Sequencing of the insert region confirmed that vHVT110 contains the correct sequences of mCMV promoter, the wildtype NDV-F gene and the SV40 polyA. The sequence also exactly matches the sequence described for the donor plasmid pCD046+NDV-F wt in SEQ ID NO:21.

### vHVT111

The plasmid pHM103 plasmid (Merial proprietary material) containing the Intergenic I arms of HVT FC126, SV40 promoter and SV40 polyA was digested with NotI, dephosphorylated, and the 5.6 kb fragment was gel extracted. A NotI flanked 1.7 kb fragment of a chemically synthesized NDV-F gene containing wildtype F sequence (SEQ ID NO:3, coding for SEQ ID NO:4) was also NotI digested and a 1.7kb fragment was gel extracted. The 5.6 and 1.7 kb fragments were ligated to create a donor plasmid (SEQ ID NO:22 for vHVT1110) used in transfection to generate recombinant vHVT111. Sequencing of the insert region confirmed that vHVT111 contains the correct sequences of SV40 promoter, the wildtype NDV-F gene and the SV40 polyA as shown in the sequence of the donor plasmid pHM103+NDV-F wt (SEQ ID NO:22).

### vHVT112

A fragment encompassing the synthetic NDV-F YZCQ wild type gene (SEQ ID NO:34 encoding SEQ ID NO:35) was excised from pUC57 NDV-F YZCQ plasmid (synthesized by GeneScript) using NotI and inserted into the same site of pCD046 plasmid containing mCMV promoter and SV40 polyA tail. Ligated material was transformed using Top10 Oneshot kit (cat#C404002, Invitrogen). Bacterial colonies were grown in LBamp broth, plasmid extracted by using Qiagens MiniSpin Prep kit, and screened for insert orientation. The correct donor plasmid was designated pCD046+NDV-F VII YZCQ. Large scale cultures were grown and plasmid extraction was done by using Qiagens Maxi Prep kit. Transient expression of the maxi preps was verified using Fugene Transfection Reagent in Chicken Embryo Fibroblast Cells (CEF's) and chicken polyclonal sera against NDV.

Plasmid pCD046+NDV-F VII YZCQ (SEQ ID NO:29) was used in transfection to generate recombinant vHVT112. Sequencing of the insert region confirmed that vHVT112 contains the correct sequences of mCMV promoter, the wildtype NDV-F YZCQ gene and the SV40 polyA. The sequence also exactly matches the sequence described for the donor plasmid pCD046+NDV-F VII YZCQ in SEQ ID NO:29.

### vHVT113

A fragment encompassing the synthetic NDV Texas F gene (SEQ ID NO:36 encoding SEQ ID NO:37) was excised from pUC57 NDV Texas F plasmid (synthesized by GeneScript) using NotI and inserted into the same site of pCD046 plasmid containing mCMV promoter and SV40 polyA tail. Ligated material was transformed using Top10 Oneshot kit (cat#C404002, Invitrogen). Bacterial colonies were grown in LBamp broth, plasmid extracted by using Qiagens MiniSpin Prep kit, and screened for insert orientation. The correct donor plasmid was designated pCD046+Texas NDV-F. Large scale cultures were grown and plasmid extraction was done by using Qiagens Maxi Prep kit. Transient expression of the maxi preps was verified using Fugene Transfection Reagent in Chicken Embryo Fibroblast Cells (CEF's) and chicken polyclonal sera against NDV.

Plasmid pCD046+Texas NDV-F (SEQ ID NO:30) was used in transfection to generate recombinant vHVT113. Sequencing of the insert region confirmed that vHVT113 contains the correct sequences of mCMV promoter, the wild-type NDV-F Texas F gene and the SV40 polyA. The sequence also exactly matches the sequence described for the donor plasmid pCD046+Texas NDV-F in SEQ ID NO:30.

### vHVT039

The MDV gB promoter (SEQ ID NO:38) was amplified from MDV1 RB1B strain extracted DNA by PCR using the primers HM101 (5'-CCG-GAA-TTC-CGA-TGT-TTA-GTC-ACG-ATA-GAC-3') (SEQ ID NO:44) and HM102 (5'-ATA-AGA-GCG-GCC-GCA-GTG-AGA-TGA-TCT-TAA-TGA-TG-3') (SEQ ID NO:45). The former contains an EcoRI site and the latter contains a NotI site for ligation of the EcoRI/NotI digested 630 bp PCR product into EcoRI/NotI digested pCD046 plasmid. The ligation product was used to transform DH5α competent cells. Colonies were picked and screened for the presence of the inserted PCR fragment by restriction analysis with EcoRI and NotI. The resulting plasmid was designated pHM102.

The velogenic NDV Texas strain (genotype IV) was grown on 11-day-old SPF embryonated eggs and semi-purified. Total RNA was extracted and an RT PCR was performed using two primers F-ATG (5' TAT-AGC-GGC-CGC-AAG-ATG-GGC-TCC-AGA-TCT-TCT-ACC-AG 3') (SEQ ID NO:46) and F-STOP (5' CGA-GGC-GGC-CGC-TCA-TAT-TTT-TGT-AGT-GGC-TCT-C 3') (SEQ ID NO:47). They allow the whole amplification of the NDV F gene with addition of NotI site upstream ATG and downstream STOP codons. The 1.7 kb PCR fragment was digested with NotI and ligated into NotI-digested pHM102. The resulting plasmid was designated pHM119 and was used as a donor plasmid in *in vitro* recombination study by co-transfection of CEF cells with HVT parental DNA to generate vHVT039 as described above. Sequencing of the insert region confirmed that vHVT039 contains the correct sequences of MDV gB promoter, the wildtype unmodified NDV-F gene from Texas strain (SEQ ID NO:32 encoding SEQ ID NO:33) and the SV40 polyA as shown in the partial sequence of the donor plasmid pHM119 (SEQ ID NO:31).

### vHVT116

The plasmid pHM103 plasmid (Merial proprietary material) containing the Intergenic I arms of HVT FC126, SV40 promoter and SV40 polyA was digested with NotI, dephosphorylated, and the 5.6kb fragment was gel extracted. A NotI flanked 1.7 kb fragment of a chemically synthesized, codon-optimized, CA02 genotype V NDV-F gene (SEQ ID NO:5, coding for SEQ ID NO:6) was also NotI digested and the 1.7 kb fragment was gel extracted. The 5.6 and 1.7kb fragments were ligated to create pHM103 + NDV-F CA02 (SEQ ID NO:23 for vHVT116) used in transfection to generate recombinant vHVT116. Sequencing of the insert region confirmed that vHVT116 contains the correct sequences of SV40 promoter, the codon-optimized CA02 NDV-F gene and the SV40 polyA as shown in the sequence of the donor plasmid pHM103+NDV-F wt (SEQ ID NO:23).

### Discussion

Various cassettes under mCMV or non-CMV promoter were inserted at different loci of HVT genome (Table 4). Despite repeated attempts, generating a construct with a combination of mCMV and codon-optimized F sequence was not successful beyond passage 2. However, when wild-type sequence was driven by mCMV a stable construct, vHVT110 could be generated. In addition, recombinant vHVT111 with wild-type F sequence under SV40 promoter was also stable for more than 10 *in vitro* passages. Surprisingly, a codon-optimized F sequence under SV40 promoter was similarly found to be stable for more than 10 *in vitro* passages (e.g. vHVT114 and vHVT116). These results indicate the delicate balance between the strength of the promoter and the nature of the gene they control (codon-optimized or not optimized) in generating a genetically stable HVT construct.

### Example 3 Construction of vHVT306, a double HVT vector expressing NDV-F and IBDV VP2

The donor plasmid pHVT US2 SV- Fopt-synPA was constructed containing SV40 promoter, synthetic NDV F codon optimized VII gene, synthetic polyA tail flanked by the SORF3 and US2 arm sequences of HVT FC126.

### Generation of recombinant virus

A standard homologous recombination procedure was followed by co-electroporation of secondary CEF cells using donor plasmid pHVT US2 SV-Fopt-synPA and viral DNA isolated from vHVT13 (an HVT vector expressing the IBDV VP2 gene, Merial Limited). Essentially the procedure described in example 1 for vHVT114 was followed to generate, plaque purify and characterize recombinants by immunofluorescence.

After five rounds of plaque purification, pure recombinant virus (vHVT306) was isolated and the purity of vHVT306 was tested and confirmed by IFA and PCR.

### PCR analysis

Viral DNA was extracted from vHVT306 pre-master seed virus (pre-MSV) stock by QIA DNeasy Blood & Tissue Kit (Qiagen cat#69506). PCR primers were designed to identify the presence of the NDV F optimized, the NDV F wild type, the SV40 promoter, the mCMV promoter, the flanking arms of US2 HVT virus and SB-1 virus.

PCR amplification with various primers confirmed that the vHVT306 has the expected amplification patterns and amplicons.

### Expression analysis

Indirect immunofluorescent assay (IFA) was performed on the vHVT306 pre-MSV stock. The CEFs that were inoculated with vHVT306 were fixed with ice-cold 95% acetone for three minutes at room temperature and air-dried for 10 min. After three washes with PBS, two primary antibodies, chicken anti-Newcastle Disease Virus sera (Charles Rivers Laboratories cat#10100641, lot#C0117A) at 1:500 dilution and L78 monoclonal antibody against HVT (Merial Select, Gainesville, GA) at 1:3000 dilution were added and incubated for 45 min at 37°C. After three washes with PBS, two secondary antibodies, goat anti-chicken IgG--fluorescein (KPL cat#.02-24-06, lot#110020) at 1:500 dilution and donkey anti-mouse IgG-Alexa Fluor 568 (Molecular Probe #A10037, lot#989784) at 1:300 dilution were added. The plates were incubated at 37°C for 45 min and followed by three washes with PBS. The cells were observed to identify the IFA positive plaques with a fluorescent microscope using fluorescein isothiocyanate (FITC)- and tetramethylrhodamine isothiocyanate (TRITC)-filters of Nikon Eclipse Ti inverted microscope.

Similarly the expression of IBDV VP2 protein (SEQ ID NO:8 encoded by SEQ ID NO:7) of vHVT306 were examined by IFA using chicken anti-IBDV sera (Charles River Laboratories cat#10100610 lot#G0117) (1:500 dilution) and anti-NDV F monoclonal antibody 001C3 (Asceitic fluid, Batch 10/09/044, 02/11/2010) (1:300 dilution) as primary antibodies; followed by goat anti-chicken IgG-fluorescein (KPL cat#.02-24-06, lot#110020) (1:500 dilution) and donkey anti-mouse IgG-Alexa Fluor 568 (Molecular Probe #A10037, lot#989784) (1:300 dilution) as secondary antibodies.

IFA results indicate that vHVT306 expresses the NDV F genes in virus-infected CEFs.

Over 400 vHVT306 plaques were counted using the FITC-filter and TRITC-filter of microscope. The overall expression of NDV F gene and IBDV VP2 match with the HVT plaques (Table 5).

**Table 5 Dual IFA of vHVT306**

| Virus | IFA #1 (total 453 plaques) | | IFA#2 (total 478 plaques) | |
|---|---|---|---|---|
| | Anti-NDV serum positive plaques | Anti-HVT MAb positive plaques | Anti-NDV F MAb positive plaques | Anti-IBDV serum positive plaques |
| vHVT306 pre-MSV | 453 | 453 | 478 | 478 |

### Southern Blot analysis

Total genomic DNA was extracted from vHVT306 pre-MSV stock infected CEFs. The Southern blot analysis was performed according to the standard protocol.

A total 3 probes were used to confirm the NDV F cassette (SV40 promoter, NDV F codon optimized gene, synthetic poly A tail) between SORF3 and US2 of vHVT306 as well as retention of IBDV VP2 cassette (mCMV promoter, IBDV VP2 gene, SV40 poly A tail).

The Southern blot results showed the digestion patterns as expected based on Vector NTI (Invitrogen, 1600 Faraday Ave., Carlsbad, CA) map analysis. The NDV F cassette (SV40 promoter, NDV F codon optimized gene, synthetic poly A tail) is located between SORF3 and US2, and IBDV VP2 cassette (mCMV promoter, IBDV VP2 gene, SV40 poly A tail) is intact like the parent virus (vHVT13).

### Genomic analysis

The genomic DNA of vHVT306 pre-MSV stock was sequenced to verify the sequence of the recombination arm region as well as inserted gene cassette.

Primers were designed to amplify the entire inserted gene cassette including recombination arm used in donor plasmid. Analysis of vHVT306 genomic DNA was performed by PCR amplification and followed by nucleotide sequence determination.

The vHVT306 (donor plasmid pHVT US2 SV- Fopt-synPA) containing the recombinant arms, SV40 promoter and NDV F codon-optimized gene was confirmed to be correct as shown in SEQ ID NO:20.

### Western blot analysis

The CEF monolayer was infected with vHVT306 pre-MSV at MOI ∼ 0.1. After a 4-day incubation, the CEFs were pelleted and washed with PBS followed by lysis with IP Lysis/Wash buffer of Pierce Classic IP Kit (Thermo Scientific cat#26146) according to the manufacturer's protocols. The lysate was pre-cleared and incubated with 100 ul of anti-NDV F monoclonal antibody 001C3 to make the immune complex. The immune complex was captured by Protein A/G Plus Agarose and after removing of the un-bounded immune complex by washing steps, the 50 ul of sample buffer was used to elute under non-reducing conditions. The uninfected CEFs were included as controls.

The 20 ul of eluted samples were separated in a 10% Bis-Tris Gels by electrophoresis. After the electrophoresis, the separated proteins were transferred onto PVDF membrane. The Protein Detection TMB Western Blot Kit (KPL cat#54-11-50) was used to detect the NDV antigens on PVDF membrane with chicken anti-NDV serum (Charles River Laboratories Laboratories cat#10100641, lot#C0117A), and goat anti-chicken IgG-peroxidase conjugate (KPL cat#14-24-06) following the manufacturers' protocols.

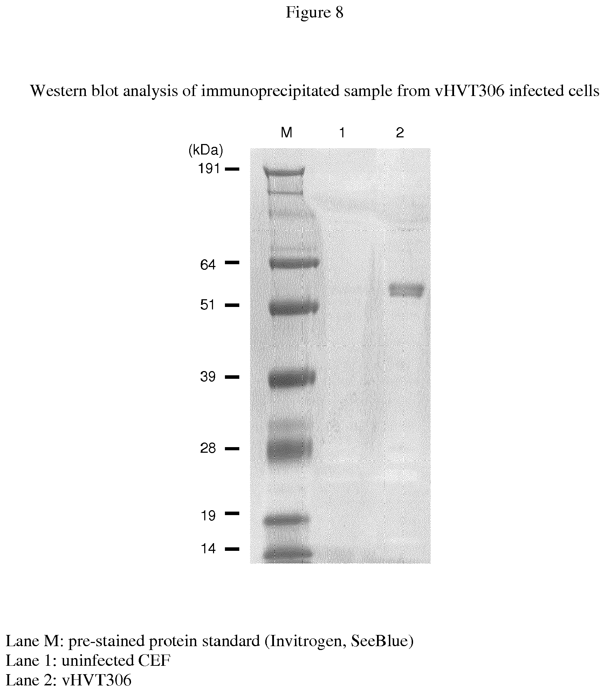

The NDV F protein expression of vHVT306 was confirmed by two-step immunodetection. First, the expressed NDV F proteins from vHVT306 infected CEF were captured by the immunoprecipitation using anti-NDV F monoclonal antibody 001C3. Subsequently Western blot analysis using anti-NDV polyclonal serum (Charles River Laboratories cat# 10100641, lot#C0117A) was applied to detect the NDV F protein in the captured samples (NDV F protein-monoclonal antibody complex) (FIG.8). A 55 kDa protein in vHVT306 pre-MSV lysates was detected by anti-NDV serum which corresponds to the expected size of NDV F1 fusion protein (FIG.8).

### Example 4 Construction of double HVT vectors vHVT301, vHVT302, vHVT303, vHVT304 and vHVT307 expressing NDV-F and IBDV VP2, and double HVT vector vHVT202 expressing IBDV VP2 variants

### Example 4.1 Construction of vHVT301, vHVT302, vHVT303, vHVT304 and vHVT307

Generation and characterization of double HVT recombinants vHVT301, vHVT302, vHVT303, vHVT304, and vHVT307 were essentially done in the same way as for vHVT306 described in example 3. Table 6.1 shows the features unique to each construct around the expression cassettes, including the respective sequences.

**Table 6.1 Characteristics of the expression cassettes of double HVT recombinants**

| Name | Parental virus | Promoter | NDV-F gene | Poly-A | Locus |
|---|---|---|---|---|---|
| vHVT301 | vHVT13 | SV40 | Wt-VIId NDV-F | SV40 | IG2 |
| vHVT302 | vHVT13 | US10 | Opt-VIId NDV-F | US10 | US10 |
| vHVT303 | vHVT13 | US10 | Opt-V NDV-F | US10 | US10 |
| vHVT304 | vHVT13 | SV40 | Opt-VIId NDV-F | Synthetic | IG2 |
| vHVT306 | vHVT13 | SV40 | Opt-VIId NDV-F | Synthetic | SORF3-US2 |
| vHVT307 | vHVT13 | SV40 | Opt-V NDV-F | Synthetic | SORF3-US2 |

### vHVT301

The plasmid pHVT IG2 SbfI (Merial proprietary material) containing the Intergenic 2 arm sequences of vHVT13 was digested with *Sma*I*,* dephosphorylated, and the 4.3kb fragment was gel extracted. The donor plasmid pHM103+NDV-F wt containing an SV40 promoter, wildtype NDV-F genotype VIId, SV40 poly A tail was *EcoR*I and *Sal*I digested, klenow treated, and the 2.3kb fragment was gel extracted. The two fragments were ligated to create a donor plasmid pHVT IG2 SV Fwt SbfI (SEQ ID NO: 24) used in transfection to generate recombinant vHVT301.

### vHVT302

A synthetically synthesized plasmid, pHVT US 10 cds, containing the US 10 arm sequences of vHVT13 was digested with *Not*I*,* dephosphorylated, and the 4.7kb fragment was gel extracted. A *Not*I flanked 1.7kb fragment of a chemically synthesized, codon-optimized, NDV-F genotype VIId was *Not*I digested and gel extracted. The two fragments were ligated to create a donor plasmid pHVT US 10 cds F opt used in transfection to generate recombinant vHVT302. The transcription of the inserted F gene should be driven by the native US10 promoter and be stopped by the native US10 polyA signal. No exogenous promoter or polyA is added to express this insert. Sequencing of the insert region confirmed that vHVT302 contains the correct sequence of the codon-optimized VIId NDV-F gene as shown in the sequence of the donor plasmid pHVT US10 cds F opt (SEQ ID NO: 25).

### vHVT303

The synthetically synthesized plasmid pHVT US 10 cds containing the US 10 arm sequences of vHVT13 was digested with *Not*I*,* dephosphorylated, and the 4.7kb fragment was gel extracted. A *Not*I flanked 1.7kb fragment of a chemically synthesized, codon-optimized, NDV-F genotype V was *Not*I digested and gel extracted. The two fragments were ligated to create a donor plasmid pHVT US 10 cds F CAO2 opt used in transfection to generate recombinant vHVT303. As with vHVT302, the transcription of this inserted F gene should also be driven by the native US 10 promoter and be stopped by the native US10 polyA signal. No exogenous promoter or polyA is added to express this insert. Sequencing of the insert region confirmed that vHVT303 contains the correct sequence of the codon-optimized NDV-F genotype V as shown in the sequence of the donor plasmid pHVT US 10 cds F CA02 (SEQ ID NO: 26).

### vHVT304

The donor plasmid pHVT IG2 SbfI containing the Intergenic 2 arm sequences of vHVT13 was digested with *Sbf*I*,* dephosphorylated, and the 4.3 kb fragment was gel extracted. A synthetically synthesized plasmid containing an SV40 promoter + codon optimized NDV-F genotype VIId + synthetic poly A tail flanked by *Sbf*I was digested with *Sbf*I and the 2.3kb fragment was gel extracted. The two fragments were ligated to create a donor plasmid pHVT IG2 SV Fopt syn tail used in transfection to generate recombinant vHVT304. Sequencing of the insert region confirmed that vHVT304 contains the correct sequences of SV40 promoter, the codon-optimized VIId NDV-F gene, and the synthetic poly A tail as shown in the sequence of the donor plasmid pHVT IG2 SV Fopt syn tail (SEQ ID NO:27).

### vHVT307

The donor plasmid pHVT US2-SORF3 containing the US2 and SORF3 arm sequences of vHVT13 was digested with *Sbf*I*,* dephosphorylated, and the 5.1kb fragment was gel extracted. The plasmid SB-1 UL55 SV CaF syn tail SbfI containing an SV40 promoter + codon optimized NDV-F genotype V + synthetic poly A tail flanked by *Sbf*I was digested with *Sbf*I and the 2.3kb fragment was gel extracted. The two fragments were ligated to create a donor plasmid pHVT US2 SV-FCA02 opt-synPA used in transfection to generate recombinant vHVT307. Sequencing of the insert region confirmed that vHVT307 contains the correct sequences of SV40 promoter, the codon-optimized VIId NDV-F gene, and the synthetic poly A tail as shown in the sequence of the donor plasmid pHVT US2 SV-FCA02 opt-synPA (SEQ ID NO: 28).

### Discussion

One of the main goals of this work was to develop a multivalent avian Herpesvirus-based vector by incorporating multiple protective genes of interest to one avian Herpesvirus backbone (e.g. HVT). A prerequisite for this approach is to define expression cassettes containing appropriate promoter-gene-plyA combinations and evaluate for their genetic stability and ability to protect against the specific disease.

For the purpose of creating an efficacious MD-IBD-ND trivalent vector vaccine, either codon-optimized or non-optimized Newcastle Disease Virus (NDV)-F gene sequences were cloned into vHVT13 backbone (HVT-IBD, a licensed vaccine to simultaneously protect chickens against MD and IBD) under human CMV (mouse CMV is already used in vHVT13). All vHVT-IBD-F constructs under human CMV promoter lost F-protein expression within six passages whether or not the NDV-F sequence is codon-optimized and regardless of the insertion site. The loss of F protein expression was rapid (within two passes) when hCMV was combined with codon-optimized F protein as compared to a combination of hCMV with wild-type F-sequence (loss of F protein expression within 6 passages). Taken together, the data shows that human CMV is not an ideal promoter for the generation of stable HVT recombinants expressing NDV-F protein. Surprisingly, this example shows that SV40 promoter and HVT endogenous promoter (US 10 promoter) generated stable HVT recombinants expressing NDV-F protein.

### Example 4.2 Construction of vHVT202

### Donor plasmid HVT SORF3-US2 gpVar-Ewtsyn construction

A fragment encompassing the synthetic Variant E wild type IBDV VP2 gene (SEQ ID NO:41 encoding SEQ ID NO:42) was excised from pUC57 Variant E wt plasmid (synthesized by GeneScript) using NotI and inserted into the same site of SORF3 and US2 plasmid containing gpCMV promoter and synthetic polyA tail. Ligated material was transformed using Top10 Oneshot kit (cat#C404002, Invitrogen). Bacterial colonies were grown in LBamp broth, plasmid extracted by using Qiagens MiniSpin Prep kit, and screened for insert orientation using *SacI*+*HindIII* digestion. The correct donor plasmid was designated pHVT SORF3-US2 gpVar-Ewt Syn. Table 6.2 shows the features unique to the construct around the expression cassettes, including the respective sequences. Large scale cultures were grown and plasmid extraction was done by using Qiagens Maxi Prep kit. Transient expression of the maxi preps was verified using Fugene Transfection Reagent in Chicken Embryo Fibroblast Cells (CEF's) and chicken polyclonal sera against IBDV.

**Table 6.2 Characteristics of the expression cassettes of double HVT recombinants**

| Name | Parental virus | Promoter | IBDV VP2 gene | Poly-A | Locus |
|---|---|---|---|---|---|
| vHVT202 | vHVT306 | Guinea pig CMV | IBDV E VP2 | Synthetic | SORF3-US2 |

### Recombinant generation

A standard homologous recombination procedure was followed by co-electroporation of secondary CEF cells using pHVTSORF3-US2 gpVar-Ewt Syn donor plasmid and viral DNA isolated from vHVT306 and digested with SbfI. vHVT306, expressing classical VP2 of IBDV and NDV-F, was chosen as a parent to simplify the section process as described below. The variant E VP2 donor plasmid was designed to replace the F gene and recombinants were initially selected for the absence of F gene expression and later by PCR for the presence of variant E VP2. Co-electroporation was performed using 1x10⁷ 2° CEF in 300 µl Opti-MEM and shocked at 150 volts with 950 capacitance in a 2 mm electroporation cuvette. The transfected cells were seeded into 96-well plate and incubated for 5-7 days. The cells grown in the 96-well plate were then duplicated into two 96-well plates and incubated for 5 more days. One set of 96-well plates was used for IFA using chicken polyclonal sera against NDV-F to identify positive wells containing the vHVT306 parents and another set of 96-well plates was used for recovering the infected cells from the IFA negative wells.

The recombinant viral purification methods were preformed first by 96-well plate duplication and IFA selection for the wells containing the most IFA negative (against NDV-F) plaques with the least amount of IFA positive plaques. Wells matching those criteria were then harvested and adjusted to 1ml in DMEM+2% FBS. From the 1ml stock 5-20 µl (depending on the number of visible plaques) were removed and mixed with 1x10⁷ CEFs in 10ml DMEM+2% FBS and aliquoted onto a new 96-well plate in an attempt to have single HVT plaques per well. The 96-well plates were duplicated after 4 days of incubation and wells that contained plaques were tested for the presence of recombinant HVT and absence of parental virus by IFA and PCR. Again the wells that appeared to have more recombinant virus and less parent virus, by comparing the PCR banding results, were harvested and adjusted to 1ml and aliquoted onto new 96-well plates (the same as before). After five rounds of purification of virus infected cells, recombinant HVT carrying two IBDV VP2 proteins was isolated and the purity of the recombinant virus was tested by PCR to confirm the absence of parental virus.

Sequencing of the insert region confirmed that vHVT202 contains the correct sequences of guinea pig CMV promoter, the IBDV Variant E wildtype VP2 gene, and the synthetic poly A tail as shown in the sequence of the donor plasmid HVT SORF3-US2 gpVar-Ewtsyn (SEQ ID NO:39).

### Analysis of recombinant by PCR

DNA was extracted from a stock virus by phenol/chloroform extraction, ethanol precipitated, and resuspended in 20mM HEPES. PCR primers were designed to specifically identify the Variant E wt gene, the promoter, the polyA, as well as, the purity of the recombinant virus from HVT parental virus. PCR was performed using 200 µg of DNA template along with the specified primers pairs indicated in Table 1. PCR cycling conditions are as follows (unless otherwise noted): 94°C - 2 min; 30 cycles of 94°C - 30 sec, 55°C - 30 sec, 68°C - 3 min; 68°C - 5 min.

Purity of recombinant virus was verified by PCR using primer pairs that are specific to the HVT flanking arms, the gpCMV promoter, the Variant E gene and the syn tail. Primers, specific to SB1, MDV serotype 2 (SB1US1.FP +SB1Sorf4.RP) were also included in the analysis. The PCR results demonstrate that recombinant virus vHVT202 carries the intended expression cassette and the virus stock is free from detectable amounts of parental HVT virus.

### Immunofluorescent staining of recombinant vHVT202 virus expressing two VP2 proteins of IBDV

For immunofluorescence testing, the P3 material was diluted 1:100 in media. Approximately 50 µl of the diluted virus was added to 10 ml of DMEM+2% FBS with 1x10⁷ CEFs and then aliquoted onto a 96 well plate (100 µl/well). The plates were incubated for 4 days at 37°C+5% CO₂ until viral plaques were visible. The plates were fixed with 95% ice-cold acetone for three minutes and washed three times with PBS. One well was used for chicken anti-sera against Newcastle Disease Virus (lot#C0139, Charles Rivers Laboratory) at 1:1000 was added and the plates were incubated at 37°C for 1 hour. The other well was used for chicken anti-sera against IBDV (lot#G0117) After one hour incubation, the plates were washed three times with PBS and FITC anti-chicken (cat# F8888, Sigma) was added at 1:500. Again the plates were incubated at 37°C for 1 hour. After one hour incubation the cells were rinsed three times with PBS and visualized with a fluorescent microscope using fluorescein isothiocyanate (FITC) filter.

The immuno fluorescent staining results indicate that vHVT202 exhibited a very strong expression of the VP2 protein when the polyclonal sera against both classical and variant E VP2 proteins were used.

### Conclusion

Based on PCR and immunofluorescence analysis, vHVT202 is a recombinant HVT in which a VP2 gene of variant E IBDV under the control of gpCMV promoter was successfully inserted into a recombinant HVT background that already expresses the VP2 gene of classical IBDV. Consequently vHVT202 carries both VP2 genes of variant E and classical IBDV and it is free of any detectable parental vHVT306 virus.

### Example 5 Construction of recombinant vSB1-009, vSB1-004, vSB1-006, vSB1-007, vSB1-008, and vSB1-010 expressing NDV-F

### Example 5.1 Construction of vSB1-009, vSB1-004, vSB1-006, vSB1-007, and vSB1-008

The aim of the study is to construct a recombinant SB-1 viral vector vSB 1-009 in which an expression cassette containing SV40 promoter and Newcastle disease virus fusion protein (NDV-F) is inserted to replace UL44 coding sequence (gC) of SB-1.

A donor plasmid pSB1 44 cds SV FCAopt was constructed containing UL44 flanking arms of SB1 virus, SV40 promoter and NDV F codon optimized gene sequence (SEQ ID NO:5, coding for SEQ ID NO:6).

### Generation of recombinant virus

A standard homologous recombination procedure was followed by co-electroporation of secondary CEF cells using donor plasmid pSB1 44 cds SV FCAopt and viral DNA isolated from SB-1 virus infected CEFs. Essentially the procedure described in example 1 for vHVT114 was followed to generate, plaque purify and characterize recombinants by immunofluorescence.

After five rounds of plaque purification, pure recombinant virus (vSB1-009) was isolated and the purity of vSB1-009 was tested by IFA and PCR to validate the appropriate insertion as well as no remnant parental virus.

### PCR analysis

Viral DNA was extracted from vSB 1-009 pre-master seed virus (pre-MSV) stock by QIA DNeasy Blood & Tissue Kit (Qiagen cat#69506). PCR primers were designed to identify the presence of the NDV F optimized, the NDV F wild type, the SV40 promoter, the mCMV promoter, the UL44 flanking arms of SB-1 virus and HVT virus. PCR amplifications were performed using approximately 200ng of DNA template along with the primer pairs.

PCR amplification with various primers confirmed that the vSB1-009 has the expected amplification patterns and amplicons.

### Expression analysis

Indirect immunofluorescent assay (IFA) was performed on the vSB 1-009 pre-MSV stock to examine the expression of NDV F gene and SB-1 virus antigen. The CEFs that were inoculated with vSB 1-009 were fixed with ice-cold 95% acetone for three minutes at room temperature and air-dried for 10 min. The plates were washed with PBS, then two primary antibodies, chicken anti-Newcastle Disease Virus sera (Charles Rivers Laboratories cat#10100641, lot#C0117A) at 1:500 dilution and Y5.9 monoclonal antibody against SB-1 virus (Merial Select, Gainesville, GA) at 1:3000 dilution were added and the plates were incubated for 45 min at 37°C. After three washes with PBS, two secondary antibodies, goat anti-chicken IgG-fluorescein (KPL cat#.02-24-06, lot#110020) at 1:500 dilution and donkey anti-mouse IgG-Alexa Fluor 568 (Molecular Probe #A10037, lot#989784) at 1:250 dilution were added. The plates were incubated at 37°C for 45 min and followed by three washes with PBS. The wells were screened for IFA positive plaques with a fluorescent microscope using fluorescein isothiocyanate (FITC) and tetramethylrhodamine isothiocyanate (TRITC)-filters of Nikon Eclipse Ti inverted microscope. Similarly, reactivity of vSB 1-009 with NDV F Mab was examined by Dual IFA using anti-MDV serum (Charles River Laboratories, cat#10100628, lot#D0111) (1/300 dilution) and anti-NDV F monoclonal antibody (1/300 dilution) as primary antibody. The goat anti-chicken IgG-fluorescein (KPL cat#.02-24-06, lot#110020) (1:500 dilution) and donkey anti-mouse IgG-Alexa Fluor 568 (Molecular Probe #A10037, lot#989784) (1:250 dilution) were used as secondary antibodies. The wells were observed to identify the IFA positive plaques with a fluorescent microscope using FITC- and TRITC-filters of Nikon Eclipse Ti inverted microscope.

IFA results indicate that vSB 1-009 expresses the NDV F protein in virus-infected CEF. Over 500 vSB 1-009 plaques were counted for NDV F protein expression as well as SB-1 virus specific protein expression with dual IFA. The expression of NDV F protein completely matched with SB-1 virus antigen expression in each virus plaque (Table 7).

**Table 7 Dual IFA of vSB1-009**

| Virus | Dual IFA plate#1 (total 189 plaques) | | Dual IFA plate#2(total 361 plaques) | |
|---|---|---|---|---|
| | Anti-NDV serum positive plaques | Anti-SB-1 MAb positive plaques | Anti-NDV serum positive plaques | Anti-SB-1 MAb positive plaques |
| vSB1-009 pre-MSV | 189 | 189 | 361 | 361 |

NDV F Mab reactivity was confirmed by Dual IFA. Over 200 vSB1-009 plaques were examined for NDV F Mab reactivity as well as anti-MDV serum reactivity. The reactivity with NDV F Mab completely matched with anti-MDV serum reactivity in each virus plaque (Table 8).

**Table 8 Reactivity of vSB1-009 with anti-NDV F Mab**

| Virus | Dual IFA (total 254 plaques) | |
|---|---|---|
| | Anti-MDV serum positive plaques | Anti-NDV F MAb positive plaques |
| vSB1-009 pre-MSV | 254 | 254 |

### Southern Blot analysis

Total genomic DNA was extracted from vSB 1-009 pre-MSV stock infected CEFs. The genomic DNA of vSB1-009, SB-1 virus (negative control), pSB1 44 cds SV FCA opt donor plasmid were digested at 37°C with EcoRI, Ncol, and KpnI restriction endonucleases separately. The restriction fragments were separated by a 0.8 % agarose gel electrophoresis and transferred onto a positively charged Nylon membrane. After transfer, the membrane was treated with 0.4M NaOH and then neutralized with 2XSSC-HCl buffer. The membrane was then air dried and UV crosslinked.

Following the North2South Chemiluminescent Hybridization and Detection Kit (Thermo Scientific cat#89880) manufacturers' instructions, the membrane was pre-hybridized for 1 hr and then hybridized with the probe at 55°C for overnight. For hybridization, two probes were used; 1) the SbfI fragment of pSB1 44 cds SV FCA opt as NDV F cassette probe, 2) the SmaI-EcoRI fragment of pUC57 SB1 44 arm (GenScript) as recombination arm probe. After the overnight hybridization, several stringency washes were conducted until the membrane was placed in blocking buffer with the addition of Streptavidin-HRP. After rinsing the membrane of any unbound Streptavidin-HRP, the substrate solution of Luminal and peroxide were added. The membrane was then exposed to X-ray film and the film was developed.

The Southern blot results were as expected based on Vector NTI map analysis. The NDV F cassette (SV40 promoter, NDV-F CA02 codon optimized gene) replaced the UL44 coding sequences of SB-1 virus.

### Genomic analysis

The genomic DNA of vSB 1-009 pre-MSV stock was conducted by nucleotide sequence determination of the region of recombination arm as well as inserted gene cassette. Primers were designed and used to amplify the entire NDV-F gene cassette including the recombination arms.

The vSB1-009 sequence (donor plasmid pSB1 44 cds SV FCAopt) containing the recombinant arms, SV40 promoter and NDV F codon-optimized gene was confirmed to be correct as shown in SEQ ID NO: 19.

### Western blot analysis

The CEF monolayer was infected with vSB 1-009 pre-MSV at MOI ∼ 0.1. After a 5-day incubation, the CEFs were pelleted and washed with PBS followed by lysis with IP Lysis/Wash buffer of Pierce Classic IP Kit (Thermo Scientific cat#26146) according to the manufacturers' protocols. The lysate was pre-cleared and incubated with 100µl of anti-NDV F monoclonal antibody to make the immune complex. The immune complex was captured by Protein A/G Plus Agarose and after removing of the un-bounded immune complex by washing steps, the 50µl of sample buffer was used to elute under non-reducing conditions. The uninfected CEFs were included as a control. The 20µl of eluted samples were separated in 10% Bis-Tris gels by electrophoresis. After the electrophoresis, the separated proteins in a gel were transferred onto PVDF membrane. The Protein Detection TMB Western Blot Kit (KPL cat#54-11-50) was used to detect the NDV antigens onto PVDF membrane with chicken anti-NDV serum (Charles River Laboratories Laboratories cat#10100641, lot#C0117A), and goat anti-chicken IgG-peroxidase conjugate (KPL cat#14-24-06) following the manufacturers' protocols.

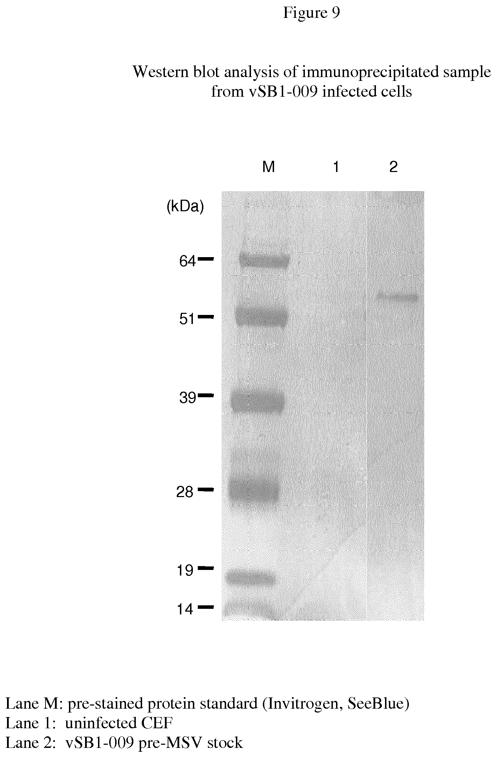

The NDV F protein expression of vSB1-009 was confirmed by two-step immunodetection. First, the expressed NDV F proteins from vSB 1-009 infected CEF lysate were captured by the immunoprecipitation using anti-NDV F monoclonal antibody 001C3. Subsequently Western blot analysis using anti-NDV polyclonal serum (Charles River Laboratories cat# 10100641, lot#C0117A) was applied to detect the NDV F protein in the captured samples (NDV F protein-monoclonal antibody complex) (FIG.9). An approximately 55 kDa protein in vSB 1-007 pre-MSV lysates was detected by anti-NDV serum that corresponding the expected size of NDV F1 fusion protein (FIG.9).

Generation and characterization of HVT recombinants vSB1-004, vSB1-006, vSB 1-007 and vSB 1-008 were essentially done in the same way as for vSB1-009 described in this example. Table 9.1 shows the features unique to each construct around the expression cassettes, including the respective sequences. The generation and characterization of recombinant SB1 viral vectors were also described in US patent application serial No. USSN 13/689, 572 filed on November 29, 2012 (Merial limited), which is incorporated herein by reference in their entirety.

**Table 9.1 Characteristics of the expression cassettes of SB1 recombinants**

| Name | Parental virus | Promoter | F gene | Locus |
|---|---|---|---|---|
| vSB1-009 | SB1 | SV40 | Opt-CA02 | UL44 (gC) |
| vSB1-004 | SB1 | mCMV IE | Wt-VIId | US10 |
| vSB1-006 | SB1 | SV40 | Opt-VIId | UL55/LORF5 |
| vSB1-007 | SB1 | SV40 | Opt-VIId | UL44 (gC) |
| vSB1-008 | SB1 | SV40 | Opt-CA02 | UL55/LORF5 |

### Example 5.2 Construction of double construct vSB1-010

### Donor plasmid SB1US2 gpVIIdwtsyn construction

Using the plasmid HVT SOrf3-US2 gpVar-Ewt Syn, the gpCMV, Variant E, Syn tail was removed by SbfI digestion. This fragment was ligated into the SB1 US2 donor plasmid. The Variant E gene was cut out by *NotI* and replaced by NDV-F VIId wt. The synthetic NDV-F VIId wild type gene (SEQ ID NO:3 encoding SEQ ID NO:4) was excised from pUC57 NDV-F VIId wt plasmid (synthesized by GeneScript) using NotI digestion. Ligated material was transformed using Top10 Oneshot kit (cat#C404002, Invitrogen). Bacterial colonies were grown in LBamp broth, plasmid extracted by using Qiagens MiniSpin Prep kit, and screened for insert orientation using *NcoI*+*SalI* digestion. The correct donor plasmid was designated pSB1 US2 gpVIIdwt Syn. Table 9.2 shows the features unique to the construct around the expression cassettes, including the respective sequences. Large scale cultures were grown and plasmid extraction was done by using Qiagens Maxi Prep kit. Transient expression of the maxi preps was verified using Fugene Transfection Reagent in Chicken Embryo Fibroblast Cells (CEF's) and chicken polyclonal sera against NDV-F.

### Recombinant generation

A standard homologous recombination procedure was followed by co-electroporation of secondary CEF cells using pSB1 US2 gpVIIdWt Syn donor plasmid and viral DNA isolated from vSB 1-009 (vSB 1-009 is already a recombinant virus expressing CA02 F gene of NDV). Essentially the procedure described in example 1 for vHVT114 was followed to generate, plaque purify and characterize recombinants by immunofluorescence.

After five rounds of plaque purification, pure recombinant virus (vSB1-010) was isolated and the purity of vSB1-010 was tested by IFA and PCR to validate the appropriate insertion as well as no remnant parental virus.

**Table 9.2 Characteristics of the expression cassette of vSB1-010**

| Name | Parental virus | Promoter | F gene | Locus |
|---|---|---|---|---|
| vSB1-010 | vSB 1-009 | Guinea pig CMV | NDV-F VIId | SORF4-US2 |

Sequencing of the insert region confirmed that vSB1-010 contains the correct sequences of guinea pig CMV promoter and the NDV-F VIId wt gene as shown in the sequence of the donor plasmid SB1US2 gpVIIdwtsyn (SEQ ID NO:40).

### Analysis of recombinant by PCR

DNA was extracted from a stock virus by phenol/chloroform extraction, ethanol precipitated, and resuspended in 20mM HEPES. PCR primers were designed to specifically identify the NDV-F VIId wt gene, the promoter, the polyA, as well as, the purity of the recombinant virus from SB1 parental virus. PCR was performed using 200 µg of DNA template along with the specified primers pairs indicted in Table 1. PCR cycling conditions are as follows (unless otherwise noted): 94°C - 2 min; 30 cycles of 94°C - 30 sec, 55°C - 30 sec, 68°C - 3 min; 68°C - 5 min.

Purity of recombinant virus was verified by PCR using primer pairs that are specific to the SB1 flanking arms, the gpCMV promoter, the NDV-F VIId wt gene and the syn tail. Primers, specific to HVT, MDV serotype 3 (MB080 +MB081) were also included in the analysis. The PCR results demonstrate that recombinant virus vSB1-010 carries the intended expression cassette and the virus stock is free from detectable amounts of parental SB 1-009 virus.

### Immunofluorescent staining of recombinant vSB1-010 virus expressing two NDV-F proteins

For immunofluorescence testing, the P3 material was diluted 1:100 in media. Approximately 50µl of the diluted virus was added to 10 ml of DMEM+2% FBS with 1x10⁷ CEFs and then aliquoted onto a 96 well plate (100 µl/well). The plates were incubated for 5 days at 37°C+5% CO₂ until viral plaques were visible. The plates were fixed with 95% ice-cold acetone for three minutes and washed three times with PBS. Chicken anti-sera against Newcastle Disease Virus (lot#C0139, Charles Rivers Laboratory) at 1:1000 was added and the plates were incubated at 37°C for 1 hour. After one hour incubation, the plates were washed three times with PBS and FITC anti-chicken (cat# F8888, Sigma) was added at 1:500. Again the plates were incubated at 37°C for 1 hour. After one hour incubation the cells were rinsed three times with PBS and visualized with a fluorescent microscope using fluorescein isothiocyanate (FITC) filter.

The immunofluorescent staining results indicate that vSB1-010 exhibited a very strong expression of the NDV-F protein when the polyclonal sera against both CA02 and VIId F proteins of NDV were used.

### Conclusion

Based on PCR testing and immunofluorescence analysis, vSB1-010 is a recombinant SB-1 in which VIId-F gene of NDV under the control of gpCMV promoter was successfully inserted into a vSB1-009, which already expresses the CA02-F gene of NDV. Consequently vSB1-010 carries both VIId and CA02 F genes of NDV genotypes and it is free of any detectable parental vSB 1-009.

### Example 6 Efficacy of vHVT110, vHVT111, vHVT114 and vSB1-004 expressing the NDV F gene against challenges with NDV Chimalhuacan and Malaysian (MAL04-01) strains at 14 days of age in SPF chickens

The aim of the study was to assess the efficacy of three HVT recombinant constructs (vHVT110, vHVT111 and vHVT114) and one SB1 recombinant construct (vSB1-004) expressing the NDV F gene against Newcastle disease challenges (Chimalhuacan and Malaysian virus strains) performed at 14 days of age in SPF chickens.

The characteristics of these 5 vaccine candidates are described in Table 10 below.

**Table 10 Characteristics of the vectors used in the challenge study**

| Name | Parental virus | Promoter | F gene | Poly-A | Locus |
|---|---|---|---|---|---|
| vHVT110 | HVT | mCMV IE | Wt-VIId | SV40 | IG1 |
| vHVT111 | HVT | SV40 | Wt-VIId | SV40 | IG1 |
| vHVT114 | HVT | SV40 | Opt-VIId | SV40 | IG1 |
| vSB1-004 | SB-1 | mCMV IE | Wt-VIId | SV40 | US10 |

On D0, 100 one-day-old SPF chickens were randomly allocated into 10 groups of 10 birds. The birds were injected by subcutaneous injection in the neck at D0 with 0.2 mL of recombinant vaccines containing a target dose of 2000 pfu as described in Table 11 below. It should be mentioned that the titer of vSB1-004 (31600 pfu) administered to birds of groups 6 was well above the target. The birds were challenged by the intramuscular route on D14 with velogenic ND Malaysia (genotype VIId) strain (sub-groups "a") or with virulent ND Chimalhuacan (genotype V) strain (sub-groups "b").

**Table 11 Challenge study with vHVT110, vHVT111, vHVT114 and vSB1-004**

| **Group** | **Vaccine at day-old (D0)** | **NDV serology at D14*** | **% protection against mortality/morbidity after Newcastle challenge at 14 days of age (D14)** | |
|---|---|---|---|---|
| | | | **Malaysian strain** | **Chimalhuacan strain** |
| **G1a** | - | 0/10 | 0%/0% | - |
| **G1b** | - | | - | 0%/0% |
| **G2a** | vHVT110 | 7/10 | 100%/89% | - |
| **G2b** | vVHT110 | | | 100%/70% |
| **G3a** | vHVT111 | 2/10 | 30%/20% | - |
| **G3b** | vHVT111 | | - | 67%/11% |
| **G4a** | vHVT114 | 9/10 | 100%/100% | - |
| **G4b** | vHVT114 | | - | 89%/89% |
| **G5a** | vSB 1-004 | 3/10 | 70%/50% | - |
| **G5b** | vSB 1-004 | | - | 40%/30% |

| | | | | |
|---|---|---|---|---|
| *Number of birds positive by NDV HI test/total tested | | | | |

Each group was monitored before and after challenge. Clinical signs after challenge were scored daily as follows: healthy / with specific symptoms (ruffled feathers, prostration, torticollis, tremor) / dead. On D14, serum samples were taken in each group for serology (Newcastle Disease virus haemagglutination inhibition (HI) test).

As expected, the unvaccinated animals (G1a and G1b) displayed no NDV antibodies on D14. A low titer seroconversion (mean HI titer <0.6 log10) was obtained in each vaccinated group (sub-groups "a" and "b" of G2 to G5) confirming the vaccine takes. The number of positive birds/total tested was group-dependent and was the highest (90%) in vHVT114 vaccinated birds (see Table above).

Percentages of protection against mortality and morbidity are reported in the table above. Full susceptibility was observed in the control groups G1a and G1b thus validating the high severity of both challenges. Lowest protection levels were observed in the groups vaccinated with vHVT111 or vSB1-004. Highest protection rates against morbidity and mortality were obtained in the groups vaccinated with vHVT110 or vHVT114 whatever the challenge strain used (homologous strain *i.e.* Malaysian genotype VIId or heterologous strain *i.e.* Chimalhuacan genotype V). There was a correlation between the % of birds positive by HI test before challenge and the % protection.

The difference of protection obtained between vHVT110 and vHVT111 clearly illustrates the importance of the promoter, the mCMV IE promoter being more potent than the SV40 promoter for the transcription of the wild type (wt) genotype VIId F gene. The difference of protection obtained between vHVT111 and vHVT114 illustrates the importance of the nucleotide sequence of the F gene, the optimized sequence being more potent than the wild type (or native) sequence.

In conclusion, the results of this study showed the importance of the promoter and the nucleotide sequence of the F gene in the ND protection induced by Marek's disease vector vaccines. An optimal combination of these factors needs to be found to reach the best efficacy performances as for vHVT114.

### Example 7 Efficacy of vHVT114, vHVT116, vHVT301, vHVT302 and vHVT303 expressing the NDV F gene against challenges with NDV Texas GB strain at 14 days of age in SPF chickens

The aim of the study was to assess the efficacy of 2 single HVT recombinant constructs (vHVT114 and vHVT116) expressing the NDV F gene and 3 double HVT recombinant constructs (vHVT-301, vHVT302 and vHVT303) expressing both NDV F and IBDV VP2 genes against Newcastle disease challenge (Texas GB strain, genotype II) performed at 14 days of age in SPF chickens.

The characteristics of these 4 vaccine candidates are described in Table 12 below.

**Table 12 Characteristics of the vectors used in the challenge study**

| Name | Parental virus | Promoter | F gene | Poly-A | Locus |
|---|---|---|---|---|---|
| vHVT114 | HVT | SV40 | Opt-VIId | SV40 | IG1 |
| vHVT116 | HVT | SV40 | Opt-V | SV40 | IG1 |
| vHVT301 | vHVT13* | SV40 | Wt-VIId | SV40 | IG2 |
| vHVT302 | vHVT13 | US10 | Opt-VIId | US10 | US10 |
| vHVT303 | vHVT13 | US10 | Opt-V | US10 | US10 |

| | | | | | |
|---|---|---|---|---|---|
| *vHVT13 is the active ingredient of the licensed Vaxxitek HVT-IBD vaccine based on an HVT vector expressing the IBDV VP2 gene (see US 5,980,906 and EP 0 719 864). | | | | | |

On D0, 120 one-day-old SPF chickens were randomly allocated into 6 groups of 20 birds. The birds were injected by subcutaneous injection in the neck at D0 with 0.2 mL of recombinant vaccines containing a target dose of 1000 pfu as described in Table 13 below. The birds were challenged by the intramuscular route on D14 with 4.5 log10 EID50 velogenic ND Texas GB (genotype II) strain.

**Table 13 Results of efficacy**

| **Group** | **Vaccine at day-old (D0)** | **% clinical protection (number infected/total) after Newcastle challenge at 14 days of age (D14)** |
|---|---|---|
| **G1** | **-** | 0% (20/20) |
| **G2** | vHVT114 | 80% (4/20) |
| **G3** | vHVT116 | 70% (6/20) |
| **G4** | vHVT301 | 15% (17/20) |
| **G5** | vHVT302 | 52.6% (9/19)* |
| **G6** | vHVT303 | 15% (17/20) |

| | | |
|---|---|---|
| *1 bird died before challenge | | |

Each group was monitored before and after challenge. NDV clinical signs and mortality were recorded after challenge.

Percentages of clinical protection are reported in the table above. Full susceptibility was observed in the non-vaccinated challenged control group G1 thus validating the high severity of both challenges. Partial protection was observed for the 5 vaccine candidates, the best performances being obtained with vHVT114 and vHVT116. Among the double HVT recombinants, the vHVT302 was the most protective. It performed better than vHVT303 suggesting that the optimized genotype VIId NDV F gene may be better cross-protective against genotype II challenge than the optimized genotype V NDV F gene. A similar tendency was observed with single HVT, the vHVT14 (VIId gene) performing slightly better than vHVT16 (V gene) but the difference was less pronounced. These results indicated that both genotypes VIId and V NDV F genes inserted in the HVT vector provide cross-protection against a heterologous genotype II NDV challenge; the VIId gene may potentially be more cross-protective. The vHVT302 induced a better ND protection than vHVT301 confirming the importance of the promoter, poly-A and locus of insertion. In conclusion, the results of this study showed the very good early ND protection induced by tested Marek's disease vector vaccines, especially for the tested single HVT-ND.

### Example 8 Efficacy of vHVT114, vHVT116, vSB1-007, vSB1-008 (alone or with vHVT13) and vHVT 304 against challenges with NDV ZJ1 (genotype VIId) and California/02 (genotype V) at 21 days of age in SPF chickens

The aim of the study was to assess the efficacy of 2 single HVT recombinant constructs (vHVT114 and vHVT116), 2 SB1 recombinant constructs (vSB1-007 & vSB 1-008) expressing the NDV F gene and a double HVT recombinant (vHVT304) against Newcastle disease challenge with NDV ZJ1 (genotype VIId) and California/02 (genotype V) performed at 21 days of age in SPF chickens.

The characteristics of these 5 vaccine candidates are described in Table 14 below.

**Table 14 Characteristics of the vectors used in the challenge study**

| Name | Parental virus | Promoter | F gene | Poly-A | Locus |
|---|---|---|---|---|---|
| vHVT114 | HVT | SV40 | Opt-VIId | SV40 | IG1 |
| vHVT116 | HVT | SV40 | Opt-V | SV40 | IG1 |
| vSB1-007 | SB-1 | SV40 | Opt-VIId | gC | UL44 (gC) |
| vSB1-008 | SB-1 | SV40 | Opt-V | SV40 | IG1 |
| vHVT304 | vHVT13* | SV40 | Opt-VIId | Synth | IG2 |

| | | | | | |
|---|---|---|---|---|---|
| *vHVT13 is the active ingredient of the licensed Vaxxitek HVT-IBD vaccine based on an HVT vector expressing the IBDV VP2 gene (see US 5,980,906 and EP 0 719 864). | | | | | |

On D0, 158 one-day-old SPF chickens were randomly allocated into 6 groups of 24 birds (vaccinated) and 1 group of 12 birds (non-vaccinated controls). The birds were injected by subcutaneous injection in the neck at D0 with 0.2 mL of recombinant vaccines containing a target dose of 1000 pfu as described in Table 15 below. The birds were then separated into two sub-groups, each sub-group being challenged by the intramuscular route on D21 with 5 log10 EID50 of either NDV ZJ1 (genotype VIId) or California/02 (genotype V) velogenic strain.

**Table 15 Results of efficacy**

| **Group** | **Vaccine at day-old (D0)** | **% clinical protection** | |
|---|---|---|---|
| | | **CA/02 (genotype V)** | **ZJ1 (genotype VIId)** |
| **G1** | - | 0% | 0% |
| **G2** | vHVT1 14 | 100% | 100% |
| **G3** | vHVT116 | 100% | 90% |
| **G4** | vSB 1-007 | 92% | 100% |
| **G5** | vSB1-008 | 100% | 100% |
| **G6** | vSB1-008+vHVT13 | 100% | 83% |
| **G7** | vHVT304 | 92% | 75% |

Each group was monitored before and after challenge. Technical problems observed with isolators reduced the number of birds in group 2 (vHVT114: from 24 to 14) and in group 3 (vHVT116: from 24 to 20). NDV clinical signs were recorded after challenge. Serum was collected from blood samples taken from birds of groups 2 and 7 before challenge (D21) for NDV serology by HI test using each challenge strains as antigen.

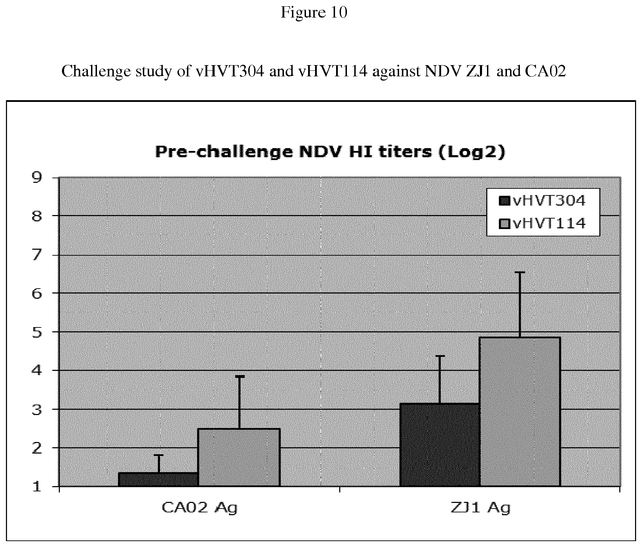

Mean serologic HI titers in G2 and G7 before challenge are shown in Figure 10. HI titers were higher with the ZJ1 antigen in both groups. The HI titers induced by vHVT114 were higher than those induced by vHVT304.

Percentages of protection against mortality and morbidity are reported in the table above. Full susceptibility was observed in the non-vaccinated challenged control group G1 thus validating the high severity of both challenges. All vaccines induced high levels (≥75%) of protection against both challenges. Full clinical protection against both challenges was induced by vHVT114 and vSB1-008. Following a similar tendency as the HI titers, the ND protection induced by vHVT304 was slightly lower than that induced by vHVT114.

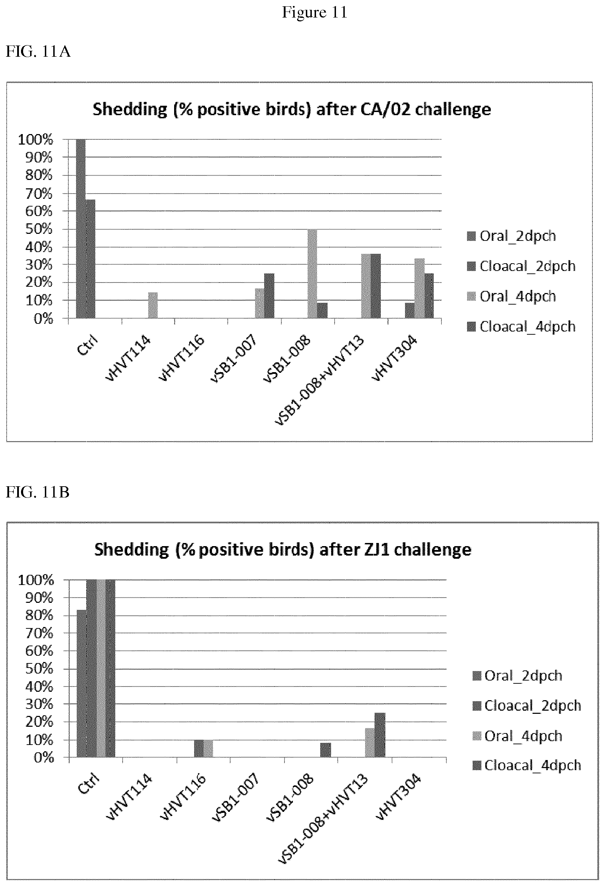

The shedding was evaluated after challenge by real time RT-PCR in oral and cloacal swabs taken 2 and 4 days post-challenge. Percentage of positive (Ct<40) birds are shown for both challenges in FIG. 11A and 11B. Note that all 6 birds were dead at 4 dpch in the control group challenged with the CA/02 isolate and only one bird (out of 6) was still alive at 4 dpch in the control group challenged with ZJ1. Shedding was detected in all control birds. Reduction of the percentage of birds positive for shedding was observed in all vaccinated groups.

In conclusion, the results of this study showed the very good ND protection at 3 weeks of age induced by tested Marek's disease vector vaccines.

### Example 9 Efficacy of vHVT114, vSB1-007, vSB1-009, vHVT306 and vHVT307 vaccines against challenges with NDV Texas GB strain at 28 days of age in SPF chickens

The aim of the study was to assess the efficacy of combinations of different Marek's disease vector vaccines expressing the NDV F and/or the IBDV VP2 gene against Newcastle disease challenge (Texas GB strain, genotype II) performed at 28 days of age in SPF chickens.

The characteristics of the 5 recombinant vaccine candidates tested in this study are described in Table 16 below.

**Table 16 Characteristics of the vectors used in the challenge study**

| Name | Parental virus | Promoter | F gene | Poly-A | Locus |
|---|---|---|---|---|---|
| vHVT114 | HVT | SV40 | Opt-VIId | SV40 | IG1 |
| vSB 1-007 | SB-1 | SV40 | Opt-VIId | gC | UL44 (gC) |
| vSB 1-009 | SB-1 | SV40 | Opt-V | gC | UL44 (gC) |
| vHVT306 | vHVT13 | SV40 | Opt-VIId | Synth | SORF3-US2 |
| vHVT307 | vHVT13 | SV40 | Opt-V | Synth | SORF3-US2 |

The Marek's disease virus serotype 1 (CVI988 (or Rispens) strain; Gallid herpesvirus 2) and serotype 2 (SB-1 strain; gallid herpesvirus 3) vaccines were used also in combination with recombinant viruses in some of the groups.

On D0, 135 one-day-old SPF chickens were randomly allocated into 9 groups of 15 birds. The birds were injected by subcutaneous injection in the neck at D0 with 0.2 mL containing a target dose of 2000 pfu for recombinant vaccines (vSB 1-007, vSB 1-009, vHVT13, vHVT306, vHVT307, vHVT114), and 1000 pfu for parental Marek's disease vaccine strains (SB-1 and CVI988). The design of the 9 groups is shown in Table 17 below. The birds were challenged by the intramuscular route on D28 with 4.0 log10 EID50 velogenic ND Texas GB (genotype II) strain.

**Table 17 Results of efficacy**

| **Group** | **Vaccine at day-old (D0)** | **% ND protection after Newcastle disease challenge at 28 days of age** |
|---|---|---|
| **G1** | - | 0% |
| **G2** | vSB1-007+vHVT13 | 80% |
| **G3** | vSB1-009 | 100% |
| **G4** | vSB 1-009+vHVT13 | 86% |
| **G5** | vSB 1-009+vHVT13+CVI988 | 93% |
| **G6** | vHVT306+SB-1 | 100% |
| **G7** | vHVT307 | 100% |
| **G8** | vHVT307+SB-1 | 93% |
| **G9** | vHVT114+vHVT13+SB-1 | 100% |

Each group was monitored before and after challenge. NDV clinical signs after challenge were recorded.

Percentages of protection against mortality and morbidity are reported in the table above. Full susceptibility was observed in the non-vaccinated challenged control group G1 thus validating the high severity of challenge. Excellent levels of protection were observed in all vaccinated groups. Birds from G3, G6, G7 and G9 were fully protected. This study shows that the vSB1-ND candidates can be co-administered with vHVT13 and CVI988 and still provide a very good ND protection. Similarly, double HVT-IBD+ND are compatible with SB-1 and vHVT-ND (vHVT114) is compatible with vHVT13 and SB-1.

In conclusion, the results of this study showed the lack of interference on ND protection induced by the tested Marek's disease parental and vector vaccines.

### Example 10 Efficacy of vHVT114, vHVT307, vSB1-007 and vSB1-009 in combination with vHVT13 against challenges with NDV Chimalhuacan strain (genotype V) at D28 in SPF chickens

The aim of the study was to assess the efficacy of 1 HVT recombinant construct (vHVT114) and 2 SB1 recombinant constructs (vSB1-007 and vSB 1-009) expressing the NDV F gene in combination with vHVT-IBD (vHVT13), as well as a double HVT vHVT307 expressing both NDV F and IBDV VP2 against Newcastle disease challenge (Chimalhuacan, genotype V) performed at 28 days of age in SPF chickens.

The characteristics of these 4 vaccine candidates are described in Table 18 below.

**Table 18 Characteristics of the vectors used in the challenge study**

| Name | Parental virus | Promoter | F gene | Poly-A | Locus |
|---|---|---|---|---|---|
| vHVT114 | HVT | SV40 | Opt-VIId | SV40 | IG1 |
| vSB 1-007 | SB-1 | SV40 | Opt-VIId | gC | UL44 (gC) |
| vSB 1-009 | SB-1 | SV40 | Opt-V | gC | UL44 (gC) |
| vHVT307 | vHVT13 | SV40 | Opt-V | Synth | SORF3-US2 |

On D0, 45 one-day-old SPF chickens were randomly allocated into 4 groups of 10 birds and 1 group of 5 birds (unvaccinated control group). The birds were injected by subcutaneous injection in the neck at D0 with 0.2 mL of recombinant vaccines containing a target dose of 2000 pfu as described in Table 19 below. The birds were challenged by the intramuscular route on D28 with 5.0 log10 EID50 velogenic Chimalhuacan (genotype V) strain.

**Table 19 Results of efficacy**

| **Group** | **Vaccine at day-old (D0)** | **% protection against mortality** | **% protection against morbidity** |
|---|---|---|---|
| **G1** | - | 0% | 0% |
| **G2** | vHVT114+vHVT13 | 100% | 100% |
| **G3** | vHVT307 | 80% | 80% |
| **G4** | vSB1-007+vHVT13 | 90% | 90% |
| **G5** | vSB1-009+vHVT13 | 90% | 90% |

Each group was monitored before and after challenge. NDV clinical signs were recorded after challenge. Oropharyngeal swabs were taken in the vaccinated groups at 5 and 7 days post-challenge to evaluate the viral load by real time RT-PCR.

Percentages of protection against mortality and morbidity are reported in the table above. Full susceptibility was observed in the non-vaccinated challenged control group G1 thus validating the high severity of challenge. Very good protection was observed in all 4 vaccinated groups, a full clinical protection being induced by vHVT114+vHVT13.

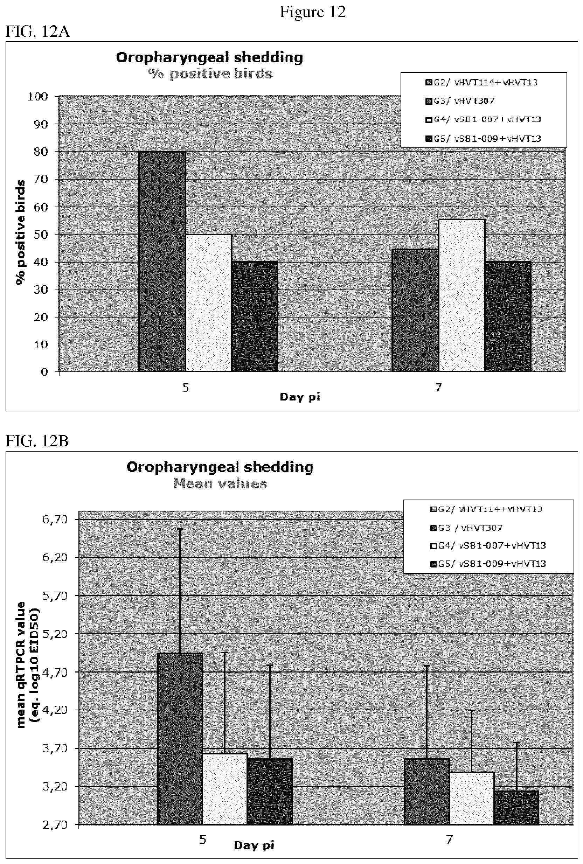

The percentage of positive birds and the mean shedding titer (expressed as log10 EID50 equivalent per mL) are shown in FIG. 12A and 12B. Surprisingly, no shedding was detected in G2 indicating a complete (against both clinical signs and shedding) ND protection induced by vHVT114 even if co-administered with vHVT13, in the tested conditions. The shedding levels detected in the other vaccinated groups were low with a slightly higher level detected in G3 (vHVT307) at 5 days post-infection (pi) only.

In conclusion, this example further illustrates the excellent ND protection induced by double HVT-IBD+ND recombinant or a combination of SB1-ND or HVT-ND and HVT-IBD (vHVT13) recombinant viruses. Contrary to the general belief in the field that a second HVT vaccine (regular HVT vaccines or recombinant HVT vaccines) interferes with the immunity to the foreign genes inserted into the first recombinant HVT vaccine, the present invention showed surprising result that vHVT114 in combination with vHVT13 offered excellent protection against NDV and no interference effect was observed.

### Example 11 Efficacy of vHVT306, vSB1-008 in combination with vHVT13 administered by SC or in ovo route against challenge with NDV Chimalhuacan strain (genotype V) at D28 in SPF chickens

The aim of the study was to assess the efficacy of the vHVT306 double HVT expressing both NDV F and IBDV VP2 genes, and the vSB 1-008 SB1 recombinant expressing the NDV F gene in combination with vHVT-IBD (vHVT13), administered by the *in ovo* or by the subcutaneous route against Newcastle disease challenge (Chimalhuacan, genotype V) performed at 28 days of age in SPF chickens.

The characteristics of these 2 ND vaccine candidates are reported in the table 14 (vSB1-008) and in table 16 (vHVT306).

The design of the groups is shown on Table 20. Sixty SPF embryonated eggs (after approximately 18 days and 18 hours of incubation; D-3) were used for the *in ovo* administration (20 per group for G1, G2 and G3). Fifty microliters of vaccine containing 2000 PFU were administered by the *in ovo* route using the IntelliLab System device from AviTech LLC (Salisbury, MD, USA). Hatchability and survival were recorded after *in ovo* administration. On D0, 20 one-day-old SPF chickens were randomly allocated into 2 groups of 10 birds (G4 and G5). The birds were injected by subcutaneous (SC) injection in the neck at D0 with 0.2 mL of recombinant vaccines containing a target dose of 2000 pfu as described in Table 20 below. Ten birds per group were challenged by the intramuscular route on D28 with 5.0 log10 EID50 velogenic Chimalhuacan (genotype V) strain.

**Table 20 Study design and results of ND efficacy**

| **Group** | **Vaccine at day-old (D0)** | **Admin. route** | **% protection against mortality** | **% protection against morbidity** |
|---|---|---|---|---|
| **G1** | vHVT13 | *In ovo* | 0% | 0% |
| **G2** | vHVT306 | *In ovo* | 100% | 100% |
| **G3** | vSB 1-008+vHVT13 | *In ovo* | 78% | 68% |
| **G4** | vHVT306 | SC | 100% | 100% |
| **G5** | vSB 1-008+vHVT13 | SC | 100% | 70% |

Each group was monitored before and after challenge. NDV clinical signs were recorded after challenge. Oropharyngeal swabs were taken in the vaccinated groups at 5 and 7 days post-challenge to evaluate the viral load by real time RT-PCR.

Full hatchability and viability were recorded up to D28 (challenge day) for birds of groups G1 and G2. Hatchability in G3 was 85% and one additional bird died after hatching in this group. The lower hatchability of that group may be due to egg incubator problems. Body weights of males and females in G1, G2 and G3 were similar at D1 and at D28.

Percentages of protection against mortality and morbidity are reported in the table 20. Full susceptibility was observed in the non-vaccinated challenged control group G1 thus validating the high severity of challenge. Very good protection was observed in all 4 vaccinated groups, a full clinical protection being induced by vHVT306 administered by both routes.

The percentage of positive birds and the mean shedding titer (expressed as log10 EID50 equivalent per mL) are shown in Table 21. Absence of detectable or very low shedding was observed in G2 and G4 vaccinated with vHVT306. The shedding levels detected in the groups vaccinated with vSB1-008+vHVT13 were higher especially at 5 days post-infection (pi).

**Table 21 Results of protection against shedding (percentage of birds with detectable shedding and mean viral load in log10) evaluated at D5 and D7 after NDV challenge**

| **Group** | **Vaccine at day-old (D0)** | **Admin. Route** | **Percent of positive birds (D5/D7 pi)** | **Mean viral load*** **(D5/D7 pi)** |
|---|---|---|---|---|
| **G2** | vHVT306 | *In ovo* | 0/0% | 2.7/2.7 |
| **G3** | vSB1-008+vHVT13 | *In ovo* | 100/38% | 5.2/3.2 |
| **G4** | vHVT306 | SC | 20/10% | 3.2/2.9 |
| **G5** | vSB1-008+vHVT13 | SC | 80/50% | 4.6/3.4 |

| | | | | |
|---|---|---|---|---|
| * Mean quantitative real time PCR value expressed in equivalent log10 EID50; the threshold is set at 2.7 log10. | | | | |

In conclusion, this example shows excellent ND protection induced by vHVT306 double HVT recombinant administered either by *in ovo* or by SC routes. The performance of vSB1-008+vHVT13 was slightly lower especially after *in ovo* administration, but it may be at least partially due to egg incubator problems. Indeed, the *in ovo* safety testing of another SB1-ND recombinant (vSB1-009) at 1000 or 4000 PFU associated with 6000 PFU of vHVT13 did not show any difference in hatchability and early survival with a group receiving 6000 PFU of vHVT13 only.

### Example 12 Efficacy of vHVT304, vHVT306, vSB1-007 and vSB1-008 in combination with vHVT13 against challenge with NDV Chimalhuacan strain (genotype V) at D42 in commercial broiler chickens

The aim of the study was to assess the efficacy of two double HVT (vHVT304 and vHVT306) expressing both NDV F and IBDV VP2 genes, and two SB1 recombinants (vSB1-007 and vSB1-008) expressing the NDV F gene in combination with vHVT-IBD (vHVT13) against Newcastle disease challenge (Chimalhuacan, genotype V) performed at 42 days of age in commercial broiler chickens.

The characteristics of these 4 ND vaccine candidates are reported in tables 14 and 16. The design of the groups is shown on Table 22. On D0, 55 one-day-old commercial broiler chickens were randomly allocated into 5 groups of 11 birds. The birds were injected by subcutaneous (SC) injection in the neck at D0 with 0.2 mL of recombinant vaccines containing a target dose of 2000 pfu as described in Table 22 below. Ten birds per group were challenged by the intramuscular route on D42 with 5.0 log10 EID50 velogenic Chimalhuacan (genotype V) strain.

**Table 22 Study design and results of ND efficacy**

| **Group** | **Vaccine at day-old (D0)** | **% protection against mortality** | **% protection against morbidity** |
|---|---|---|---|
| **G1** | vHVT13 | 0% | 0% |
| **G2** | vHVT304 | 82% | 82% |
| **G3** | vHVT306 | 100% | 100% |
| **G4** | vSB1-007+vHVT13 | 100% | 100% |
| **G5** | vSB 1-008+vHVT13 | 91% | 91% |

Each group was monitored before and after challenge. NDV clinical signs were recorded during 14 days after challenge. Oropharyngeal swabs were taken in the vaccinated groups at 5 and 7 days post-challenge to evaluate the viral load by real time RT-PCR.

Percentages of protection against mortality and morbidity are reported in the table 22. Full susceptibility was observed in the non-vaccinated challenged control group G1 thus validating the high severity of challenge. Very good protection was observed in all 4 vaccinated groups, a full clinical protection being induced by vHVT306 and by vSB1-007+vHVT13.

The percentage of positive birds and the mean shedding titer (expressed as log10 EID50 equivalent per mL) are shown in Table 23. The best reduction of shedding was induced by vHVT306 and vSB1-007+vHVT13, which were also the best candidates for clinical protection.

**Table 23 Results of protection against shedding (percentage of birds with detectable shedding and mean viral load in log10) evaluated at D5 and D7 after NDV challenge (pi)**

| **Group** | **Vaccine at day-old (D0)** | **Percent of positive birds (D5/D7 pi)** | **Mean viral load* (D5/D7 pi)** |
|---|---|---|---|
| **G2** | vHVT304 | 100/100% | 5.4/4.6 |
| **G3** | vHVT306 | 40/50% | 3.5/3.7 |
| **G4** | vSB1-007+vHVT13 | 80/70% | 3.8/4.8 |
| **G5** | vSB1-008+vHVT13 | 100/100% | 4.8/4.3 |

| | | | |
|---|---|---|---|
| * Mean quantitative real time PCR value expressed in equivalent log10 EID50; the threshold was set at 2.7 log10. | | | |

The vHVT306 ND protection was found to be better than that of vHVT304. These two double HVT contain the same NDV F expression cassette but inserted in two different loci, the IBDV VP2 one being inserted at the same position. This example therefore illustrates the importance of the locus of insertion in the design of HVT recombinants. The vSB1-007+vHVT13 was better than vSB1-008+vHVT13. The vSB1-007 genomic structure differs from that of vSB 1-008 in different aspects: locus of insertion, promoter, poly-adenylation signal and F gene origin. The combination of these foreign sequences and locus of insertion in vSB 1-007 were likely responsible for its better ND protection performances.

In summary, this example illustrates the importance of the locus of insertion and other regulatory sequences of the NDV expression cassette in the ND protection induced by HVT and MDV serotype 2 vectors.

### Example 13 Efficacy of double HVT-ND+IBD (vHVT304 and vHVT306) or SB1-ND (vSB1-008) in combination with vHVT13 recombinant vaccines, against challenge with a classical IBDV isolate on D14 in SPF chickens

The aim of the study was to assess the early IBD efficacy of double HVT recombinants vHVT304 and vHVT306 as well as that of vHVT13 co-administered with a SB1-ND (vSB1-008) recombinant constructs against a virulent infectious bursal disease virus (vIBDV) challenge (Faragher 52/70 strain) performed at 14 days of age in SPF chickens.

The characteristics of the double HVT and SB1 recombinants used in this study are shown in Tables 14 and 16.

On D0, 95 one-day-old SPF chickens were randomly allocated into 9 groups of 10 birds and 1 group of 5 birds (unvaccinated unchallenged control group). The birds were injected by subcutaneous injection in the neck at D0 with 0.2 mL of recombinant vaccines containing a target dose of 300 or 1000 pfu as described in the Table 24 below. On D14, blood sample was collected from 5 birds per group for serological testing with the Kit ProFLOK® plus IBD (Synbiotics Corp). The birds (10 birds per group except for group 7 in which 1 bird died before challenge) were challenged by the eye drop (0.05 mL per bird) on D14 with 2.5 log10 EID50.

**Table 24 Study design and results of IBD efficacy**

| **Group** | **Vaccine at day-old (dose in PFU)** | **IBD+ ELISA titer at D14¹** | **Number Dead/Sick²** | **% protect-tion³** | **Mean bursal/body weight ratio⁴** |
|---|---|---|---|---|---|
| **G1** | vSB1-008 (1000) | 0.2 | 7/10 | 0% | 0.0013 |
| **G2** | vHVT13 (300) | 2.7 | 0/0 | 100% | 0.0051 |
| **G3** | vHVT13 (1000) | 2.7 | 0/0 | 90% | 0.0049 |
| **G4** | vHVT13+vSB1-008 (300) | 1.9 | 1/1 | 60% | 0.0041 |
| **G5** | vHVT13+vSB1-008 (1000) | 2.4 | 0/0 | 70% | 0.0041 |
| **G6** | vHVT304 (300) | 2.9 | 0/0 | 60% | 0.0037 |
| **G7** | vHVT304 (1000) | 2.2 | 0/0 | 67% | 0.0047 |
| **G8** | vHVT306 (300) | 2.4 | 0/0 | 80% | 0.0033 |
| **G9** | vHVT306 (1000) | 2.7 | 0/0 | 40% | 0.0026 |

| | | | | | |
|---|---|---|---|---|---|
| ¹ Mean IBD+ ELISA titers expressed in log10 in the serum of 5 birds per group sampled at D14 before challenge; ² Birds sick for more than 2 days or still sick on D25 were considered as sick. ³ Protection against clinical signs and severe bursal lesion (bursal score <3) ⁴ The bursal/body weight ratio of the unvaccinated/unchallenged group was 0.0047. | | | | | |

Each group was monitored before and after challenge. IBDV clinical signs were recorded for 11 days after challenge (from D15 to D25). At the end of the post-challenge observation period (D33), all the surviving birds were euthanized and necropsied. Body and bursal weights were recorded. Each bursa of Fabricius (BF) was weighted then stored in individual recipients containing 4% formaldehyde for histology. Histological lesions of the bursa were scored according to the scale presented in Table 25.

**Table 25 Scoring scale of histological lesions of the bursa of Fabricius***

| **Score** | **Histology observation/lesions** |
|---|---|
| 0 | No lesion, normal bursa |
| 1 | 1% to 25% of the follicles show lymphoid depletion (i.e. less than 50% of depletion in 1 affected follicle), influx of heterophils in lesions |
| 2 | 26% to 50% of the follicles show nearly complete lymphoid depletion (i.e. more than 75% of depletion in 1 affected follicle), affected follicles show necrosis and severe influx of heterophils may be detected |
| 3 | 51% to 75% of the follicles show lymphoid depletion; affected follicles show necrosis lesions and a severe influx of heterophils is detected |
| 4 | 76% to 100% of the follicles show nearly complete lymphoid depletion; hyperplasia and cyst structures are detected; affected follicles show necrosis and severe influx of heterophils is detected |
| 5 | 100% of the follicles show nearly complete lymphoid depletion; complete loss of follicular structure, thickened and folded epithelium, fibrosis of bursal tissue |

| | |
|---|---|
| * sourced from Monograph No. 01/2008:0587 of EU Pharmacopoeia "Avian Infectious Bursal Disease vaccine (live) | |

A bird was considered as affected if it died and/or showed notable sign of disease and/or severe lesions of the bursa of Fabricius (*i.e.,* histology score ≥3).

The mean ELISA IBD+ antibody titer expressed in log10 before challenge is shown in Table 24. Significant titers were detected in all vaccinated groups that were significantly higher than that of the control group G1. The serology titer was not dose-dependent.

Severe clinical signs were observed after challenge in all birds of the control group G1. Seven out of 10 birds of that group died within the 11 days observation period indicating the high severity of challenge. None of the vaccinated birds showed severe clinical signs after challenge except 1 bird of G4 that died. Percentages of protection against severe bursal lesions are shown in the table above. Significant IBD protection was observed in all groups, the best protection being observed in G2 and G3 (vHVT13 alone). The co-administration of vSB1-008+vHVT13 and the double vHVT304 and vHVT306 constructs induced similar levels of IBD protection. The protection was not dose-dependent at the tested doses. The mean bursal/body weight ratios are also shown in Table 24. Ratios in all vaccinated groups were higher than those of the challenged control group.

In conclusion, these data indicate that both the combination of a SB1-ND vector with a single HVT-IBD or double HVT expressing both NDV-F and IBDV-VP2 induce IBD antibodies and early IBD protection in a severe IBDV challenge model.

### Example 14 Efficacy of single HVT-ND (vHVT114) or SB1-ND (vSB1-007 and vSB1-009) in combination with vHVT13 recombinant vaccines, against challenge with a very virulent IBDV isolate on D23 in commercial broiler chickens

The aim of the study was to assess the IBD efficacy of vHVT13 co-administered with an HVT-ND (vHVT114) or SB1-ND (vSB1-007 and vSB1-009) recombinant constructs against a very virulent infectious bursal disease virus (vvIBDV) challenge (91-168/980702) performed at 23 days of age in commercial broiler chickens.

The characteristics of these 4 vaccine candidates are described in Tables 14 and 16. On D0, 90 one-day-old broiler chickens were randomly allocated into 7 groups of 12 birds and 1 group of 6 birds (unvaccinated unchallenged control group). The birds were injected by subcutaneous injection in the neck at D0 with 0.2 mL of recombinant vaccines containing a target dose of 3000 pfu as described in the Table 26. On D14, blood sample was collected from 5 birds per group for serological testing with the Kit ProFLOK® plus IBD (Synbiotics Corp). The serum of 10 extra one-day-old broiler chickens was tested at D0 with the same kit to evaluate the level of IBDV maternal antibody. The birds (10 birds per group) were challenged by the eye drop (0.05 mL per bird) on D23 with 4.3 log10 EID50 of the wIBDV 91-168 isolate.

Each group was monitored before and after challenge. IBDV clinical signs were recorded for 11 days after challenge (from D23 to D33). At the end of the post-challenge observation period (D33), all the surviving birds were euthanized and necropsied. Body and bursal weights were recorded. Each bursa of Fabricius (BF) was weighted then stored in individual recipients containing 4% formaldehyde for histology. Histological lesions of the bursa were scored according to the scale presented in Table 25.

A bird was considered as affected if it died and/or showed notable signs of disease and/or severe lesions of the bursa of Fabricius (*i.e.,* histology score ≥3).

**Table 26 Study design and serology results**

| **Group** | **Vaccine at day-old (D0)** | **IBD+ ELISA titer at D23¹** | **Mean bursal/body weight ratio²** |
|---|---|---|---|
| **G1** | - | 3.9 | 0.0007 |
| **G2** | vHVT13 | 4.0 | 0.0015 |
| **G3** | vHVT114+vHVT13 | 4.1 | 0.0015 |
| **G4** | vSB1-007+vHVT13 | 3.8 | 0.0018 |
| **G5** | vSB1-009+vHVT13 | 4.0 | 0.0019 |

| | | | |
|---|---|---|---|
| ¹ Mean IBD+ ELISA titers expressed in log10 in the serum of 5 birds per group sampled at D23 before challenge; ² The bursal/body weight ratio of the unvaccinated/unchallenged group was 0.0047 | | | |

The mean ELISA IBD+ serological titer at D0 was 4.36±0.01 log10 indicating a very high level of IBD maternal antibody at hatch. At D23, the mean ELISA IBD+ titer was still high (3.9) in the control G1. ELISA mean titers in the vaccinated groups were not significantly different from those of the control group.

Neither morbidity nor mortality was observed in any of the groups after challenge. Percentages of protection against severe bursal lesions are shown in the table 26 above. The result showed that co-administration of vHVT114, vSB1-007 or vSB1-009 did not interfere with vHVT13-induced IBD protection indicating a lack of interference. Similarly, the mean bursal/body weight ratios of the vaccinated groups were similar and clearly higher than that of the control group, indicating IBD protection and no difference between the vaccination regimens.

In conclusion, the data indicate the compatibility between vHVT114, vSB1-007 or vSB1-009 and vHVT13 for IBD protection. The lack of interference between the two HVT vectors for IBD protection was again surprising and confirmed the results observed for ND protection (see example 10),

### Example 15 Efficacy of double HVT-ND+IBD (vHVT304 and vHVT306) associated or not with SB-1 and of SB1-ND (vSB1-007 and vSB1-008) in combination with vHVT13 recombinant vaccines, against challenge with a variant E IBDV isolate on D28 in SPF chickens

The aim of the study was to assess the efficacy of two double HVT (HVT-ND+IBD: vHVT304 and vHVT306) or two vSB-1-NDV in combination with vHVT13 (vSB1-007+vHVT13, vSB1-008+vHVT13) vectored vaccines administered subcutaneously (SC) to day-old SPF chicks and challenged with IBDV-Variant (VAR-E) 28 days post-vaccination.

On D0, 105 one-day-old SPF chickens were randomly allocated into 7 groups of 15 birds including a group of challenged controls (G6) and unchallenged controls (G7). The birds of groups G1 to G5 were injected by subcutaneous injection in the neck at D0 with 0.2 mL of recombinant and/or SB-1 vaccines containing each a target dose of 2000 pfu. The design of the study is shown in Table 27 below. On D28, all birds from groups G1 to G6 were challenged by the eye drop (0.03 mL containing 3 log10 EID50 per bird) of the IBDV variant E isolate from University of Delaware (USA). Each group was monitored before and after challenge. Eleven days post-challenge, birds were weighed and necropsied. The bursa were collected and weighed. The bursal/body weight ratios (bursa weight/body weight ratio X 100) were calculated.

**Table 27 Study design and results of IBD efficacy**

| **Group** | **Vaccine at day-old** | **Mean bursal/body weight ratio (*100)** |
|---|---|---|
| **G1** | vHVT304 | 0.33 |
| **G2** | vHVT304+SB-1 | 0.33 |
| **G3** | vHVT306 | 0.29 |
| **G4** | vHVT13+vSB1-007 | 0.49 |
| **G5** | vHVT13+vSB1-008 | 0.47 |
| **G6** | - (challenged) | 0.13 |
| **G7** | - (unchallenged) | 0.46 |

The mean bursal/body weight ratios are shown in the Table 27. The challenged control birds had a severe bursal atrophy compared to unchallenged ones. The vSB1-007 and vSB1-008 vaccines did not interfere on vHVT13-induced protection (G4 and G5). The bursal/body weight ratios of birds vaccinated with the double HVT (HVT-ND+IBD) were slightly lower than the unchallenged control group but were clearly higher than the challenged control groups. Furthermore, the SB-1 serotype 2 Marek's disease vaccine did not interfere with vHVT304-induced IBD protection.

In conclusion, these data indicate that both the combination of a SB1-ND vector with a single HVT-IBD or double HVT expressing both NDV-F and IBDV-VP2 induce IBD protection in a variant E IBDV challenge model.

### Example 16 Lack of interference of vHVT114, vSB1-009 and/or SB-1 on vHVT13 induced variant E IBD protection in SPF chickens

The aim of the study was to assess the IBD efficacy of vHVT13 when administered by SC or in ovo route concomitantly with vHVT114, vSB 1-009 and/or SB-1 in SPF chicks in an IBDV-Variant (VAR-E) at D28 challenge model.

75 one-day-old SPF chickens and 75 SPF 18 to 19 day-old chicken embryo were randomly allocated into 5 groups (G1 to G5 and G6 to G10, respectively) including a group of challenged controls (G4 and G9, respectively) and unchallenged controls (G5 and G10, respectively). The birds of groups G1 to G3 were injected by subcutaneous injection in the neck at D0 with 0.2 mL of vaccines containing each a target dose of 3000 pfu except for SB-1 which had a target dose of 1000 PFU. Birds from G6 to G8 received the same vaccine doses but in 0.05 mL volume by the *in ovo* route 2-3 days before hatch. The design of the study is shown in Table 28 below. At 28 days of age, all birds from groups G1 to G4 and G6 to G9 were challenged by the eye drop (0.03 mL containing 3 log10 EID50 per bird) of the IBDV variant E isolate from University of Delaware (USA). Each group was monitored before and after challenge. Eleven days post-challenge, birds were weighed and necropsied. The bursa were collected and weighed. The bursal/body weight ratios (bursa weight/body weight ratio X 100) were calculated.

**Table 28 Study design and results of IBD efficacy**

| **Group** | **Vaccine at day-old** | **Administration route** | **Mean bursal/body weight ratio (*100)** |
|---|---|---|---|
| **G1** | vHVT13+vHVT114+SB-1 | SC | 0.56 |
| **G2** | vHVT13+vHVT114+vSB1-009 | SC | 0.58 |
| **G3** | vHVT13+vSB1-009 | SC | 0.52 |
| **G4** | -(challenged) | SC | 0.13 |
| **G5** | - (unchallenged) | SC | 0.51 |
| **G6** | vHVT13+vHVT114+SB-1 | *In ovo* | 0.54 |
| **G7** | vHVT13+vHVT114+vSB1-009 | *In ovo* | 0.47 |
| **G8** | vHVT13+vSB1-009 | *In ovo* | 0.53 |
| **G9** | - (challenged) | *In ovo* | 0.14 |
| **G10** | - (unchallenged) | *In ovo* | 0.58 |

The mean bursal/body weight ratios are shown in the Table 28. The challenged control birds (G4 and G9) had a severe bursal atrophy compared to unchallenged ones. The bursal/body weight ratios of the vaccinated groups (G1 to G3 and G6 to G8) were similar to those of the unchallenged control groups (G5 and G10) and well above those of the challenged control groups (G4 and G9). The lack of interference of vHVT114 on vHVT13-induced IBD protection after both SC or *in ovo* routes was surprising and confirmed data obtained in examples 10 and 14.

In conclusion, these data indicate clearly the compatibility of vHVT114+vSB1-009 or +SB-1 and of vSB1-009 with vHVT13 when administered by SC or *in ovo* route in a variant E IBDV challenge model.

### Example 17 Efficacy of vHVT114 and vHVT13 and SB1 or vSB1-009 vectors against very virulent plus Marek's disease challenge

The aim of this study was to evaluate the Marek's disease efficacy induced by different combinations of vaccines including vHVT114, vHVT13, SB-1 and/or vSB1-009 administered by the SC route to one-day-old SPF chicks and challenged 4 days later with the very virulent plus Marek's disease virus (vv+MDV) T-King isolate.

On D0, 100 one-day-old SPF chickens were randomly allocated into 5 groups of 20 birds. The birds from groups 1 to 3 were injected by subcutaneous injection in the neck at D0 with 0.2 mL of vaccines containing a target dose of 2000 pfu for each vaccine except for SB-1 for which the target dose was 1000 pfu. Birds from groups 4 and 5 were non-vaccinated and were used as sham controls challenged (group 4) or unchallenged (group 5). The study design is shown in the Table 29. On D4, All birds from groups 1 to 4 were challenged with 0.2 mL of the vv+MDV T-King isolate using the intraperitoneal route of administration.

**Table 29 Study design and MD protection results**

| **Group** | **Vaccine at day-old (D0)** | **Number of MD positive/total** | **Percentage of protection** |
|---|---|---|---|
| **G1** | vHVT13+SB-1 | 7/20 | 65% |
| **G2** | vHVT114+SB-1 | 7/20 | 65% |
| **G3** | vHVT13+vHVT114+vSB1-009 | 7/20 | 65% |
| **G4** | - (challenged) | 20/20 | 0% |
| **G5** | - (unchallenged) | 0/20 | 100% |

Each group was monitored daily for any unfavourable reactions before and after challenge. At day 49, all live birds were terminated and necropsied to examine for gross lesions associated with Marek's disease. Chickens were classified as positive for infection with Marek's disease if nervous signs, such as paralysis, locomotive signs attributable to the disease, and severe emaciation or depression are observed, if mortality directly attributable to Marek's Disease occurs, or if gross lesions are observed at necropsy. Lesions might include, but not be limited to, the following: liver, heart, spleen, gonads, kidneys, and muscle lesions

Results of protection are shown in the Table 29 above. All vaccinated groups (G1 to G3) performed equally, inducing a partial (65%) MD protection as expected in this very severe and early challenge model. These results indicated that the vector vaccine candidates retain their ability to protect against Marek's disease.

### Example 18 Efficacy of recombinant HVT and SB1 vectors against Marek's disease

Marek's disease efficacy is also demonstrated for the HVT vectored recombinants and the SB-1 vectored recombinants either alone or in combination. The challenge strains include a virulent Marek's disease (vMD) challenge such as GA22, a very virulent Marek's disease (vvMD) challenge such as RB1B and/or a very virulent plus Marek's disease (vv+MD) challenge such as the T. King virus. One-day-old chickens are inoculated subcutaneously or 18-19-day-old embryonated eggs are inoculated with a 0.2 ml dose or 0.05 ml dose, respectively, of the test viruses. At five days of age the vaccinated chickens and naive controls are challenged with the relevant Marek's challenge virus (v, vv, or vv+ MDV). The challenged birds are observed until seven weeks of age. All birds are terminated and necropsied to observe for grossly visible lesions associated with Marek's disease as described in Example 17.

### Example 19 Interference of HVT on vHVT13-induced IBDV antibodies in commercial pullets

The objective of this study was to determine if co-administration of HVT with vHVT13 had an impact on vHVT13-induced IBDV antibody response in commercial pullets.

Eighty day-old commercial brown pullets were used in three isolation units. Fifteen were blood sampled at day-old to test IBD maternally derived antibodies (MDA). The remaining birds were split into three groups as shown in Table 30. Birds from group 2 and 3 were vaccinated by the SC route in the nape of the neck with commercial doses of vHVT13 (VAXXITEK HVT+IBD; Merial SAS, Lyon, France) and/or HVT cell-associated Bio HVT (Merial S.p.A., Noventa, Italy). Blood sampling was performed at the age of 25, 35 and 45 days of age. The ELISA kit used to evaluate IBDV serological response was the PROFLOK PLUS IBD (IBD+) Ab ELISA kit from Synbiotics (Synbiotics Corp., Kansas City, MO, USA).

**Table 30 Study design and serology results**

| **Group** | **Vaccine at day-old (D0)** | **ELISA titre D1** | **ELISA titre D25** | **ELISA titre D35** | **ELISA titre D45** |
|---|---|---|---|---|---|
| **G1** | - | 10,502 | 7,814 | 6,237 | 3,664 |
| **G2** | vHVT13 | 10,502 | 8,023 | 9,360 | 9,486 |
| **G3** | vHVT13+HVT | 10,502 | 6,896 | 4,763 | 3,795 |

Mean ELISA titers are shown in Table 30. Titers in the unvaccinated group G1 decreased from D1 to D45, which corresponded to the decline of IBDV maternal antibodies. As expected; ELISA titers in the vHVT13 group G2, remains high up to D45 indicating maternal antibodies were progressively replaced by vHVT13-induced antibodies. The addition of HVT to vHVT13 had a clear negative impact since the antibody titers observed in G3 were similar to G1. These results contrast with those obtained with vHVT114+vHVT13 since the vHVT114 did not decrease vHVT13-induced IBD+ ELISA titers (see example 14, Table 26). They confirm the unexpected property of vHVT114 in not interfering with vHVT13 immunogenicity.

In conclusion, in contrast to what was observed with vHVT114, the addition of HVT to vHVT13 had a clear negative impact on vHVT13-induced IBDV humoral immunity.

### Example 20 Interference of commercial HVT-ND on vHVT13-induced IBD protection

The objective of this study was to determine if co-administration of commercial HVT-ND vector vaccines with vHVT13 had an impact on vHVT13-induced IBD protection in SPF chickens.

Seventy five SPF chickens (3 groups (G2, G3 and G4) of 25) were vaccinated at one day-of-age by the SC route with a commercial dose of vHVT13 (VAXXITEK HVT+IBD) with or without one commercial dose of licensed HVT-vectored ND vaccine (vHVT-ND 1 and vHVT-ND2) as shown in the Table 31. Fifteen birds were kept as non-vaccinated controls (G1). Three weeks post-vaccination, birds (20 chickens in G2, G3 and G4 and 10 chickens in G1) were challenged with at least 2.0 log10 EID50 in 0.05ml of IBD virus Ph/B1 strain (isolated in the Philippines) administered via ocular route. All chickens were observed for 5 days for clinical signs or death from causes attributable to IBD challenge virus and euthanatized humanely at end of post-challenge observation for necropsy examination of IBD lesion, especially from the bursa of Fabricius. Birds were considered as protected if their bursa did not show bursal lesions typical of IBD: bursal atrophy, peri-bursa edema and/or hemorrhages in bursa tissues.

**Table 31 Study design and IBD protection data**

| **Group** | **Vaccine at day-old (D0)** | **Number of sick (dead)/total** | **Number of positive bursa/total** | **Percent of protection** |
|---|---|---|---|---|
| **G1** | - | 10(8)/10 | 10/10 | 0% |
| **G2** | vHVT13+vHVT-ND1 | 3(3)/20 | 9/20 | 55% |
| **G3** | vHVT13+vHVT-ND2 | 3(1)/20 | 7/20 | 65% |
| **G4** | vHVT13 | 0(0)/20 | 0/20 | 100% |

Results are shown in Table 31. All 10 challenged control birds showed clinical signs and 8 out of 10 died 4 or 5 dpi indicating that the IBDV challenge was very severe. All of them had severe lesions of bursa including severe atrophy and haemorrhagic patches. The vHVT13 alone induced full protection whereas both combinations with vHVT-ND induced partial clinical and bursal protection.

In conclusion, these results clearly indicate that the 2 commercial HVT-vectored ND vaccines interfere with vHVT13-induced IBD protection.

### Example 21 Efficacy of vSB1-004, vSB1-006, vSB1-007, vSB1-008, SB1-vectored ND vaccine alone or in association with vHVT13 HVT-vectored IBD vaccine, and the vHVT302 and vHVT304 vaccines against challenges with NDV Texas GB strain at 14 and/or 28 days of age in SPF chickens

The aim of the study was to assess the efficacy of combinations of different Marek's disease vector vaccines expressing the NDV F and/or the IBDV VP2 gene against Newcastle disease challenge (Texas GB strain, genotype II) performed at 14 and/or 28 days of age in SPF chickens.

The characteristics of the 6 NDV recombinant vaccine candidates tested in this study are described in the Table 32 below.

**Table 32 characteristics of the 6 NDV recombinant vaccine candidates tested in this study**

| Name | Parental virus | Promoter | F gene | Poly-A | Locus |
|---|---|---|---|---|---|
| vSB 1-004 | SB-1* | mCMV IE | Wt-VIId | SV40 | SORF4/US10 |
| vSB 1-006 | SB-1 | SV40 | Opt-VIId | Synthetic | UL55/LORF5 |
| vSB 1-007 | SB-1 | SV40 | Opt-VIId | (endogeneous from gC gene) | gC |
| vSB 1-008 | SB-1 | SV40 | Opt-CA02 | Synthetic | UL55/LORF5 |
| vHVT302 | vHVT13 | US10 | Opt-VIId | US10 | US10 |
| vHVT304 | vHVT13 | SV40 | Opt-VIId | Synthetic | IG2 |

On D0, 225 one-day-old SPF chickens were randomly allocated into 9 groups of 15 birds (G1a to G9a challenged at D14) and 6 groups of 15 birds (G1b, G3b, G4b, G5b, G8b, G9b challenged at D28). The birds were injected by subcutaneous injection in the neck at D0 with 0.2 mL containing a target dose of 2000 pfu for recombinant vaccines. The design of the study is shown in Table 33 below. The birds were challenged by the intramuscular route on D14 or D28 with 4.3 and 4.2 log10 EID50 (0.1 mL) velogenic ND Texas GB (genotype II) strain, respectively.

**Table 33 Results of ND efficacy**

| **Group** | **Vaccine at day-old (D0)** | **% ND protection after ND challenge at 14 days of age** | **% ND protection after ND challenge at 28 days of age** |
|---|---|---|---|
| **G1a & 1b** | - | 0% | 0% |
| **G2a** | vSB1-004 | 20% | ND* |
| **G3a & 3b** | vSB1-006 | 26.6% | 73.3% |
| **G4a & 4b** | vSB1-007 | 33.3% | 93.3% |
| **G5a & 5b** | vSB1-008 | 46.6% | 86.6% |
| **G6a** | vSB1-006+vHVT13 | 14% | ND |
| **G7a** | vSB1-008+vHVT13 | 21.4% | ND |
| **G8a & 8b** | vHVT302 | 13.3% | 80% |
| **G9a & 9b** | vHVT304 | 33.3% | 93.3% |

| | | | |
|---|---|---|---|
| *ND = not done | | | |

Each group was monitored before and after challenge. NDV clinical signs after challenge were recorded. One bird died in G6 and G7 before challenge reducing the number of birds from 15 to 14 in these groups.

Percentages of clinical protection (including protection against both mortality and morbidity) are reported in Table 33 above. Full susceptibility was observed in the non-vaccinated challenged control group G1a and G1b thus validating the high severity of challenge. Partial protections ranging from 13.3 to 46.6% were observed after challenge at D14, the highest levels of protection being induced by vSB1-008, vSB1-007 and vHVT304. Protection levels after ND challenge at D28 were much higher for all vaccinated groups and were again slightly higher in the groups vaccinates with vSB1-008, vSB1-007 or vHVT304. These results indicated that ND protection levels were dependent on the date of challenge and on the construct. The vSB 1-008 and vSB1-007 constructs performed slightly better than vSB 1-004 and vSB1-006, and the vHVT304 performed slightly better than vHVT302, indicating that different characteristics of the constructs are playing a role in the performances of MDV-based vector vaccines.

In conclusion, the results of this study showed that ND protection levels induced by Marek's disease vectors expressing NDV F gene may depend on different parameters including the vector, the locus of insertion, the F gene, the promoter, the poly-adenylation site and the challenge conditions.

### Example 22 Efficacy of double HVT-ND+IBD vHVT304 and vHVT306 vaccines against challenges with NDV Texas GB strain at 14 and/or 28 days of age in SPF chickens

The aim of the study was to assess the efficacy of HVT-vectored vaccine expressing both NDV F and IBDV VP2 genes against Newcastle disease challenge (Texas GB strain, genotype II) performed at 14 and/or 28 days of age in SPF chickens.

The characteristics of the 2 recombinant vaccine candidates tested in this study are described in the Table 34 below.

**Table 34 Characteristics of the recombinant vaccine candidates used in this study**

| Name | Parental virus | Promoter | F gene | Poly-A | Locus |
|---|---|---|---|---|---|
| vHVT304 | vHVT13 | SV40 | Opt-VIId | Synthetic | IG2 |
| vHVT306 | vHVT13 | SV40 | Opt-VIId | Synthetic | SORF3-US2 |

On D0, 90 one-day-old SPF chickens were randomly allocated into 3 groups of 15 birds (G1a to G3a challenged at D14) and 3 groups of 15 birds (G1b to G3b challenged at D28). The birds were injected by subcutaneous injection in the neck at D0 with 0.2 mL containing a target dose of 2000 pfu for recombinant vaccines. The design of the study is shown in Table 35 below. The birds were challenged by the intramuscular route on D14 or D28 with a target dose of 4.0 log10 EID50 (0.1 mL) velogenic ND Texas GB (genotype II) strain.

**Table 35 Results of ND efficacy**

| **Group** | **Vaccine at day-old (D0)** | **% ND protection after ND challenge at 14 days of age** | **% ND protection after ND challenge at 28 days of age** |
|---|---|---|---|
| **G1a & 1b** | - | 0% | 0% |
| **G2a & 2b** | vHVT304 | 26.7% | 92.9% |
| **G3a & 3b** | vHVT306 | 33.3% | 86.7% |

Each group was monitored before and after challenge. NDV clinical signs after challenge were recorded. One bird died in G2b before challenge reducing the number of birds from 15 to 14 in this group.

Percentages of clinical protection (including protection against both mortality and morbidity) are reported in Table 35 above. Full susceptibility was observed in the non-vaccinated challenged control group G1a and G1b thus validating the high severity of challenge. Protections levels after challenge at D14 were much lower than those obtained after challenge at D28. These vaccine candidates had the same NDV F expression cassette inserted into 2 different loci of vHVT13 genome. They performed equally in terms of ND protection in the tested conditions, indicating that both insertion loci (IG2 and SORF3-US2) are equally suitable for NDV F cassette insertion.

In conclusion, the results of this study showed that ND protection levels induced by Marek's disease vectors expressing NDV F gene depend on different parameters including the vector, the locus of insertion, the F gene, the promoter, the poly-adenylation site and the challenge conditions.

### Example 23 ND early efficacy induced by double HVT-ND+IBD (vHVT302, vHVT303, and vHVT304) or SB1-vectors (vSB1-006 and vSB1-007) in one day-old SPF chickens against a velogenic genotype V NDV challenge

The objective of the study was to evaluate the efficacy of three double HVT-ND+IBD (vHVT302, vHVT303, and vHVT304) and two SB1-ND vectors (vSB1-006 and vSB 1-007) in one day-old SPF chickens against a velogenic genotype V (Chimalhuacan) NDV challenge performed at D14.

The characteristics of the 5 recombinant vaccine candidates tested in this study are described in Table 36 below.

**Table 36 Characteristics of the recombinant vaccine candidates used in this study**

| Name | Parental virus | Promoter | F gene | Poly-A | Locus |
|---|---|---|---|---|---|
| vHVT302 | vHVT13 | US10 | Opt-VIId | US10 | US10 |
| vHVT303 | vHVT13 | US10 | Opt-V (CA02) | US10 | US10 |
| vHVT304 | vHVT13 | SV40 | Opt-VIId | Synthetic | IG2 |
| vSB 1-006 | SB-1 | SV40 | Opt-VIId | Synthetic | UL55/LORF5 |
| vSB 1-007 | SB-1 | SV40 | Opt-VIId | (endogeneous from gC gene) | gC |

Six groups (1 and 2) of ten one-day-old specific pathogen free (SPF) white Leghorn chicks were randomly constituted. Birds from groups 2 to 6 were vaccinated by the subcutaneous route (nape of the neck) with a target dose of 2000 PFU as shown in the Table 37 below. Chickens from group 1 were not vaccinated and were kept as control birds. At 2 week-of-age, all birds were challenged with the genotype V Mexican Chimalhuacan (Mex V) velogenic NDV strain. The challenge was performed by the intramuscular (IM) route using 10⁵ Egg Infectious Dose 50 (EID50) diluted in 0.2 ml of physiological sterile water. All birds were monitored until 14 days post-challenge. After challenge, health status of each bird was scored daily as follows: healthy / with specific symptoms (ruffled feathers, prostration, torticollis, tremor) / dead. Any bird that showed specific symptoms for more than 2 days or was noted sick on D28 was taken into account for calculation of morbidity.

**Table 37 Results of early ND protection induced by different MDV vectored candidates expressing NDV F gene in SPF day-old chicks**

| Group | Vaccine | Target dose (PFU) under 0.2 mL (actual dose) | Protection against mortality | Protection against morbidity |
|---|---|---|---|---|
| G1 | - | - | 0% | 0% |
| G2 | vHVT302 | 2000 (4427) | 50% | 10% |
| G3 | vHVT303 | 2000 (ND) | 10% | 0% |
| G4 | vHVT304 | 2000 (1169) | 80% | 60% |
| G5 | vSB1-006 | 2000 (1720) | 60% | 40% |
| G6 | vSB1-007 | 2000 (1564) | 80% | 50% |

Results of protection are summarized in Table 37. All control birds died after ND challenge. Variable levels of ND protection were induced by the different tested vaccines ranging from 10% to 80% and from 0% and 60% in terms of protection against mortality and morbidity, respectively. The vHVT304 candidate induced a better protection than the vHVT303 and vHVT302 candidates; this may be due to the exogenous SV40 promoter placed in front of the NDV F gene. The vSB 1-007 performed slightly better than the vSB 1-006. Furthermore, performances obtained with vHVT304 were comparable to those obtained with vSB 1-007 indicating that different Marek's disease vectors can reach the same level of ND protection.

In conclusion, this study demonstrates that both double HVT-ND+IBD and SB1-ND vectored vaccines can reach significant levels of ND protection in a very severe and early NDV challenge model.

### Example 24 ND efficacy induced by the double HVT-ND+IBD vHVT306 administered by in ovo or SC route to one day-old SPF chickens against a velogenic genotype V NDV challenge performed at D28

The objective of the study was to evaluate the efficacy of one double HVT-ND+IBD (vHVT306) administered by the in ovo or SC route to SPF chickens against a velogenic genotype V (Chimalhuacan) NDV challenge performed at 28 days of age.

The characteristics of the vHVT306 recombinant vaccine candidate tested in this study are described in Table 38 below. The single HVT-IBD vector vaccine vHVT13 was used as a control.

**Table 38 Characteristics of the recombinant vaccine candidate used in this study**

| Name | Parental virus | Promoter | F gene | Poly-A | Locus |
|---|---|---|---|---|---|
| vHVT306 | vHVT13 | SV40 | Opt-VIId | Synthetic | SORF3-US2 |

On day -3, 40 SPF embryonated eggs aged around 18 days and 18 hours of incubation were randomly allocated into 2 groups of 20 eggs each. On D0, one group of 12 day-old SPF chicks was added. The definition of groups is given in Table 39 below. The vaccination was performed on D-3 (*in ovo* route) or on D0 (SC route, in the back of the neck) and the target dose of vHVT306 and vHVT13 was 2000PFU/bird. For the *in ovo* route, hatchability, viability (until D28) and growth of the birds (between hatching and D28) were monitored.

On D28, 10 birds per group were challenged with virulent ND Chimalhuacan strain. The challenge was performed by the intramuscular (IM) route using 10⁵ Egg Infectious Dose 50 (EID50) diluted in 0.2 ml of physiological sterile water. Birds were monitored until 14 days post-challenge. Specific clinical signs and mortality were recorded. Any bird that showed specific symptoms for more than 2 days or was noted sick on D42 was taken into account for calculation of morbidity. Five and seven days post-challenge (i.e. on D33 and D35), oropharyngeal swab was taken from each surviving bird. All the swabs were analyzed by specific NDV qRT-PCR.

**Table 39 Results of ND protection induced by vHVT306 MDV vectored candidate expressing both NDV F and IBDV VP2 genes administered by the SC or in ovo route into SPF chicks**

| Group | Vaccine/route | Hatchability/viability (%) | Protection against mortality/morbidity | % birds shedding at 5 dpi/7 dpi (mean log10 titer*) |
|---|---|---|---|---|
| G1 | vHVT13/*in ovo* | 100%/100% | 0%/0% | (not tested) |
| G2 | vVHT306/*in ovo* | 100%/100% | 100%/100% | 0% (2.7)/0% (2.7) |
| G3 | vHVT306/SC | - | 100%/100% | 20% (3.2)/10% (2.9) |

| | | | | |
|---|---|---|---|---|
| * The threshold titer of the real time RT PCR was set at 2.7 log10 equivalent EID50 | | | | |

Full hatchability was recorded after *in ovo* vaccination in groups 1 and 2 and all hatched birds survived up to D28. No difference in body weights was detected between the two groups at both D0 and D28 confirming the perfect safety of vHVT306 when administered *in ovo.* Results of protection are summarized in Table 39. All vHVT13-vaccinated control birds died by 4 days after ND challenge. Full clinical ND protection was induced by vHVT306 administered by both routes. Furthermore, no shedding was detected after *in ovo* administration whereas only a few birds shed detectable amount of challenge virus after SC administration.

In conclusion, this study demonstrates that the double HVT-ND+IBD vHVT306 induce excellent level of ND protection by SC or *in ovo* administration routes in a very severe heterologous NDV challenge model.

### Example 25 Efficacy of double HVT-ND+IBD (vHVT302, vHVT303 and vHVT304) recombinant vaccines, against challenge with a classical IBDV isolate on D15 in SPF chickens

The aim of the study was to assess the early IBD efficacy of double HVT recombinants vHVT302, vHVT303 and vHVT304 recombinant constructs against a virulent infectious bursal disease virus (vIBDV) challenge (Faragher 52/70 strain) performed at 15 days of age in SPF chickens.

The characteristics of the 3 double HVT-ND+IBD recombinant vaccine candidates tested in this study are described in the Table 40 below.

**Table 40 Characteristics of the expression cassettes of double HVT recombinants**

| Name | Parental virus | Promoter | F gene | Poly-A | Locus |
|---|---|---|---|---|---|
| vHVT302 | vHVT13 | US10 | Opt-VIId | US10 | US10 |
| vHVT303 | vHVT13 | US10 | Opt-V (CA02) | US10 | US10 |
| vHVT304 | vHVT13 | SV40 | Opt-VIId | Synthetic | IG2 |

On D0, 40 one-day-old SPF chickens were randomly allocated into 4 groups of 10 birds including one control groups (G1) that was vaccinated with vSB1-004, a SB-1 vector expressing NDV F gene. Five other SPF birds were kept unvaccinated and unchallenged for bursal/body weights evaluation. The birds were injected by subcutaneous injection in the neck at D0 with 0.2 mL of recombinant vaccines containing a target dose of 2000 pfu as described in the Table 41 below. On D15, blood sample was collected from all birds per group (10 birds per group except for groups 1 and 3 in which 1 bird died before blood sampling) for serological testing with the Kit ProFLOK® plus IBD (Synbiotics Corp). On D15, birds from all 4 groups were challenged by the eye drop (0.05 mL per bird) with 2.5 log10 EID50.

**Table 41 Study design and results of IBD efficacy**

| **Group** | **Vaccine at day-old** | **ELISA IBD+ titer (log10)** | **Number Dead /Sick (total)¹** | **% protection²** | **Mean bursal/body weight ratio⁴** |
|---|---|---|---|---|---|
| **G1** | vSB 1-004 | 0.25 | 1/9 (9) | 0% | 0.0014 |
| **G2** | vHVT302 | 2.6 | 0/1 (10) | 80% | 0.0043 |
| **G3** | vHVT303 | 3.0 | 0/0 (9) | 100% | 0.0053 |
| **G4** | vHVT304 | 2.4 | 0/0 (10) | 80% | 0.0034 |

| | | | | | |
|---|---|---|---|---|---|
| ¹ Birds sick for more than 2 days or still sick on D25 were considered as sick. The number in brackets is the total number of birds in the group that were challenged. ² Protection against clinical signs and severe bursal lesion (bursal score <3) ⁴ The bursal/body weight ratio of the unvaccinated/unchallenged group was 0.0043. | | | | | |

Each group was monitored before and after challenge. IBDV clinical signs were recorded for 11 days after challenge (from D15 to D25). At the end of the post-challenge observation period (D25), all the surviving birds were euthanized and necropsied. Body and bursal weights were recorded. Each bursa of Fabricius (BF) was weighted then stored in individual recipients containing 4% formaldehyde for histology. Histological lesions of the bursa were scored according to the scale presented in Table 42.

**Table 42 Scoring scale of histological lesions of the bursa of Fabricius***

| **Score** | **Histology observation/lesions** |
|---|---|
| 0 | No lesion, normal bursa |
| 1 | 1% to 25% of the follicles show lymphoid depletion (i.e. less than 50% of depletion in 1 affected follicle), influx of heterophils in lesions |
| 2 | 26% to 50% of the follicles show nearly complete lymphoid depletion (i.e. more than 75% of depletion in 1 affected follicle), affected follicles show necrosis and severe influx of heterophils may be detected |
| 3 | 51% to 75% of the follicles show lymphoid depletion; affected follicles show necrosis lesions and a severe influx of heterophils is detected |
| 4 | 76% to 100% of the follicles show nearly complete lymphoid depletion; hyperplasia and cyst structures are detected; affected follicles show necrosis and severe influx of heterophils is detected |
| 5 | 100% of the follicles show nearly complete lymphoid depletion; complete loss of follicular structure, thickened and folded epithelium, fibrosis of bursal tissue |

| | |
|---|---|
| * sourced from Monograph No. 01/2008:0587 of EU Pharmacopoeia "Avian Infectious Bursal Disease vaccine (live) | |

A bird was considered as affected if it died and/or showed notable sign of disease and/or severe lesions of the bursa of Fabricius (*i.e.,* histology score ≥3).

The mean ELISA IBD+ antibody titer expressed in log10 before challenge is shown in Table 41. Significant titers were detected in all vaccinated groups that were significantly higher than that of the control group G1. The serology titer was slightly higher in G3 (vHVT303).

Severe clinical signs were observed after challenge in all 9 birds of the control group G1, which lead to the death of 1 bird. Only one vaccinated bird in G2 (vHVT302) showed clinical signs after challenge. Percentages of protection against severe bursal lesions are shown in Table 41 above. Significant IBD protection was observed in all vaccinated groups, a full protection being observed in G3 (vHVT303). The mean bursal/body weight ratios are also shown in Table 41. Ratios in all vaccinated groups were higher than those of the challenged control group G1 and not significantly different from the unvaccinated and unchallenged control group.

In conclusion, these data indicate that the three double HVT-IBD+ND tested in this study induced IBD antibodies and early IBD protection in a severe IBDV challenge model.

### Example 26 Efficacy of five different HVT-ND vaccine candidates against challenges with velogenic NDV ZJ1 (genotype VIId) isolate at 14 days of age in SPF chickens

The aim of the study was to assess the efficacy of 5 single HVT recombinant constructs (vHVT39, vHVT110, vHVT111, vHVT112 and vHVT113) expressing the NDV F gene against Newcastle disease challenge with velogenic NDV ZJ1 (genotype VIId) isolate performed at 14 days of age in SPF chickens.

The characteristics of these 5 vaccine candidates are described in Table 43 below.

**Table 43 Characteristics of the HVT-ND recombinant viruses used in the challenge study**

| Name | Parental virus | Promoter | F gene* | Poly-A | Locus |
|---|---|---|---|---|---|
| vHVT039 | HVT | MDV gB | Wtnm-Texas | SV40 | IG1 |
| vHVT110 | HVT | MCMV IE | Wt-VIId | SV40 | IG1 |
| vHVT111 | HVT | SV40 | Wt-VIId | SV40 | IG1 |
| vHVT112 | HVT | MCMV IE | Wt-YZCQ | SV40 | IG1 |
| vHVT113 | HVT | MCMV IE | Wt-Texas | SV40 | IG1 |

| | | | | | |
|---|---|---|---|---|---|
| *Wt means that the wild type velogenic F gene sequence was used but the cleavage site was modified to that of a lentogenic virus. Wtnm means that the cleavage site of the wild type sequence was not modified. The Texas velogenic strain belongs to genotype IV and YZCQ to the genotype VIId. | | | | | |

On D0, 72 one-day-old SPF chickens were randomly allocated into 5 groups of 12 birds (vaccinated) and 1 group of 12 birds (non-vaccinated controls). The birds were injected by subcutaneous injection in the neck at D0 with 0.2 mL of recombinant vaccines containing a target dose of 6000 pfu as described in Table 44 below. The birds were challenged by the intramuscular route on D14 with 5 log10 EID50 of NDV ZJ1/2000 (genotype VIId) velogenic strain.

**Table 44 Results of ND efficacy**

| **Group** | **Vaccine at day-old (D0)** | **% clinical protection** | |
|---|---|---|---|
| | | **Protection against mortality/morbidity** | **Mean shedding titer (log10) at 2/4 dpi** |
| **G1** | - | 0%/0% | 3.5/-(all dead) |
| **G2** | vHVT039 | 25%/8% | 2.5/4.8 |
| **G3** | vHVT110 | 100%/83% | 1.8/2.0 |
| **G4** | vHVT111 | 100%/67% | 1.8/2.8 |
| **G5** | vHVT112 | 75%/42% | 1.7/3.4 |
| **G6** | vHVT113 | 83%/25% | 1.4/3.3 |

Each group was monitored before and after challenge. NDV clinical signs and mortality were recorded after challenge. Oropharyngeal swabs were taken at 2 and 4 days post-infection (dpi) for evaluation of viral load by real time RT-PCR using the method described by Wise et al. (2004; Development of a Real-Time Reverse-Transcription PCR for Detection of Newcastle Disease Virus RNA in Clinical Samples. J Clin Microbiol 42, 329-338).

Percentages of protection against mortality and morbidity are reported in the table 44 above. Full susceptibility was observed in the non-vaccinated challenged control group G1 thus validating the high severity of the challenge. Vaccines induced variable levels of protection against mortality (25-100%) or against morbidity (8%-83%). The best protection level was induced by vHVT110 whereas the lowest one was induced by vHVT039, the other candidates giving intermediate results. Results of oropharyngeal shedding at 2 and 4 dpi are also shown in Table 44 above and are in line with those of clinical protection. These vaccine candidates differ in their promoter and F gene sequence. These results show that both of these parameters are important for the design of optimal HVT-ND vaccine candidate.

In conclusion, the results of this study showed the importance of promoter and F gene sequence in the ND efficacy induced by HVT-vectored ND vaccine candidates.

### Example 27 Evaluation of the Newcastle disease efficacy induced by double SB1 constructs expressing IBDV VP2 and NDV F.

The aim of the study is to assess the efficacy of double SB1 constructs expressing IBDV VP2 and NDV F against Newcastle disease challenge.

On D0, one-day-old SPF chickens are randomly allocated into several groups of 10-20 birds, including vaccinated and non-vaccinated groups. The birds of the vaccinated groups are injected by subcutaneous injection in the neck at D0 with 0.2 mL containing a target dose of 1000 to 5000 pfu of recombinant vaccines. Alternatively, the same dose in 0.05 mL may be administered *in ovo* 2 or 3 days before hatch. The birds (at least one vaccinated and one non vaccinated group) are challenged by the intramuscular route at different time after vaccination: for instance, D14, D28 or D42 with about 4.0 log10 EID50 (0.1 mL) of a velogenic NDV strain such as Texas GB (genotype II), ZJ1 (genotype VIId), Chimalhuacan (genotype V) strain.

Each group is monitored clinically before and after challenge. NDV clinical signs (morbidity) and mortality are recorded after challenge. Percentages of clinical protection in all groups are calculated. At least 90% of non-vaccinated challenged SPF birds should die or be severely sick after challenge to validate the severity of challenge. Oropharyngeal and cloacal swabs can be samples at different times after challenge such as 3, 5, 7 and 9 days post-challenge and the viral load can be estimated by real-time RT-PCR. The best candidates will be those who induced the highest level of clinical protection and the lowest level of viral load in the swabs. A similar study can be performed in broilers containing NDV maternal antibodies; however, these maternal antibodies may potentially protect the non-vaccinated birds if the challenge is performed early. The double SB1 construct may also be tested in combination with other Marek's disease vaccine or vector vaccines.

### Example 28 Evaluation of the infectious bursal disease efficacy induced by double SB1 constructs expressing IBDV VP2 and NDV F

The aim of the study is to assess the IBD efficacy of double SB1 expressing both the IBDV VP2 and the NDV F.

One-day-old SPF chickens are randomly allocated into several groups of 10 to 20 birds including vaccinated and non-vaccinated controls. Non-vaccinated controls will be separated into 2 subgroups including challenged and unchallenged birds. The birds of vaccinated groups are injected by subcutaneous injection in the neck at D0 with 0.2 mL of vaccines containing each a target dose of 1000 to 5000 pfu. Alternatively, the same dose in 0.05 mL may be administered *in ovo* 2 or 3 days before hatch. At different times after vaccination such as 14, 21, 28 or 42 days post-vaccination, all birds from vaccinated groups and the challenged controls are challenged by the eye drop (0.03 mL containing 2 to 4 log10 EID50 per bird) of a virulent IBDV (such as the Faragher or the US standard strain), a very virulent IBDV such as the 91-168 isolate or a variant IBDV isolate such as the US Delaware variant E isolate. Each group is clinically monitored before and after challenge. Birds can be necropsied 4 or 5 days post-challenge for bursal gross lesions evaluation. They can also be necropsied 10 to 11 days post-challenge. Gross and/or histological lesions can be evaluated. Furthermore, birds and bursa are weighed the bursal/body weight ratios (bursa weight/body weight ratio X 100) are calculated compared to those of the non-vaccinated unchallenged group. Control SPF challenged birds must show clinical signs and/or have significant gross and/or histological lesions, and/or should have a bursal/body weight ratio significantly lower than the unvaccinated unchallenged control birds to validate the severity of challenge. The efficacy of the vaccine is evaluated by comparing these parameters with unvaccinated/challenged and unvaccinated/unchallenged groups. Such study may be performed in broiler chickens containing IBDV maternal antibodies; however, these maternal antibodies may potentially protect the non-vaccinated birds if the challenge is performed early. The double SB1 construct may also be tested in combination with other Marek's disease vaccine or vector vaccines.

### Example 29 Evaluation of the Marek's disease efficacy induced by double SB1 constructs expressing IBDV VP2 and NDV F

The aim of the study is to evaluate Marek's disease efficacy induced by the SB1 vectors expressing both IBDV VP2 and NDVF.

One-day-old SPF chickens are randomly allocated into several groups of 20 to 50 birds including vaccinated and non-vaccinated controls. Non-vaccinated controls may be separated into 2 subgroups including challenged and unchallenged birds. The birds of vaccinated groups are injected by subcutaneous injection in the neck at D0 with 0.2 mL of vaccines containing each a target dose of 1000 to 5000 pfu. Alternatively, the same dose in 0.05 mL may be administered *in ovo* 2 or 3 days before hatch. At different times after vaccination such as 3 to 10 days post-vaccination, all birds from vaccinated groups and the challenged controls are challenged by the intraperitoneal route with 0.2 mL of a Marek's disease virus (MDV) strain. MDV strain may be of several pathotypes such as virulent MDV (vMDV) including the JM or GA22 isolate, very virulent MDV (vvMDV) such as the RB-1B or Md5 isolate, very virulent plus (vv+MDV) such as the T-King or 648A isolate. MDV challenge strain inoculum are prepared by infecting chickens, harvesting and freezing their blood cells into liquid nitrogen in presence of a cryopreservative such as DMSO. The chicken infectious dose 50 (CID50) is established for each challenge batch before performing vaccination/challenge studies. Each group is clinically monitored before and after challenge. Birds are necropsied after at least 7 weeks post-vaccination and the presence Marek's disease gross lesions is checked in each bird. Lesions might include, but not be limited to, the following: liver, heart, spleen, gonads, kidneys, nerve and muscle lesions. Such study may be performed in broiler chickens containing MDV maternal antibodies. The double SB1 construct may also be tested in combination with other Marek's disease vaccine (for instance HVT and or CVI988 Rispens strains) or MD vector vaccines. MD challenge may also be performed by contact between vaccinated birds and MDV infected non-vaccinated SPF chicks.

Having thus described in detail preferred embodiments of the present invention, it is to be understood that the invention defined by the above examples is not to be limited to particular details set forth in the above description as many apparent variations thereof are possible without departing from the spirit or scope of the present invention.

All documents cited or referenced herein ("herein cited documents"), and all documents cited or referenced in herein cited documents, together with any manufacturer's instructions, descriptions, product specifications, and product sheets for any products mentioned herein or in any document incorporated by reference herein, are hereby incorporated herein by reference, and may be employed in the practice of the invention.

The invention is described in the following numbered paragraphs:
1. A composition or vaccine comprising one or more recombinant herpesvirus of turkeys (HVT) vectors comprising at least one or more heterologous polynucleotides coding for and expressing at least one antigen of an avian pathogen.
2. The composition or vaccine of paragraph 1, wherein the HVT vector comprises a heterologous polynucleotide coding for and expressing a Newcastle Disease Virus F (NDV-F) antigen having at least 80% sequence identity to the amino acid sequence as set forth in SEQ ID NO:2, 4, 6, 33, 35, or 37.
3. The composition or vaccine of paragraph 2, wherein the polynucleotide encoding the NDV-F polypeptide is operably linked to a promoter selected from the group consisting of SV40 promoter, MDV gB promoter and guinea pig CMV promoter.
4. The composition or vaccine of paragraph 3, wherein the polynucleotide encoding the NDV-F polypeptide is operably linked to an SV40 polyA signal.
5. The composition or vaccine of paragraph 4, wherein the polynucleotide encoding the NDV-F polypeptide , the operably linked promoter and SV40 polyA signal are inserted in the IG1 locus of HVT genome.
6. The composition of any one of paragraphs 1-5, wherein the composition further comprises a second recombinant HVT vector comprising a heterologous polynucleotide coding for and expressing IBDV VP2 antigen.
7. The composition of paragraph 6, wherein the second recombinant HVT vector is vHVT13.
8. The composition of any one of paragraphs 1-7, wherein the composition further comprises one or more recombinant SB1 vectors.
9. The composition of paragraph 8, wherein the recombinant SB1 vector comprises one or more heterologous polynucleotides coding for and expressing a Newcastle Disease Virus F (NDV-F) antigen.
10. The composition of paragraph 9, wherein the SB1 vector is selected from the group consisting of vSB1-009, vSB1-010, vSB1-004, vSB1-006, vSB1-007 and vSB1-008.
11. The composition or vaccine of paragraph 1, wherein the HVT vector comprises a first heterologous polynucleotide coding for and expressing a Newcastle Disease Virus F (NDV-F) antigen having at least 80% sequence identity to the amino acid sequence as set forth in SEQ ID NO:2, 4, 6, 33, 35, or 37 and a second heterologous polynucleotide coding for and expressing an Infectious Bursal Disease Virus (IBDV) VP2 antigen having at least 80% sequence identity to the amino acid sequence as set forth in SEQ ID NO:8 or 42.
12. The composition or vaccine of paragraph 11, wherein the HVT vector comprises two heterologous polynucleotides coding for and expressing two IBDV VP2 antigen having at least 80% sequence identity to the amino acid sequence as set forth in SEQ ID NO:8 or 42.
13. The composition or vaccine of paragraph 11, wherein the polynucleotide encoding the NDV-F antigen is operably linked to a promoter selected from the group consisting of SV40 promoter, MDV gB promoter, and guinea pig CMV promoter, and wherein the polynucleotide encoding the IBDV VP2 antigen is operably linked to a CMV promoter.
14. The composition or vaccine of paragraph 13, wherein the polynucleotide encoding the NDV-F antigen is operably linked to an SV40 polyA signal and the polynucleotide encoding the IBDV VP2 antigen is operably linked to an SV40 polyA signal.
15. The composition or vaccine of paragraph 14, wherein the polynucleotide encoding the NDV-F antigen, the operably linked promoter, and SV40 polyA signal are inserted in the IG1 locus of the HVT genome, and wherein the polynucleotide encoding the IBDV VP2 antigen and the operably linked CMV promoter and SV40 polyA signal are inserted in the IG1 locus or the SORF3-US2 locus of the HVT genome.
16. The composition of any one of paragraphs 11-15, wherein the composition further comprises a recombinant HVT vector comprising a heterologous polynucleotide coding for and expressing IBDV VP2 antigen.
17. The composition of paragraph 16, wherein the recombinant HVT vector is vHVT13.
18. The composition of any one of paragraph 11-17, wherein the composition further comprises one or more recombinant SB1 vectors.
19. The composition of paragraph 17, wherein the recombinant SB1 vector comprises one or more heterologous polynucleotides coding for and expressing a Newcastle Disease Virus F (NDV-F) antigen.
20. The composition of paragraph 19, wherein the SB1 vector is selected from the group consisting of vSB1-009, vSB1-004, vSB1-006, vSB1-007, vSB1-008 and vSB1-010.
21. A recombinant HVT vector comprising one or more heterologous polynucleotides coding for and expressing at least one antigen of an avian pathogen.
22. The recombinant HVT vector of paragraph 21, wherein the vector comprises a heterologous polynucleotide coding for and expressing a Newcastle Disease Virus F (NDV-F) antigen having at least 80% sequence identity to the amino acid sequence as set forth in SEQ ID NO:2, 4, 6, 33, 35, or 37.
23. The recombinant HVT vector of paragraph 22, wherein the polynucleotide encoding the NDV-F polypeptide is operably linked to a promoter selected from the group consisting of SV40 promoter, MDV gB promoter, and guinea pig CMV promoter.
24. The recombinant HVT vector of paragraph 23, wherein the polynucleotide encoding the NDV-F polypeptide is operably linked to an SV40 polyA signal.
25. The recombinant HVT vector of paragraph 24, wherein the polynucleotide encoding the NDV-F polypeptide, the operably linked promoter, and SV40 polyA signal are inserted in the locus selected from the group consisting of IG1 , IG2, US10, SORF3-US2 and gD of HVT genome.
26. The recombinant HVT vector of any one of paragraphs 21-25, wherein the vector comprises a first heterologous polynucleotide coding for and expressing a Newcastle Disease Virus F (NDV-F) antigen having at least 80% sequence identity to the amino acid sequence as set forth in SEQ ID NO:2, 4, 6, 33, 35, or 37 and a second heterologous polynucleotide coding for and expressing an Infectious Bursal Disease Virus (IBDV) VP2 antigen having at least 80% sequence identity to the amino acid sequence as set forth in SEQ ID NO:8 or 42.
27. The recombinant HVT vector of any one of paragraphs 21-25, wherein the HVT vector comprises two heterologous polynucleotides coding for and expressing two IBDV Vp2 antigen having at least 80% sequence identity to the amino acid sequence as set forth in SEQ ID NO:8 or 42.
28. The recombinant HVT vector of paragraph 26, wherein the polynucleotide encoding the NDV-F antigen is operably linked to a promoter selected from the group consisting of SV40 promoter, MDV gB promoter, and guinea pig CMV promoter, and wherein the polynucleotide encoding the IBDV VP2 antigen is operably linked to a CMV promoter.
29. The recombinant HVT vector of paragraph 28, wherein the polynucleotide encoding the NDV-F antigen is operably linked to an SV40 polyA signal and the polynucleotide encoding the IBDV VP2 antigen is operably linked to an SV40 polyA signal.
30. The recombinant HVT vector of paragraph 29, wherein the polynucleotide encoding the NDV-F antigen, the operably linked promoter, and SV40 polyA signal are inserted in the IG1 locus of the HVT genome, and wherein the polynucleotide encoding the IBDV VP2 antigen and the operably linked CMV promoter and SV40 polyA signal are inserted in the IG1 locus or the SORF3-US2 locus of the HVT genome.
31. A method of vaccinating an animal comprising at least one administration of the composition or vector of any one of paragraphs 1-30.
32. A method for inducing an immunogenic or protective response in an animal against one or more avian pathogens comprising at least one administration of the composition or vector of any one of paragraphs 1-30.
33. The method of paragraph 32, wherein the avian pathogen is selected from the group consisting of Newcastle Disease Virus (NDV), Infectious Bursal Disease Virus (*i.e.,* IBDV or Gumboro Disease virus), Marek's Disease Virus (MDV), Infectious Laryngotracheitis Virus (ILTV), avian encephalomyelitis virus, avian reovirus, avian paramyxovirus, avian metapneumovirus, avian influenza virus, avian adenovirus, fowl pox virus, avian coronavirus, avian rotavirus, avian parvovirus, avian astrovirus and chick anemia virus coccidiosis (*Eimeria sp.*)*, Campylobacter sp., Salmonella sp., Mycoplasma gallisepticum, Mycoplasma synoviae, Pasteurella sp., Avibacterium sp., E. coli* and *Clostridium sp.*
34. The method of any one of paragraphs 31 -33, wherein the animal is avian.

## Claims

1. A composition or vaccine comprising a recombinant SB-1 viral vector wherein the SB-1 viral vector comprises one or more heterologous polynucleotides coding for and expressing at least one antigen or polypeptide of an avian pathogen.

2. The composition or vaccine of claim 1, wherein the strain of the recombinant SB-1 viral vector is the commercial Marek's Disease Vaccine, or the strain having the genome sequence as defined by GenBank Accession Number HQ840738.1.

3. The composition or vaccine of claim 1 or 2, wherein the one or more heterologous polynucleotides are codon-optimised for a given host.

4. The composition or vaccine of any one of claims 1-3, wherein the one or more heterologous polynucleotides are under the control of an immediate early cytomegalovirus (CMV) promoter; a guinea pig CMV promoter; an SV40 promoter; a Pseudorabies Virus promoter; a Herpes Simplex Virus-1 promoter; a Marek's disease virus (MDV) promoter including an MDV-1, MDV-2 or HVT promoter; an Infectious Laryngotracheitis Virus promoter, or another herpesvirus promoter.

5. A composition or vaccine of any one of claims 1-4, wherein the recombinant SB-1 viral vector comprises a first heterologous polynucleotide coding for and expressing a Newcastle Disease Virus F (NDV-F) antigen with at least 80% sequence identity to a polynucleotide having the sequence as set forth in SEQ ID NO: 1, 3, 5, 32, 34 or 36.

6. The composition or vaccine of claim 5, wherein the expression of the first heterologous polynucleotide coding for and expressing an NDV-F antigen is regulated by the SV40 promoter having the sequence as set forth in SEQ ID NO: 9.

7. The composition or vaccine of claim 5 or 6, wherein the expression of the first heterologous polynucleotide coding for and expressing an NDV-F antigen is regulated by the polyA signal having the sequence as set forth in SEQ ID NO: 11.

8. The composition or vaccine of any one of claims 5-7, wherein the heterologous polynucleotide coding for and expressing an NDV-F antigen is one of the following:
i) A codon-optimised NDV-F gene of the CA02 genotype operably linked to an SV40 promoter and inserted into the UL44 locus of the SB-1 genome;
ii) An NDV-F gene of the VIId genotype operably linked to an mCMV IE promoter and inserted into the US 10 locus;
iii) A codon-optimised NDV-F gene of the VIId genotype operably linked to an SV40 promoter and inserted into the UL55/LORF5 locus;
iv) A codon-optimised NDV-F gene of the VIId genotype operably linked to an SV40 promoter and inserted into the UL44 locus;
v) A codon-optimised NDV-F gene of the CA02 genotype operably linked to an SV40 promoter and inserted into the UL55/LORF5 locus.

9. The composition or vaccine of any one of claims 5-8 wherein the heterologous polynucleotide coding for and expressing an NDV-F antigen is a codon-optimised NDV-F gene of the CA02 genotype operably linked to an SV40 promoter and inserted into the UL44 locus of the SB-1 genome, further wherein the recombinant SB-1 vector further comprises a second heterologous polynucleotide coding for and expressing an NDV-F Variant E gene of the VIId genotype, under the control of a guinea pig CMV promoter and inserted into the SORF4-US2 locus.

10. The composition or vaccine of any one of claims 1-8, wherein the composition or vaccine is a polyvalent composition or vaccine further comprising vHVT13.

11. The composition or vaccine of any one of claims 1-10, further comprising a pharmaceutically or veterinarily acceptable carrier, excipient, vehicle or adjuvant.

12. The composition or vaccine of any one of claims 1-11 for use in a method of vaccinating an animal.

13. The composition or vaccine of any one of claims 1-11 for use in a method of inducing a protective response in an animal comprising at least one administration of the composition or vaccine.

14. The composition or vaccine for use of claim 13 wherein the protective response is provided against Marek's Disease Virus MDV, NDV and/or IBDV.

15. The composition or vaccine for use of any one of claims 12-14, wherein the animal is an avian.
